# EUROPEAN PATENT APPLICATION

(11) **EP 1 975 255 A2**
(43) Date of publication of application: **01.10.2008**
(21) Application number: 07826251.6
(22) Date of filing: 11.01.2007
(51) Int. Cl.: C12Q 1/68

(54) **METHOD AND APPARATUS FOR THE DETERMINATION OF GENETIC ASSOCIATIONS**

(30) Priority: 11.01.2006 ES 200600061
(71) Applicant: Neocodex, S.L., 41092 Sevilla (ES)
(72) Inventor: RUÍZ LAZA, Agustín, 41092 Sevilla (ES)
(74) Representative: Illescas, Manuel
(86) International application number: PCT/IB2007/053555
(87) International publication number: WO 2008/010195

(57) **Abstract**

Procedure and tool to determine genetic associations. The method allows to identify, without the need for predictive hypothesis, genes that influence, either individually or preferably collectively, the appearance of any phenotypic trait shared by several groups of individuals; groups in each of which the characteristic appears in a different context as they can be different diseases, a different reaction to the same treatment or different manifestations of the same disease. For each phenotypic context, a study is carried out of cases and controls, giving rise to associations of genes or combinations of genes with statistical significance. These associations are filtered, eliminating those that also appear when comparing controls versus controls. Of the remaining associations, those that have appeared in all the cases and controls are selected, preferably rationalized, and are validated by analysing their presence in larger groups.

## Description

### Technical Field

This invention describes new methods and apparatuses for carrying out genetic association studies. In particular, the invention describes a range of methods that serve for the carrying out of the cited studies, requiring less performance time for the analytical assays and less time consuming of the investigators, allowing the use of a lesser number of clinical patient samples and permitting the identification of a polygenetic base for any trait, phenotype, disease or characteristic discernible between individuals.

### State of the Art

Genetic association studies encompass a group of techniques designed to isolate genes and genetic mutations involved in *simple* (monogenic) and complex (multigenic and/or polygenic) biological conditions, as they can be illnesses, syndromes, clinical symptoms, or adverse effects induced by drugs. The elucidation of the genes implicated in a biological condition provides useful insight into the disease pathogenesis, or at least partial explanations regarding the mechanism and course of the disease and other biological statuses. Consequently, this knowledge allows the selection of potential strategies for prevention and treatment, and suggests new targets for treatment. Thus, with these studies we can identify treatment strategies such as the administration of a deficient protein, a change of diet, gene therapy, or the isolation of treatment targets for therapies with small molecules (conventional pharmacology). In addition, these studies also allow the development of clinical tests based on the presence of polymorphisms, mutant proteins, concentration profiles of biomolecules and other biological markers. All of these can be employed in performing an early diagnosis, an accurate diagnosis, establishment of a susceptibility to a particular disease (predictive medicine). Additionally it could be used in patient segmentation strategies in clinical trials, as well as in establishing an optimum personalised treatment for an individual through the assessment of the risks and potential effectiveness of a drug in a particular individual (pharmacogenomic and pharmacogenetic tests).

The fundamental underlying concept for all research into genetic association is that both normal phenotypic characteristics as well as clinical features such as diseases are due to an interaction between or combination of environmental factors that operate on a specific and individual genetic background. Many common diseases are said to be *Complex*; this means that they are either polygenic or multifactorial, or the end result of the complex effects of several (or many) genes interacting with environmental factors. The general approach in genetic association studies is to carry out a systematic examination of the genome of individual "cases" and individual "controls" with the aim of identifying statistically significant associations between the trait being studied (present in cases and absent in controls) and particular elements of the genome of those individuals being studied. This has been successfully achieved for monogenic or Mendelian traits. More recently, protocols have been developed for tackling a much more complex problem: the isolation of the genetic basis of polygenetic and/or multifactorial traits.

Mendel's laws on inheritance establish that phenotypes are inherited independently, although in reality genes are in fact often linked. In other words, they are inherited in long segments of DNA. This phenomenon is called linkage disequilibrium. (LD) and is very important because these long segments of DNA, known as haplotypes, are often associated with complex traits such as diseases or adverse reaction to drugs that have a complex origin or cause. The existence of LD means that the markers, that can be genotypically identified or characterised throughout the length of the genome as they are changes of a single nucleotide (single nucleotide polymorphisms or SNPs), are often associated in a very consistent manner with other physical elements of the adjacent DNA and that consequently, research on association can be carried out using the origin of the markers along the length of the genome as a strategy for the isolation or discovery of loci or a single locus that traces genetic elements associated with a particular trait. Once the locus has been identified, using this strategy, we can use the map of the human genome to identify which genes are present in the area and, thus, what function do they have. This allows us to establish more refined hypotheses that can be validated or checked by more specific association assays, other non-genetic types of research and, finally to further the advancement of understanding of the genetic basis for the trait studied. For quite a few years, various techniques have been used for carrying out genetic association studies, but with the development of new DNA based technologies for genotyping, the isolation of SNPs, and the completion of the human genome project, the volume of genetic association research has increased considerably.

There are two fundamental approaches to genetic association research. One of these is focused on the study of one/several candidate genes. The candidate genes are loci selected before carrying out the research, on the basis of a working hypothesis. This hypothesis depends on the situation and knowledge of the molecular basis of the condition being studied (e.g. knowledge of the aetiology or pathogenesis of a particular disease). Some examples of this candidate genes approach are those involved in the synthesis of enzymes, different receptors, transporters, growth factors, or other biomolecules that have been attributed to a particular biochemical pathway that is suspected to be related to the aetiology of a particular disease. For example see: Dryja TP et al., 1990 and Zee RY *et al*., 1992.
This research requires significant intellectual effort on the part of those performing it, as the grounds for selecting a particular candidate gene have to be selected, in a very laborious manner, from the literature. Furthermore, the hypotheses being constructed may or may not be correct, and this may only be known for sure upon completion of a series of, usually, very costly studies. On the other hand, these studies allow investigational efforts to be focused on specific areas of the genome where the candidate genes of interest are located. Therefore, this approach usually requires a number of relatively rare cases and controls in order to be able to identify statistically significant associations. Finally, these studies can be used to validate hypotheses including those established by means of more general association studies. Nonetheless, the 'candidate genes' approach is limited by the knowledge of the disease being studied
The other fundamental strategy employed in the genetic association studies consists in the identification of genes through the characterisation of the whole genome ("*shot gun approach*", association studies on the whole genome, etc). See as examples: Pericak-Vance MA et al., 2000, and Horikawa Y, et al. 2000.

In this strategy, which relies on linkage disequilibrium, multiple markers are employed throughout the genome in comparing individual genes that are unrelated but that present a feature under study when controls do not demonstrate this feature. Currently, it is possible to examine the complete genome of an individual by employing commercial products for genetic investigation based on micro devices of oligonucleotides that detect SNPs throughout the length of the genome with a capacity for the identification and genotyping of 10,000 or more SNPs in each individual and being able to reveal whether SNPs are present or absent in the cases and controls of a particular genetic association study. The data generated in these studies can be conceptualised as a table of values in which each column represents an individual genome, each row a particular SNP in these genomes with a + or - symbol in each cell representing the presence or absence of the SNP in question in each genome investigated. Numerous computer programs such as, for example, Sumstat (Ott J, Hoh J., 2003) can analyse these data with the objective of identifying loci statistically correlated with the characteristic being studied. These same programs, moreover, can calculate the probability of this association being true or simply an artefact of the data. Ultimately, by scanning the map of the human genome it is possible to draw up a more refined hypothesis based on the information about the genes close to the associated marker and to design strategies for confirmation or validation of the results obtained. This approach has been used in the study of monogenetic traits. However, it is also possible to apply it to complex (polygenetic) traits, including those in which a single gene has only a very minor and even undetectable effect on its own. See as examples: Hoh et al, 2003; or Marchini et al, 2005.

Genetic association studies are very costly and its main problem is rooted in the continuous appearance of spurious or random associations (generally referred to as "noise") that must be identified by means of a process of verification requiring relatively large case and control population sizes. In this respect, the huge problems of those who attempt to perform genetic association studies have now become broadly accepted. (see, Neurology, 2001; 57: 30-1354). For example, one of the most notable genetic association research studies in the past decade has been the association of the APOE gene with Alzheimer's disease (AD). This association stimulated new ideas on the causes and pathobiology of AD and other related illnesses. In contrast, as many as 50 associations related to AD have been described and several new markers have been proposed although most of them could not be replicated. Thus the majority of these associations have not been accepted, and the rest are subject of controversy in the scientific community.

The potential problems of the study of genetic association can be traced by the example that the researcher makes of genetic data that, inherently, can be quite confused, vague or subject to a certain degree of subjectivity on the part of the researcher. These problems include:
1. Viability of the diagnostic criteria of the characteristic being studied. Do all the individuals have the same disease?
2. Selection of an appropriate control group. Especially relevant are the age, sex and ethnic pool of the population being studied.
3. Choice of research strategy, as it is the use of approaches based on linkage studies (based on studies in families employing analysis techniques for the transmission of characteristics in individuals related by a single ancestry) or approaches based on association studies with cases and controls of unrelated individuals.
4. The problem with multiple comparisons (multiple testing), is the high probability of getting false positive results by random through the use of a large number of comparisons during the study.
5. The choice of the type of statistical analysis and the threshold of significance.
6. The great tendency of authors and journal editors to publish solely studies with positive results rather than those with negative results (publication bias).

The 'candidate genes' approach inherently has a good signal to noise ratio and results less expensive than association research characterising the whole genome, as it usually requires less samples and the collection of less genetic data in order to come to a conclusion. Nonetheless, in order to be successful, it requires a strong initial analysis, great creativity and the intellectual work of highly qualified scientific staff. Furthermore, it carries the risk that after successive validations and a long period of execution it could result in a negative result. Lastly, this type of analysis can only be based, either directly or indirectly, on earlier scientific results.

Lately, some authors have postulated that the common variants present in the genome could contribute significantly to the risk of common diseases. If this common-disease common-variant hypothesis is true, it will, in theory, permit a conceptually very simple approach to the identification of mutations responsible for diseases: that is to say, the building of an exhaustive catalogue of a limited number of common mutations in the genes of human populations. These can be analysed directly to evaluate their association with multiple clinical phenotypes. (Cargill et al., 1999).

An extension of this hypothesis was recently proposed by Becker (Becker, KG, 2004). Becker proposes a general model for the genetics of common diseases that emphasises the shared nature of common alleles in intrinsically related common disorders, as are schizophrenia and bipolar disorders, type II diabetes and obesity, or among autoimmune disorders. This model emphasizes that many genes are not disease specific. Consequently, common deleterious alleles with a relatively high occurrence in the population can play a role in phenotypes that are clinically related in terms of different genetic background and with distinct exposition to environment factors. In this sense, it is broadly established that similarities between common diseases can be caused by either genetic or epigenetic (environmental, etc) factors. Unfortunately, we lack the tools to dissect this important question.
The possibility of acquiring a deeper understanding of the relationships between the genome and phenotypes is the basis for the generalised optimism that leads us to believe we are very close to a new era in the area of human health. Consequently, methodological tools enabling us to conduct these studies more efficiently will be very valuable.

### Summary of the Invention

This invention provides new and efficient methods for the development of new genomic hypotheses. For example: the discovery that loci, and, ultimately that a gene or combination of genes are involved in or responsible for the appearance or persistence of some phenotypic characteristic worth investigating. In other words, that a mono, di, trigenic or polygenic mutation, (understanding by mutation a polymorphism of a single nucleotide (SNP), null alleles, mutations that conduct aberrant splicing, etc) is responsible for the phenotype being studied. The method can be employed in the investigation of monogenic traits, but has peculiarities that make it ideal for the study of polygenic or complex traits. As will be described in this document, the method combines the analysis and systematic processing *in vitro* of multiple groups of control samples of healthy individuals and samples of individuals having one or several phenotypes of interest, resulting in the identification of one or multiple genetic locations (Loci) that have a high probability of being implicated in the phenotype being studied. The genetic association identified can later be refined and verified (or alternatively rejected and discarded from the study). Moreover, the molecular mechanism of the association identified can be checked by employing other well established techniques.
This method has the advantage of not only avoiding a large part of the complex analytical work of the 'candidate genes' approach, but also, the optimisation of the costly and inefficient process of complete genome characterisation. The method has been named Hypothesis Free Clinical Cloning or **HFCC,** given that it enables the identification of genes responsible for virtually any phenotype or disease and the working hypotheses are generated by the system and not through inductive reasoning and the analysis of biochemical pathways based on the thinking of the investigators. In other words, the hypotheses are formed or revealed directly from observation of the clinical phenotypes in the study. The end result is the identification of a gene or multiple genes (pairs, trios or tetrads, etc) of interest.

HFCC uses a new method of background noise filtration. This is based on the combined study of clinically related phenotypes, allowing the simultaneous extraction of common elements of the genetic profiles of SNPs (or any other genetic marker) in multiple, clinically related, phenotypic characteristics. This permits exploration of the combined effects of either multiple markers or independent genes in practically any group of genetically linked characteristics. These characteristics may be, for example, susceptibility to any disease, or the progression or course of a disorder. Alternatively, HFCC can be employed for the analysis of the adverse effects and the effectiveness of drugs or to find biomarkers that indicate whether a particular individual is going to exhibit a particular phenotype.

Therefore, and in its most fundamental aspect, this method provides an engine for the generation of new scientific directions or hypotheses that can be verified and on which further work can be carried out *a posterioi*. These hypotheses emerge from the system that analyses the results of the pan-genomic investigation of multiple samples from individual controls (for example healthy individuals) as well as from individuals in which specific phenotypes are verified (for example a disease linked to a particular diagnostic method).

In one of its most fundamental aspects, this invention is going to provide a method for the determination of the association of one or more loci within the genome of a particular species that appears in a subgroup of individuals of the species. The method consists of the steps:
a) To obtain from the genomes of multiple individuals of the same species that define the control group, and that do not exhibit the phenotype under investigation, and then the data for the presence or absence of a great number of predetermined genetic markers located in different loci along the length of the genome.
b) To generate a noise data filter the method correlates the presence of markers of diverse loci of different members in a subgroup within the control group defined in point a) with the presence of the same markers within specified loci in a second subgroup of controls of the same control group .
c) To obtain from the genome of multiple individuals of the same species that configure the target or studied group sharing and expressing the same phenotype (F), genetic data from multiple and predetermined genetic markers located in multiple and physically separated loci.
d) To formulate diverse hypothetical correlations between mentioned phenotype (F) and selected loci analysed in the genome of studied individuals
e) To filter obtained and hypothetical correlations with de noise data filter to separate and eventually delete spurious correlations.
The study has to be initiated with the acquisition of the genetic profile of each individual incorporated in the study using the same set of pre-selected genetic markers. These data can be obtained, for example, by application of DNA samples assimilated from the individuals at a microdevice (DNA array) that consists of thousands of oligonucleotides, each one specific to an SNP type marker located in one site of the genome of the species being studied. This array permits to identify whether or not the polymorphic markers are present or absent in every genotyped individual. This array can contain 3,500, 10,000, 50,000 or more different oligonucleotides, each one corresponding to a distinct genetic marker. By including more markers, these genetic maps allow a more refined association analysis, but, the amount of computational analysis required is increased. The markers can be of the SNP type but the system of the invention can also include other types of genetic markers. Depending on the characteristics of the study and to generate genotype raw data from individuals the method can employ any other available techniques that permit the genomic analysis and the identification of the presence or absence of selected genetic markers in both chromosomes (giving information or not of both alleles) such pyrosequencing technique or many PCR derived protocols (i.e. real time PCR coupled to fluorescence resonance energy transfer). Another possibility is to obtain the raw genotyping data to be employed during the study directly from public databases under construction by multiple international initiatives and consortiums that are uploading to their websites the result of genotyping in a whole genome basis of multiple individuals with numerous high social impact diseases and its corresponding healthy control groups. The method can mix different diseases that have to share a common phenotype and look for genomic panels shared by all selected individuals to conduct the study.
The control groups mentioned in the previous paragraph are a series of healthy individuals, randomly selected from a population with the same ethnic and geographic background as the patients that present the phenotype of interest. From these control groups, we can select two subgroups of individuals, preferably at random. The presence or genotyping of multiple loci in all members of the first control subgroup labelled as control group "cases" (Cfl) is compared with the presence or genotyping of multiple loci in all members of the second control subgroup labelled as "controls" (Cc1). This study even generates a great number of apparent associations (positive theoretical correlations that could indicate, in the case of a comparison of cases and controls, an association between the phenotype studied and the appearance with greater frequency in one of the groups of one or more of the mutations detected) that, in the case of a comparison between two control groups, can only be explained by selection bias within the control group, technical problems during the genotyping, or occurring simply by chance. This group of detected associations can serve, as described here, as a noise filter for the data obtained and enables the elimination of spurious associations from the subsequent study of cases (real) and controls as described in step c) and conducted using the same techniques and panel of markers employed during the "control-control" study, which served as a filter. The associations eliminated will be those that appear in the study of cases and controls that have been detected in the noise filter (comprised of the associations detected in the comparative study of controls against controls).

Before, simultaneously, or following constitution of the noise data filter, raw data can be obtained from the presence in the same group of prespecified set of markers obtained from the genomes of different individuals that we define as the study groups (test groups, F groups). These groups are configured on the basis of individuals (for example patients that exhibit the phenotype of interest). Using available genotypes in the F group expressing the targeted phenotype, it is possible to formulate diverse hypothetical correlations between the phenotype and loci analysed in the genomes of F group. To formulated such hypothetical correlations, the F groups are compared with the controls (Cc) obtaining the generation, by means of computational tools, of a series of diverse potential or hypothetical correlations.
Finally, in order to identify, label or delete spurious correlations, the hypothetical correlations can be filtered by the application of the noise data filter generated in previous steps. This last step includes the comparison and filtering of the correlations obtained in step d), with those correlations obtained in the previous step b), allowing elimination of those that, with a high degree of probability are due to bias in the control group, technical problems or random occurrences. In other words, the correlations obtained when comparing the case group (F) and the controls (Cc) but that also appear when comparing the controls with each other (Cc versus Cf) can be discarded immediately, weighted or separated in the table of results. This process diminishes the number of correlations obtained during the study and facilitates the evaluation of candidate markers in the next steps of the research by employing, for example, a further depuration of markers based of previous knowledge about the phenotype based on gene or gene regulation related to the phenotype, the function of selected gene, the role of selected genes within known candidate metabolic pathways for the disease, previous data about gene-gene interactions for selected gene combinations or simply for the selection of the best markers for further validation in large case-control series.
The ideal configuration of the study groups (F groups, test groups) for HFCC includes n study groups (F1 to Fn), each one of which includes N individuals of the same species, for example groups of humans that exhibit different diseases or biological conditions, but that share a characteristic, common phenotype or identical risk factor present in all of them. In this configuration, the method includes the determination of the association between the phenotype, characteristic or common risk factor present in all the study groups and one or more loci, an association that can be observed for each and every one of the study groups (F1 to Fn). For example, the subgroups (F1 to Fn) can be human beings who have been diagnosed with different diseases, the same disease in different clinical stages or any other phenotype combinations but who all share a common clinical phenotype, a common risk factor, or a common complication, or the F groups can even have the same phenotype or disorder but with different clinical courses. It is also possible to apply this method to different subgroups of patients that have just the same disease or biological state. Although it is better (as previously mentioned) to apply the method to different diseases, biological states or drug responses that share specific and common phenotypes, the invented method can be applied to subgroups of randomized patients with an identical phenotype without any criteria to differentiate among phenotypic subgroups.
Preferably, at least three F groups should be configured. The sample sizes of these subgroups can include less than 1,000 individuals, or have even less than 150 members or less than 100 members. The optimisation of the size of each F subgroup can depend on the genetic model that we wish to apply, the density of markers and the number of genetic combinations that we wish to study (see Table 1 and following explanations).

Another crucial aspect of HFCC is that the method selects and analyses two control subgroups (Cc and Cf) selected at random from the overall control group (control pool) for each group F analysed. The objective is that each comparative Cc vs F study with its filter (Cc vs Cf) should be independent of the rest of the analysis. This means, for example, that for the analysis of F1, it is necessary to select at random two control subgroups (Cc1 and Cf1) from the general combination of controls (C). Both control subgroups (Cfl and Cc1) are compared with each other to obtain the noise filter 1 (Ns1) that is employed during the first round of comparison (Cc1 versus F1), that will ultimately provide the results R1. In order to ensure the independence between studies of the groups (F1... Fn) and the controls (Cc1...Ccn), this operation of random selection of control subgroups is repeated for each group F studied. Thus, n noise filters (Ns1...Nsn) will be obtained, depending on the number of F groups being studied (F1...Fn). Ultimately, all the noise filters will be assembled in a record R0 that will include all the associations obtained through permutation and analysis of the control subgroups (Nsl+Ns2+Ns3...+Nsn).

The noise filter is applied to the preliminary results obtained in each subgroup of associations (R1...Rn) generated by the comparison of each phenotypic group (F) with its corresponding control subgroup (Cc). The method of noise filter application can vary: that is to say the corresponding noise filter (Ns1, Ns2....,Nsn) can either be applied initially to each one of the result groups R1...Rn Ns or the first filter for the preliminary results can be performed by direct comparison with the general background noise record R0. In any case, the objective is to select a group of potentially valid digenic (or trigenic, etc) associations for each one of the pairs of control subgroup and phenotypic study group (Cc1 vs F1, Cc2 vs F2,..., Ccn vs Fn). With this complete compendium of data, the application selects those associations that appear in all the result groups (R1, R2,...Rn) but are not present in the archive R0, thus yielding a group of associated variables (RP, rationalised results) that appear in all the R groups (R1...Rn) and never in R0. Consequently, HFCC determines the association between a pair (or a triad etc) of loci within the genome of the individuals of the test groups (F) and the phenotype under study and which is common to all the individuals belonging to the F groups.

In another subsequent stage, the method could include the successive steps for the comparison of the markers obtained after filtration, correlating them with the map of the genome of the species being studied with the object of determining which genes are near to the selected markers and then consulting the literature to circumscribe the hypothesis, thus, reducing the number of hypothetical correlations by means of a rational inspection of the genes adjacent to the markers, and their functions. Alternatively and/or additionally, the method will include the subsequent steps for refining the correlations by the comparison of the loci associated with the map of markers of the species in order to select and add new markers, flanking those previously selected, in order to perform a later confirmation re-analysis of previously established correlations.

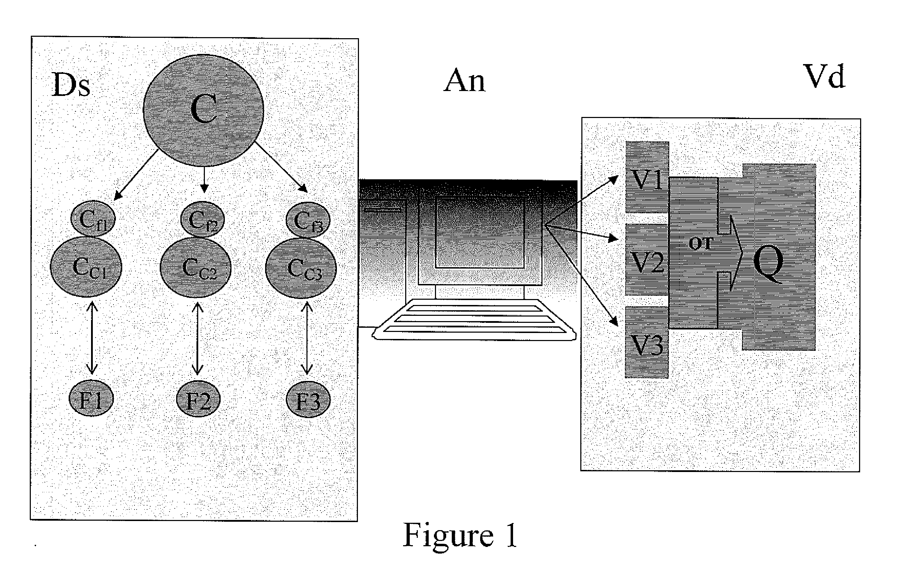

Typically, the correlations that are selected by this discovery procedure will be re-analysed in a group of independent individuals, usually of a greater sample size (see Figure 1), in order to validate the correlations previously identified, checking that the results can be reproduced. This part of the procedure would constitute what in Figure 1 has been labelled "Validation engine)".

In another aspect, this invention provides a noise filter in order to reduce the associations detected between loci of the genome of the species being studied and the phenotypes exhibited by group F individuals. The filter consists of a database that specifies the spurious associations (empirically rationalised by the calculation of their significance by using permutation tests, a very specific statistical test in which the level of statistical significance of an association does not rely on conventional statistical calculations but on an empirical calculation of statistical association based on the automatic relabelling a number of times, which would be the number of permutations, and in a random form of the status of the case or control of each individual in the study series, computing in each permutation of these labels the degree of association observed in the study, a calculation that is carried out by dividing the number of associated permutations by the total number of permutations carried out). This database encompasses all the multi-locus combinations of markers that appear commonly associated on carrying out comparisons between the distinct control subgroups obtained from the control pool (C) and having a positive association above a determined threshold of statistical significance. This noise filter is used as a computational tool to eliminate or mathematically rationalise the combinations of markers that appear to be associated by random occurrence, a poor choice of controls during the design of the case-control study or the selection processes for control selection. This information is important for the prioritisation of marker combinations in association studies and, moreover, it can identify potentially conflicting markers or combinations of markers that generate noise in association studies.

In addition, this invention provides an apparatus for the generation of hypotheses of association detected between one or more loci of the genome of a species and a phenotype exhibited by a subgroup of individuals of the species. This apparatus is another important part of this invention. The apparatus consists of a programmed computational system or a network of computers containing the following programs or modules: 1. a system or device for receiving the data entered for a panel of predetermined genetic markers located in independent sites or loci throughout the length of the genome of the species exhibiting the phenotype (F groups). 2. A system or device that stores and records the spurious associations including the all the multi-locus combinations obtained from the studies of association between control groups (Cc1 to Ccn against Cf1 to Cfn respectively) with statistical values below a statistical significant or confident thresholds (usually p<0.01). 3. A system or device for the calculation, based on the data that registers the presence of a panel of predetermined markers in the case groups (F groups, test group), the associations with markers registered between the markers studied and the common phenotype observed in the F groups, i.e. a system or device to calculate based on multiple and predetermined genotypic data of specified individuals different assays or hypothetical associations between loci carrying selected genetic markers and the selected phenotype corresponding to associations contained in F groups 4. A filter device for eliminating or rationalising the associations selected using device three but which have been also registered in the control-control device (device 2, noise filter), removing therefore those combinations due to noise from those obtained for device 3.

This kind of apparatus helps to analyze the raw genotypic data of a big number of individuals interrogating about the presence or absence of hundred thousand of markers and its combinations in individuals affected of targeted diseases. However, it is also possible that available sample size for controls could be very small and the for this reason the noise data filter cannot by applied or simple cannot render any advantage to the study. For this reason, and in order to improve the versatility of the method, the configuration of our device admits the incorporation or not of the system 2. i.e. the noise data filter. This option can be useful to identify genetic correlations without noise data filter restrictions that can help for example to increase the number of selected associations or to proceed to further weight or evaluation of obtained correlations using other available methods.
Taking into account all specified characteristics, this invention and its related apparatus can be employed to generate hypothesis of association between a single or multilocus combination of loci in the genome of any species with a phenotype exhibited by a subgroup of individuals of the specified species. It is possible to employ the device and whole invention with or without noise data filter. It is also possible to employ the device and the whole invention to conduct multilocus or monogenic association studies in the genome.

Another important aspect of this invention is the production of an informatics software comprising a computer readable device and a computer readable program code registered in the computer readable device and appropriated to give instructions to the computer or cluster of computers included in the apparatus described in the invention to conduct the following stages:
a) To receive raw data including the presence of multiple predetermined genetic markers located within separate loci along the genome in multiple individuals sharing a specified phenotype;
b) To receive raw data including the presence of multiple predetermined genetic markers located within separate loci along the genome in multiple individuals that are not exhibiting the specified phenotype;
c) To evaluate hypothetical associations between the presence of genetic markers and a selected phenotype using two control groups that are not exhibiting the specified considering one of the control groups like a group exhibiting the phenotype.
d) To evaluate hypothetical associations between the presence of genetic markers and a selected phenotype using two groups of individuals one of them are not exhibiting the specified phenotype the other exhibiting the targeted phenotype.
e) To identify, separate and or remove hypothetical associations obtained in stage d) but also present in stage c).

### Brief Description of the figures

Figure 1 is a block diagram that illustrates an example of the method and system of the invention and that contains three different functions: A discovery engine, an analysis tool and a validation engine. The left side of Figure 1, with the heading "Ds", represents the discovery engine, where the circles represent groups of individuals. C=controls, F=patients. Cf1 to Cf3: subgroups of control individuals, each one composed of equal numbers of individuals. Cc1 to Cc3: subgroups of control individuals, each containing an equal number of individuals but of a larger size than subgroups Cf. F1 to F3: subgroups of patients, each of which is composed of an equal number of individuals, which preferably also coincides with the size of each of the subgroups Cf1 to Cf3. The members of each and every one of the subgroups F1 to F3 share a common characteristic, which can be to show the same phenotypic characteristic, to suffer from the same disease or to have received the same treatment, but the members of each one of the subgroups share a common distinctive characteristic that differentiates them from the other subgroups (for example, the disorder by which the same phenotypic characteristic manifests itself is different in each subgroup, the disease progresses in a different manner in each subgroup, or the effects of the same treatment are different). The arrows between each one of the pairs Cc1:F1, Cc2:F2, Cc3:F3 indicate the performance of comparisons between each pair in searching for marker associations. The central part of Figure 1, headed by the letters "An", represents the analysis tool that encompasses all the mathematical procedures for carrying out the studies of gene-gene interactions, which allow the selection of the combinations of genes or SNPs isolated by the search engine.
The right side of Figure 1, headed by the letters "Vd", represents the validation engine, which allows validation of the associations obtained with the search engine by means of the analysis of subgroups of a larger size. V1, V2 and V3 represent groups of subgroups with the same common characteristic as subgroups F1, F2 and F3, but of a much larger size than these subgroups. The members of each one of the V groups presents the same differentiating characteristic as the members of the corresponding F subgroup. Q1 represents a sample of individuals of the general population of a size equal to or greater than that of each one of the V groups. The arrow connecting the V groups with Q1 represents the carrying out of comparative analyses between each one of the V groups and Q1 in order to confirm the associations found with the discovery engine or even to classify them according to criteria such as diagnostic usefulness, potential for use as treatment targets or usefulness in the discovery of new drugs.

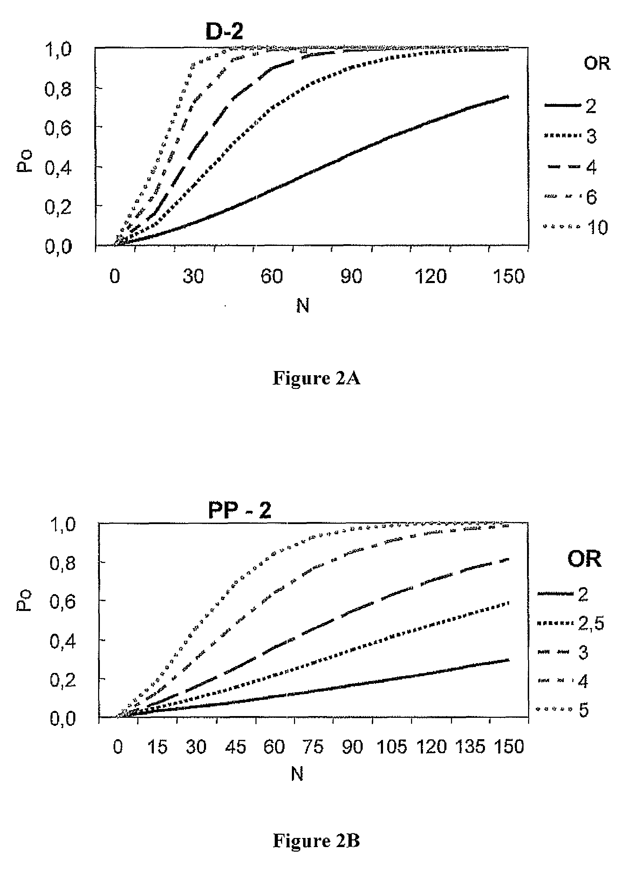

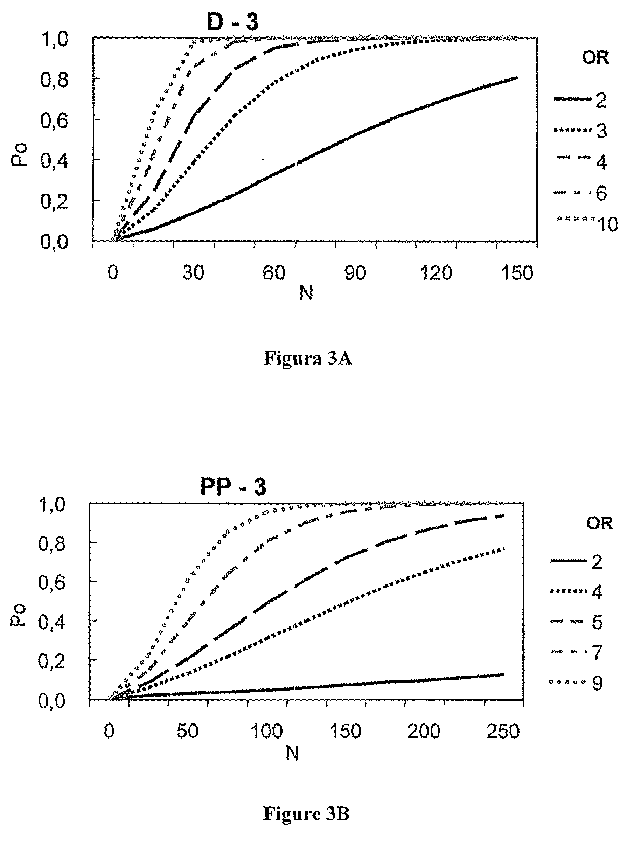

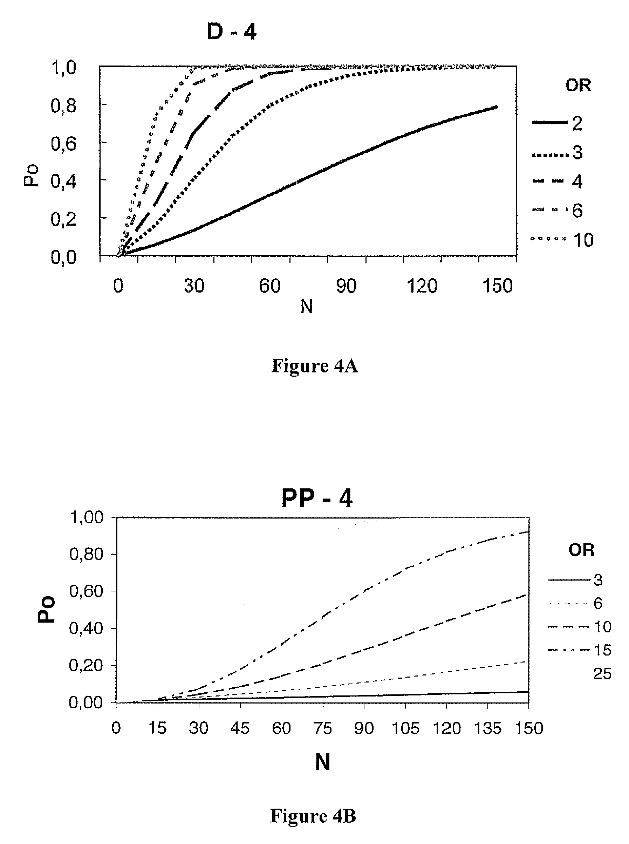

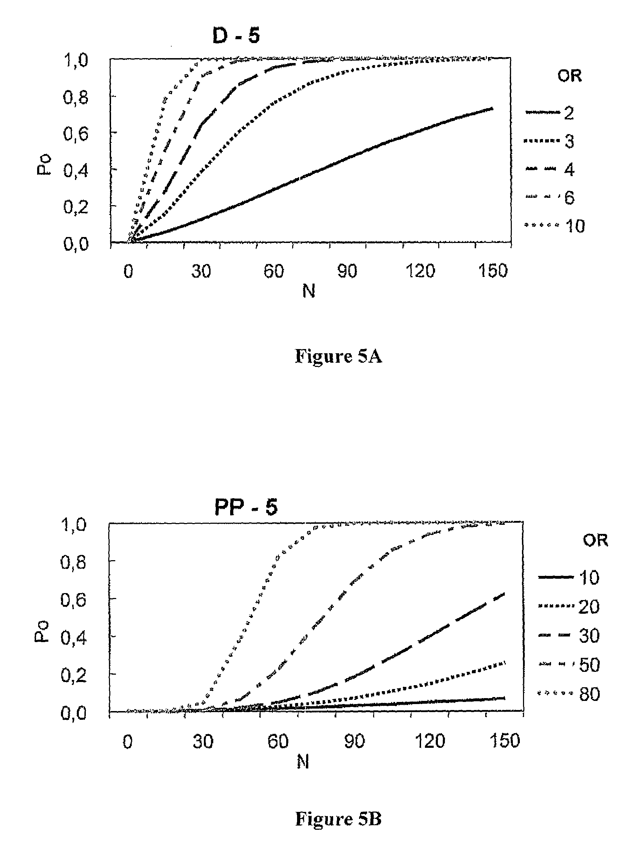

Figures 2-5 are graphical representations of the estimates of statistical power (Po) as a function of the number of cases (N) for interaction studies that are digenic (Figures 2A and 2B), trigenic (Figures 3A and 3B), tetragenic (Figures 4A and 4B) and pentagenic (Figures 5A and 5B) under a dominant model (Figures 2A, 3A, 4A and 5A, in which the correspondence with the dominant model is indicated with the letter D above the graphs) or a model that studies all possible genetic combinations and takes all factors into consideration (pan-factor) (Figures 2B, 3B, 4B and 5B, in which the correspondence with the pan-factor model is indicated by the letters FF above the graphs).

### Description

The current model of complex disease establishes that complex features or phenotypes are caused by or constituted of multiple, physically unrelated genetic elements. Normally, each genetic element *per se* has a very small magnitude and is insufficient, alone, to cause a given phenotype. This means that these genes need another variant and/or additional environmental or exogenous risk factors in order to lead to the appearance of a determined phenotype. In contrast, when the concurrence of two or more factors is produced in the same individual, the penetrance (understood as the proportion of individual carriers of a genotype or combination of genotypes displaying a phenotype) is habitually increased. The OR (odds ratio) is a measure of the extent of the effect of a determined factor. This measure is generally used in case-control studies and is a valid estimate for the cause or probability ratio that an event (in our case the presence of a genotype) will occur in un group de individuals (cases) divided by the probability of the same event in another group (controls). This concept is very important because if applied beforehand it implies or signifies that the OR or the penetrance of specific genetic combinations is higher than those observed in the studies of the markers in isolation (Hoh J and Ott J, 2003).
OR_{single} marker < OR_{digenic}<OR _{trigenic} < OR _{tetragenic}, and so on and so forth.

In fact it has recently been postulated that the search for gene-gene interactions (interactomes) would be more fruitful than tackling phenotypes with marker by marker study strategies in the context of scanning the whole genome (Marchini J, et al. 2005)

One of the great challenges of association studies is the problem of multiple comparisons: how to identify and isolate valid associations of gene combinations (or marker combinations) that confer susceptibility to each feature or disease (see Hoh J and Ott J, 2003). For example, if there are ten million common SNP markers throughout the length of the genome, then there are 10,000,000!/(2! x 9,999,998!) or around 5 x10¹³ combinations of pairs of markers potentially involved for each characteristic (see for example Weiss KM, et al. 2000; Altshuler D, et al. 2000; Zondervan KT, et al. 2004). To carry out association studies, on a selected phenotype, in an exhaustive and systematic manner is a discouraging task, and thus, the method of the invention has been developed with the object of discovering those genuinely associated genotypic combinations in a relatively economic manner and furthermore reducing the risk of "false positives" or random associations, or other spurious causes. The design of filters for analysis of the data in the process of the invention can reduce the noise due to the great volume of association tests that must be performed when we employ a large number of markers and combinations of markers.

Protocols for genotyping dozens or hundreds of thousands of SNP markers in just one trial have been developed in the last decade (Syvanen A.C., 2005, comes to mind for reference). These advances have the potential for identification of the proteins associated with each disease, and their corresponding biochemical pathways as therapeutic targets (Craig D.W. et al. 2005). The fundamental characteristic of whole genome mapping is that this search for associations is not based on specific genes *per se*; there is no hypothesis referring to any element of the genome being studied (positional cloning approach or hypothesis free approach).

The method of the invention employs a strategy based on the complete mapping of the genome, using SNPs, to obtain a full genetic profile or footprint of the SNPs of the individuals being studied (cases and controls). The realisation of this, in which commercial micro arrays such as GeneChip® low of Affymetrix are employed, attempts to exploit a map of 10,000 markers distributed throughout the genome with a resolution of one marker for every 200,000 pairs of bases. However, the method of the invention also uses higher resolution genetic maps, which can easily be achieved using, as an alternative, emerging technologies (Syvanen A.C. 2005). This will depend on the precision of the initial results and an exhaustive cost-benefit study.

The method exploits two concepts or assumptions widely accepted in genetic research that have never been systematically evaluated. The first assumption is that the clinical symptoms included in the study share common genetic factors. The second is that the cause of the clinical symptoms is a combination of different markers with no genetic link between them (in other words, a genetic pattern composed of two or more unrelated genetic markers). Therefore, the inspection method gives preference to the genetic combinations involved in several clinically related features or phenotypes (although the role of the individual markers can be interrogated very simply with this method).

As previously described, the samples labelled as C are referred to as the control group and the F groups represent the groups of patients being studied. Each one of these groups must be divided into subgroups. In Figure 1 an example is shown for the existence of three subgroups of patients (F1, F2, F3), which means the creation of 2 series of control subgroups: Cf1, Cf2, Cf3, on one hand and Cc1, Cc2, Cc3 on the other. The sample size of each group can vary, but should, preferably, be at least large enough to enable significant genetic associations to be obtained. The size of each of the F subgroups and C should preferably be based on the calculations that are going to be performed to validate the presence/absence of a pair or greater combination of markers (polymorphisms, for example), as the model that follows in order to perform these calculations and the values pre-fixed will determine the statistical weight of the study. The statistical power represents the probability of rejecting the null hypothesis (which, in the case of the method of the invention, would be the absence of association between a combination of markers and an illness, phenotypic characteristic, standard of response to treatment, etc., considered) when the null hypothesis is false, that is, it represents the capacity of a test or experiment to detect as statistically significant differences or associations of a determined magnitude. The usefulness of an association study depends to a large extent on its capacity to detect the association between factors when this association exists. When this capacity (the statistical power) is insufficient, and following completion of a study, a null result is obtained (no statistical significance), it is not possible to rule out the possibility that results were obtained as a consequence of the lack of power of the study, in other words that a "false negative" is present. This situation invalidates the results obtained and therefore, the study. Consequently, it is important to estimate the power of a study before carrying it out to completion, since if the result of this estimate is insufficient, it would be advisable not to carry out the study. Thus, we come to the method of the invention. The most advisable strategy to be used consists of exploring different scenarios and obtaining a different estimate for each one of them. However, as it is an estimate, when the calculation is performed parameters must be assumed that will not be known with any degree of certainty until the study is completed, fixing the parameters that affect the statistical power of a series in studies of cases and controls and which are:
i) error type alpha (α), which represents the probability of obtaining a positive result when an association does not exist and that is often fixed at 5% (which means setting as an acceptable maximum a probability of 0.05 of accepting as positive a result of association between two or more markers when the association does not actually exist); thus, the associations for which a value p (probability that the results obtained in an investigation may be due to chance with the assumption that there are no differences between the study groups) were obtained of equal to or greater than 0.05 would be rejected as they would not be regarded as statistically significant;
ii) ratio of controls to cases (preferably at least 150 controls in each subgroup Cc versus 75 cases in each F subgroup);
iii) prevalence of the genetic pattern in controls (in other words, the number of controls with a specific genetic pattern divided by the total number of controls analysed), which is fixed according to the genetic model considered and assuming that for each one of the markers considered in each combination there are two possible alleles, each one of which presents with an equivalent frequency, (0.5), in such a way that, in a dominant digenic model (in which combinations of markers are considered two at a time) for example, exhibition of the genetic pattern (in other words, a specific combination of one of the possible alleles of the first marker with one of the possible alleles of the second marker) in the controls would be fixed at 25%,
iv) the OR that results from comparing the carriers and non-carriers of a determined genetic pattern in cases and controls, and
v) the genetic models and models of marker combinations.

Each one of the models of marker combinations means considering a given number of markers in each combination. For example, in a digenic model, there are two markers considered in each combination. In the ideal scenario for this invention, the two markers are two SNP type polymorphisms (1 and 2) with two possible alleles, A and B, each one (1A, 1B, 2A, 2B) of which, generally, is considered allele B at the presence of the polymorphic site, which usually means that it is considered to be allele B that the nucleotide encountered in the position considered the less frequent of the two possibilities. As shown in Table 1, in this model, each variable consists of a digenic combination with nine different strata (possible genotypic structures), that in the table are specified by indicating first the combination of markers (SNPs in this case) which is being considered (12: polymorphism 1 with polymorphism 2) and then the specific genotypic structure that corresponds to it:

**Table 1.- Possible strata in a model**

| **Genotypes Polymorphism 1** | **Genotypes Polymorphism 2** | | |
|---|---|---|---|
| | **AA** | **AB** | **BB** |
| AA | 12AAAA | 12AAAB | 12AABB |
| AB | 12ABAA | 12ABAB | 12ABBB |
| BB | 12BBAA | 12BBAB | 12BBBB |

For each one of the variables (pairs or combinations of distinct polymorphisms considered) of the digenic model there will be a configuration similar to that which is presented in Table 1.

In a trigenic model, the number of markers considered possible in each variable is three. In a similar way to the previous case, taking it as an example of marker polymorphisms, three polymorphisms have to be considered (1, 2, 3) for each one of which it is considered that two possible alleles exist (1A, 1B; 2A, 2B; 3A, 3B) of which, generally, allele B is the less frequent. As shown in Table 2, in this model, each variable consists of a trigenic combination with 27 different strata, the structure of which is also shown in Table 2 with an annotation analogous to that of Table 1.

**Table 2.- Strata possible in a trigenic model**

| **Genotypes Polymorphism 1** | **Genotypes Polymorphism 2** | **Genotypes Polymorphism 3** | | |
|---|---|---|---|---|
| | | **AA** | **AB** | **BB** |
| **AA** | **AA** | 123AAAAAA | 123AAAAAB | 123AAAABB |
| | **AB** | 123AAABAA | 123AAABAB | 123AAABBB |
| | **BB** | 123AABBAA | 123AABBAB | 123AABBBB |
| **AB** | **AA** | 123ABAAAA | 123ABAAAB | 123ABAABB |
| | **AB** | 123ABABAA | 123ABABAB | 123ABABBB |
| | **BB** | 123ABBBAA | 123ABBBAB | 123ABBBBB |
| **BB** | **AA** | 123BBAAAA | 123BBAAAB | 123BBAABB |
| | **AB** | 123BBABAA | 123BBABAB | 123BBABBB |
| | **BB** | 123BBBBAA | 123BBBBAB | 123BBBBBB |

Each one of the configurations of variables generated can be analysed by using two different genetic models: either the dominant model or the pan-factor model. The dominant model is equivalent to the classical thresholds model (Marchini *et al*., 2005) and the number of possible combinations obtained in any model can easily be obtained using the classical formula corresponding to the number of possible variations without repetition, M!/(G! x (M-G)!), where M would be the number of markers analyzed and G the number of markers that have to be taken to form each one of the possible combinations (in the digenic model, G=2 markers per combination; in the trigenic model, G=3 markers per combination, and so on and so forth). The dominant model analyses the information of the genetic markers in two groups: presence of at least one copy of each SNP against the rest of the combinations. In a digenic model, for example, this would mean 4 possibilities to be considered: presence of B in both polymorphisms (a condition that would complete the strata 12ABAB, 12ABBB, 12BBAB, 12BBBB), presence of A in both polymorphisms (a condition that would complete the strata 12AAAA, 12AAAB, 12ABAA, 12ABAB), presence of A in polymorphism 1 and of B in polymorphism 2 (a condition that would complete the strata 12AAAB, 12AABB, 12ABAB, 12ABBB) and presence of B in polymorphism 1 and of A in polymorphism 2 (a condition that would complete the strata 12ABAA, 12ABAB, 12BBAA, 12BBAB). In Table 3 the possible genotypic combinations for a digenic model have been reproduced, indicating in bold and italics the strata that would complete each one of the possibilities of presence of at least one copy of a determined marker. For each one of these possibilities, it would be necessary to consider the strata that complete it, verifying, both in cases and in controls, the frequency of appearance of any of these against the frequency of appearance of any of the remainder and comparing the value obtained in each group of cases (F1, F2...Fn) with the value obtained in its corresponding control groups (Cc1, Cc2...Cn).

**Table 3.- Strata that complete each one of the possible options to be considered in a dominant digenic model**

| **Dominant 1: presence of B in both polymorphisms** | | | |
|---|---|---|---|
| **Genotypes Polymorphism 1** | **Genotypes Polymorphism 2** | | |
| | **AA** | **AB** | **BB** |
| AA | 12AAAA | 12AAAB | 12AABB |
| AB | 12ABAA | ***12ABAB*** | ***12ABBB*** |
| BB | 12BBAA | ***12BBAB*** | ***12BBBB*** |

| **Dominant 2: presence of A in both polymorphisms** | | | |
|---|---|---|---|
| **Genotypes Polymorphism 1** | **Genotypes Polymorphism 2** | | |
| | **AA** | **AB** | **BB** |
| AA | ***12AAAA*** | ***12AAAB*** | 12AABB |
| AB | ***12ABAA*** | ***12ABAB*** | 12ABBB |
| BB | 12BBAA | 12BBAB | 12BBBB |

| **Dominant 3: presence of A in polymorphism 1 and of B in polymorphism 2** | | | |
|---|---|---|---|
| **Genotypes Polymorphism 1** | **Genotypes Polymorphism 2** | | |
| | **AA** | **AB** | **BB** |
| AA | 12AAAA | ***12AAAB*** | ***12AABB*** |
| AB | 12ABAA | ***12ABAB*** | ***12ABBB*** |
| BB | 12BBAA | 12BBAB | 12BBBB |

| **Dominant 4: presence of B in polymorphism 1 and of A in polymorphism 2** | | | |
|---|---|---|---|
| **Genotypes Polymorphism 1** | **Genotypes Polymorphism 2** | | |
| | **AA** | **AB** | **BB** |
| AA | 12AAAA | 12AAAB | 12AABB |
| AB | ***12ABAA*** | ***12ABAB*** | 12ABBB |
| BB | ***12BBAA*** | ***12BBAB*** | 12BBBB |

The pan-factor model divides the information into multiple strata and selects for analysis the strata that reach a size of minimum effect, a parameter that has been marked by the study of statistical power and that refers to the minimal OR that can be detected in a case-control study with a power of greater than 80% given a fixed number of cases and controls in the study. Each stratum of each variable is considered an independent variable that is compared against the rest (in the case of a digenic model, 12AAAA against the rest, then 12ABAA against the rest, and so on and so forth. Alternatively a chi-squared table can be constructed with n degrees of freedom (with n=number of strata-1). In this case each digenic model is calculated 9 times (the nine strata of each variable), with the OR, confidence interval, standard error and p of each stratum all being indicated.

The pan-factor model has less statistical power than the dominant model, but it captures genetic combinations without relying on previous assumptions.

Fixing the necessary parameters and choosing the distinct scenarios (number of markers in combination to consider and genetic model used), diverse statistical calculations have been made to evaluate the statistical power and the viability of HFCC. Table 4 shows further on an analysis of the number of genetic tests, phenotypic groups necessary (which is called "n", the number of F groups of patients to analyse) and calculates, for a theoretical study in which the number of markers distributed throughout the genome to be analysed were 10,000 (10K) and the level of significance for an association to be considered as positive were fixed at alpha=0.01, the number of random positive associations observed in different configurations of HFCC according to the distinct combinations of simultaneous markers and genetic models used, just as the number of experiments necessary (in other words, the number of individuals, cases+controls, having to be genotyped in each configuration) depending on the number of individuals N composing each one of the F groups. It must be stressed that each group of individuals can be reanalysed independently employing different genetic models and other assumptions and the system can be programmed accordingly. Evidently, it is necessary to stress that each time more complex models (tetragenic, pentagenic, hexagenic...) are evaluated, the number of combinations increases steeply and, therefore, the number of F groups (n) must also be progressively increased.

**Table 4: Number of phenotypic groups (n) and of experiments necessary to complete execution of HFCC for distinct genetic models and combinations of marker, using 10,000 SNPs.**

| Combination of Markers | Model | Combinations with 10000 SNPs | No. groups of study F (n) | Loci identified by chance** | No. experiments N=75 | No. experiments N=150 |
|---|---|---|---|---|---|---|
| Digenic | Dominant | 50 x 10⁶ | 3 | 50 | 525 | 950 |
| Digenic | FF* | 50 x 10⁸ | 4 | 9 | 600 | 1100 |
| Trigenic | Dominant | 1.66 x 10¹¹ | 5 | 17 | 675 | 1225 |
| Trigenic | FF* | 4.5 x 10¹² | 6 | 5 | 750 | 1475 |
| Tetragenic | Dominant | 4.16 x 10¹⁴ | 7 | 4 | 825 | 1550 |
| Tetragenic | FF* | 3.77 x 10¹⁶ | 8 | 4 | 900 | 1700 |
| Pentagenic | Dominant | 8.32 x 1017 | 8 | 83 | 900 | 1700 |
| Pentagenic | FF* | 2 x 10²⁰ | 9 | 202 | 975 | 1850 |

| | | | | | | |
|---|---|---|---|---|---|---|
| *FF: pan-factorial **The loci associated by chance are calculated as the product of possible combinations for an alpha error to the power of n, (alpha)ⁿ. n is the number of F groups and alpha is the level of significance of each study (alpha=0.01 in this case ) | | | | | | |

Calculations of sample size can be performed using the software Statcalc (EpiInfo 5.1, Centre of Disease Control and Prevention, Atlanta) and with the software Episheet (Rothman KJ, 2002).

Table 5 shows the size of minimal effect, that is, the minimal *odds ratio* (OR) detected using HFCC, in studies with a statistical power of above 80%, employing two different sample sizes in the F groups, as a function of the type of marker combinations considered and the genetic model used. These ORs have been calculated using the software Episheet, assuming an alpha error =0.01, P=Q=0.5, 2:1 ratio of cases:controls and an exposure in controls to genetic masters depending on the combinations of markers and the genetic model used, as indicated below:
- dominant digenic model: Exposure = 0.56
- pan-factor digenic model: Exposure = 0.0625
- dominant trigenic model: Exposure = 0.42
- pan-factor trigenic model: Exposure = 0.015625
- dominant tetragenic model: Exposure = 0.31
- pan-factor tetragenic model: Exposure = 0.0039062
- dominant pentagenic model: Exposure = 0.23
- pan-factor pentagenic model: Exposure = 0.00097

**Table 5.- Minimum Odds ratio (OR) detected by HFCC using two different sample sizes in the F groups, three distinct combinations of markers and two distinct genetic models.**

| Combination of Markers | Dominant Model | Pan-factor Model |
|---|---|---|
| N=75 | | |
| Digenic | 2.95 | 4.2 |
| Trigenic | 2.7 | 8.5 |
| Tetragenic | 2.7 | 21.4* |
| Pentagenic | 2.8 | 64.5* |

| N=150 | | |
|---|---|---|
| Digenic | 2.05 | 3 |
| Trigenic | 2 | 5.5 |
| Tetragenic | 2.05 | 12.6 |
| Pentagenic | 2.1 | 35.5* |

| | | |
|---|---|---|
| * values clearly outside the range, as these huge levels of effects can not be expected for polygenic combinations. For the pan-factorial model of tetragenic and pentagenic genetic combinations it is more realistic to use larger sample sizes. | | |

In Figures 2A to 5B, for their part, graphics can be seen that demonstrate the variation of the estimation of power as a function of the number of cases for distinct values of OR. The actual values of the calculations of power corresponding to the different configurations of HFCC are shown in Tables 6 (dominant digenic model), 7 (pan-factor digenic model), 8 (dominant trigenic model), 9 (pan-factor trigenic model), 10 (dominant tetragenic model), 11 (pan-factor tetragenic model), 12 (Dominant pentagenic model) and 13 (pan-factor pentagenic model).

**Table 6.- Power calculation for a dominant digenic model**

| **CASES** | OR | | | | | | |
|---|---|---|---|---|---|---|---|
| | **2** | **3** | **4** | **5** | **6** | **8** | **10** |
| **0** | 0.0049400 | 0.0049400 | 0.0049400 | 0.0049400 | 0.0049400 | 0.0049400 | 0.0049400 |
| **15** | 0.0478573 | 0.1078043 | 0.1656079 | 0.2169877 | 0.2618297 | 0.3352410 | 0.3924016 |
| **30** | 0.1121369 | 0.3033132 | 0.4814452 | 0.6198112 | 0.7211203 | 0.8462908 | 0.9115602 |
| **45** | 0.1923226 | 0.5160276 | 0.7464499 | 0.8727256 | 0.9364072 | 0.9834273 | 0.9952866 |
| **60** | 0.2817049 | 0.6943226 | 0.8964933 | 0.9673202 | 0.9897485 | 0.9989145 | 0.9998670 |
| **75** | 0.3741459 | 0.8213226 | 0.9631988 | 0.9931017 | 0.9987130 | 0.9999499 | 0.9999976 |
| **90** | 0.4646819 | 0.9020192 | 0.9882667 | 0.9987476 | 0.9998663 | 0.9999982 | 1.0000000 |
| **105** | 0.5497100 | 0.9490698 | 0.9965755 | 0.9997984 | 0.9999880 | 0.9999999 | 1.0000000 |
| **120** | 0.6269242 | 0.9747045 | 0.9990711 | 0.9999706 | 0.9999991 | 1.0000000 | 1.0000000 |
| **135** | 0.6951233 | 0.9879199 | 0.9997632 | 0.9999960 | 0.9999999 | 1.0000000 | 1.0000000 |
| **150** | 0.7539642 | 0.9944252 | 0.9999428 | 0.9999995 | 1.0000000 | 1.0000000 | 1.0000000 |

**Table 7.- Power calculation for a pan-factor digenic model**

| **CASES** | **OR** | | | | | | |
|---|---|---|---|---|---|---|---|
| | **2** | **2.5** | **3** | **3.5** | **4** | **4.5** | **5** |
| **0** | 0.0049400 | 0.0049400 | 0.0049400 | 0.0049400 | 0.0049400 | 0.0049400 | 0.0049400 |
| **15** | 0.0316998 | 0.0510686 | 0.0736407 | 0.0991410 | 0.1273288 | 0.1579434 | 0.1906884 |
| **30** | 0.0543923 | 0.1003233 | 0.1586581 | 0.2270688 | 0.3026264 | 0.3820893 | 0.4622321 |
| **45** | 0.0793026 | 0.1568683 | 0.2561389 | 0.3685229 | 0.4842882 | 0.5945994 | 0.6929134 |
| **60** | 0.1063250 | 0.2183277 | 0.3576660 | 0.5048710 | 0.6417461 | 0.7561968 | 0.8435254 |
| **75** | 0.1351667 | 0.2824402 | 0.4567242 | 0.6250551 | 0.7635477 | 0.86318555 | 0.9270134 |
| **90** | 0.1655019 | 0.3472472 | 0.5488536 | 0.7243956 | 0.8504514 | 0.9274328 | 0.9682789 |
| **105** | 0.1970067 | 0.4111372 | 0.6314176 | 0.8025679 | 0.9087674 | 0.9632734 | 0.9869902 |
| **120** | 0.2293718 | 0.4728459 | 0.7032330 | 0.8617168 | 0.9460415 | 0.9821364 | 0.9949173 |
| **135** | 0.2623078 | 0.5314334 | 0.7641735 | 0.9050511 | 0.9689353 | 0.9916030 | 0.9980949 |
| **150** | 0.2955487 | 0.5862469 | 0.8148153 | 0.9359488 | 0.9825342 | 0.9961686 | 0.9993110 |

**Table 8.- Power calculation for a dominant trigenic model**

| **CASES** | **OR** | | | | | | |
|---|---|---|---|---|---|---|---|
| | **2** | **3** | **4** | **5** | **6** | **8** | **10** |
| **0** | 0.0049400 | 0.0049400 | 0.0049400 | 0.0049400 | 0.0049400 | 0.0049400 | 0.0049400 |
| **15** | 0.0601859 | 0.1520730 | 0.2486240 | 0.3373956 | 0.4152028 | 0.5395025 | 0.6306252 |
| **30** | 0.1377048 | 0.3923357 | 0.6161650 | 0.7682699 | 0.8620338 | 0.9504935 | 0.9813061 |
| **45** | 0.2309317 | 0.6200428 | 0.8515241 | 0.9471354 | 0.9816846 | 0.9977399 | 0.9996900 |
| **60** | 0.3312079 | 0.7862771 | 0.9528582 | 0.9909456 | 0.9983224 | 0.9999391 | 0.9999974 |
| **75** | 0.4313340 | 0.8894268 | 0.9870753 | 0.9987375 | 0.9998816 | 0.9999989 | 1.0000000 |
| **90** | 0.5260465 | 0.9465364 | 0.9968375 | 0.9998494 | 0.9999931 | 1.0000000 | 1.0000000 |
| **105** | 0.6119816 | 0.9755569 | 0.9992936 | 0.9999841 | 0.9999997 | 1.0000000 | 1.0000000 |
| **120** | 0.6873914 | 0.9893398 | 0.9998536 | 0.9999985 | 1.0000000 | 1.0000000 | 1.0000000 |
| **135** | 0.75117649 | 0.9955350 | 0.9999715 | 0.9999999 | 1.0000000 | 1.0000000 | 1.0000000 |
| **150** | 0.8054522 | 0.9981943 | 0.9999948 | 1.0000000 | 1.0000000 | 1.0000000 | 1.0000000 |

**Table 9.- Power calculation for a pan-factor trigenic model**

| **CASES** | **OR** | | | | | | |
|---|---|---|---|---|---|---|---|
| | **2** | **4** | **5** | **6** | **7** | **8** | **9** |
| **0** | 0.0049400 | 0.0049400 | 0.0049400 | 0.0049400 | 0.0049400 | 0.0049400 | 0.0049400 |
| **25** | 0.00211744 | 0.0613694 | 0.0862255 | 0.1153408 | 0.1491971 | 0.1879954 | 0.2316501 |
| **50** | 0.0314495 | 0.1339930 | 0.2097001 | 0.2999788 | 0.4006827 | 0.5058206 | 0.6086585 |
| **75** | 0.0418754 | 0.2196387 | 0.3519240 | 0.4971386 | 0.6380701 | 0.7596280 | 0.8531978 |
| **100** | 0.0526974 | 0.3115057 | 0.4920716 | 0.6660100 | 0.8059993 | 0.9009943 | 0.9557269 |
| **125** | 0.0639720 | 0.4038895 | 0.6169643 | 0.7919281 | 0.9053268 | 0.9640781 | 0.9886458 |
| **150** | 0.0757035 | 0.4924935 | 0.7205455 | 0.8770731 | 0.9571642 | 0.9882061 | 0.9974314 |
| **175** | 0.0878764 | 0.5744086 | 0.8018744 | 0.9305500 | 0.9817896 | 0.9964291 | 0.9994745 |
| **200** | 0.1004662 | 0.6479331 | 0.8630098 | 0.9622332 | 0.9926534 | 0.9989891 | 0.9999010 |
| **225** | 0.1134443 | 0.7123304 | 0.9073515 | 0.9801305 | 0.9971658 | 0.9997296 | 0.9999826 |
| **250** | 0.1267803 | 0.7675763 | 0.9385607 | 0.9898448 | 0.9989482 | 0.9999311 | 0.9999971 |

| | | | | | | | |
|---|---|---|---|---|---|---|---|
| Note: In order to examine this model using HFCC, 150-250 samples are recommended for the F groups in place of 75. | | | | | | | |

**Table 10.- Power calculation for a dominant tetragenic model.**

| **CASES** | **OR** | | | | | | |
|---|---|---|---|---|---|---|---|
| | **2** | **3** | **4** | **5** | **6** | **8** | **10** |
| **0** | 0.0049400 | 0.0049400 | 0.0049400 | 0.0049400 | 0.0049400 | 0.0049400 | 0.0049400 |
| **15** | 0.631454 | 0.1686961 | 0.2860635 | 0.3965561 | 0.4933910 | 0.6436967 | 0.7466626 |
| **30** | 0.1394180 | 0.4134241 | 0.6567685 | 0.8144865 | 0.9034320 | 0.9745893 | 0.9931443 |
| **45** | 0.2294242 | 0.6366523 | 0.8742840 | 0.9626427 | 0.9896287 | 0.9992349 | 0.9999401 |
| **60** | 0.3255404 | 0.7963344 | 0.9619222 | 0.9943057 | 0.9992250 | 0.9999863 | 0.9999997 |
| **75** | 0.4214823 | 0.8944729 | 0.9899762 | 0.9992882 | 0.9999550 | 0.9999998 | 1.0000000 |
| **90** | 0.5126340 | 0.9486871 | 0.9976323 | 0.9999234 | 0.9999978 | 1.0000000 | 1.0000000 |
| **105** | 0.5959878 | 0.9763251 | 0.9994872 | 0.9999927 | 0.9999999 | 1.0000000 | 1.0000000 |
| **120** | 0.6699048 | 0.9895495 | 0.9998966 | 0.9999994 | 1.0000000 | 1.0000000 | 1.0000000 |
| **135** | 0.7338082 | 0.9955585 | 0.9999804 | 0.9999999 | 1.0000000 | 1.0000000 | 1.0000000 |
| **150** | 0.7878794 | 0.9981734 | 0.9999965 | 1.0000000 | 1.0000000 | 1.0000000 | 1.0000000 |

**Table 11.- Power calculation for a pan-factor tetragenic model**

| **CASES** | OR | | | | | | |
|---|---|---|---|---|---|---|---|
| | **3** | **6** | **10** | **12** | **15** | **20** | **25** |
| **0** | 0.0049400 | 0.0049400 | 0.0049400 | 0.0049400 | 0.0049400 | 0.0049400 | 0.0049400 |
| **15** | 0.0141639 | 0.0153215 | 0.0152295 | 0.0155946 | 0.0167686 | 0.0204947 | 0.0268146 |
| **30** | 0.0191639 | 0.0292547 | 0.0439029 | 0.0541158 | 0.0746765 | 0.1274232 | 0.2094213 |
| **45** | 0.0239537 | 0.0460113 | 0.0874806 | 0.1179402 | 0.1795331 | 0.3284336 | 0.5207519 |
| **60** | 0.0287385 | 0.0654857 | 0.1443860 | 0.2031117 | 0.3174563 | 0.5561344 | 0.7815538 |
| **75** | 0.0335887 | 0.0874687 | 0.2117458 | 0.3022354 | 0.4660789 | 0.7443902 | 0.9216928 |
| **90** | 0.0385354 | 0.1117096 | 0.2860637 | 0.4069523 | 0.6053345 | 0.8693291 | 0.9769427 |
| **105** | 0.0435936 | 0.1379352 | 0.3637716 | 0.5097637 | 0.7227941 | 0.9396713 | 0.9942261 |
| **120** | 0.0487707 | 0.1658615 | 0.4416216 | 0.6050174 | 0.8139957 | 0.9744794 | 0.9987370 |
| **135** | 0.0540698 | 0.1952013 | 0.5169215 | 0.6891824 | 0.8801914 | 0.9899904 | 0.9997536 |
| **150** | 0.0594916 | 0.2256707 | 0.5876332 | 0.7606531 | 0.9256061 | 0.9963247 | 0.9999564 |

| | | | | | | | |
|---|---|---|---|---|---|---|---|
| Note: This model can not be examined using HFCC with the proposed sample sizes. | | | | | | | |

**Table 12.- Power calculation for a dominant pentagenic model**

| **CASES** | **OR** | | | | | | |
|---|---|---|---|---|---|---|---|
| | **2** | **3** | **4** | **5** | **6** | **8** | **10** |
| **0** | 0.0049400 | 0.0049400 | 0.0049400 | 0.0049400 | 0.0049400 | 0.0049400 | 0.0049400 |
| **15** | 0.0597166 | 0.1629234 | 0.2839444 | 0.4024856 | 0.5087981 | 0.6750246 | 0.7863940 |
| **30** | 0.1267543 | 0.3893566 | 0.6399359 | 0.8090994 | 0.9049771 | 0.9784711 | 0.9952680 |
| **45** | 0.2051951 | 0.6015251 | 0.8580232 | 0.9586421 | 0.9892492 | 0.9993752 | 0.9999650 |
| **60** | 0.2895969 | 0.7614886 | 0.9528098 | 0.9930652 | 0.9991333 | 0.9999889 | 0.9999999 |
| **75** | 0.3752872 | 0.8666956 | 0.9861830 | 0.9990316 | 0.9999448 | 0.9999999 | 1.0000000 |
| **90** | 0.4586216 | 0.9295382 | 0.9963335 | 0.9998823 | 0.9999971 | 1.0000000 | 1.0000000 |
| **105** | 0.5369942 | 0.9644433 | 09991010 | 0.9999872 | 0.9999999 | 1.0000000 | 1.0000000 |
| **120** | 0.6087311 | 0.9827481 | 0.9997935 | 0.9999987 | 1.0000000 | 1.0000000 | 1.0000000 |
| **135** | 0.6729348 | 0.9919074 | 0.9999551 | 0.9999999 | 1.0000000 | 1.0000000 | 1.0000000 |
| **150** | 0.7293110 | 0.9963139 | 0.9999907 | 1.0000000 | 1.0000000 | 1.0000000 | 1.0000000 |

**Table 13.- Power calculation for a pan-factor pentagenic model**

| **CASES** | **OR** | | | | | | |
|---|---|---|---|---|---|---|---|
| | **10** | **20** | **30** | **40** | **50** | **60** | **80** |
| **0** | 0.0049400 | 0.0049400 | 0.0049400 | 0.0049400 | 0.0049400 | 0.0049400 | 0.0049400 |
| **15** | 0.0049400 | 0.0049400 | 0.0049400 | 0.0049400 | 0.0049400 | 0.0049400 | 0.0049400 |
| **30** | 0.0067016 | 0.0049400 | 0.0049400 | 0.0054418 | 0.0085306 | 0.0147094 | 0.0477584 |
| **45** | 0.0108672 | 0.0112759 | 0.0175520 | 0.0331309 | 0.0664840 | 0.1305829 | 0.3914865 |
| **60** | 0.0159762 | 0.0238848 | 0.0488263 | 0.1078047 | 0.2241673 | 0.4071354 | 0.8153211 |
| **75** | 0.0220639 | 0.0432866 | 0.1039851 | 0.2385041 | 0.4592008 | 0.7081740 | 0.9732721 |
| **90** | 0.0291511 | 0.0702964 | 0.1840354 | 0.4072875 | 0.6880722 | 0.8954282 | 0.9979189 |
| **105** | 0.0372469 | 0.1051523 | 0.2843761 | 0.5807238 | 0.8497978 | 0.9717316 | 0.9999023 |
| **120** | 0.0463501 | 0.1475134 | 0.3964506 | 0.7296255 | 0.9385848 | 0.9940105 | 0.9999970 |
| **135** | 0.0564501 | 0.1965298 | 0.5104191 | 0.8400885 | 0.9782873 | 0.9989713 | 0.9999999 |
| **150** | 0.0675274 | 0.2509619 | 0.6175854 | 0.9126420 | 0.9932513 | 0.9998527 | 1.0000000 |

| | | | | | | | |
|---|---|---|---|---|---|---|---|
| Note this model can not be examined using HFCC with the proposed sample sizes. | | | | | | | |

It is evident that the dominant models can be analysed by employing sample sizes in F in the range of from 75 to 150 individuals in each group (see the boxes highlighted in tables 6,8,10 and 12, 75 samples in each F group seems the optimum for evaluating the dominant models). In contrast, the pan-factor models, employing 4 or more loci in combination, are going to require a greater number of samples in each F subgroup.

In general the calculations of power indicate that 69 and 138 controls are sufficient to achieve viable associations on the assumption base of HFCC: dominant model, digenic combinations (markers taken two by two), minimal size of effect (OR>3), ratio of controls:cases=2:1, alpha error=0.01 and power of 80%. Evidently, other configurations will require specific adjustments. However, the calculations displayed indicate that employing 75 patients, HFCC can approach the classic threshold model (dominant) even for pentagenic configurations (Table 5).

One of the most significant questions concerning association studies by complete mapping of the genome and employing multi-locus studies is the large number of possible combinations when pairs or trios of markers are used in place of individual markers (10000!/(2!x 9998!) and 10000!/(3! x 9997!), respectively, for pairs or trios of genes and so on for dominant models). The method of this invention initially compares the control groups with each other (Cc1 and Cf1) searching for genetic associations. The positive results that are obtained from this comparison can only be explained in a very limited fashion: either by chance, bias in the selection of controls, technical problems during the identification of the markers, or some combination of these. In reference to the associations obtained by chance, employing the pre-fixed level of significance (usually 1%), it is expected to obtain a positive association for every 100 combinations analysed. In other words, in the analysis of a phenotype, the calculations suggest that we can obtain roughly 500,000 positive associations by chance, employing the dominant and digenic model (1% of 10,000!/(2! X 9998!). In reference to bias in selection of controls, the system can detect suspicious control groups that have a poor selection of individuals and suggest the need for re-sampling of controls. This type of association can be quantitatively analysed as an excess of positive associations, over and above that expected by chance, during the process of comparing controls against controls.

Employing this strategy, HFCC can measure the noise of the study on the results for the analysis of the whole genome and not the selection of a small group of markers selected as neutrals a priori. In this respect, the statistics for cases and controls can be analysed in detail. In addition, the deviations from the Hardy-Weinberg (HWD) equilibrium (Hardy GH, 1908; Weinberg W, 1908) provide a base line that allows the determination of the level of bias of the controls, as a deviation from the Hardy-Weinberg equilibrium in controls not selected usually indicates technical problems in the polymorphism being studied (although there can be other reasons). By computing this parameter in controls, a classification can be performed for the markers in the study, and can later be applied to rationalise the true associations. The classification can be established, for example, by multiplying the figure for the Hardy-Weinberg equilibrium for two loci (delta) (Weir B.S. et al. 1976) by the figure used in the control-control association studies. Consequently, the most deviant and associated (between controls) markers appear first in the R0 classification. This combination of information from the control-control associations and HWD can be employed in the HFCC analysis to establish the true noise due to a poor selection of controls and/or problems of genotyping. Ultimately, the information provided by the comparison between control groups is the tool that is employed to rationalise, filter and prioritise the associations observed between patients (F) and controls (Cc).

One of the most notable characteristics of the design of the discovery engine of HFCC is the flexibility of the system for interrogating different aspects of the risk, pharmacogenetic evaluation (adverse effects and effectiveness) or the pathogenesis of multiple diseases. The power of this platform resides in the combination of phenotypes (F1 to Fn) that the investigator can introduce into the discovery engine. As we will explain later, on increasing the number of phenotypes in the engine, the specificity of the tests is also increased. For example, using the HFCC platform we can interrogate genomes in the search for genetic patterns involved in the appearance of carcinomas. Thus, we can select three or more distinct carcinomas (F1 to F3, for example F1= carcinoma of the breast, F2= carcinoma of the breast, F3= carcinoma of the larynx) and apply the HFCC search engine to extract those genetic combinations common to all types of carcinomas in the study, with great precision. In this way, we can infer that HFCC can also be used to create hierarchical classifications of multiple diseases based on genetic footprints of the whole genome of the individuals, or to interrogate different groups of individuals suffering from the same pathology but with different complications or symptoms. This last concept that we have introduced could revolutionise the classification of many related phenotypes and also help to explain a multitude of common adverse effects that are observed during the clinical trials of potentially useful drugs. Thus, it is also easy to comprehend the potential of the system for simultaneously dissecting a great number of complex diseases or phenotypes.

Another important feature of this system lies in its treatment of the results in a quantitative manner. In fact, the excess or the absence of genetic combinations shared among the phenotypes F1 to Fn can serve as an indirect measure of the weighting of the genetic factors involved in the phenotypes being studied, explaining or failing to explain the clinical similarities between the phenotypes or the adverse effects of the drugs.

The raw data that is introduced to the discovery engine is processed by analysis software that systematically applies a series of filters for selecting the most important genetic combinations. More specifically, the program can include four mathematical algorithms to be sequentially applied to the raw data:
a. Initially the system computes all the combinations of markers taken two by two, digenic (and/or three by three, trigenic or n-genic) that are observed in the groups Cc1, Cc2, Cc3, Cf1, Cf2, Cf3, F1, F2, F3 (for the schematic model).
b. Second, the system establishes the level of noise selected associations in the studies of combinations of 2-loci, 3-loci or n-loci among the control groups Ccn and Cfn, producing a number of statistically significant results or combinations of markers (p<0.01) and evaluated by the Hardy-Weinberg calculations of disequilibrium that are included in the archive R0.
c. Third, the system compares F1 versus Cc1 giving a table of results R1, F2 versus Cc2 (R2) and F3 versus Cc3 (R3).
d. Fourth, the system searches for positive associations of combinations that are common to R1, R2 and R3, but do not appear in R0, and it selects them.

It is interesting to note that simply by chance and the establishment of the significance level, each comparison theoretically identifies 1% of associated combinations. However, the probability of obtaining these associations by chance decreases exponentially by comparing the results of each F group with each Cc group and selecting only those combinations contained in all the groups (in other words, just 1/100x 1/100 combinations are shared by chance by two, clinically related phenotypes and so on). On the basis of the theory of probability one can estimate that using a model of two markers combined (digenic or 2-loci), and 10,000 markers studied, there are 10000!/2!x9998! possible combinations of markers, in other words, about 50,000,000 possible combinations (assuming a dominant model). Using just three F groups (Fn=F1,F2 and F3), and α=0.01, we can only expect 50 combinations to be shared by the three groups simultaneously and by chance ((10000!/2!x9998!)/ αⁿ). Therefore, this approach drastically reduces the complexity of the combinations that must be later evaluated in the HFCC validation engine. Comparing the results for related features and verifying still further the selection of combinations applied to the noise filter we reduce the number of combinations obtained simply by chance (for details see table 1).

With a trigenic model, the system continues to function appropriately, that is, using 10,000 markers and this model we obtain 160 billion different combinations that must be analysed in each study of cases and controls. Assuming the dominant model (threshold model), by chance we would obtain 1.6 billion positive associations in each case control study. Nonetheless, by using six independent phenotypic groups (for example six types of carcinomas or six drugs that act on the same protein or biochemical pathway, we would obtain just 160 associated trigenic combinations shared randomly between the six groups. These can be accepted or rejected during the validation process. Thus, this system and method could be of enormous use in the selection of genetic markers and combinations that must be studied during pharmacogenetic research coupled with the clinical trials for higher sample sizes (Phase III and Phase IV), drastically reducing the cost of pharmacogenetic tests during the final stages of the development of new drugs (for example).
Once the potential loci or combinations of loci have been selected, the software optionally and/or with the help of scientific experts can apply other secondary filters for the selection of the pairs or triads of more plausible genes: this is the stage that was earlier referred to as the "Analysis tool". For example, the system can search for shared multipoint genetic segments. Subsequently, the system can automatically localise these segments in the map of the human genome and even re-evaluate the flanking markers in order to contrast their links to the phenotypes. In addition, "biological" filters "can also be applied during this phase of the analysis, the system being able to extract the genes close to the candidate regions identifying the information of interest, using a text mining approach in each region selected (current calculations indicate that it will be necessary to trawl a region of around 200,000 base pairs (bp) surrounding the selected SNP):
a. A locus that appears in excess in the selected combinations
b. Extraction of all the related biochemical and metabolic pathways for the genes close to the selected locus
c. Linkage studies in the area of the selected marker
d. Association studies on the genes of the region
e. Patterns of gene expression of the loci of the region
f. Information on the gene-gene and protein-protein interactions between the genes of the regions involved.

The validation engine is not an innovation in itself. It is rather that the process of validation includes the employment of classic strategies of cases and controls and a study of locus linkages to quantitative features (QTL analysis) in order to re-evaluate the results obtained by the system. Any combination selected is usually reanalysed on large series of patients in order to confirm its association in the selected phenotypes. The replication of genetic association studies in larger series, independent of earlier studies, is the best option for selection of markers for diagnosis, pharmacogenetic trials and/or the tracing of biochemical pathways that are important for the process of discovering drugs (see Hirschhorn JN, et al. 2002a; and Hirschhorn JN, et al. 2002b)

As an example of HFCC, our study of DNA microchips can be applied to 525 individuals: three groups of 75 patients (F1,F2,F3) and three groups of 150 controls (Cc1, Cc2, Cc3) and three groups of internal controls in order to measure the noise (Cfl, Cf2, Cf3). All the controls are extracted and selected randomly from a group taken from a normal population (usually 300 healthy individuals when the corresponding F groups have a size of 75, and 500 individuals when the corresponding F groups have a size of 150). The patients (F) are taken, for example, from individuals diagnosed with three different diseases but characterised by the fact that the clinical profile of all of them share important features among them (it is postulated that these shared features have a common genetic base in the profile of all the patients). For example, patients diagnosed with the metabolic syndrome, PCOS, and hypertension/cardiopathy are selected. All of these are prone to the development of high blood pressure, resistance to insulin, usually sharing a diabetic component. Many other studies could be designed. For example, the group of controls can consist of individuals that have been medicated with a drug or drug group for a certain disease, in which no incidence of adverse effects was registered. On the other hand the three test groups (F) can be individuals that have taken the same drug, but that have experienced an allergic rash (F1), a respiratory irritation (F2) or an intestinal inflammation (F3), all of these sharing the phenotype compatible with an iatrogenic and inflammatory problem. If there is a genetic link that explains the three adverse effects, HFCC can be expected to find it. Another interesting example would be to apply HFCC to three different drugs but having the same biochemical pathway (or that have the same therapeutic target) and that cause the same adverse effect (for example headache) in a subgroup of individuals. In this model we can use as controls (Cc1 to Cc3) patients with the treatments 1, 2, 3 but with no adverse effects registered, and, in the groups F1 to F3 patients with headache registered for each one of the drugs being studied, and look for common factors in F1 to F3 that do not exist in Cc1 to Cc3. As an alternative for the control group in this case we can use individuals from the general population in place of treated patients, as it is better to observe genetic combinations in the general population than in individuals subject to a bias factor.

Another illustrative example of our technology would be its application to the study of carcinomas. In this case the question to be posed would be: Is there a common genetic component in all the types of carcinoma? Therefore, it would be possible to reuse the comparison of controls Cc1, Cc2 and Cc3, and Cf1, Cf2 and Cf3 respectively, previously described as noise filters, and to include in the phenotypic groups a battery of different types of carcinomas. For example, F1: carcinoma of the Breast, F2: carcinoma of the colon, F3: carcinoma of the lung, F4: carcinoma of the larynx (and so on to Fn). The objective would be to identify what is common in F1 to Fn and different from R0 (including all the noise or false associations detected by randomisation and association studies between the control subgroups).

The genetic analyses can be executed employing well established technologies, for example, microchips with 10,000 points set against the DNA of each one of the 525 individuals. Each element of the array can contain a different fixed oligonucleotide that codes for an SNP having these characteristics:
1. It is present in human populations with a reasonably high frequency (allele p>0.2)
2. All of these are localised in distinct positions within the genome.

For example, it would be possible to select some 400 oligonucleotides of each chromosome that could occur every 300 kilobases and have a frequency in the population of about 40 or 50% of the individuals of a population. The DNA of the individual could be distributed throughout the whole array and the pattern for hybridisation could be determined for each individual. Superficially, what would be happening is that one DNA segment of the individual in evaluation would be hybridizing with each specific oligonucleotide for each SNP. The reagents and equipment for performing these studies and generating the raw data for carrying out HFCC are commercially available. For example, the commercial chips for scanning the whole genome, from the companies Illumina or Affymetrix (or any other technology developed in the future).

Records are prepared for the registration of the data for each individual (we will typically employ I.T. memory support tools). These records, for example, contain, for each one of the individuals (patients y controls) on whom the study is carried out:
- information for identification of the individual (identification code)
- one or more cells with the symptoms and phenotypic characteristics of the individual
- for each one of the SNPs considered (10,000 if the micro-array used were Affymetrix 10K), a cell with the sign "+" or "-" or "aa, ab, bb" or any other code, indicating the presence or absence of the polymorphism being considered in this individual,
- in the case of a dominant digenic model, 10,000!/(2!x9.998!) digenic combinations of SNPs, and/or 10,000!/(3!x9997!) combinations in the case of a dominant trigenic model, etc.
This would result in 675 columns, corresponding to the three sets of subgroups of cases and controls, each one with 225 individuals (75 cases and 150 individual controls, although it is possible that some of the individuals of the control could appear in more than one subgroup). Each one of the 675 columns would contain at least 1.66 x 10¹¹ items of data (one of them identifying the phenotype (F) en study). This type of huge matrix represents an authentic computational challenge. However, the discovery of hypothetical associations can be simplified by various strategies. For example, multiple groups of independent matrices can be generated for each pair of distinct SNPs and distinct algorithms executed in each study (some 1,000 million calculations for the study). A conventional PC can execute some seven hundred million of these calculations every second. In this respect, the computational requirements have been estimated according to the total number of SNPs considered in each combination (two: digenic, three: trigenic, four: tetragenic, five: pentagenic), and are shown in Table 11.

**Table 14: Computational viability of HFCC employing 10,000 SNPs.**

| Combination of markers | Digenic | Trigenic | Tetragenic | Pentagenic |
|---|---|---|---|---|
| SNPs genotyped | 10⁴ | 10⁴ | 10⁴ | 10⁴ |
| Combinations possible | 49.9 x 10⁶ | 1.66 x 10¹¹ | 4.16 x 10¹⁴ | 8.32 x 10¹⁷ |
| No of individual operations^{*} | 4.8 x 10⁹ | 2.39 x 10¹³ | 7.99 x 10¹⁶ | 1.79 x 10²⁰ |
| Computational speed | 700 mflops | 17.5 gflops | 70 gflops | 70 gflops |
| Time of computing | 6.85s | 22.8min | 13.2 days | 82 years*** |
| No of computers in cluster** | 1 | 25 | 100 | 100 |

| | | | | |
|---|---|---|---|---|
| * It covers both models, pan-factorial and dominant (flops is a measure of computational speed and equals the number of individual operations per second) ** Assuming a 3Ghz computer (700 Megaflops) (mflops=megaflops, gflops = gigaflops) *** Computational calculation outside range. The pentagenic models require super-computation. Computers that work in the range of teraflops (tflops) like the bluegene cluster of the Livermore National Laboratory (U.S) | | | | |

For triads of genes or greater marker combinations, the dimension of the calculation also grows exponentially. In fact, when 1.66 x 10¹¹ variables, with trigenics in each group as well as in the control groups, are analysed using only two models, it is necessary to perform 2.39 x10¹³ calculations in each study. In this case, a grouping of computers, for example 25 clustered PCs will be necessary in order to complete the task in a reasonable time; however, the analysis is still possible using conventional computing. The computing workload has even been estimated for pentagenic models, but in this case it is appreciated that super-computation with equipment working in the range of (teraflops, 1 teraflop=1 Spanish billion (1.000 american billions) floating point operations per second) is required for the efficient management of the calculations.

The objective of the computational task is to obtain two (or three, etc) SNPs that together are formally associated with the phenotype being studied. This task consists of a systematic checking of each genetic combination in all the groups and the selection of those that are common to all groups. Thus, HFCC selects loci that are commonly associated in the three (or more) phenotypes being studied and that, moreover, are little represented in the control groups. Consequently, for each combination present in the patients, the system analyses the statistical differences between the cases and controls and will compare them with the results of the controls-control studies. In an extreme example, an analysis could lead to the conclusion that a determined genetic combination would be present in all the patients and none of the controls. Then we could be very sure that this combination (or more probably a genetic variation close (of less than 200,000 pb) to our markers) is associated with the phenotype. In other words, that it influences the risk of an individual being prone to a phenotype. With this information, the map of the human genome can be consulted and those genes investigated that are located in the vicinity of the selected SNPs and that are known to have a function close to their function. This screening would lead to new molecular analyses (on occasion already based on a hypothesis) and finally to the elucidation of a pathway that could select different therapeutic targets, suggest treatments using recombinant proteins, or at least lead to a better understanding of the aetiology of the phenotype being studied. This information could generate data for the development of diagnostic tests that predict the probability of suffering from a determined phenotype, a determined prognosis or the appearance of an adverse effect during the consumption of a drug and even its lowered efficiency in an individual. This could be carried out by means of a genetic test, or a test based on the concentration of a protein in a particular fluid or tissue of the patient, a test that determines whether the protein of the patient is mutated or not or any other measurable characteristic that may be a consequence of the particular genetic determinant that isolated itself.

Of course, this extreme hypothetical example almost never occurs. The reality of these trials will be a series of apparent associations, many of which will be due to chance and with only a few being genuine. Conventional data analysis by computer programs attempts to isolate associations by the comparative analysis of the cells of a matrix and then determine which of the associations are apparently genuine and which are not. It usually requires a great number of patients in order to be able to obtain "true" associations. However, even using large numbers of cases and controls, there is always a finite probability that what is observed is not random and that it is related to the phenotype being studied. This is what we refer to as the systematic genome approach (shotgun analysis), using screening of random and unknown sites in the genome (like the random determination of the sites obtained by shooting a cartridge of pellets from a shotgun) and, thus, attempting to capture associations.

In association studies, 525 samples are not sufficient to carry out a viable analysis. However, the HFCC system and its technical characteristics allow the identification of viable associations even using this low number of samples. This is possible because the system calculates the ratio of combinations associated between control groups (all of them due to chance, a poor selection of controls or technical problems). Using this information the system can not only fix the error type I (a lower value of p observed in the control against control association studies, for example) but also consider or fix each positive association with the data derived from a detailed analysis of possible confusing factors introduced into the study by a poor selection of controls. In addition, the system will also compare the positive results from the comparisons of Cc1 to Cc3 vs Cf1 to Cf3 for the labelling or its elimination in the studies carried out comparing the Fn groups against Ccn.

Thus, HFCC differs from genetic identification techniques in, at least, three aspects that are expressed in an integrated form: 1) The system uses a new data filter that allows the generation of significant results with a much lower number of samples. 2) The system preferably employs the analysis of samples coming from distinct groups of patients with distinct diagnostics but with common features, symptoms or phenotypes. 3) The system preferably searches for polygenic associations. Taken as a whole, these characteristics inherently define a greatly optimised method of checking of the genetic base (if it exists) for a determined phenotype, and can be employed to determine whether there is a genetic base for any phenotype or disease or to analyse the effectiveness or toxicity of drugs. The associations selected during the process of sizing control groups and groups of individuals with different phenotypes are very probably (or we can hypothesise that they are) responsible for the phenotypes being studied.

### Examples

In order to demonstrate the application of the HFCC technology, real examples of its application are included below. To help with the understanding of these examples, we begin by giving a brief description of the computer program developed to verify the application of HFCC as a tool for the selection of genetic markers for complex phenotypes, describing below the three Examples carried out with real data and the results of these.

### HFCC software

The technology summarised in Figure 1 has been developed and implemented in a computer program that has been called the "HFCC" program. This computer program incorporates (in a systematic and automatic manner) all the numerous analysis and evaluation processes in the HFCC discovery engine. In addition, the program has been provided with some additional utilities which facilitate the execution of the HFCC experiments and the HFCC validation engine. The description of the procedure performed by the program is better understood when divided into its three tools:

### 1. Tool 1: Matrix generator.

With this utility the data derived from the genotyping tool is prepared and converted into matrixes, which are generated as independent plain text files (.txt). These matrixes contain all the genotypic results of the cases (F files), controls (Cc files) and, when used, the controls that are going to be used for comparison against the Cc controls (Cf files). Therefore, for each study, as many F matrixes have to be generated as the number of F groups that are going to be considered, as many Cc matrixes as the number of Cc groups that are going to be used and, when necessary, as many Cf matrixes as the number of Cf groups that are going to be used. Each matrix of raw data or source data for the HFCC software has as many columns as the number of individuals in the group and as many rows as the number of markers in the study. In each position of the matrix we find a value: 0,1,2,3. From its position (column No and row No) we can locate each genotypic result for each individual. Using the same nomenclature and the same equivalencies commented on when introducing Tables 1, 2 and 3, the significances of these values would be the following:
a. the value zero corresponds to null, i.e., to those cases in which there is no genotypic data available for this marker in the individual considered;
b. the value 1 corresponds to a wild-type genotype or "AA", i.e., if the characteristic analysed is an SNP type polymorphism, the value 1 would indicate that the individual presents in both chromosomes, in the corresponding position, the most frequently occurring nucleotide of the two possible nucleotides that can appear in this position, which would be considered as the wild-type variant of the polymorphism;
c. the value 2 corresponds to a heterozygous genotype or "AB", i.e., the individual presents in one chromosome the most common form of the polymorphism, which is considered the wild-type ("A") allele and, in the other chromosome of the pair, the less frequent nucleotide of the two possible ones that are being considered appears in the corresponding position ("B"), the mutant allele;
d. the value 3 corresponds to a homozygous mutant genotype or "BB".

### 2. Tool 2: Calculation module or Z Test (implements the discovery engine).

- Definition: This is the core algorithm of the HFCC software and enables the multilocus analysis to be performed as conceived in the original report of the HFCC invention. In other words, the system uses the prepared raw genotypic data obtained through any genotyping method and converted into plain text matrixes by Tool 1. Using these files, the module performs an assessment of each and every one of the possible interactive variables derived from the digenic, trigenic etc. combination of all the markers in all the groups of cases and controls used in the study. At present, the HFCC software is developed to perform the pan-factorial model, in which, as previously mentioned, each stratum of each variable is considered an independent variable that is compared with the rest, with the strata that reach a minimum size of effect being selected for analysis. Thus, the system identifies the number of positive and negative individuals for each stratum and computes the nulls for each stratum. With the four resulting values of the counting of the matrixes for each variable (a, b, c, and d, where a = number of positive cases for the stratum in study, b = number of positive controls for the stratum, c = number of negative cases for the stratum, d = number of negative controls for the stratum), the system applies the Wald Test (Z=ln(OR)/SE(in(OR)), where OR is the odds ratio (OR= ad/bc), SE is the standard error (SE (In OR)=the square root of (1/a+1/b+1/c+1/d). The Wald test is used in the manner of an example, however, the system is compatible with other algorithms and computing utilities, among which are those that allow the utilisation of the calculation module using the dominant model or others specifically designed for the user of the program.
- Parameterisation of the calculation module application: To allow its proper use, the calculation module has the following menu of options or parameters, which allow the development and optimisation of HFCC experiments and which must be input to the system in order to apply the calculation module:
   a. Input of the maximum number of cases in each comparison group (F).
      (The system accepts a range of 1-1000 cases per group)
   b. Input of the maximum number of controls in each Cc comparison group
      (The system accepts a range of 1-1000 cases per group).
   c. Input of the maximum number of controls included in each Cf comparison group.
      (The system accepts a range of 1-1000 cases per group)
   d. Input of the number of comparison groups
      (The system allows the simultaneous analysis of up to 10 F groups, 10 Cc groups and 10 independent Cf groups)
   e. Input of the number of genetic markers in the study.
      (The system has been simulated and accepts between 2 and 500,000 independent markers)
   f. Input of the statistical threshold for the selection of positive combinations is a statistical value equal to the square of the Z-test or Wald test, which is employed to define a positive result, in order to choose a stratum.
      (In Example 1 described below the normal value has been employed, Z²>6.65 which corresponds to p=0.01. Notwithstanding, the system accepts any range of positive numerical values and 0. The better the value of Z² selected, the more restrictive is the study).
   g. Input of the correction factor for the a, b, c, and d null values
      (usually 0.33. It is important to introduce the null values, since they have to be subtracted in each calculation from the maximum sample size for each study group)
   h. Input of the localisation path of the F, Cc and Cf files.
   i. Input of the noise filter application: the system permits the choice, whether or not it is going to be applied, of a noise filter which, if applied, would result in the comparison of the Cc groups with the Cf groups.
      (Yes or No: Y/N)
   j. Input of the multilocus module or combination selected:
      i. Monogenic
      ii. Digenic
      iii. Trigenic
      iv. Tetragenic
      v. Pentagenic
   k. Input: printing of intermediate data (y/n). Indicating "Yes" (Y), the system records the results for each stratum and variable analysed: number of observations, number of nulls, odds ratio, Z, Z².
   l. Input of the analysis type
      i. Hard: the stratum selected must be positive in each comparison of F versus Cc and, if the option to apply a noise filter has been selected, the stratum must not be positive in any comparison of Cc versus Cf.
      ii. Fuzzy logic: selects any variable (i.e. any combination of markers) in which at least one of the strata is positive in all the F versus Cc comparisons and, if the option to apply the noise filter has been selected, this combination or variable (all the strata for this variable) will not be positive in any Cc versus Cf comparison.
   m. Input of the statistical model applied.
      i. Exhaustive. This model selects all the markers of the study in order to compile the interactive variables, which would be all possible combinations that could exist between these markers according to the combination model selected (digenic, trigenic...).
      ii. Conditional. The system selects only markers with a marginal effect (those markers having some of their strata with a statistical significance below a determined threshold in monogenic studies) and compares them with the rest of the variables.
      iii. Simultaneous. This only uses markers with a marginal effect for the construction of interactive variables.
- Output records of the calculation module: based on all the parameters input, the calculation module identifies which strata (HARD) or variables (Fuzzy logic) are positive for the study. The result of the procedure programmed in the calculation module consists of a list of interactive variables selected during the process, which the system writes to an output file. In addition, if we apply the "noise filter" option, the system identifies those variables that are positive in the control against control tests (Cc versus Cf) and identifies and saves them in a separate file. The output files are plain text files (.txt) which provide a list of the combinations of markers which have proved positive for the study. In the case of the HARD analysis only the positive stratum appears (for example 23 2 178 1, which corresponds to the combination of the markers 23 and 178, the first is heterozygous or AB and the second wild-type or AA). In the case of the Fuzzy logic analysis, the system saves all possible strata of the study variable to the output record.

### 3. Tool 3: post-hoc ("a posteriori") analysis.

In order to improve the capacity and speed of calculation of the calculation module, the system possesses a version that does not print intermediate data in any case. Therefore, the module does not store any of the results of the negative or positive strata, but only produces the file of the positive strata or variables according to the original input parameters. The *post-hoc* analysis is used to display all the values or results of the positive variables. In this case, the system uses the stored positive results as the exclusive analysis variables and performs all the corresponding calculations in each group of the study on these; in other words, the data forms correspond only to positive variables, with the data of all the strata corresponding to these obtained in all the groups being printed. This printed data allows the investigator to perform a detailed study of the values obtained with the positive variables and to analyse them according to complementary criteria, in order to be able to establish additional filters for the results obtained or to draw conclusions regarding these. An example of the results obtained in a printout of the *post-hoc* analysis can be observed below in Table 15, which appears in Example 1.
It is worth noting that tool 3 can also be used as the reference tool for the validation tests that are proposed in the validation engine described in Figure 1 and which are referred to throughout the report.

Three practical examples are included below, in which the HFCC software is applied to real genotypic data.

### - Example 1: Low-scale application of HFCC to pharmacogenetic trials for controlled ovarian stimulation (COS).

### 1.1 Aim of the study:

The applicant company has wide experience in the identification of genetic factors linked to the response of follicle stimulating hormone (FSH) in women subjected to assisted reproduction techniques (reviewed in De Castro et al., 2005a). These studies set out to identify which genetic markers determine a normal response to treatment, a low efficacy or a pathologic over-response when recombinant FSH (rFSH) is administered pharmacologically. The aim of the study is to discover if there is some multilocus genetic pattern which could be common to a bad response (regardless of whether this is a high or low response) to this treatment. HFCC technology was used in order to be able to answer this question. The idea is to prioritise or select the genes that are most likely to be involved in both phenotypes and on which future developments should be focused.

### 1.2. Description of the study phenotype:

In order to perform this study, a series of cases and controls have been employed for which there was data available in the laboratory of the inventor's group and which had already been broadly disseminated in international scientific journals (De Castro et al., 2003; 2004; 2005a; 2005b; Morón et al., 2006).
a. F groups: Two F groups were employed with phenotypes that were considered "extreme" and opposed as regards the response to FSH hormone, in accordance with the inclusion criteria previously published by the inventor's group (Morón et al., 2006). The idea is to check whether both phenotypes share some common gene or combination of genes that could be simultaneously involved in both phenotypes.
   i. F1: contains the genotypic result for the panel of markers selected from 33 women who have in common a low response to FSH. The selection criteria for these women have been published (De Castro et al., 2003): they are applied to women subjected to assisted reproduction treatments employing recombinant FSH, with a low response being considered as obtaining less than three ovarian follicles during the laparoscopy performed at the end of the hormonal treatment. Those women diagnosed with any ovarian dysfunction are excluded.
   ii. F2: contains the genotypic result for the panel of markers selected from 35 women who have in common a high response to FSH. The selection criteria for these women have been published (Morón et al., 2006): they are applied to women subjected to assisted reproduction treatment employing recombinant FSH, considering a high response as more than 11 ovarian follicles during the laparoscopy performed at the end of the hormonal treatment. Those women diagnosed with any ovarian dysfunction are excluded.
b. Cc groups: two control groups were employed with women with a normal response to FSH. The size of each group is of 275 individuals.
c. Cf groups: two random control groups were employed of 75 women with a normal response to FSH. This is a random selection from women with a normal response to the hormone available in the laboratory of the inventor's group.

### 1.3. Obtaining genotypes in the patients and controls in the study.

In order to perform the genotyping, conventional DNA reading techniques were employed which have been previously described by our group in the scientific works mentioned earlier (pyrosequencing and/or real-time PCR). A total of 10 SNP (*single nucleotide polymorphism*) type markers were selected, distributed in seven different genes: FMR1 (two markers ATL1 and ATL2), GNAS1, CYP19, FSH-receptor, ESR1, ESR2, NRIP1 and BMP15 (two markers bmp15-1 and bmp15-2). In particular, the markers utilised were those which are shown below in Table 15:

**Table 15.- Markers used in the application of HFCC to COS**

| **GENE** | **SNP** | **rs** | **Identification No in the study** |
|---|---|---|---|
| *FSHR* | Ser680Asn | 6166 | 1 |
| *CYP19A1* | 3'UTR | 10046 | 2 |
| *ESR1* | PvulI | 2234693 | 3 |
| *ESR2* | *39A>G | 4986938 | 4 |
| *NRIP1* | Gly75Gly | 2229741 | 5 |
| *BMP15-1* | -9C>G | 3810682 | 6 |
| *BMP15-2* | IVS1+905A>G | 3897937 | 7 |
| *FMR1* | ATL1 | 4949 | 8 |
| *FMR1* | ALT2 :ATL1+244 | 1805423 | 9 |
| *GNAS1* | C>T | 2057291 | 10 |

According to this, there are 10 lines in all the matrixes, which correspond to each one of the genotyped markers in the patients, while the number of columns will correspond to the number of individuals included in each group (33 columns for F1, 35 columns for F2, 275 for Cc1 and Cc2 and 75 columns for Cf1 and Cf2, respectively). Consequently, in each position, the calculated value (0, 1, 2, or 3) for the marker is found by linking the row for the marker to the corresponding column for the individual. The matrixes used in the study, for each group, were those presented below; in the case of groups Cc1 and Cc2, it must be understood that each group of 4 lines would represent a single row in the file of the matrix, the whole row corresponding to the same marker:

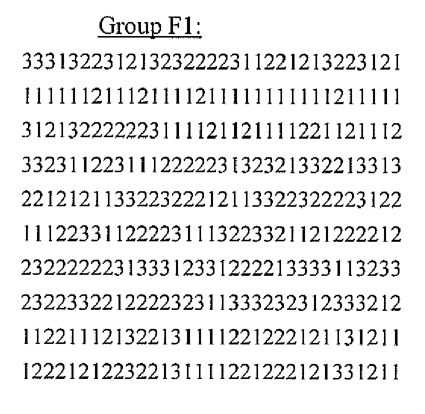

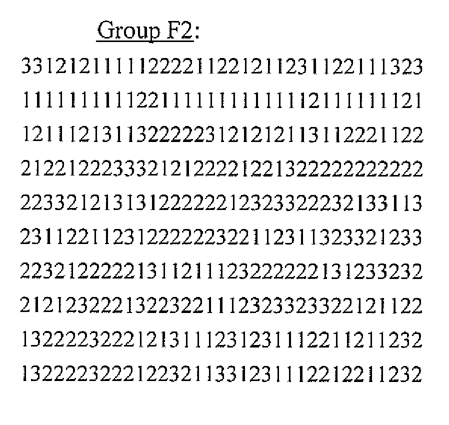

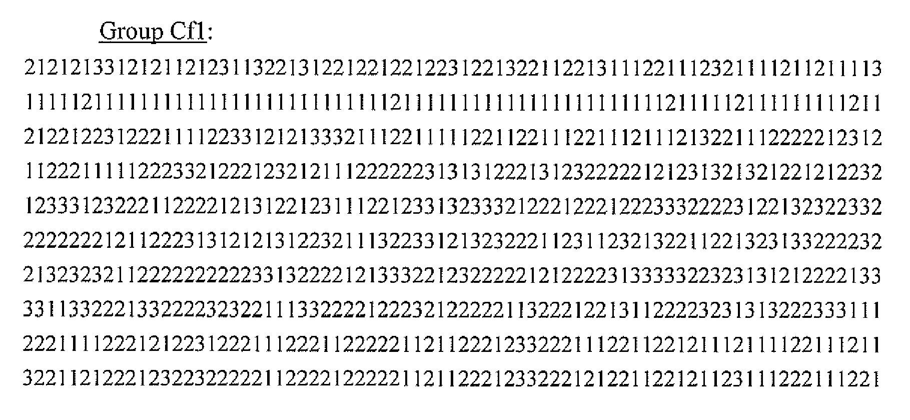

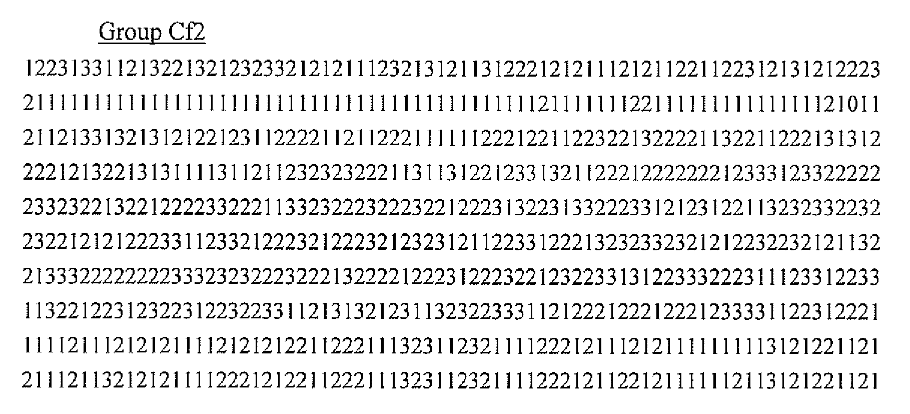

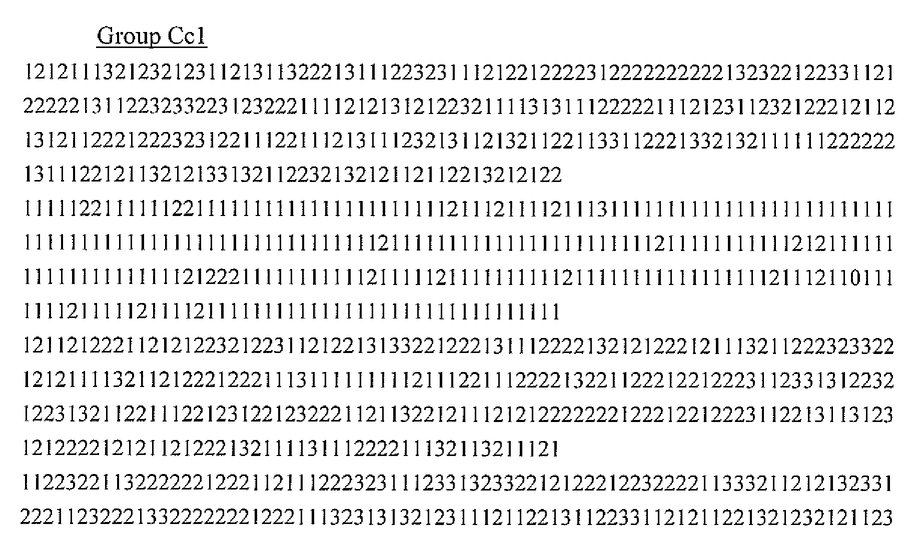

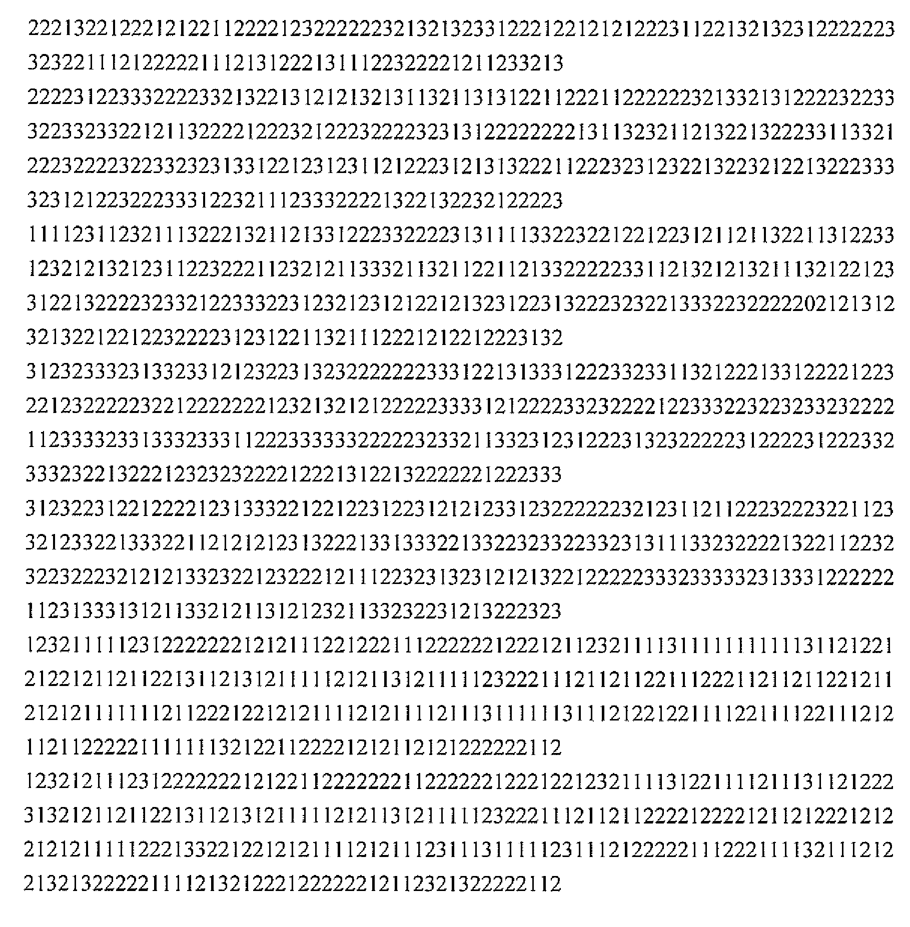

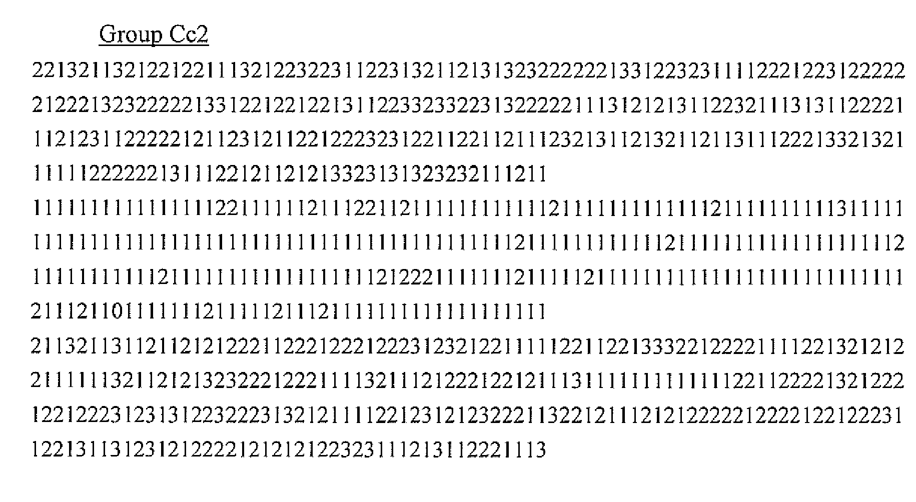

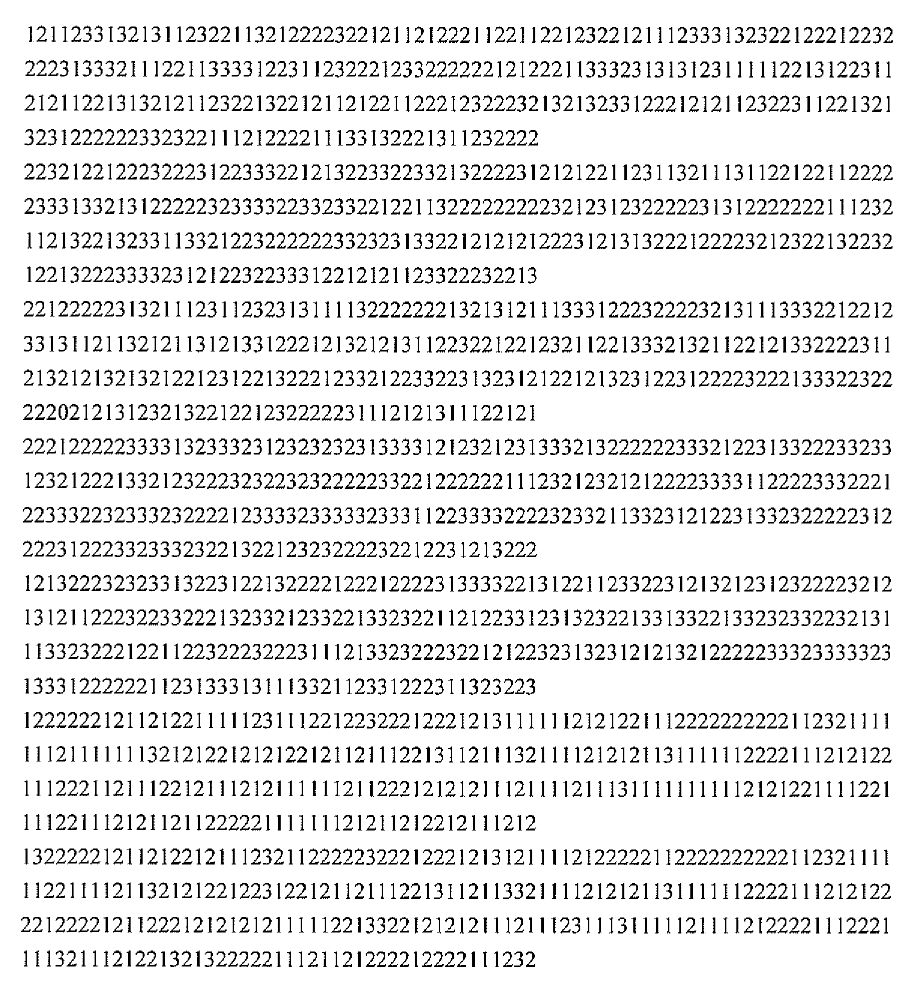

### 1.4 Calculation module parameters applied to the study.

The HFCC software is applied to these input matrixes in accordance with the input values entered in the various parameters of the calculation module, i.e.:
○ Input of the maximum number of cases in F=35
○ Input of the maximum number of controls in Cc=275.
○ Input of the maximum number of controls in Cf=75
○ Input of the number of comparison groups =2
○ Input of the number of genetic markers in study=10
○ Input of the statistical threshold for the selection: Z²>6.65
○ Input of the correction factor for null values=0.33
○ Input of the location path of the F, Cc, Cf. and output files
   ■ /home/aruiz/hfcc/pruebas phase 3/
○ Input of the noise filter application=Yes
○ Input of the multilocus model selected=Digenic
○ Input of printing of intermediate data (y/n)=Yes
○ Input of the type of analysis=Fuzzy logic (chosen because the groups contain distinct phenotypes, because of which it is expected that they will share the variable, but not the stratum)
○ Input of the statistical model applied=Exhaustive

### 1.5. Obtaining specific results.

a. Calculation of interactive variables and number of calculations performed. Using these study parameters, the HFCC software must carry out a combined analysis of ten elements taken two at a time (45 variables) and 405 strata (9 strata per variable when dealing with a digenic model). From each stratum four comparisons must be processed (F1 vs Cc1: G1, F2 vs Cc2: G2, Cc1 vs Cf1: FR G1, and Cc2 vs Cf2: FR G2) which results in the calculation being made on a total of 1620 Wald tests in this study.
b. Results. The system selected only the positive combinations of the 405 possible combinations for both groups (Variable 7_9), corresponding to the combined analysis of the markers within ESR2 and BMP15-2. The probability of getting this result randomly is p=0.0081. On being subjected to a Fuzzy logic analysis, the archive of results contained all the strata of this variable:
   7 1 9 1
   7 1 9 2
   7 1 9 3
   7 2 9 1
   7 2 9 2
   7 2 9 3
   7 3 9 1
   7 3 9 2
   7 3 9 3

The application of the post-hoc analysis gave rise to the results which are displayed below in Table 16:

**Table 16. Results of the post-hoc analysis of the data from Example 1**

| **Variable** | **Group** | **Stratum** | **OR** | **Z** | **Z²** |
|---|---|---|---|---|---|
| 7 9 | (G1) | (7 1 9 1) | 0.88675 | -0.167984 | 0.0282186 |
| 7 9 | (G2) | (7 1 9 1) | 2.4479 | 1.69468 | 2.87193 |
| 7 9 | (FR G1) | (7 1 9 1) | 11.9236 | 1.00258 | 1.00518 |
| 7 9 | (FR G2) | (7 1 9 1) | 13.9296 | 1.06482 | 1.13384 |
| 7 9 | (G1) | (7 1 9 2) | 0.54552 | -0.661823 | 0.43801 |
| 7 9 | (G2) | (7 1 9 2) | 1.52349 | 0.671707 | 0.45119 |
| 7 9 | (FR G1) | (7 1 9 2) | 13.2607 | 1.04517 | 1.09238 |
| 7 9 | (FR G2) | (7 1 9 2) | 14.7911 | 1.0888 | 1.18548 |
| 7 9 | (G1) | (7 1 9 3) | 8.9789 | 2.74952 | 7.55989 |
| 7 9 | (G2) | (7 1 9 3) | 1.95038 | 0.677456 | 0.458947 |
| 7 9 | (FR G1) | (7 1 9 3) | 81.7808 | 1.74569 | 3.04743 |
| 7 9 | (FR G2) | (7 1 9 3) | 50.3433 | 1.5673 | 2.45644 |
| 7 9 | (G1) | (7 2 9 1) | 0.61594 | -1.05041 | 1.10337 |
| 7 9 | (G2) | (7 2 9 1) | 0.45431 | -1.61468 | 2.60718 |
| 7 9 | (FR G1) | (7 2 9 1) | 2.65937 | 0.396704 | 0.157374 |
| 7 9 | (FR G2) | (7 2 9 1) | 2.58522 | 0.385238 | 0.148408 |
| 7 9 | (G1) | (7 2 9 2) | 1.08396 | 0.181762 | 0.0330376 |
| 7 9 | (G2) | (7 2 9 2) | 1.68487 | 1.30596 | 1.70554 |
| 7 9 | (FR G1) | (7 2 9 2) | 3.89366 | 0.551157 | 0.303774 |
| 7 9 | (FR G2) | (7 2 9 2) | 4.13145 | 0.575145 | 0.330792 |
| 7 9 | (G1) | (7 2 9 3) | 0.31774 | -0.642512 | 0.412821 |
| 7 9 | (G2) | (7 2 9 3) | 8.59655 | 3.05004 | 9.30276 |
| 7 9 | (FR G1) | (7 2 9 3) | 32.0924 | 1.39479 | 1.94543 |
| 7 9 | (FR G2) | (7 2 9 3) | 62.2009 | 1.6462 | 2.70997 |
| 7 9 | (G1) | (7 3 9 1) | 1.64973 | 1.24604 | 1.55261 |
| 7 9 | (G2) | (7 3 9 1) | 0.44972 | -1.50168 | 2.25504 |
| 7 9 | (FR G1) | (7 3 9 1) | 3.72587 | 0.533322 | 0.284432 |
| 7 9 | (FR G2) | (7 3 9 1) | 3.254 | 0.47847 | 0.228934 |
| 7 9 | (G1) | (7 3 9 2) | 1.0378 | 0.0679304 | 0.00461454 |
| 7 9 | (G2) | (7 3 9 2) | 0.45667 | -1.11863 | 1.25134 |
| 7 9 | (FR G1) | (7 3 9 2) | 7.02971 | 0.789987 | 0.62408 |
| 7 9 | (FR G2) | (7 3 9 2) | 6.53262 | 0.760374 | 0.578169 |
| 7 9 | (G1) | (7 3 9 3) | 2.04221 | 0.36553 | 0.133612 |
| 7 9 | (G2) | (7 3 9 3) | 32.0886 | 1.77581 | 3.1535 |
| 7 9 | (FR G1) | (7 3 9 3) | 206.263 | 2.04122 | 4.16657 |
| 7 9 | (FR G2) | (7 3 9 3) | 828.273 | 2.22811 | 4.96445 |

It was proved by manual counting in the matrixes and employing conventional statistical techniques (SPSS) as well as the HFCC software post-hoc analysis that the resulting positive strata are those that are shown in boxes in Table 16, i.e.:
7 1 9 3 for F1 (OR=8.9789, Z²=7.55)
7 2 9 3 for F2 (OR=8.5965, Z²=9.30)

To conclude, the HFCC system reveals a single digenic genetic combination for the two extreme phenotypes. This combination indicates which of the seven genes analysed is most likely to be involved in the response to FSH. As shown in Table 16, none of the studies of the control groups (FR G1 and FR G2) exceeds the threshold of statistical significance of Z²>6.65 because of which the variable 7_9 is the only one not to be rejected during the application of the calculation module including the noise filter.

### 1.6. Evaluation of positives obtained.

The results obtained using the HFCC software during this study are completely compatible with our results obtained in previous works (Morón, 2006). Although the interaction of the genes BMP15 and ESR2 is completely new, both had been identified by the inventor's group in previous works (although the powerful interaction between the two and their role in both extreme phenotypes was not known, since ESR2 was only linked to a low response (Phenotype 1. F1), see de Castro, 2004), and BMP15 had been independently associated only with an exaggerated response to FSH (F2), (Morón 2006).
Specifically, patent requests had already been presented for the protection of pharmacogenetic application of the gene BMP15 and its role in ovarian function is endorsed by independent international publications (reviewed in Morón 2006). In addition, the extension of its role in human ovarian function has also been endorsed in recent works and by the group of the inventor himself (Dixit et al., 2006; Di Pascuale et al., 2006; Laissue et al., 2006; Morón et al., not yet published).
Employing conventional statistical techniques we detect the existence of statistical epistasis (gene interaction) between the two markers selected (p<0.01). In addition, the functional regulation of the genes of the BMP family by oestrogens have been documented in the literature and regions of the DNA sequence of the promoter of BMP15 have been identified which join the oestrogen receptor (Morón 2006 and other unpublished data).

This all reinforces the biological plausibility of the results obtained in this experiment and classifies these genes as strong candidates for large-scale pharmacogenetic trials, suggesting their prioritisation ahead of the rest of the markers studied simultaneously.

### - Example 2: Use of HFCC technology for the prioritisation of genetic markers in genetic association studies for Parkinson disease on a massive genomic scale

### 2.1. Aim of the study:

Having proved the reliability of the program on internal data generated in our laboratory, it was decided to prove the robustness and capacity of the invention's selection system of markers, using publicly accessible high-volume genotypic data. To this end, advantage was taken of the existence of a series of international initiatives to carry out the download of raw data of the whole genome genotyping in a series of cases and controls for common illnesses with a high social impact. In particular, the National Institute on Ageing, under the umbrella of the USA's National Institutes of Health (NIH), has an ongoing initiative for Parkinson disease, in which the raw data for the whole genome genotyping is being distributed for both patients and controls for Parkinson disease (Fung et al.., 2006; raw data accessible via http://queue.coriell.org). In order to perform this study, the genotypic data of 270 patients and 270 healthy controls was downloaded and the 31,532 markers corresponding to the human chromosome 1 for these patients and controls (a total of 16,932,684 genotypes) were selected. In view of this information, a concept test was performed in the HFCC system using high-volume real data.

### 2.2. Description of the study phenotype:

Parkinson disease is a quite common chronic neurodegenerative process (incidence 1:1000 in individuals above 65 years). In addition, its global incidence is on the increase due, for the most part, to the progressive ageing of the occidental population. The genetic base of the illness has not been sufficiently clarified. The existence of contributory genetic factors is suspected on the basis of epidemiological risk studies comparing the incidence of the illness in the families of patients affected by the disease and in the general population. In addition, monogenic forms have been described that support the existence of transmittable factors linked to the appearance of this pathology, revised in the database of human genes and genetic alterations at OMIM (Online Medelian Inheritance in Man, accessible at http://www.ncbi.nlm.nih.gov/entrez/query.fcgi?db=OMIM, Ref. 168600). However, it is suspected that the great majority of cases of this illness will have a complex and multifactorial etiology (Farrer, 2006). The illness develops with a progressive neurological deterioration linked to a characteristic loss of the dopaminergic neurons of the black substance, and alteration of the basal ganglions (neuron centres responsible for the initiation and control of movements controlled by the brain). The main clinical feature of the illness is *parkinsonism,* understood as an alteration in the movement of individuals that is characterised by shaking of the extremities, bradykinesia, muscular rigidity and unstable posture.
In order to perform this study a series of publicly available cases and controls publicly available in the NIH archives were employed, which have already been widely approved by panels of international experts in Neurology (to analyse the details of this series of cases and controls see Fung et al., 2006).
a. F Groups: Three F groups were employed, with 90 individuals, all different (F1, F2 and F3). In contrast to the earlier example, these phenotypic groups are considered "clinically identical" with respect to their status: individuals affected by Parkinson's disease. The idea is to check whether genetic combinations exist which are shared by the three homogenous groups and whether, therefore, they will be solidly associated with the pathology. Consequently, the results, predictably, must be completely in harmony for the whole series and, thus, they must have a high probability of being linked to the disease.
b. Cc Groups: similarly, three groups of 90 independent controls were employed with no sign of the illness as defined in the NIH (for details see Fung et al., 2006).
c. Cf Groups: given that the Coriell's database (http://queue.coriell.org) does not contain sufficient controls to form Cf groups with individuals different from those used to form the Cc groups, the Cf groups were established using individuals that formed part of the Cc groups, in such a way that Cf1=Cc2, Cf2=Cc3, Cf3=Cc1).

### 2.3. Obtaining genotypes in the patients and controls of the study.

The genotypic results of the samples contained in groups F1-F3 and the control groups of this example have been genotyped in the Neurogenetic Laboratory and the Unit of Molecular Genetics of the National Institute of Health (NIH, Bethesda, Maryland). These raw genotypic results were generated using the Infinium I and Infinium Human Hap300 technologies of the Illumina company (San Diego, California, United States). The localisation data and information on the raw results from the 31,532 genotypes of chromosome 1 in the 540 cases and controls selected for our HFCC experiment can be freely obtained from the above mentioned Coriell's database, . The quality controls for the genotyping processes employed to obtain these genotypes in the patients included in this study have been previously described (Fung et al., 2006).

Therefore, each one of the matrixes (F1,F2, F3, Cc1, Cc2, Cc3, Cf1, Cf2, Cf3) would contain 31,532 lines, that each correspond to one of the markers genotyped in the individuals selected and 90 columns corresponding to the number of individuals included in each group respectively).

### 2.4. Calculation module parameters applied in the study.

The HFCC software was applied to these input matrixes and in accordance with the input values entered in the various parameters of the calculation module , i.e.:
○ Input of the maximum number of cases in F=90
○ Input of the maximum number of controls in Cc=90.
○ Input of the maximum number of controls in Cf=90
○ Input of the number of comparison groups=3
○ Input of the number of genetic markers in study=31,532
○ Input of the statistical threshold for the selection: Z²>7.879
○ Input of the correction factor for null values=0.33)
○ Input of the location path for F, Cc, Cf. and output files
   ■ /home/aruiz/hfcc/pruebas chromosome 1-3 groups/
○ Input of the application of the noise filter=Yes
○ Input of the multilocus model selected=Digenic
○ Input of printing of intermediate data (y/n)=No
○ Input of the type of analysis=Hard
○ Input of the statistical model applied=Exhaustive

### 2.5. Obtaining specific results

On this occasion, the prioritisation system is confronted by a significant problem since, in contrast to Example 1, in this Example 2 it is sought to prioritise, for Parkinson's disease, a pair of markers from among the 497,117.746 possible ones that are derived from the combination of the 31,532 markers studied and taken two by two (digenic model). Furthermore, these results in a number of mathematical calculations which is nine times greater per group (4,474,059,714), since each interactive variable consists of nine strata, as was explained earlier in this report. Performing the corresponding probability calculations of randomly obtained variables, it was calculated that the number of positive variables must be 559.2 according to our estimates based on the theory of probability.
Using the HFCC software and the matrixes derived from the raw data, the output record produced 657 variables, dispersed throughout chromosome 1. This represents an over-representation of positives of 17% above that expected by chance. The list of the variables obtained can be observed in Table 17.

**Table 17: Strata of variables of chromosome 1 positive for Parkinson disease**

| | | | | | |
|---|---|---|---|---|---|
| 21 6321 2 | 19 1 6321 2 | 36 1 6321 2 | 50 3 6321 2 | 92 1 8330 1 | 141 1 6321 2 |
| 201 3 6321 3 | 271 3 27090 2 | 303 1 6321 2 | 325 3 6321 2 | 372 1 6321 2 | 380 3 6321 2 |
| 457 3 6321 2 | 461 3 6321 2 | 534 1 6321 2 | 597 3 6321 2 | 781 3 6321 2 | 1120 3 6321 2 |
| 1221 1 24281 3 | 1223 3 24281 3 | 1366 1 6321 2 | 1495 1 6321 2 | 1558 1 6321 2 | 1603 3 6321 2 |
| 1610 3 6321 2 | 1623 3 6321 2 | 1696 3 6321 2 | 1750 1 6321 2 | 1758 1 6321 2 | 1759 1 6321 2 |
| 18581 6321 2 | 1925 3 6321 2 | 2026 1 6321 2 | 2088 3 6321 2 | 2142 1 6321 2 | 2311 2 3597 3 |
| 2583 3 6321 2 | 2589 3 6321 2 | 2620 3 4427 2 | 2819 1 6321 2 | 2852 1 6321 2 | 2855 3 6321 2 |
| 28671 6321 2 | 3164 3 6321 2 | 3166 3 6321 2 | 3170 2 22115 2 | 3179 1 6321 2 | 3189 3 6321 2 |
| 32031 6321 2 | 3206 3 6321 2 | 3266 1 6321 2 | 3289 3 6321 2 | 3320 3 6321 2 | 3322 3 6321 2 |
| 3359 3 6321 2 | 3361 3 6321 2 | 3362 3 6321 2 | 3365 3 6321 2 | 3371 3 6321 2 | 3373 3 6321 2 |
| 3374 3 6321 2 | 3465 1 6321 2 | 3525 3 6321 2 | 3547 3 6321 2 | 3553 1 6321 2 | 3642 3 6321 2 |
| 3678 1 6321 2 | 3715 3 6321 2 | 3716 3 6321 2 | 3718 3 6321 2 | 3807 1 6321 2 | 3909 3 6321 2 |
| 3944 3 6321 2 | 3955 3 6321 2 | 3973 3 6321 2 | 4050 1 6568 1 | 4094 3 6321 2 | 4153 3 6321 2 |
| 4169 3 6321 2 | 4181 3 6321 2 | 4182 3 6321 2 | 4184 3 6321 2 | 4204 3 6321 2 | 4220 3 6321 2 |
| 4250 1 6321 2 | 4283 1 23188 3 | 4294 1 6321 2 | 4331 3 6321 2 | 4427 2 6068 1 | 4427 2 12846 1 |
| 4633 3 6321 2 | 4634 1 6321 2 | 4636 1 6321 2 | 4702 3 6321 2 | 4722 3 6321 2 | 4773 1 6321 2 |
| 4782 1 6321 2 | 4784 1 6321 2 | 4793 3 6321 2 | 4796 3 6321 2 | 4802 1 6321 2 | 4830 3 6321 2 |
| 4845 1 6321 2 | 4964 3 6321 2 | 5002 1 6321 2 | 5014 3 6321 2 | 5066 1 6321 2 | 5067 1 6321 2 |
| 5149 3 17806 3 | 5230 1 6321 2 | 5231 3 6321 2 | 5241 3 6321 2 | 5257 1 6321 2 | 5274 3 6321 2 |
| 5288 1 6321 2 | 5299 3 6321 2 | 5300 1 6321 2 | 5302 1 30013 2 | 5320 3 6321 2 | 5375 3 9353 3 |
| 5375 3 15323 1 | 5408 1 6321 2 | 5426 3 6321 2 | 5450 3 6321 2 | 5453 1 6321 2 | 5456 3 6321 2 |
| 5473 1 6321 2 | 5475 1 6321 2 | 5543 1 6321 2 | 5717 1 6321 2 | 5739 1 6321 2 | 5761 1 6321 2 |
| 5867 1 6321 2 | 5878 3 6321 2 | 5884 1 6321 2 | 5885 1 6321 2 | 6097 1 6321 2 | 6200 3 6321 2 |
| 6201 1 6321 2 | 6266 1 6321 2 | 6276 1 6321 2 | 6281 3 6321 2 | 6289 1 6321 2 | 6321 2 6352 1 |
| 6321 2 6423 3 | 6321 2 6509 3 | 6321 2 6617 1 | 6321 2 6710 1 | 6321 2 6714 3 | 6321 2 6779 1 |
| 6321 2 6816 3 | 6321 2 6925 3 | 6321 2 6944 1 | 6321 2 6968 1 | 6321 2 7044 3 | 6321 2 7131 1 |
| 6321 2 7144 1 | 6321 2 7186 3 | 6321 2 7197 3 | 6321 2 7233 1 | 6321 2 7244 3 | 6321 2 7248 3 |
| 6321 2 7287 1 | 6321 2 7329 1 | 6321 2 7338 3 | 6321 2 7345 1 | 6321 2 7347 1 | 6321 2 7349 1 |
| 6321 2 7370 3 | 6321 2 7371 1 | 6321 2 7400 3 | 6321 2 7461 1 | 6321 2 7488 3 | 6321 2 7516 1 |
| 6321 2 7535 1 | 6321 2 7538 1 | 6321 2 7593 3 | 6321 2 7595 3 | 6321 2 7596 1 | 6321 2 7598 1 |
| 6321 2 7601 1 | 6321 2 7656 1 | 6321 2 7686 1 | 6321 2 7708 1 | 6321 2 7709 1 | 6321 2 7710 1 |
| 6321 2 7834 1 | 6321 2 7878 1 | 6321 2 7907 1 | 6321 2 8186 1 | 6321 2 8324 1 | 6321 2 8326 3 |
| 6321 2 8515 1 | 6321 2 8527 1 | 6321 2 8559 1 | 6321 2 8702 3 | 6321 2 8713 3 | 6321 2 8743 3 |
| 6321 2 8748 1 | 6321 2 8750 3 | 6321 2 8751 1 | 6321 2 8784 1 | 6321 2 8862 3 | 6321 2 8864 1 |
| 6321 2 8902 1 | 6321 2 8908 1 | 6321 2 8944 1 | 6321 2 9148 1 | 6321 2 9171 3 | 6321 2 9370 1 |
| 6321 2 9462 3 | 6321 2 9518 3 | 6321 2 9564 1 | 6321 2 9791 1 | 6321 2 9808 1 | 6321 2 9842 3 |
| 6321 2 9846 1 | 6321 2 9847 3 | 6321 2 9849 1 | 6321 2 9869 1 | 6321 2 9895 3 | 6321 2 9915 3 |
| 6321 2 9918 1 | 6321 2 9921 1 | 6321 2 9963 3 | 6321 2 10282 3 | 6321 2 10609 3 | 6321 2 10612 3 |
| 6321 2 10728 3 | 6321 2 10858 1 | 6321 2 10934 3 | 6321 2 10940 3 | 6321 2 10991 1 | 6321 2 10995 3 |
| 6321 2 10996 3 | 6321 2 10997 3 | 6321 2 11001 1 | 6321 2 11010 1 | 6321 2 11044 3 | 6321 2 11145 1 |
| 6321 2 11203 1 | 6321 2 11208 1 | 6321 2 11245 3 | 6321 2 11435 3 | 6321 2 11591 1 | 6321 2 11600 1 |
| 6321 2 11603 1 | 6321 2 11604 3 | 6321 2 11688 1 | 6321 2 11705 1 | 6321 2 11865 3 | 6321 2 11867 1 |
| 6321 2 11868 3 | 6321 2 11916 1 | 6321 2 11950 3 | 6321 2 12008 1 | 6321 2 12194 1 | 6321 2 12415 1 |
| 6321 2 12472 1 | 6321 2 12485 1 | 6321 2 12552 1 | 6321 2 12559 1 | 6321 2 12562 1 | 6321 2 12563 3 |
| 6321 2 12564 1 | 6321 2 12605 3 | 6321 2 12736 1 | 6321 2 12745 3 | 6321 2 12807 3 | 6321 2 13136 1 |
| 6321 2 13208 1 | 6321 2 13211 1 | 6321 2 13214 1 | 6321 2 13217 1 | 6321 2 13228 1 | 6321 2 13365 1 |
| 6321 2 13552 1 | 6321 2 13558 1 | 6321 2 13620 3 | 6321 2 13672 1 | 6321 2 13684 3 | 6321 2 13750 3 |
| 6321 2 13763 3 | 6321 2 13764 1 | 6321 2 13899 3 | 6321 2 14056 1 | 6321 2 14102 1 | 6321 2 14112 3 |
| 6321 2 14200 1 | 6321 2 14205 3 | 6321 2 14206 3 | 6321 2 14208 3 | 6321 2 14214 3 | 6321 2 14342 1 |
| 6321 2 14376 1 | 6321 2 14379 1 | 6321 2 14405 1 | 6321 2 14420 1 | 6321 2 14445 3 | 6321 2 14460 1 |
| 6321 2 14584 1 | 6321 2 14593 3 | 6321 2 14620 3 | 6321 2 14800 3 | 6321 2 14803 1 | 6321 2 14838 1 |
| 6321 2 14844 1 | 6321 2 14983 1 | 6321 2 15210 1 | 6321 2 15263 3 | 6321 2 15353 3 | 6321 2 15652 1 |
| 6321 2 15910 1 | 6321 2 15992 3 | 6321 2 15998 3 | 6321 2 16023 3 | 6321 2 16030 1 | 6321 2 16074 3 |
| 6321 2 16159 1 | 6321 2 16200 1 | 6321 2 16207 1 | 6321 2 16213 3 | 6321 2 16220 1 | 6321 2 16221 1 |
| 6321 2 16239 1 | 6321 2 16319 1 | 6321 2 16363 3 | 6321 2 16518 3 | 6321 2 16521 1 | 6321 2 16527 3 |
| 6321 2 16528 3 | 6321 2 16529 3 | 6321 2 16536 3 | 6321 2 16609 1 | 6321 2 16644 1 | 6321 2 16662 3 |
| 6321 2 16742 3 | 6321 2 16812 1 | 6321 2 16814 1 | 6321 2 16833 1 | 6321 2 16859 1 | 6321 2 16915 1 |
| 6321 2 16921 1 | 6321 2 16973 3 | 6321 2 16988 3 | 6321 2 16989 1 | 6321 2 16990 3 | 6321 2 16994 3 |
| 6321 2 16995 1 | 6321 2 17000 1 | 6321 2 17003 1 | 6321 2 17067 1 | 6321 2 17110 1 | 6321 2 17161 3 |
| 6321 2 17162 1 | 6321 2 17165 1 | 6321 2 17175 1 | 6321 2 17190 1 | 6321 2 17200 1 | 6321 2 17202 3 |
| 6321 2 17209 1 | 6321 2 17263 3 | 6321 2 17299 1 | 6321 2 17470 3 | 6321 2 17572 3 | 6321 2 17574 1 |
| 6321 2 17575 1 | 6321 2 17669 3 | 6321 2 17672 1 | 6321 2 17813 1 | 6321 2 17819 1 | 6321 2 17825 1 |
| 6321 2 17894 1 | 6321 2 17895 3 | 6321 2 17898 3 | 6321 2 17900 1 | 6321 2 17920 1 | 6321 2 17997 1 |
| 6321 2 18049 1 | 6321 2 18050 1 | 6321 2 18052 1 | 6321 2 18090 3 | 6321 2 18181 1 | 6321 2 18340 3 |
| 6321 2 18374 1 | 6321 2 18453 3 | 6321 2 18759 1 | 6321 2 18761 1 | 6321 2 18762 1 | 6321 2 18773 1 |
| 6321 2 18778 1 | 6321 2 18787 3 | 6321 3 18965 1 | 6321 2 18978 1 | 6321 2 18979 3 | 6321 2 19002 1 |
| 6321 2 19003 3 | 6321 2 19004 1 | 6321 2 19131 3 | 6321 2 19242 1 | 6321 2 19429 1 | 6321 2 19430 1 |
| 6321 2 19518 3 | 6321 2 19536 3 | 6321 2 19538 1 | 6321 2 19541 1 | 6321 2 19551 1 | 6321 2 19692 1 |
| 6321 2 19797 3 | 6321 2 20086 1 | 6321 2 20127 3 | 6321 2 20218 3 | 6321 2 20226 1 | 6321 2 20233 3 |
| 6321 2 20328 1 | 6321 2 20465 1 | 6321 2 20534 3 | 6321 2 20690 3 | 6321 2 20722 1 | 6321 2 20728 1 |
| 6321 2 20734 3 | 6321 2 20783 1 | 6321 2 20793 1 | 6321 2 20873 3 | 6321 2 20955 3 | 6321 2 21108 1 |
| 6321 2 21218 3 | 6321 2 21258 1 | 6321 2 21259 1 | 6321 2 21260 1 | 6321 2 21263 1 | 6321 2 21305 1 |
| 6321 2 21438 3 | 6321 2 21601 3 | 6321 2 21622 3 | 6321 2 21635 3 | 6321 2 21646 3 | 6321 2 21697 1 |
| 6321 2 21796 1 | 6321 2 21797 1 | 6321 2 21813 1 | 6321 2 21948 1 | 6321 2 21957 3 | 6321 2 22018 3 |
| 6321 2 22045 3 | 6321 2 22051 1 | 6321 2 22054 1 | 6321 2 22061 3 | 6321 2 22062 3 | 6321 2 22063 1 |
| 6321 2 22066 3 | 6321 2 22067 1 | 6321 2 22068 1 | 6321 2 22129 1 | 6321 2 22386 1 | 6321 2 22560 3 |
| 6321 2 22567 1 | 6321 2 22591 3 | 6321 2 22710 1 | 6321 2 22714 1 | 6321 2 22749 3 | 6321 2 22915 1 |
| 6321 2 22921 3 | 6321 2 22936 1 | 6321 2 23013 3 | 6321 2 23018 3 | 6321 2 23042 3 | 6321 2 23046 1 |
| 6321 2 23110 3 | 6321 2 23156 1 | 6321 2 23170 3 | 6321 2 23262 1 | 6321 2 23264 1 | 6321 2 23266 1 |
| 6321 2 23290 3 | 6321 2 23319 3 | 6321 2 23366 1 | 6321 2 23405 1 | 6321 2 23498 3 | 6321 2 23540 1 |
| 6321 2 23543 1 | 6321 2 23660 3 | 6321 2 23828 3 | 6321 2 23831 1 | 6321 2 23963 3 | 6321 3 24038 1 |
| 6321 2 24066 3 | 6321 2 24230 1 | 6321 2 24262 1 | 6321 2 24348 1 | 6321 2 24354 3 | 6321 2 24465 1 |
| 6321 2 24542 3 | 6321 2 24711 3 | 6321 2 24714 1 | 6321 2 24782 3 | 6321 2 25024 3 | 6321 2 25107 3 |
| 6321 2 25145 1 | 6321 2 25148 1 | 6321 2 25202 1 | 6321 2 25310 3 | 6321 2 25350 3 | 6321 2 25504 1 |
| 6321 2 25505 1 | 6321 2 25512 1 | 6321 2 25513 3 | 6321 2 25558 1 | 6321 2 25560 1 | 6321 2 25588 3 |
| 6321 2 25611 1 | 6321 2 25617 1 | 6321 2 25723 1 | 6321 2 25744 1 | 6321 2 25826 3 | 6321 2 25905 1 |
| 6321 2 25919 1 | 6321 2 25935 1 | 6321 2 25950 1 | 6321 2 25951 3 | 6321 2 25957 1 | 6321 2 25963 1 |
| 6321 2 25987 3 | 6321 2 25988 3 | 6321 2 26125 1 | 6321 2 26179 3 | 6321 2 26186 3 | 6321 2 26295 3 |
| 6321 2 26437 3 | 6321 2 26448 1 | 6321 2 26449 1 | 6321 2 26628 1 | 6321 2 26718 1 | 6321 2 26833 3 |
| 6321 2 27040 3 | 6321 2 27042 1 | 6321 2 27046 1 | 6321 2 27078 1 | 6321 2 27083 3 | 6321 2 27087 3 |
| 6321 2 27089 1 | 6321 2 27138 2 | 6321 3 27196 3 | 6321 3 27198 1 | 6321 3 27200 3 | 6321 3 27204 3 |
| 6321 3 27205 3 | 6321 3 27207 1 | 6321 2 27546 1 | 6321 2 27608 3 | 6321 2 27610 3 | 6321 2 27612 3 |
| 6321 2 27614 3 | 6321 2 27616 3 | 6321 2 27617 1 | 6321 2 27618 1 | 6321 2 27619 1 | 6321 3 27622 1 |
| 6321 3 27623 1 | 6321 3 27626 1 | 6321 2 27651 3 | 6321 2 27712 3 | 6321 2 27771 3 | 6321 2 27788 3 |
| 6321 2 27936 3 | 6321 2 28010 1 | 6321 2 28049 1 | 6321 2 28051 3 | 6321 2 28052 3 | 6321 2 28061 1 |
| 6321 2 28070 3 | 6321 2 28131 1 | 6321 2 28185 1 | 6321 2 28236 1 | 6321 2 28238 1 | 6321 2 28325 3 |
| 6321 2 28333 1 | 6321 2 28578 1 | 6321 2 28663 3 | 6321 2 28673 1 | 6321 2 287001 | 6321 2 28765 3 |
| 6321 2 28779 1 | 6321 2 28789 3 | 6321 2 28923 3 | 6321 2 28926 1 | 6321 2 290481 | 6321 2 29092 3 |
| 6321 2 29171 3 | 6321 2 29250 1 | 6321 2 29254 3 | 6321 2 29314 1 | 6321 2 293291 | 6321 2 29523 3 |
| 6321 2 29572 3 | 6321 2 29575 3 | 6321 2 29622 1 | 6321 2 29627 3 | 6321 2 29672 1 | 6321 2 29740 1 |
| 6321 2 29833 1 | 6321 2 29841 3 | 6321 2 30010 1 | 6321 2 30177 3 | 6321 2 30187 1 | 6321 2 30412 3 |
| 6321 2 30529 1 | 6321 2 30689 1 | 6321 2 30729 1 | 6321 2 30766 1 | 6321 2 30866 1 | 6321 2 30909 3 |
| 6321 2 30952 3 | 6321 2 31110 3 | 6321 2 31391 3 | 6321 2 31428 3 | 6321 2 31531 1 | 65681 22216 1 |
| 65693 2 2216 1 | 6570 1 22216 1 | 6571 3 22216 1 | 8466 3 22099 3 | 8897 3 15305 1 | 10073 3 15218 2 |
| 10073 3 15224 2 | 10386 2 16101 3 | 10788 3 26873 1 | 11993 1 22099 3 | 13145 2 27852 3 | 13221 1 22109 3 |
| 13221 1 22112 1 | 13221 1 22113 3 | 13221 1 22114 1 | 13861 3 22083 3 | 15993 2 30662 2 | 16005 2 30662 2 |
| 17177 3 22099 3 | 18014 2 30063 1 | 18262 1 27805 1 | 18264 3 27805 1 | 18266 3 27805 1 | 18267 3 27805 1 |
| 184133 2 10701 1 | 21014 3 24281 2 | 21689 1 23137 3 | 22109 3 24450 2 | 22109 3 29832 3 | 22112 1 29832 3 |
| 22113 3 29832 3 | 22114 1 29832 3 | 22216 1 28979 3 | 22235 3 23137 3 | 22293 2 23036 2 | 22296 2 23036 2 |
| 22300 2 23036 2 | 22879 3 24281 3 | 24281 3 29792 2 | | | |

A summary study of the matrixes of results allowed to identify a single marker (num. 6321, corresponding to the marker rs12069733 of chromosome 1) that was present in 601 of the combinations obtained (91% of the positive combinations). The simple interpretation of this result only offers two possibilities:
- That marker 6321 is tracking a gene that is very important in Parkinson disease.
- That the results for this marker were due to its poor genotyping (a much more plausible hypothesis, given the characteristics of the illness).

Coriell's database also contains the univariate data for all the markers studied and the Hardy-Weinberg equilibrium (HWE) value for all of these. Thus, the second hypothesis can be confirmed, since this marker has a marked HWE disequilibrium (HWD, p=0.0000000131), which is completely compatible with a genotyping problem for this SNP (Ho and Ott, 2003).

The rest of the positive variables (55 combinations, 110 markers) derived from the study, in which marker 6321 was not included, were subjected to a post-hoc analysis in order to evaluate the direction and genetic model of the potential interactions detected. As previously explained, the HFCC post-hoc analysis allows the systematic evaluation of all the strata and positive groups of simultaneous studies. The output obtained in the printout following the elimination of all the variables in which marker 6321 intervened is displayed in Table 18, in which the strata that demonstrated a homogenous direction of effect have been indicated by means of spotted lines.

**Table 18.- Statistical data corresponding to each and every one of the strata of the positive variables detected by HFCC.**

| **Variable** | | **Group** | **Stratum** | | | | **OR** | **Z** | **Z²** | **epist*** |
|---|---|---|---|---|---|---|---|---|---|---|
| 92 | 8330 | (G1) | (92 | 1 | 8330 | 1) | 0,280281 | -3,05809 | 9,3519 | |
| 92 | 8330 | (G2) | (92 | 1 | 8330 | 1) | 3,71514 | 2,87874 | 8,28716 | |
| 92 | 8330 | (G3) | (92 | 1 | 8330 | 1) | 0,269169 | -2,87874 | 8,28716 | |
| 92 | 8330 | (G1) | (92 | 1 | 8330 | 2) | 1,77396 | 1,28826 | 1,6596 | |
| 92 | 8330 | (G2) | (92 | 1 | 8330 | 2) | 0,716495 | -0,812023 | 0,659381 | |
| 92 | 8330 | (G3) | (92 | 1 | 8330 | 2) | 1,13382 | 0,250349 | 0,0626747 | |
| 92 | 8330 | (G1) | (92 | 1 | 8330 | 3) | 1 | 0 | 0 | |
| 92 | 8330 | (G2) | (92 | 1 | 8330 | 3) | 1 | 0 | 0 | |
| 92 | 8330 | (G3) | (92 | 1 | 8330 | 3) | 1 | 0 | 0 | |
| 92 | 8330 | (G1) | (92 | 2 | 8330 | 1) | 1,51106 | 1,27852 | 1,63462 | |
| 92 | 8330 | (G2) | (92 | 2 | 8330 | 1) | 1,24544 | 0,661505 | 0,437589 | |
| 92 | 8330 | (G3) | (92 | 2 | 8330 | 1) | 1 | 0 | 0 | |
| 92 | 8330 | (G1) | (92 | 2 | 8330 | 2) | 1,20695 | 0,432875 | 0,18738 | |
| 92 | 8330 | (G2) | (92 | 2 | 8330 | 2) | 0,775473 | -0,7113 | 0,505948 | |
| 92 | 8330 | (G3) | (92 | 2 | 8330 | 2) | 1,71639 | 1,62825 | 2,6512 | |
| 92 | 8330 | (G1) | (92 | 2 | 8330 | 3) | 1 | 0 | 0 | |
| 92 | 8330 | (G2) | (92 | 2 | 8330 | 3) | 1 | 0 | 0 | |
| 92 | 8330 | (G3) | (92 | 2 | 8330 | 3) | 1 | 0 | 0 | |
| 92 | 8330 | (G1) | (92 | 3 | 8330 | 1) | 1,09508 | 0,213015 | 0,0453756 | |
| 92 | 8330 | (G2) | (92 | 3 | 8330 | 1) | 0,583713 | -1,01904 | 1,03844 | |
| 92 | 8330 | (G3) | (92 | 3 | 8330 | 1) | 2,28648 | 1,5094 | 2,27829 | |
| 92 | 8330 | (G1) | (92 | 3 | 8330 | 2) | 1 | 0 | 0 | |
| 92 | 8330 | (G2) | (92 | 3 | 8330 | 2) | 0,396817 | -1,70693 | 2,91363 | |
| 92 | 8330 | (G3) | (92 | 3 | 8330 | 2) | 0,766782 | -0,513054 | 0,263224 | |
| 92 | 8330 | (G1) | (92 | 3 | 8330 | 3) | 1 | 0 | 0 | |
| 92 | 8330 | (G2) | (92 | 3 | 8330 | 3) | 1 | 0 | 0 | |
| 92 | 8330 | (G3) | (92 | 3 | 8330 | 3) | 1 | 0 | 0 | |
| 271 | 27090 | (G1) | (271 | 1 | 27090 | 1) | 1,01119 | 0,00451366 | 2,04E-05 | |
| 271 | 27090 | (G2) | (271 | 1 | 27090 | 1) | 4,0303 | 0,714596 | 0,510647 | |
| 271 | 27090 | (G3) | (271 | 1 | 27090 | 1) | 1 | 0 | 0 | |
| 271 | 27090 | (G1) | (271 | 1 | 27090 | 2) | 0,769053 | -0,352671 | 0,124377 | |
| 271 | 27090 | (G2) | (271 | 1 | 27090 | 2) | 0,988679 | -0,0121294 | 0,00014712 | |
| 271 | 27090 | (G3) | (271 | 1 | 27090 | 2) | 1,77171 | 0,521337 | 0,271792 | |
| 271 | 27090 | (G1) | (271 | 1 | 27090 | 3) | 2,59077 | 0,918164 | 0,843024 | |
| 271 | 27090 | (G2) | (271 | 1 | 27090 | 3) | 0,751441 | -0,383785 | 0,147291 | |
| 271 | 27090 | (G3) | (271 | 1 | 27090 | 3) | 1 | 0 | 0 | |
| 271 | 27090 | (G1) | (271 | 2 | 27090 | 1) | 1,9237 | 0,791554 | 0,626558 | |
| 271 | 27090 | (G2) | (271 | 2 | 27090 | 1) | 2,81338 | 1,32389 | 1,75268 | |
| 271 | 27090 | (G3) | (271 | 2 | 27090 | 1) | 1,44555 | 0,424857 | 0,180503 | |
| 271 | 27090 | (G1) | (271 | 2 | 27090 | 2) | 1,81091 | 1,53599 | 2,35927 | |
| 271 | 27090 | (G2) | (271 | 2 | 27090 | 2) | 0,577455 | -1,27971 | 1,63766 | |
| 271 | 27090 | (G3) | (271 | 2 | 27090 | 2) | 2,17138 | 1,70467 | 2,90589 | |
| 271 | 27090 | (G1) | (271 | 2 | 27090 | 3) | 0,858145 | -0,374774 | 0,140455 | |
| 271 | 27090 | (G2) | (271 | 2 | 27090 | 3) | 2,22018 | 2,10986 | 4,45151 | |
| 271 | 27090 | (G3) | (271 | 2 | 27090 | 3) | 1,9545 | 1,70117 | 2,89399 | |
| 271 | 27090 | (G1) | (271 | 3 | 27090 | 1) | 0,863574 | -0,259937 | 0,067567 | |
| 271 | 27090 | (G2) | (271 | 3 | 27090 | 1) | 1,23095 | 0,312535 | 0,0976783 | |
| 271 | 27090 | (G3) | (271 | | 27090 | 1) | 0,260712 | -1,77642 | 3,15566 | |
| 271 | 27090 | (G1) | (271 | 3 | 27090 | 2) | 0,36024 | -2,81196 | 7,90711 | 1 |
| 271 | 27090 | (G2) | (271 | 3 | 27090 | 2) | 0,349528 | -2,89383 | 8,37428 | 1 |
| 271 | 27090 | (G3) | (271 | 3 | 227090 | 2) | 0.296503 | -3,30387 | 10,9155 | 1 |
| 271 | 27090 | (G1) | (271 | 3 | 27090 | 3) | 1,66611 | 1,43608 | 2,06233 | |
| 271 | 27090 | (G2) | (271 | 3 | 27090 | 3) | 1,53092 | 1,1917 | 1,42016 | |
| 271 | 27090 | (G3) | (271 | 3 | 27090 | 3) | 1,4634 | 1,14899 | 1,32017 | |
| 1221 | 24281 | (G1) | (1221 | 1 | 24281 | 1) | 1,08412 | 0,0327342 | 0,00107153 | |
| 1221 | 24281 | (G2) | (1221 | 1 | 24281 | 1) | 1,18654 | 0,0693131 | 0,00480431 | |
| 1221 | 24281 | (G3) | (1221 | 1 | 24281 | 1) | 1,45183 | 0,151063 | 0,0228202 | |
| 1221 | 24281 | (G1) | (1221 | 1 | 24281 | 2) | 0,754266 | -0,713771 | 0,50947 | |
| 1221 | 24281 | (G2) | (1221 | 1 | 24281 | 2) | 0,516903 | -1,59954 | 2,55854 | |
| 1221 | 24281 | (G3) | (1221 | 1 | 24281 | 2) | 0,260236 | -2,60136 | 6,76705 | |
| 1221 | 24281 | (G1) | (1221 | 1 | 24281 | 3) | 2,66654 | 2,87707 | 8,27755 | 2 |
| 1221 | 24281 | (G2) | (1221 | 1 | 24281 | 3) | 2,94766 | 3,01139 | 9,06849 | 2 |
| 1221 | 24281 | (G3) | (1221 | 1 | 24281 | 3) | 2,91258 | 2,96354 | 8,78257 | 2 |
| 1221 | 24281 | (G1) | (1221 | 2 | 24281 | 1) | 1,08412 | 0,0327342 | 0,00107153 | |
| 1221 | 24281 | (G2) | (1221 | 2 | 24281 | 1) | 1,18654 | 0,0693131 | 0,00480431 | |
| 1221 | 24281 | (G3) | (1221 | 2 | 24281 | 1) | 1,45183 | 0,151063 | 0,0228202 | |
| 1221 | 24281 | (G1) | (1221 | 2 | 24281 | 2) | 0,696923 | -0,74925 | 0,561376 | |
| 1221 | 24281 | (G2) | (1221 | 2 | 24281 | 2) | 0,37446 | -1,67434 | 2,80343 | |
| 1221 | 24281 | (G3) | (1221 | 2 | 24281 | 2) | 0,0364959 | -1,86497 | 3,47812 | |
| 1221 | 24281 | (G1) | (1221 | 2 | 24281 | 3) | 0,417349 | -2,08311 | 4,33936 | |
| 1221 | 24281 | (G2) | (1221 | 2 | 24281 | 3) | 0,822092 | -0,514259 | 0,264463 | |
| 1221 | 24281 | (G3) | (1221 | 2 | 24281 | 3) | 1,4345 | 0,925991 | 0,85746 | |
| 1221 | 24281 | (G1) | (1221 | 3 | 24281 | 1) | 1,08412 | 0,0327342 | 0,00107153 | |
| 1221 | 24281 | (G2) | (1221 | 3 | 24281 | 1) | 1,18654 | 0,0693131 | 0,00480431 | |
| 1221 | 24281 | (G3) | (1221 | 3 | 24281 | 1) | 1,45183 | 0,151063 | 0,0228202 | |
| 1221 | 24281 | (G1) | (1221 | 3 | 24281 | 2) | 1,08412 | 0,0327342 | 0,00107153 | |
| 1221 | 24281 | (G2) | (1221 | 3 | 24281 | 2) | 1,18654 | 0,0693131 | 0,00480431 | |
| 1221 | 24281 | (G3) | (1221 | 3 | 24281 | 2) | 1,45183 | 0,151063 | 0,0228202 | |
| 1221 | 24281 | (G1) | (1221 | 3 | 24281 | 3) | 1,08412 | 0,0327342 | 0,00107153 | |
| 1221 | 24281 | (G2) | (1221 | 3 | 24281 | 3) | 1,18654 | 0,0693131 | 0,00480431 | |
| 1221 | 24281 | (G3) | (1221 | 3 | 24281 | 3) | 1,45183 | 0,151063 | 0,0228202 | |
| 1223 | 24281 | (G1) | (1223 | 1 | 24281 | 1) | 1,08412 | 0,0327342 | 0,00107153 | |
| 1223 | 24281 | (G2) | (1223 | 1 | 24281 | 1) | 1,18368 | 0,0683391 | 0,00467023 | |
| 1223 | 24281 | (G3) | (1223 | 1 | 24281 | 1) | 1,46991 | 0,156078 | 0,0243604 | |
| 1223 | 24281 | (G1) | (1223 | 1 | 24281 | 2) | 1,08654 | 0,0881756 | 0,00777494 | |
| 1223 | 24281 | (G2) | (1223 | 1 | 24281 | 2) | 0,289898 | -0,634314 | 0,402354 | |
| 1223 | 24281 | (G3) | (1223 | 1 | 24281 | 2) | 0,106516 | -1,23423 | 1,52333 | |
| 1223 | 24281 | (G1) | (1223 | 1 | 24281 | 3) | 1,36085 | 0,460701 | 0,212245 | |
| 1223 | 24281 | (G2) | (1223 | 1 | 24281 | 3) | 2,11149 | 0,679464 | 0,461671 | |
| 1223 | 24281 | (G3) | (1223 | 1 | 24281 | 3) | 1,49685 | 0,507191 | 0,257243 | |
| 1223 | 24281 | (G1) | (1223 | 2 | 24281 | 1) | 1,08412 | 0,0327342 | 0,00107153 | |
| 1223 | 24281 | (G2) | (1223 | 2 | 24281 | 1) | 1,18368 | 0,0683391 | 0,00467023 | |
| 1223 | 24281 | (G3) | (1223 | 2 | 24281 | 1) | 1,46991 | 0,156078 | 0,0243604 | |
| 1223 | 24281 | (G1) | (1223 | 2 | 24281 | 2) | 0,696923 | -0,74925 | 0,561376 | |
| 1223 | 24281 | (G2) | (1223 | 2 | 24281 | 2) | 0,37408 | -1,67731 | 2,81338 | |
| 1223 | 24281 | (G3) | (1223 | 2 | 24281 | 2) | 0,0370223 | -1,85696 | 3,44832 | |
| 1223 | 24281 | (G1) | (1223 | 2 | 24281 | 3) | 0,39224 | -2,24116 | 5,02278 | |
| 1223 | 24281 | (G2) | (1223 | 2 | 24281 | 3) | 0,835039 | -0,48239 | 0,2327 | |
| 1223 | 24281 | (G3) | (1223 | 2 | 24281 | 3) | 1,45789 | 0,968334 | 0,93767 | |
| 1223 | 24281 | (G1) | (1223 | 3 | 24281 | 1) | 1,08412 | 0,0327342 | 0,00107153 | |
| 1223 | 24281 | (G2) | (1223 | 3 | 24281 | 1) | 1,18368 | 0,0683391 | 0,00467023 | |
| 1223 | 24281 | (G3) | (1223 | 3 | 24281 | 1) | 1,46991 | 0,156078 | 0,0243604 | |
| 1223 | 24281 | (G1) | (1223 | 3 | 24281 | 2) | 0,723475 | -0,779948 | 0,608318 | |
| 1223 | 24281 | (G2) | (1223 | 3 | 24281 | 2) | 0,55073 | -1,44124 | 2,07717 | |
| 1223 | 24281 | (G3) | (1223 | 3 | 24281 | 2) | 0,319474 | -2,18399 | 4,76981 | |
| 1223 | 24281 | (G1) | (1223 | 3 | 24281 | 3) | 2,77508 | 2,8893 | 8,34807 | 2 |
| 1223 | 24281 | (G2) | (1223 | 3 | 24281 | 3) | 2,75389 | 2,81042 | 7,89844 | 2 |
| 1223 | 24281 | (G3) | (1223 | 3 | 24281 | 3) | 2,82148 | 2,86591 | 8,21345 | 2 |
| 2311 | 3597 | (G1) | (2311 | 1 | 3597 | 1) | 0,245373 | -0,720315 | 0,518854 | |
| 2311 | 3597 | (G2) | (2311 | 1 | 3597 | 1) | 1 | 0 | 0 | |
| 2311 | 3597 | (G3) | (2311 | 1 | 3597 | 1) | 0,138495 | -1,05945 | 1,12244 | |
| 2311 | 3597 | (G1) | (2311 | 1 | 3597 | 2) | 0,138495 | -1,05945 | 1,12244 | |
| 2311 | 3597 | (G2) | (2311 | 1 | 3597 | 2) | 1,90142 | 0,777535 | 0,604561 | |
| 2311 | 3597 | (G3) | (2311 | 1 | 3597 | 2) | 4,07542 | 0,720315 | 0,518854 | |
| 2311 | 3597 | (G1) | (2311 | 1 | 3597 | 3) | 10,4376 | 1,28084 | 1,64054 | |
| 2311 | 3597 | (G2) | (2311 | 1 | 3597 | 3) | 1,44555 | 0,424857 | 0,180503 | |
| 2311 | 3597 | (G3) | (2311 | 1 | 3597 | 3) | 10,4376 | 1,28084 | 1,64054 | |
| 2311 | 3597 | (G1) | (2311 | 2 | 3597 | 1) | 0,668197 | -0,62784 | 0,394183 | |
| 2311 | 3597 | (G2) | (2311 | 2 | 3597 | 1) | 6,79567 | 2,02527 | 4,10173 | |
| 2311 | 3597 | (G3) | (2311 | 2 | 3597 | 1) | 0,544496 | -1,07661 | 1,1591 | |
| 2311 | 3597 | (G1) | (2311 | 2 | 3597 | 2) | 1,39568 | 0,812023 | 0,659381 | |
| 2311 | 3597 | (G2) | (2311 | 2 | 3597 | 2) | 1,57209 | 1,15395 | 1,3316 | |
| 2311 | 3597 | (G3) | (2311 | 2 | 3597 | 2) | 1,62364 | 0,970715 | 0,942288 | |
| 2311 | 3597 | (G1) | (2311 | 2 | 3597 | 3) | 0,104089 | -3,17161 | 10,0591 | 3 |
| 2311 | 3597 | (G2) | (2311 | 2 | 3597 | 3) | 0,230497 | -2,85028 | 8,12408 | 3 |
| 2311 | 3597 | (G3) | (2311 | 2 | 3597 | 3) | 0,282926 | -3,15056 | 9,92603 | 3 |
| 2311 | 3597 | (G1) | (2311 | 3 | 3597 | 1) | 1,83656 | 1,07661 | 1,1591 | |
| 2311 | 3597 | (G2) | (2311 | 3 | 3597 | 1) | 0,433489 | -1,23221 | 1,51835 | |
| 2311 | 3597 | (G3) | (2311 | 3 | 3597 | 1) | 1 | 0 | 0 | |
| 2311 | 3597 | (G1) | (2311 | 3 | 3597 | 2) | 0,943129 | -0,171081 | 0,0292687 | |
| 2311 | 3597 | (G2) | (2311 | 3 | 3597 | 2) | 0,547407 | -1,79905 | 3,23658 | |
| 2311 | 3597 | (G3) | (2311 | 3 | 3597 | 2) | 1,52398 | 1,29112 | 1,667 | |
| 2311 | 3597 | (G1) | (2311 | 3 | 3597 | 3) | 1,75885 | 1,74593 | 3,04826 | |
| 2311 | 3597 | (G2) | (2311 | 3 | 3597 | 3) | 2,29868 | 2,26311 | 5,12169 | |
| 2311 | 3597 | (G3) | (2311 | 3 | 3597 | 3) | 1,64061 | 1,394 | 1,94324 | |
| 2620 | 4427 | (G1) | (2620 | 1 | 4427 | 1) | 1,34792 | 0,543268 | 0,29514 | |
| 2620 | 4427 | (G2) | (2620 | 1 | 4427 | 1) | 0,793562 | -0,34779 | 0,120958 | |
| 2620 | 4427 | (G3) | (2620 | 1 | 4427 | 1) | 1,01186 | 0,0186669 | 0,00034845 | |
| 2620 | 4427 | (G1) | (2620 | 1 | 4427 | 2) | 1 | 0 | 0 | |
| 2620 | 4427 | (G2) | (2620 | 1 | 4427 | 2) | 0,843094 | -0,302471 | 0,0914888 | |
| 2620 | 4427 | (G3) | (2620 | 1 | 4427 | 2) | 2,53429 | 1,84915 | 3,41937 | |
| 2620 | 4427 | (G1) | (2620 | 1 | 4427 | 3) | 4,19538 | 1,46172 | 2,13662 | |
| 2620 | 4427 | (G2) | (2620 | 1 | 4427 | 3) | 1,42918 | 0,411694 | 0,169492 | |
| 2620 | 4427 | (G3) | (2620 | 1 | 4427 | 3) | 1,51452 | 0,646305 | 0,41771 | |
| 2620 | 4427 | (G1) | (2620 | 2 | 4427 | 1) | 2,10735 | 1,78189 | 3,17513 | |
| 2620 | 4427 | (G2) | (2620 | 2 | 4427 | 1) | 0,536713 | -1,46357 | 2,14205 | |
| 2620 | 4427 | (G3) | (2620 | 2 | 4427 | 1) | 0,865877 | -0,362591 | 0,131472 | |
| 2620 | 4427 | (G1) | (2620 | 2 | 4427 | 2) | 0,941439 | -0,173679 | 0,0301644 | |
| 2620 | 4427 | (G2) | (2620 | 2 | 4427 | 2) | 1,18165 | 0,479196 | 0,229629 | |
| 2620 | 4427 | (G3) | (2620 | 2 | 4427 | 2) | 0,741625 | -0,841121 | 0,707485 | |
| 2620 | 4427 | (G1) | (2620 | 2 | 4427 | 3) | 1,46191 | 0,748081 | 0,559625 | |
| 2620 | 4427 | (G2) | (2620 | 2 | 4427 | 3) | 1,13643 | 0,241356 | 0,0582525 | |
| 2620 | 4427 | (G3) | (2620 | 2 | 4427 | 3) | 1,54733 | 0,811362 | 0,658309 | |
| 2620 | 4427 | (G1) | (2620 | 3 | 4427 | 1) | 1,2017 | 0,302401 | 0,0914462 | |
| 2620 | 4427 | (G2) | (2620 | 3 | 4427 | 1) | 0,431841 | -1,53214 | 2,34746 | |
| 2620 | 4427 | (G3) | (2620 | 3 | 4427 | 1) | 2,88387 | 1,80697 | 3,26513 | |
| 2620 | 4427 | (G1) | (2620 | 3 | 4427 | 2) | 0,253861 | -3,01956 | 9,11777 | |
| 2620 | 4427 | (G2) | (2620 | 3 | 4427 | 2) | 4,1199 | 3,12895 | 9,79035 | |
| 2620 | 4427 | (G3) | (2620 | 3 | 4427 | 2) | 0,246907 | -2,90848 | 8,45923 | |
| 2620 | 4427 | (G1) | (2620 | 3 | 4427 | 3) | 0,610457 | -0,690368 | 0,476608 | |
| 2620 | 4427 | (G2) | (2620 | 3 | 4427 | 3) | 0,503695 | -1,41003 | 1,9882 | |
| 2620 | 4427 | (G3) | (2620 | 3 | 4427 | 3) | 1,21612 | 0,321972 | 0,103666 | |
| 3170 | 22115 | (G1) | (3170 | 1 | 22115 | 1) | 0,307159 | -1,17687 | 1,38502 | |
| 3170 | 22115 | (G2) | (3170 | 1 | 22115 | 1) | 10,4416 | 1,281 | 1,64096 | |
| 3170 | 22115 | (G3) | (3170 | 1 | 22115 | 1) | 3,44867 | 1,23437 | 1,52366 | |
| 3170 | 22115 | (G1) | (3170 | 1 | 22115 | 2) | 0,667072 | -0,85064 | 0,723589 | |
| 3170 | 22115 | (G2) | (3170 | 1 | 22115 | 2) | 0,907166 | -0,2206 | 0,0486644 | |
| 3170 | 22115 | (G3) | (3170 | 1 | 22115 | 2) | 0,572556 | -1,13219 | 1,28186 | |
| 3170 | 22115 | (G1) | (3170 | 1 | 22115 | 3) | 2,65799 | 2,37555 | 5,64322 | |
| 3170 | 22115 | (G2) | (3170 | 1 | 22115 | 3) | 0,528581 | -1,84629 | 3,4088 | |
| 3170 | 22115 | (G3) | (3170 | 1 | 22115 | 3) | 0,558819 | -1,46581 | 2,1486 | |
| 3170 | 22115 | (G1) | (3170 | 2 | 22115 | 1) | 1,69781 | 0,740122 | 0,547781 | |
| 3170 | 22115 | (G2) | (3170 | 2 | 22115 | 1) | 1 | 0 | 0 | |
| 3170 | 22115 | (G3) | (3170 | 2 | 22115 | 1) | 0,141631 | -1,04742 | 1,09708 | |
| 3170 | 22115 | (G1) | (3170 | 2 | 22115 | 2) | 0,309544 | -2,91243 | 8,48227 | |
| 3170 | 22115 | (G2) | (3170 | 2 | 22115 | 2) | 3,03138 | 2,82916 | 8,00412 | |
| 3170 | 22115 | (G3) | (3170 | 2 | 22115 | 2) | 3,73802 | 3,03425 | 9,20665 | |
| 3170 | 22115 | (G1) | (3170 | 2 | 22115 | 3) | 1,31371 | 0,809192 | 0,654791 | |
| 3170 | 22115 | (G2) | (3170 | 2 | 22115 | 3) | 0,929447 | -0,191221 | 0,0365653 | |
| 3170 | 22115 | (G3) | (3170 | 2 | 22115 | 3) | 0,980431 | -0,0612115 | 0,00374684 | |
| 3170 | 22115 | (G1) | (3170 | 3 | 22115 | 1) | 7,47433 | 1,07791 | 1,16189 | |
| 3170 | 22115 | (G2) | (3170 | 3 | 22115 | 1) | 0,245343 | -0,720355 | 0,518912 | |
| 3170 | 22115 | (G3) | (3170 | 3 | 22115 | 1) | 1,02264 | 0,00907804 | 8,24E-05 | |
| 3170 | 22115 | (G1) | (3170 | 3 | 22115 | 2) | 3,45922 | 1,61655 | 2,61324 | |
| 3170 | 22115 | (G2) | (3170 | 3 | 22115 | 2) | 0,869264 | -0,264348 | 0,0698801 | |
| 3170 | 22115 | (G3) | (3170 | 3 | 22115 | 2) | 1,47943 | 0,451488 | 0,203841 | |
| 3170 | 22115 | (G1) | (3170 | 3 | 22115 | 3) | 0,605244 | -0,949726 | 0,90198 | |
| 3170 | 22115 | (G2) | (3170 | 3 | 22115 | 3) | 0,433248 | -1,23266 | 1,51945 | |
| 3170 | 22115 | (G3) | (3170 | 3 | 22115 | 3) | 0,619962 | -1,15101 | 1,32483 | |
| 4050 | 6568 | (G1) | (4050 | 1 | 6568 | 1) | 2,38742 | 2,82759 | 7,99529 | |
| 4050 | 6568 | (G2) | (4050 | 1 | 6568 | 1) | 0,404612 | -2,95624 | 8,73937 | |
| 4050 | 6568 | (G3) | (4050 | 1 | 6568 | 1) | 2,63865 | 2,86975 | 8,23549 | |
| 4050 | 6568 | (G1) | (4050 | 1 | 6568 | 2) | 0,519957 | -1,86721 | 3,48647 | |
| 4050 | 6568 | (G2) | (4050 | 1 | 6568 | 2) | 1,11303 | 0,327123 | 0,10701 | |
| 4050 | 6568 | (G3) | (4050 | 1 | 6568 | 2) | 0,853047 | -0,487888 | 0,238034 | |
| 4050 | 6568 | (G1) | (4050 | 1 | 6568 | 3) | 0,507995 | -0,975963 | 0,952505 | |
| 4050 | 6568 | (G2) | (4050 | 1 | 6568 | 3) | 2,36798 | 1,07768 | 1,16139 | |
| 4050 | 6568 | (G3) | (4050 | 1 | 6568 | 3) | 0,104247 | -2,43253 | 5,91719 | |
| 4050 | 6568 | (G1) | (4050 | 2 | 6568 | 1) | 0,432243 | -1,76073 | 3,10017 | |
| 4050 | 6568 | (G2) | (4050 | 2 | 6568 | 1) | 2,01135 | 1,52238 | 2,31764 | |
| 4050 | 6568 | (G3) | (4050 | 2 | 6568 | 1) | 0,650085 | -1,02782 | 1,05641 | |
| 4050 | 6568 | (G1) | (4050 | 2 | 6568 | 2) | 0,900389 | -0,228907 | 0,0523984 | |
| 4050 | 6568 | (G2) | (4050 | 2 | 6568 | 2) | 4,34894 | 1,96431 | 3,85851 | |
| 4050 | 6568 | (G3) | (4050 | 2 | 6568 | 2) | 0,760749 | -0,637918 | 0,406939 | |
| 4050 | 6568 | (G1) | (4050 | 2 | 6568 | 3) | 4,07542 | 0,720315 | 0,518854 | |
| 4050 | 6568 | (G2) | (4050 | 2 | 6568 | 3) | 4,07542 | 0,720315 | 0,518854 | |
| 4050 | 6568 | (G3) | (4050 | 2 | 6568 | 3) | 3,36877 | 1,21116 | 1,46691 | |
| 4050 | 6568 | (G1) | (4050 | 3 | 6568 | 1) | 2,5611 | 0,907111 | 0,822851 | |
| 4050 | 6568 | (G2) | (4050 | 3 | 6568 | 1) | 0,564425 | -0,521337 | 0,271792 | |
| 4050 | 6568 | (G3) | (4050 | 3 | 6568 | 1) | 1,77171 | 0,521337 | 0,271792 | |
| 4050 | 6568 | (G1) | (4050 | 3 | 6568 | 2) | 4,07542 | 0,720315 | 0,518854 | |
| 4050 | 6568 | (G2) | (4050 | 3 | 6568 | 2) | 4,07542 | 0,720315 | 0,518854 | |
| 4050 | 6568 | (G3) | (4050 | 3 | 6568 | 2) | 0,245373 | -0,720315 | 0,518854 | |
| 4050 | 6568 | (G1) | (4050 | 3 | 6568 | 3) | 1 | 0 | 0 | |
| 4050 | 6568 | (G2) | (4050 | 3 | 6568 | 3) | 1 | 0 | 0 | |
| 4050 | 6568 | (G3) | (4050 | 3 | 6568 | 3) | 1 | 0 | 0 | |
| 4283 | 23188 | (G1) | (4283 | 1 | 23188 | 1) | 0,437215 | -1,37506 | 1,89078 | |
| 4283 | 23188 | (G2) | (4283 | 1 | 23188 | 1) | 0,433388 | -1,85264 | 3,43227 | |
| 4283 | 23188 | (G3) | (4283 | 1 | 23188 | 1) | 0,399248 | -2,05015 | 4,20311 | |
| 4283 | 23188 | (G1) | (4283 | 1 | 23188 | 2) | 0,686358 | -1,2227 | 1,495 | |
| 4283 | 23188 | (G2) | (4283 | 1 | 23188 | 2) | 0,926643 | -0,248317 | 0,0616613 | |
| 4283 | 23188 | (G3) | (4283 | 1 | 23188 | 2) | 0,866467 | -0,463434 | 0,214771 | |
| 4283 | 23188 | (G1) | (4283 | 1 | 23188 | 3) | 2,53391 | 2,82128 | 7,95962 | 4 |
| 4283 | 23188 | (G2) | (4283 | 1 | 23188 | 3) | 2,88255 | 3,08694 | 9,52918 | 4 |
| 4283 | 23188 | (G3) | (4283 | 1 | 23188 | 3) | 2,70188 | 2,89695 | 8,39234 | 4 |
| 4283 | 23188 | (G1) | (4283 | 2 | 23188 | 1) | 1,24537 | 0,330111 | 0,108973 | |
| 4283 | 23188 | (G2) | (4283 | 2 | 23188 | 1) | 0,6997 | -0,411694 | 0,169492 | |
| 4283 | 23188 | (G3) | (4283 | 2 | 23188 | 1) | 1,77171 | 0,521337 | 0,271792 | |
| 4283 | 23188 | (G1) | (4283 | 2 | 23188 | 2) | 1,30415 | 0,513054 | 0,263224 | |
| 4283 | 23188 | (G2) | (4283 | 2 | 23188 | 2) | 0,501533 | -1,12712 | 1,27039 | |
| 4283 | 23188 | (G3) | (4283 | 2 | 23188 | 2) | 0,296523 | -1,87352 | 3,51007 | |
| 4283 | 23188 | (G1) | (4283 | 2 | 23188 | 3) | 0,229941 | -1,96431 | 3,85851 | |
| 4283 | 23188 | (G2) | (4283 | 2 | 23188 | 3) | 0,863574 | -0,259937 | 0,067567 | |
| 4283 | 23188 | (G3) | (4283 | 2 | 23188 | 3) | 1 | 0 | 0 | |
| 4283 | 23188 | (G1) | (4283 | 3 | 23188 | 1) | 7,22047 | 1,05945 | 1,12244 | |
| 4283 | 23188 | (G2) | (4283 | 3 | 23188 | 1) | 0,24812 | -0,714596 | 0,510647 | |
| 4283 | 23188 | (G3) | (4283 | 3 | 23188 | 1) | 1 | 0 | 0 | |
| 4283 | 23188 | (G1) | (4283 | 3 | 23188 | 2) | 0,138495 | -1,05945 | 1,12244 | |
| 4283 | 23188 | (G2) | (4283 | 3 | 23188 | 2) | 1,01119 | 0,00451366 | 2,04E-05 | |
| 4283 | 23188 | (G3) | (4283 | 3 | 23188 | 2) | 1 | 0 | 0 | |
| 4283 | 23188 | (G1) | (4283 | 3 | 23188 | 3) | 0,245373 | -0,720315 | 0,518854 | |
| 4283 | 23188 | (G2) | (4283 | 3 | 23188 | 3) | 0,140045 | -1,05347 | 1,1098 | |
| 4283 | 23188 | (G3) | (4283 | 3 | 23188 | 3) | 4,07542 | 0,720315 | 0,518854 | |
| 4427 | 6068 | (G1) | (4427 | 1 | 6068 | 1) | 1,2335 | 0,559968 | 0,313564 | |
| 4427 | 6068 | (G2) | (4427 | 1 | 6068 | 1) | 0,714089 | -0,913146 | 0,833836 | |
| 4427 | 6068 | (G3) | (4427 | 1 | 6068 | 1) | 1,48749 | 1,08434 | 1,17579 | |
| 4427 | 6068 | (G1) | (4427 | 1 | 6068 | 2) | 3,00556 | 2,07148 | 4,29104 | |
| 4427 | 6068 | (G2) | (4427 | 1 | 6068 | 2) | 0,24224 | -2,22619 | 4,95594 | |
| 4427 | 6068 | (G3) | (4427 | 1 | 6068 | 2) | 1 | 0 | 0 | |
| 4427 | 6068 | (G1) | (4427 | 1 | 6068 | 3) | 1 | 0 | 0 | |
| 4427 | 6068 | (G2) | (4427 | 1 | 6068 | 3) | 1 | 0 | 0 | |
| 4427 | 6068 | (G3) | (4427 | 1 | 6068 | 3) | 1 | 0 | 0 | |
| 4427 | 6068 | (G1) | (4427 | 2 | 6068 | 1) | 0,41292 | -2,84698 | 8,1053 | |
| 4427 | 6068 | (G2) | (4427 | 2 | 6068 | 1) | 2,72323 | 3,08036 | 9,48862 | |
| 4427 | 6068 | (G3) | (4427 | 2 | 6068 | 1) | 0,387145 | -2,84456 | 8,09152 | |
| 4427 | 6068 | (G1) | (4427 | 2 | 6068 | 2) | 1,22409 | 0,448633 | 0,201272 | |
| 4427 | 6068 | (G2) | (4427 | 2 | 6068 | 2) | 0,913172 | -0,213015 | 0,0453756 | |
| 4427 | 6068 | (G3) | (4427 | 2 | 6068 | 2) | 1,57209 | 1,15395 | 1,3316 | |
| 4427 | 6068 | (G1) | (4427 | 2 | 6068 | 3) | 1 | 0 | 0 | |
| 4427 | 6068 | (G2) | (4427 | 2 | 6068 | 3) | 1 | 0 | 0 | |
| 4427 | 6068 | (G3) | (4427 | 2 | 6068 | 3) | 1 | 0 | 0 | |
| 4427 | 6068 | (G1) | (4427 | 3 | 6068 | 1) | 1,63652 | 1,09497 | 1,19897 | |
| 4427 | 6068 | (G2) | (4427 | 3 | 6068 | 1) | 0,784621 | -0,601442 | 0,361733 | |
| 4427 | 6068 | (G3) | (4427 | 3 | 6068 | 1) | 0,918187 | -0,206507 | 0,0426453 | |
| 4427 | 6068 | (G1) | (4427 | 3 | 6068 | 2) | 1 | 0 | 0 | |
| 4427 | 6068 | (G2) | (4427 | 3 | 6068 | 2) | 0,710105 | -0,580522 | 0,337005 | |
| 4427 | 6068 | (G3) | (4427 | 3 | 6068 | 2) | 6,79567 | 2,02527 | 4,10173 | |
| 4427 | 6068 | (G1) | (4427 | 3 | 6068 | 3) | 1 | 0 | 0 | |
| 4427 | 6068 | (G2) | (4427 | 3 | 6068 | 3) | 1 | 0 | 0 | |
| 4427 | 6068 | (G3) | (4427 | 3 | 6068 | 3) | 1 | 0 | 0 | |
| 4427 | 12846 | (G1) | (4427 | 1 | 12846 | 1) | 1,42044 | 0,93165 | 0,867973 | |
| 4427 | 12846 | (G2) | (4427 | 1 | 12846 | 1) | 0,511822 | -1,79608 | 3,22592 | |
| 4427 | 12846 | (G3) | (4427 | 1 | 12846 | 1) | 1,42044 | 0,93165 | 0,867973 | |
| 4427 | 12846 | (G1) | (4427 | 1 | 12846 | 2) | 2,29647 | 1,63744 | 2,68121 | |
| 4427 | 12846 | (G2) | (4427 | 1 | 12846 | 2) | 0,544496 | -1,07661 | 1,1591 | |
| 4427 | 12846 | (G3) | (4427 | 1 | 12846 | 2) | 1,11063 | 0,228907 | 0,0523984 | |
| 4427 | 12846 | (G1) | (4427 | 1 | 12846 | 3) | 1 | 0 | 0 | |
| 4427 | 12846 | (G2) | (4427 | 1 | 12846 | 3) | 1 | 0 | 0 | |
| 4427 | 12846 | (G3) | (4427 | 1 | 12846 | 3) | 1 | 0 | 0 | |
| 4427 | 12846 | (G1) | (4427 | 2 | 12846 | 1) | 0,383944 | -2,9406 | 8,64715 | |
| 4427 | 12846 | (G2) | (4427 | 2 | 12846 | 1) | 2,70289 | 2,98512 | 8,91091 | |
| 4427 | 12846 | (G3) | (4427 | 2 | 12846 | 1) | 0,369974 | -2,98512 | 8,91091 | |
| 4427 | 12846 | (G1) | (4427 | 2 | 12846 | 2) | 1,15351 | 0,377559 | 0,142551 | |
| 4427 | 12846 | (G2) | (4427 | 2 | 12846 | 2) | 1 | 0 | 0 | |
| 4427 | 12846 | (G3) | (4427 | 2 | 12846 | 2) | 1,72157 | 1,3597 | 1,84878 | |
| 4427 | 12846 | (G1) | (4427 | 2 | 12846 | 3) | 1 | 0 | 0 | |
| 4427 | 12846 | (G2) | (4427 | 2 | 12846 | 3) | 1 | 0 | 0 | |
| 4427 | 12846 | (G3) | (4427 | 2 | 12846 | 3) | 1 | 0 | 0 | |
| 4427 | 12846 | (G1) | (4427 | 3 | 12846 | 1) | 1,45992 | 0,863219 | 0,745146 | |
| 4427 | 12846 | (G2) | (4427 | 3 | 12846 | 1) | 0,926301 | -0,195593 | 0,0382568 | |
| 4427 | 12846 | (G3) | (4427 | 3 | 12846 | 1) | 1,83778 | 1,43203 | 2,0507 | |
| 4427 | 12846 | (G1) | (4427 | 3 | 12846 | 2) | 1,31536 | 0,368182 | 0,135558 | |
| 4427 | 12846 | (G2) | (4427 | 3 | 12846 | 2) | 0,433489 | -1,23221 | 1,51835 | |
| 4427 | 12846 | (G3) | (4427 | 3 | 12846 | 2) | 0,802973 | -0,330111 | 0,108973 | |
| 4427 | 12846 | (G1) | (4427 | 3 | 12846 | 3) | 1 | 0 | 0 | |
| 4427 | 12846 | (G2) | (4427 | 3 | 12846 | 3) | 1 | 0 | 0 | |
| 4427 | 12846 | (G3) | (4427 | 3 | 12846 | 3) | 1 | 0 | 0 | |
| 5149 | 17806 | (G1) | (5149 | 1 | 17806 | 1) | 0,564425 | -0,521337 | 0,271792 | |
| 5149 | 17806 | (G2) | (5149 | 1 | 17806 | 1) | 1,01119 | 0,00451366 | 2,04E-05 | |
| 5149 | 17806 | (G3) | (5149 | 1 | 17806 | 1) | 1 | 0 | 0 | |
| 5149 | 17806 | (G1) | (5149 | 1 | 17806 | 2) | 2,5611 | 0,907111 | 0,822851 | |
| 5149 | 17806 | (G2) | (5149 | 1 | 17806 | 2) | 0,140045 | -1,05347 | 1,1098 | |
| 5149 | 17806 | (G3) | (5149 | 1 | 17806 | 2) | 0,390457 | -0,907111 | 0,822851 | |
| 5149 | 17806 | (G1) | (5149 | 1 | 17806 | 3) | 0,138495 | -1,05945 | 1,12244 | |
| 5149 | 17806 | (G2) | (5149 | 1 | 17806 | 3) | 0,300205 | -1,19986 | 1,43966 | |
| 5149 | 17806 | (G3) | (5149 | 1 | 17806 | 3) | 7,22047 | 1,05945 | 1,12244 | |
| 5149 | 17806 | (G1) | (5149 | 2 | 17806 | 1) | 0,525922 | -0,777535 | 0,604561 | |
| 5149 | 17806 | (G2) | (5149 | 2 | 17806 | 1) | 1,65754 | 0,706762 | 0,499512 | |
| 5149 | 17806 | (G3) | (5149 | 2 | 17806 | 1) | 0,760247 | -0,368182 | 0,135558 | |
| 5149 | 17806 | (G1) | (5149 | 2 | 17806 | 2) | 0,680202 | -0,974651 | 0,949945 | |
| 5149 | 17806 | (G2) | (5149 | 2 | 17806 | 2) | 0,761059 | -0,718444 | 0,516162 | |
| 5149 | 17806 | (G3) | (5149 | 2 | 17806 | 2) | 0,621142 | -1,1822 | 1,39761 | |
| 5149 | 17806 | (G1) | (5149 | 2 | 17806 | 3) | 1 | 0 | 0 | |
| 5149 | 17806 | (G2) | (5149 | 2 | 17806 | 3) | 0,550993 | -1,40194 | 1,96544 | |
| 5149 | 17806 | (G3) | (5149 | 2 | 17806 | 3) | 1,39568 | 0,812023 | 0,659381 | |
| 5149 | 17806 | (G1) | (5149 | 3 | 17806 | 1) | 0,61736 | -0,837641 | 0,701642 | |
| 5149 | 17806 | (G2) | (5149 | 3 | 17806 | 1) | 0,590717 | -0,996202 | 0,992418 | |
| 5149 | 17806 | (G3) | (5149 | 3 | 17806 | 1) | 0,832154 | -0,302401 | 0,0914462 | |
| 5149 | 17806 | (G1) | (5149 | 3 | 17806 | 2) | 0,634039 | -1,2522 | 1,568 | |
| 5149 | 17806 | (G2) | (5149 | 3 | 17806 | 2) | 0,958898 | -0,124241 | 0,0154358 | |
| 5149 | 17806 | (G3) | (5149 | 3 | 17806 | 2) | 2,77207 | 3,1015 | 9,61933 | |
| 5149 | 17806 | (G1) | (5149 | 3 | 17806 | 3) | 3,0634 | 3,0277 | 9,16695 | |
| 5149 | 17806 | (G2) | (5149 | 3 | 17806 | 3) | 3,40397 | 3,14671 | 9,90178 | |
| 5149 | 17806 | (G3) | (5149 | 3 | 17806 | 3) | 0,295875 | -2,9173 | 8,51061 | |
| 5302 | 30013 | (G1) | (5302 | 1 | 30013 | 1) | 1,20234 | 0,580687 | 0,337197 | |
| 5302 | 30013 | (G2) | (5302 | 1 | 30013 | 1) | 0,983695 | -0,0517107 | 0,00267399 | |
| 5302 | 30013 | (G3) | (5302 | 1 | 30013 | 1) | 1 | 0 | 0 | |
| 5302 | 30013 | (G1) | (5302 | 1 | 30013 | 2) | 0,20261 | -2,86188 | 8,19033 | |
| 5302 | 30013 | (G2) | (5302 | 1 | 30013 | 2) | 3,67055 | 2,85126 | 8,12969 | |
| 5302 | 30013 | (G3) | (5302 | 1 | 30013 | 2) | 4,33845 | 2,85028 | 8,12408 | |
| 5302 | 30013 | (G1) | (5302 | 1 | 30013 | 3) | 1,0229 | 0,0183276 | 0,0003359 | |
| 5302 | 30013 | (G2) | (5302 | 1 | 30013 | 3) | 0,988806 | -0,00911231 | 8,30E-05 | |
| 5302 | 30013 | (G3) | (5302 | 1 | 30013 | 3) | 7,22047 | 1,05945 | 1,12244 | |
| 5302 | 30013 | (G1) | (5302 | 2 | 30013 | 1) | 0,929403 | -0,225546 | 0,0508712 | |
| 5302 | 30013 | (G2) | (5302 | 2 | 30013 | 1) | 0,758446 | -0,854832 | 0,730738 | |
| 5302 | 30013 | (G3) | (5302 | 2 | 30013 | 1) | 0,689235 | -1,13638 | 1,29136 | |
| 5302 | 30013 | (G1) | (5302 | 2 | 30013 | 2) | 1,66563 | 1,02119 | 1,04283 | |
| 5302 | 30013 | (G2) | (5302 | 2 | 30013 | 2) | 0,675522 | -0,772523 | 0,596791 | |
| 5302 | 30013 | (G3) | (5302 | 2 | 30013 | 2) | 0,580865 | -1,3597 | 1,84878 | |
| 5302 | 30013 | (G1) | (5302 | 2 | 30013 | 3) | 4,16875 | 0,731899 | 0,535677 | |
| 5302 | 30013 | (G2) | (5302 | 2 | 30013 | 3) | 0,242627 | -0,726074 | 0,527184 | |
| 5302 | 30013 | (G3) | (5302 | 2 | 30013 | 3) | 0,138495 | -1,05945 | 1,12244 | |
| 5302 | 30013 | (G1) | (5302 | 3 | 30013 | 1) | 2,15202 | 1,4915 | 2,22458 | |
| 5302 | 30013 | (G2) | (5302 | 3 | 30013 | 1) | 0,822286 | -0,321972 | 0,103666 | |
| 5302 | 30013 | (G3) | (5302 | 3 | 30013 | 1) | 0,832154 | -0,302401 | 0,0914462 | |
| 5302 | 30013 | (G1) | (5302 | 3 | 30013 | 2) | 1,02317 | 0,0243966 | 0,0005952 | |
| 5302 | 30013 | (G2) | (5302 | 3 | 30013 | 2) | 0,235564 | -1,47363 | 2,17158 | |
| 5302 | 30013 | (G3) | (5302 | 3 | 30013 | 2) | 1,44555 | 0,424857 | 0,180503 | |
| 5302 | 30013 | (G1) | (5302 | 3 | 30013 | 3) | 1,02264 | 0,00907804 | 8,24E-05 | |
| 5302 | 30013 | (G2) | (5302 | 3 | 30013 | 3) | 0,988929 | -0,00451366 | 2,04E-05 | |
| 5302 | 30013 | (G3) | (5302 | 3 | 30013 | 3) | 1 | 0 | 0 | |
| 5375 | 9353 | (G1) | (5375 | 1 | 9353 | 1) | 1,06148 | 0,0241873 | 0,00058503 | |
| 5375 | 9353 | (G2) | (5375 | 1 | 9353 | 1) | 1,06727 | 0,0263878 | 0,00069632 | |
| 5375 | 9353 | (G3) | (5375 | 1 | 9353 | 1) | 1,19092 | 0,0708269 | 0,00501645 | |
| 5375 | 9353 | (G1) | (5375 | 1 | 9353 | 2) | 1,06148 | 0,0241873 | 0,00058503 | |
| 5375 | 9353 | (G2) | (5375 | 1 | 9353 | 2) | 1,06727 | 0,0263878 | 0,00069632 | |
| 5375 | 9353 | (G3) | (5375 | 1 | 9353 | 2) | 1,19092 | 0,0708269 | 0,00501645 | |
| 5375 | 9353 | (G1) | (5375 | 1 | 9353 | 3) | 1,06148 | 0,0241873 | 0,00058503 | |
| 5375 | 9353 | (G2) | (5375 | 1 | 9353 | 3) | 1,06727 | 0,0263878 | 0,00069632 | |
| 5375 | 9353 | (G3) | (5375 | 1 | 9353 | 3) | 1,19092 | 0,0708269 | 0,00501645 | |
| 5375 | 9353 | (G1) | (5375 | 2 | 9353 | 1) | 1,06148 | 0,0241873 | 0,00058503 | |
| 5375 | 9353 | (G2) | (5375 | 2 | 9353 | 1) | 1,06727 | 0,0263878 | 0,00069632 | |
| 5375 | 9353 | (G3) | (5375 | 2 | 9353 | 1) | 1,19092 | 0,0708269 | 0,00501645 | |
| 5375 | 9353 | (G1) | (5375 | 2 | 9353 | 2) | 0,146855 | -1,02778 | 1,05634 | |
| 5375 | 9353 | (G2) | (5375 | 2 | 9353 | 2) | 1,8972 | 0,582729 | 0,339573 | |
| 5375 | 9353 | (G3) | (5375 | 2 | 9353 | 2) | 0,673409 | -0,359961 | 0,129572 | |
| 5375 | 9353 | (G1) | (5375 | 2 | 9353 | 3) | 0,516043 | -1,50537 | 2,26614 | |
| 5375 | 9353 | (G2) | (5375 | 2 | 9353 | 3) | 0,298146 | -2,29082 | 5,24787 | |
| 5375 | 9353 | (G3) | (5375 | 2 | 9353 | 3) | 0,242292 | -2,24724 | 5,05007 | |
| 5375 | 9353 | (G1) | (5375 | 3 | 9353 | 1) | 1,06148 | 0,0241873 | 0,00058503 | |
| 5375 | 9353 | (G2) | (5375 | 3 | 9353 | 1) | 1,06727 | 0,0263878 | 0,00069632 | |
| 5375 | 9353 | (G3) | (5375 | 3 | 9353 | 1) | 1,19092 | 0,0708269 | 0,00501645 | |
| 5375 | 9353 | (G1) | (5375 | 3 | 9353 | 2) | 0,282653 | -1,96232 | 3,85072 | |
| 5375 | 9353 | (G2) | (5375 | 3 | 9353 | 2) | 0,136719 | -2,11288 | 4,46425 | |
| 5375 | 9353 | (G3) | (5375 | 3 | 9353 | 2) | 0,357979 | -1,33506 | 1,78238 | |
| 5375 | 9353 | (G1) | (5375 | 3 | 9353 | 3) | 3,02755 | 2,89664 | 8,39054 | 5 |
| 5375 | 9353 | (G2) | (5375 | 3 | 9353 | 3) | 3,93137 | 3,12546 | 9,76848 | 5 |
| 5375 | 9353 | (G3) | (5375 | 3 | 9353 | 3) | 3,8575 | 2,80744 | 7,88171 | 5 |
| 5375 | 15323 | (G1) | (5375 | 1 | 15323 | 1) | 1,06148 | -0,0241873 | 0,00058503 | |
| 5375 | 15323 | (G2) | (5375 | 1 | 15323 | 1) | 1,06727 | 0,0263878 | 0,00069632 | |
| 5375 | 15323 | (G3) | (5375 | 1 | 15323 | 1) | 1,19092 | 0,0708269 | 0,00501645 | |
| 5375 | 15323 | (G1) | (5375 | 1 | 15323 | 2) | 1,06148 | 0,0241873 | 0,00058503 | |
| 5375 | 15323 | (G2) | (5375 | 1 | 15323 | 2) | 1,06727 | 0,0263878 | 0,00069632 | |
| 5375 | 15323 | (G3) | (5375 | 1 | 15323 | 2) | 1,19092 | 0,0708269 | 0,00501645 | |
| 5375 | 15323 | (G1) | (5375 | 1 | 15323 | 3) | 1,06148 | 0,0241873 | 0,00058503 | |
| 5375 | 15323 | (G2) | (5375 | 1 | 15323 | 3) | 1,06727 | 0,0263878 | 0,00069632 | |
| 5375 | 15323 | (G3) | (5375 | 1 | 15323 | 3) | 1,19092 | 0,0708269 | 0,00501645 | |
| 5375 | 15323 | (G1) | (5375 | 2 | 15323 | 1) | 0,367611 | -2,13809 | 4,57142 | |
| 5375 | 15323 | (G2) | (5375 | 2 | 15323 | 1) | 0,388744 | -1,87699 | 3,52309 | |
| 5375 | 15323 | (G3) | (5375 | 2 | 15323 | 1) | 0,381568 | -1,65294 | 2,73222 | |
| 5375 | 15323 | (G1) | (5375 | 2 | 15323 | 2) | 1,06224 | 0,0488498 | 0,0023863 | |
| 5375 | 15323 | (G2) | (5375 | 2 | 15323 | 2) | 0,601952 | -0,461886 | 0,213338 | |
| 5375 | 15323 | (G3) | (5375 | 2 | 15323 | 2) | 0,0866056 | -1,34938 | 1,82082 | |
| 5375 | 15323 | (G1) | (5375 | 2 | 15323 | 3) | 4,33133 | 0,751364 | 0,564548 | |
| 5375 | 15323 | (G2) | (5375 | 2 | 15323 | 3) | 1,06727 | 0,0263878 | 0,00069632 | |
| 5375 | 15323 | (G3) | (5375 | 2 | 15323 | 3) | 1,19092 | 0,0708269 | 0,00501645 | |
| 5375 | 15323 | (G1) | (5375 | 3 | 15323 | 1) | 2,6795 | 2,83768 | 8,05243 | 6 |
| 5375 | 15323 | (G2) | (5375 | 3 | 15323 | 1) | 3,1081 | 2,92422 | 8,55106 | 6 |
| 5375 | 15323 | (G3) | (5375 | 3 | 15323 | 1) | 4,22987 | 3,48616 | 12,1533 | 6 |
| 5375 | 15323 | (G1) | (5375 | 3 | 15323 | 2) | 0,489235 | -1,56327 | 2,4438 | |
| 5375 | 15323 | (G2) | (5375 | 3 | 15323 | 2) | 0,463647 | -1,39192 | 1,93743 | |
| 5375 | 15323 | (G3) | (5375 | 3 | 15323 | 2) | 0,340723 | -2,04386 | 4,17735 | |
| 5375 | 15323 | (G1) | (5375 | 3 | 15323 | 3) | 0,260323 | -0,689834 | 0,475871 | |
| 5375 | 15323 | (G2) | (5375 | 3 | 15323 | 3) | 0,147347 | -1,02566 | 1,05198 | |
| 5375 | 15323 | (G3) | (5375 | 3 | 15323 | 3) | 0,292107 | -0,630688 | 0,397767 | |
| 6568 | 22216 | (G1) | (6568 | 1 | 22216 | 1) | 2,89031 | 3,1493 | 9,91808 | |
| 6568 | 22216 | (G2) | (6568 | 1 | 22216 | 1) | 0,345984 | -3,1493 | 9,91808 | |
| 6568 | 22216 | (G3) | (6568 | 1 | 22216 | 1) | 3,04887 | 3,14415 | 9,88565 | |
| 6568 | 22216 | (G1) | (6568 | 1 | 22216 | 2) | 0,745546 | -0,854118 | 0,729518 | |
| 6568 | 22216 | (G2) | (6568 | 1 | 22216 | 2) | 1,13053 | 0,350059 | 0,122541 | |
| 6568 | 22216 | (G3) | (6568 | 1 | 22216 | 2) | 0,658084 | -1,09332 | 1,19535 | |
| 6568 | 22216 | (G1) | (6568 | 1 | 22216 | 3) | 0,138495 | -1,05945 | 1,12244 | |
| 6568 | 22216 | (G2) | (6568 | 1 | 22216 | 3) | 2,30686 | 1,23221 | 1,51835 | |
| 6568 | 22216 | (G3) | (6568 | 1 | 22216 | 3) | 4,12156 | 0,726074 | 0,527184 | |
| 6568 | 22216 | (G1) | (6568 | 2 | 22216 | 1) | 0,469948 | -1,98907 | 3,95638 | |
| 6568 | 22216 | (G2) | (6568 | 2 | 22216 | 1) | 1,28954 | 0,7113 | 0,505948 | |
| 6568 | 22216 | (G3) | (6568 | 2 | 22216 | 1) | 1,64058 | 1,41645 | 2,00633 | |
| 6568 | 22216 | (G1) | (6568 | 2 | 22216 | 2) | 1,18057 | 0,406867 | 0,165541 | |
| 6568 | 22216 | (G2) | (6568 | 2 | 22216 | 2) | 1,85551 | 1,33292 | 1,77667 | |
| 6568 | 22216 | (G3) | (6568 | 2 | 22216 | 2) | 0,377515 | -2,29014 | 5,24472 | |
| 6568 | 22216 | (G1) | (6568 | 2 | 22216 | 3) | 0,138495 | -1,05945 | 1,12244 | |
| 6568 | 22216 | (G2) | (6568 | 2 | 22216 | 3) | 1 | 0 | 0 | |
| 6568 | 22216 | (G3) | (6568 | 2 | 22216 | 3) | 0,073653 | -1,43936 | 2,07175 | |
| 6568 | 22216 | (G1) | (6568 | 3 | 22216 | 1) | 0,525922 | -0,777535 | 0,604561 | |
| 6568 | 22216 | (G2) | (6568 | 3 | 22216 | 1) | 2,5611 | 0,907111 | 0,822851 | |
| 6568 | 22216 | (G3) | (6568 | 3 | 22216 | 1) | 0,513879 | -0,959236 | 0,920133 | |
| 6568 | 22216 | (G1) | (6568 | 3 | 22216 | 2) | 1 | 0 | 0 | |
| 6568 | 22216 | (G2) | (6568 | 3 | 22216 | 2) | 7,22047 | 1,05945 | 1,12244 | |
| 6568 | 22216 | (G3) | (6568 | 3 | 22216 | 2) | 0,427137 | -1,06334 | 1,13069 | |
| 6568 | 22216 | (G1) | (6568 | 3 | 22216 | 3) | 1 | 0 | 0 | |
| 6568 | 22216 | (G2) | (6568 | 3 | 22216 | 3) | 1 | 0 | 0 | |
| 6568 | 22216 | (G3) | (6568 | 3 | 22216 | 3) | 0,24812 | -0,714596 | 0,510647 | |
| 6569 | 22216 | (G1) | (6569 | 1 | 22216 | 1) | 0,531944 | -0,763686 | 0,583217 | |
| 6569 | 22216 | (G2) | (6569 | 1 | 22216 | 1) | 2,5611 | 0,907111 | 0,822851 | |
| 6569 | 22216 | (G3) | (6569 | 1 | 22216 | 1) | 0,513879 | -0,959236 | 0,920133 | |
| 6569 | 22216 | (G1) | (6569 | 1 | 22216 | 2) | 0,24812 | -0,714596 | 0,510647 | |
| 6569 | 22216 | (G2) | (6569 | 1 | 22216 | 2) | 7,22047 | 1,05945 | 1,12244 | |
| 6569 | 22216 | (G3) | (6569 | 1 | 22216 | 2) | 0,427137 | -1,06334 | 1,13069 | |
| 6569 | 22216 | (G1) | (6569 | 1 | 22216 | 3) | 1,01132 | 0,00911231 | 8,30E-05 | |
| 6569 | 22216 | (G2) | (6569 | 1 | 22216 | 3) | 1 | 0 | 0 | |
| 6569 | 22216 | (G3) | (6569 | 1 | 22216 | 3) | 0,140045 | -1,05347 | 1,1098 | |
| 6569 | 22216 | (G1) | (6569 | 2 | 22216 | 1) | 0,476105 | -1,95363 | 3,81668 | |
| 6569 | 22216 | (G2) | (6569 | 2 | 22216 | 1) | 1,28954 | 0,7113 | 0,505948 | |
| 6569 | 22216 | (G3) | (6569 | 2 | 22216 | 1) | 1,75924 | 1,59702 | 2,55047 | |
| 6569 | 22216 | (G1) | (6569 | 2 | 22216 | 2) | 1,18619 | 0,429884 | 0,1848 | |
| 6569 | 22216 | (G2) | (6569 | 2 | 22216 | 2) | 1,85551 | 1,33292 | 1,77667 | |
| 6569 | 22216 | (G3) | (6569 | 2 | 22216 | 2) | 0,377515 | -2,29014 | 5,24472 | |
| 6569 | 22216 | (G1) | (6569 | 2 | 22216 | 3) | 0,140045 | -1,05347 | 1,1098 | |
| 6569 | 22216 | (G2) | (6569 | 2 | 22216 | 3) | 1 | 0 | 0 | |
| 6569 | 22216 | (G3) | (6569 | 2 | 22216 | 3) | 0,0968804 | -1,27473 | 1,62495 | |
| 6569 | 22216 | (G1) | (6569 | 3 | 22216 | 1) | 2,8149 | 3,06145 | 9,3725 | |
| 6569 | 22216 | (G2) | (6569 | 3 | 22216 | 1) | 0,345984 | -3,1493 | 9,91808 | |
| 6569 | 22216 | (G3) | (6569 | 3 | 22216 | 1) | 2,82417 | 2,97371 | 8,84293 | |
| 6569 | 22216 | (G1) | (6569 | 3 | 22216 | 2) | 0,799437 | -0,64676 | 0,418299 | |
| 6569 | 22216 | (G2) | (6569 | 3 | 22216 | 2) | 1,13053 | 0,350059 | 0,122541 | |
| 6569 | 22216 | (G3) | (6569 | 3 | 22216 | 2) | 0,658084 | -1,09332 | 1,19535 | |
| 6569 | 22216 | (G1) | (6569 | 3 | 22216 | 3) | 0,140045 | -1,05347 | 1,1098 | |
| 6569 | 22216 | (G2) | (6569 | 3 | 22216 | 3) | 2,30686 | 1,23221 | 1,51835 | |
| 6569 | 22216 | (G3) | (6569 | 3 | 22216 | 3) | 4,12156 | 0,726074 | 0,527184 | |
| 6570 | 22216 | (G1) | (6570 | 1 | 22216 | 1) | 2,89031 | 3,1493 | 9,91808 | |
| 6570 | 22216 | (G2) | (6570 | 1 | 22216 | 1) | 0,345984 | -3,1493 | 9,91808 | |
| 6570 | 22216 | (G3) | (6570 | 1 | 22216 | 1) | 2,78618 | 2,9327 | 8,60075 | |
| 6570 | 22216 | (G1) | (6570 | 1 | 22216 | 2) | 0,745546 | -0,854118 | 0,729518 | |
| 6570 | 22216 | (G2) | (6570 | 1 | 22216 | 2) | 1,13053 | 0,350059 | 0,122541 | |
| 6570 | 22216 | (G3) | (6570 | 1 | 22216 | 2) | 0,648727 | -1,12995 | 1,27678 | |
| 6570 | 22216 | (G1) | (6570 | 1 | 22216 | 3) | 0,138495 | -1,05945 | 1,12244 | |
| 6570 | 22216 | (G2) | (6570 | 1 | 22216 | 3) | 2,30686 | 1,23221 | 1,51835 | |
| 6570 | 22216 | (G3) | (6570 | 1 | 22216 | 3) | 4,07593 | 0,720355 | 0,518912 | |
| 6570 | 22216 | (G1) | (6570 | 2 | 22216 | 1) | 0,469948 | -1,98907 | 3,95638 | |
| 6570 | 22216 | (G2) | (6570 | 2 | 22216 | 1) | 1,28954 | 0,7113 | 0,505948 | |
| 6570 | 22216 | (G3) | (6570 | 2 | 22216 | 1) | 1,86697 | 1,74064 | 3,02984 | |
| 6570 | 22216 | (G1) | (6570 | 2 | 22216 | 2) | 1,2745 | 0,601442 | 0,361733 | |
| 6570 | 22216 | (G2) | (6570 | 2 | 22216 | 2) | 1,85551 | 1,33292 | 1,77667 | |
| 6570 | 22216 | (G3) | (6570 | 2 | 22216 | 2) | 0,37207 | -2,32294 | 5,39604 | |
| 6570 | 22216 | (G1) | (6570 | 2 | 22216 | 3) | 0,138495 | -1,05945 | 1,12244 | |
| 6570 | 22216 | (G2) | (6570 | 2 | 22216 | 3) | 1 | 0 | 0 | |
| 6570 | 22216 | (G3) | (6570 | 2 | 22216 | 3) | 0,0727998 | -1,44576 | 2,09022 | |
| 6570 | 22216 | (G1) | (6570 | 3 | 22216 | 1) | 0,525922 | -0,777535 | 0,604561 | |
| 6570 | 22216 | (G2) | (6570 | 3 | 22216 | 1) | 2,5611 | 0,907111 | 0,822851 | |
| 6570 | 22216 | (G3) | (6570 | 3 | 22216 | 1) | 0,507785 | -0,976279 | 0,95312 | |
| 6570 | 22216 | (G1) | (6570 | 3 | 22216 | 2) | 0,245373 | -0,720315 | 0,518854 | |
| 6570 | 22216 | (G2) | (6570 | 3 | 22216 | 2) | 7,22047 | 1,05945 | 1,12244 | |
| 6570 | 22216 | (G3) | (6570 | 3 | 22216 | 2) | 0,422131 | -1,07796 | 1,16199 | |
| 6570 | 22216 | (G1) | (6570 | 3 | 22216 | 3) | 1 | 0 | 0 | |
| 6570 | 22216 | (G2) | (6570 | 3 | 22216 | 3) | 1 | 0 | 0 | |
| 6570 | 22216 | (G3) | (6570 | 3 | 22216 | 3) | 0,245343 | -0,720355 | 0,518912 | |
| 6571 | 22216 | (G1) | (6571 | 1 | 22216 | 1) | 0,422301 | -1,07768 | 1,16139 | |
| 6571 | 22216 | (G2) | (6571 | 1 | 22216 | 1) | 2,5611 | 0,907111 | 0,822851 | |
| 6571 | 22216 | (G3) | (6571 | 1 | 22216 | 1) | 0,676028 | -0,609596 | 0,371607 | |
| 6571 | 22216 | (G1) | (6571 | 1 | 22216 | 2) | 1 | 0 | 0 | |
| 6571 | 22216 | (G2) | (6571 | 1 | 22216 | 2) | 7,22047 | 1,05945 | 1,12244 | |
| 6571 | 22216 | (G3) | (6571 | 1 | 22216 | 2) | 0,427137 | -1,06334 | 1,13069 | |
| 6571 | 22216 | (G1) | (6571 | 1 | 22216 | 3) | 1 | 0 | 0 | |
| 6571 | 22216 | (G2) | (6571 | 1 | 22216 | 3) | 1 | 0 | 0 | |
| 6571 | 22216 | (G3) | (6571 | 1 | 22216 | 3) | 0,140045 | -1,05347 | 1,1098 | |
| 6571 | 22216 | (G1) | (6571 | 2 | 22216 | 1) | 0,511822 | -1,79608 | 3,22592 | |
| 6571 | 22216 | (G2) | (6571 | 2 | 22216 | 1) | 1,20592 | 0,529237 | 0,280092 | |
| 6571 | 22216 | (G3) | (6571 | 2 | 22216 | 1) | 1,64058 | 1,41645 | 2,00633 | |
| 6571 | 22216 | (G1) | (6571 | 2 | 22216 | 2) | 1,18057 | 0,406867 | 0,165541 | |
| 6571 | 22216 | (G2) | (6571 | 2 | 22216 | 2) | 1,63652 | 1,09497 | 1,19897 | |
| 6571 | 22216 | (G3) | (6571 | 2 | 22216 | 2) | 0,377515 | -2,29014 | 5,24472 | |
| 6571 | 22216 | (G1) | (6571 | 2 | 22216 | 3) | 0,138495 | -1,05945 | 1,12244 | |
| 6571 | 22216 | (G2) | (6571 | 2 | 22216 | 3) | 1 | 0 | 0 | |
| 6571 | 22216 | (G3) | (6571 | 2 | 22216 | 3) | 0,0968804 | -1,27473 | 1,62495 | |
| 6571 | 22216 | (G1) | (6571 | 3 | 22216 | 1) | 2,96189 | 3,17608 | 10,0875 | |
| 6571 | 22216 | (G2) | (6571 | 3 | 22216 | 1) | 0,362041 | -3,01018 | 9,06119 | |
| 6571 | 22216 | (G3) | (6571 | 3 | 22216 | 1) | 2,90751 | 3,00365 | 9,0219 | |
| 6571 | 22216 | (G1) | (6571 | 3 | 22216 | 2) | 0,745546 | -0,854118 | 0,729518 | |
| 6571 | 22216 | (G2) | (6571 | 3 | 22216 | 2) | 1,20592 | 0,529237 | 0,280092 | |
| 6571 | 22216 | (G3) | (6571 | 3 | 22216 | 2) | 0,658084 | -1,09332 | 1,19535 | |
| 6571 | 22216 | (G1) | (6571 | 3 | 22216 | 3) | 0,138495 | -1,05945 | 1,12244 | |
| 6571 | 22216 | (G2) | (6571 | 3 | 22216 | 3) | 2,30686 | 1,23221 | 1,51835 | |
| 6571 | 22216 | (G3) | (6571 | 3 | 22216 | 3) | 4,12156 | 0,726074 | 0,527184 | |
| 8466 | 22099 | (G1) | (8466 | 1 | 22099 | 1) | 1 | 0 | 0 | |
| 8466 | 22099 | (G2) | (8466 | 1 | 22099 | 1) | 1 | 0 | 0 | |
| 8466 | 22099 | (G3) | (8466 | 1 | 22099 | 1) | 1 | 0 | 0 | |
| 8466 | 22099 | (G1) | (8466 | 1 | 22099 | 2) | 1 | 0 | 0 | |
| 8466 | 22099 | (G2) | (8466 | 1 | 22099 | 2) | 1 | 0 | 0 | |
| 8466 | 22099 | (G3) | (8466 | 1 | 22099 | 2) | 1 | 0 | 0 | |
| 8466 | 22099 | (G1) | (8466 | 1 | 22099 | 3) | 1 | 0 | 0 | |
| 8466 | 22099 | (G2) | (8466 | 1 | 22099 | 3) | 1 | 0 | 0 | |
| 8466 | 22099 | (G3) | (8466 | 1 | 22099 | 3) | 1 | 0 | 0 | |
| 8466 | 22099 | (G1) | (8466 | 2 | 22099 | 1) | 0,0958079 | -1,28084 | 1,64054 | |
| 8466 | 22099 | (G2) | (8466 | 2 | 22099 | 1) | 3,36877 | 1,21116 | 1,46691 | |
| 8466 | 22099 | (G3) | (8466 | 2 | 22099 | 1) | 1 | 0 | 0 | |
| 8466 | 22099 | (G1) | (8466 | 2 | 22099 | 2) | 1,24498 | 0,466796 | 0,217899 | |
| 8466 | 22099 | (G2) | (8466 | 2 | 22099 | 2) | 1,07956 | 0,195593 | 0,0382568 | |
| 8466 | 22099 | (G3) | (8466 | 2 | 22099 | 2) | 1,0891 | 0,206507 | 0,0426453 | |
| 8466 | 22099 | (G1) | (8466 | 2 | 22099 | 3) | 0,768152 | -0,724225 | 0,524502 | |
| 8466 | 22099 | (G2) | (8466 | 2 | 22099 | 3) | 2,56826 | 2,38016 | 5,66517 | |
| 8466 | 22099 | (G3) | (8466 | 2 | 22099 | 3) | 0,872082 | -0,369664 | 0,136652 | |
| 8466 | 22099 | (G1) | (8466 | 3 | 22099 | 1) | 1 | 0 | 0 | |
| 8466 | 22099 | (G2) | (8466 | 3 | 22099 | 1) | 2,5611 | 0,907111 | 0,822851 | |
| 8466 | 22099 | (G3) | (8466 | 3 | 22099 | 1) | 4,07542 | 0,720315 | 0,518854 | |
| 8466 | 22099 | (G1) | (8466 | 3 | 22099 | 2) | 0,413049 | -2,41338 | 5,82441 | |
| 8466 | 22099 | (G2) | (8466 | 3 | 22099 | 2) | 1,31575 | 0,738426 | 0,545272 | |
| 8466 | 22099 | (G3) | (8466 | 3 | 22099 | 2) | 3,56184 | 3,53927 | 12,5264 | |
| 8466 | 22099 | (G1) | (8466 | 3 | 22099 | 3) | 2,42178 | 2,84698 | 8,1053 | |
| 8466 | 22099 | (G2) | (8466 | 3 | 22099 | 3) | 0,331544 | -3,45915 | 11,9657 | |
| 8466 | 22099 | (G3) | (8466 | 3 | 22099 | 3) | 0,336061 | -3,35705 | 11,2698 | |
| 8897 | 15305 | (G1) | (8897 | 1 | 15305 | 1) | 1 | 0 | 0 | |
| 8897 | 15305 | (G2) | (8897 | 1 | 15305 | 1) | 2,53243 | 0,8962 | 0,803174 | |
| 8897 | 15305 | (G3) | (8897 | 1 | 15305 | 1) | 1,01119 | 0,00451366 | 2,04E-05 | |
| 8897 | 15305 | (G1) | (8897 | 1 | 15305 | 2) | 13,7292 | 1,44553 | 2,08956 | |
| 8897 | 15305 | (G2) | (8897 | 1 | 15305 | 2) | 0,988806 | -0,00911231 | 8,30E-05 | |
| 8897 | 15305 | (G3) | (8897 | 1 | 15305 | 2) | 1,46229 | 0,438097 | 0,191929 | |
| 8897 | 15305 | (G1) | (8897 | 1 | 15305 | 3) | 1 | 0 | 0 | |
| 8897 | 15305 | (G2) | (8897 | 1 | 15305 | 3) | 0,136927 | -1,06554 | 1,13537 | |
| 8897 | 15305 | (G3) | (8897 | 1 | 15305 | 3) | 0,140045 | -1,05347 | 1,1098 | |
| 8897 | 15305 | (G1) | (8897 | 2 | 15305 | 1) | 0,6159 | -0,970715 | 0,942288 | |
| 8897 | 15305 | (G2) | (8897 | 2 | 15305 | 1) | 0,988 | -0,0221534 | 0,00049077 | |
| 8897 | 15305 | (G3) | (8897 | 2 | 15305 | 1) | 1,52231 | 0,922313 | 0,85066 | |
| 8897 | 15305 | (G1) | (8897 | 2 | 15305 | 2) | 0,838463 | -0,419067 | 0,175617 | |
| 8897 | 15305 | (G2) | (8897 | 2 | 15305 | 2) | 0,81782 | -0,462621 | 0,214018 | |
| 8897 | 15305 | (G3) | (8897 | 2 | 15305 | 2) | 0,849306 | -0,388324 | 0,150795 | |
| 8897 | 15305 | (G1) | (8897 | 2 | 15305 | 3) | 0,570195 | -0,898461 | 0,807233 | |
| 8897 | 15305 | (G2) | (8897 | 2 | 15305 | 3) | 0,988142 | -0,0204637 | 0,00041876 | |
| 8897 | 15305 | (G3) | (8897 | 2 | 15305 | 3) | 0,0968804 | -1,27473 | 1,62495 | |
| 8897 | 15305 | (G1) | (8897 | 3 | 15305 | 1) | 3,0216 | 2,82472 | 7,97906 | |
| 8897 | 15305 | (G2) | (8897 | 3 | 15305 | 1) | 0,325724 | -2,86302 | 8,19686 | |
| 8897 | 15305 | (G3) | (8897 | 3 | 15305 | 1) | 0,269137 | -3,16471 | 10,0154 | |
| 8897 | 15305 | (G1) | (8897 | 3 | 15305 | 2) | 0,612085 | -1,55655 | 2,42286 | |
| 8897 | 15305 | (G2) | (8897 | 3 | 15305 | 2) | 1,59223 | 1,4127 | 1,99571 | |
| 8897 | 15305 | (G3) | (8897 | 3 | 15305 | 2) | 2,08457 | 2,25945 | 5,10512 | |
| 8897 | 15305 | (G1) | (8897 | 3 | 15305 | 3) | 0,900389 | -0,228907 | 0,0523984 | |
| 8897 | 15305 | (G2) | (8897 | 3 | 15305 | 3) | 2,2199 | 1,9183 | 3,67987 | |
| 8897 | 15305 | (G3) | (8897 | 3 | 15305 | 3) | 1,55924 | 1,05959 | 1,12274 | |
| 10073 | 15218 | (G1) | (10073 | 1 | 15218 | 1) | 0,0947108 | -1,2871 | 1,65663 | |
| 10073 | 15218 | (G2) | (10073 | 1 | 15218 | 1) | 1,90142 | 0,777535 | 0,604561 | |
| 10073 | 15218 | (G3) | (10073 | 1 | 15218 | 1) | 0,51983 | -0,791554 | 0,626558 | |
| 10073 | 15218 | (G1) | (10073 | 1 | 15218 | 2) | 1,47923 | 0,609596 | 0,371607 | |
| 10073 | 15218 | (G2) | (10073 | 1 | 15218 | 2) | 1,40824 | 0,580522 | 0,337005 | |
| 10073 | 15218 | (G3) | (10073 | 1 | 15218 | 2) | 0,660273 | -0,646305 | 0,41771 | |
| 10073 | 15218 | (G1) | (10073 | 1 | 15218 | 3) | 1,42918 | 0,411694 | 0,169492 | |
| 10073 | 15218 | (G2) | (10073 | 1 | 15218 | 3) | 0,138495 | -1,05945 | 1,12244 | |
| 10073 | 15218 | (G3) | (10073 | 1 | 15218 | 3) | 0,385986 | -0,918164 | 0,843024 | |
| 10073 | 15218 | (G1) | (10073 | 2 | 15218 | 1) | 1,2169 | 0,523483 | 0,274035 | |
| 10073 | 15218 | (G2) | (10073 | 2 | 15218 | 1) | 0,544134 | -1,43203 | 2,0507 | |
| 10073 | 15218 | (G3) | (10073 | 2 | 15218 | 1) | 0,568428 | -1,59702 | 2,55047 | |
| 10073 | 15218 | (G1) | (10073 | 2 | 15218 | 2) | 3,30626 | 3,073 | 9,44332 | |
| 10073 | 15218 | (G2) | (10073 | 2 | 15218 | 2) | 2,68188 | 2,3202 | 5,38334 | |
| 10073 | 15218 | (G3) | (10073 | 2 | 15218 | 2) | 0,719452 | -0,824505 | 0,679809 | |
| 10073 | 15218 | (G1) | (10073 | 2 | 15218 | 3) | 0,988 | -0,0221534 | 0,00049077 | |
| 10073 | 15218 | (G2) | (10073 | 2 | 15218 | 3) | 0,35142 | -1,3386 | 1,79185 | |
| 10073 | 15218 | (G3) | (10073 | 2 | 15218 | 3) | 0,51983 | -0,791554 | 0,626558 | |
| 10073 | 15218 | (G1) | (10073 | 3 | 15218 | 1) | 0,670798 | -1,00924 | 1,01856 | |
| 10073 | 15218 | (G2) | (10073 | 3 | 15218 | 1) | 2,06525 | 1,95464 | 3,82062 | |
| 10073 | 15218 | (G3) | (10073 | 3 | 15218 | 1) | 1,87352 | 1,53731 | 2,36332 | |
| 10073 | 15218 | (G1) | (10073 | 3 | 15218 | 2) | 0,223399 | -3,32287 | 11,0415 | |
| 10073 | 15218 | (G2) | (10073 | 3 | 15218 | 2) | 0,298091 | -3,20471 | 10,2702 | |
| 10073 | 15218 | (G3) | (10073 | 3 | 15218 | 2) | 2,99933 | 2,89015 | 8,35296 | |
| 10073 | 15218 | (G1) | (10073 | 3 | 15218 | 3) | 1,94598 | 0,959236 | 0,920133 | |
| 10073 | 15218 | (G2) | (10073 | 3 | 15218 | 3) | 2,2872 | 1,37506 | 1,89078 | |
| 10073 | 15218 | (G3) | (10073 | 3 | 15218 | 3) | 0,501971 | -0,993006 | 0,986062 | |
| 10073 | 15224 | (G1) | (10073 | 1 | 15224 | 1) | 1,44555 | 0,424857 | 0,180503 | |
| 10073 | 15224 | (G2) | (10073 | 1 | 15224 | 1) | 0,564425 | -0,521337 | 0,271792 | |
| 10073 | 15224 | (G3) | (10073 | 1 | 15224 | 1) | 4,07542 | 0,720315 | 0,518854 | |
| 10073 | 15224 | (G1) | (10073 | 1 | 15224 | 2) | 1,24537 | 0,330111 | 0,108973 | |
| 10073 | 15224 | (G2) | (10073 | 1 | 15224 | 2) | 1,2017 | 0,302401 | 0,0914462 | |
| 10073 | 15224 | (G3) | (10073 | 1 | 15224 | 2) | 0,437215 | -1,37506 | 1,89078 | |
| 10073 | 15224 | (G1) | (10073 | 1 | 15224 | 3) | 0,390457 | -0,907111 | 0,822851 | |
| 10073 | 15224 | (G2) | (10073 | 1 | 15224 | 3) | 1,90142 | 0,777535 | 0,604561 | |
| 10073 | 15224 | (G3) | (10073 | 1 | 15224 | 3) | 0,525922 | -0,777535 | 0,604561 | |
| 10073 | 15224 | (G1) | (10073 | 2 | 15224 | 1) | 1 | 0 | 0 | |
| 10073 | 15224 | (G2) | (10073 | 2 | 15224 | 1) | 0,610457 | -0,690368 | 0,476608 | |
| 10073 | 15224 | (G3) | (10073 | 2 | 15224 | 1) | 0,525922 | -0,777535 | 0,604561 | |
| 10073 | 15224 | (G1) | (10073 | 2 | 15224 | 2) | 2,46313 | 2,33799 | 5,46622 | |
| 10073 | 15224 | (G2) | (10073 | 2 | 15224 | 2) | 2,24789 | 1,94932 | 3,79984 | |
| 10073 | 15224 | (G3) | (10073 | 2 | 15224 | 2) | 0,621142 | -1,1822 | 1,39761 | |
| 10073 | 15224 | (G1) | (10073 | 2 | 15224 | 3) | 1,57719 | 1,2522 | 1,568 | |
| 10073 | 15224 | (G2) | (10073 | 2 | 15224 | 3) | 0,544134 | -1,43203 | 2,0507 | |
| 10073 | 15224 | (G3) | (10073 | 2 | 15224 | 3) | 0,653614 | -1,21167 | 1,46815 | |
| 10073 | 15224 | (G1) | (10073 | 3 | 15224 | 1) | 2,8456 | 1,3386 | 1,79185 | |
| 10073 | 15224 | (G2) | (10073 | 3 | 15224 | 1) | 4,34894 | 1,96431 | 3,85851 | |
| 10073 | 15224 | (G3) | (10073 | 3 | 15224 | 1) | 0,507995 | -0,975963 | 0,952505 | |
| 10073 | 15224 | (G1) | (10073 | 3 | 15224 | 2) | 0,214914 | -3,42135 | 11,7056 | |
| 10073 | 15224 | (G2) | (10073 | 3 | 15224 | 2) | 0,284002 | -3,3411 | 11,1629 | |
| 10073 | 15224 | (G3) | (10073 | 3 | 15224 | 2) | 2,91647 | 2,88662 | 8,33256 | |
| 10073 | 15224 | (G1) | (10073 | 3 | 15224 | 3) | 0,680202 | -0,974651 | 0,949945 | |
| 10073 | 15224 | (G2) | (10073 | 3 | 15224 | 3) | 1,90523 | 1,7656 | 3,11733 | |
| 10073 | 15224 | (G3) | (10073 | 3 | 15224 | 3) | 1,7744 | 1,39394 | 1,94306 | |
| 10386 | 16101 | (G1) | (10386 | 1 | 16101 | 1) | 2,5611 | 0,907111 | 0,822851 | |
| 10386 | 16101 | (G2) | (10386 | 1 | 16101 | 1) | 0,0968804 | -1,27473 | 1,62495 | |
| 10386 | 16101 | (G3) | (10386 | 1 | 16101 | 1) | 0,138495 | -1,05945 | 1,12244 | |
| 10386 | 16101 | (G1) | (10386 | 1 | 16101 | 2) | 1 | 0 | 0 | |
| 10386 | 16101 | (G2) | (10386 | 1 | 16101 | 2) | 1,0123 | 0,0237601 | 0,00056454 | |
| 10386 | 16101 | (G3) | (10386 | 1 | 16101 | 2) | 0,476855 | -1,44208 | 2,0796 | |
| 10386 | 16101 | (G1) | (10386 | 1 | 16101 | 3) | 0,604332 | -1,21363 | 1,4729 | |
| 10386 | 16101 | (G2) | (10386 | 1 | 16101 | 3) | 0,858145 | -0,374774 | 0,140455 | |
| 10386 | 16101 | (G3) | (10386 | 1 | 16101 | 3) | 1 | 0 | 0 | |
| 10386 | 16101 | (G1) | (10386 | 2 | 16101 | 1) | 1 | 0 | 0 | |
| 10386 | 16101 | (G2) | (10386 | 2 | 16101 | 1) | 1,01119 | 0,00451366 | 2,04E-05 | |
| 10386 | 16101 | (G3) | (10386 | 2 | 16101 | 1) | 0,525922 | -0,777535 | 0,604561 | |
| 10386 | 16101 | (G1) | (10386 | 2 | 16101 | 2) | 0,666067 | -0,999765 | 0,99953 | |
| 10386 | 16101 | (G2) | (10386 | 2 | 16101 | 2) | 0,813831 | -0,537177 | 0,288559 | |
| 10386 | 16101 | (G3) | (10386 | 2 | 16101 | 2) | 1,63877 | 1,3008 | 1,69209 | |
| 10386 | 16101 | (G1) | (10386 | 2 | 16101 | 3) | 2,58301 | 2,84456 | 8,09152 | 7 |
| 10386 | 16101 | (G2) | (10386 | 2 | 16101 | 3) | 2,70334 | 3,01634 | 9,09833 | 7 |
| 10386 | 16101 | (G3) | (10386 | 2 | 16101 | 3) | 2,91647 | 2,88662 | 8,33256 | 7 |
| 10386 | 16101 | (G1) | (10386 | 3 | 16101 | 1) | 0,390457 | -0,907111 | 0,822851 | |
| 10386 | 16101 | (G2) | (10386 | 3 | 16101 | 1) | 1,46229 | 0,438097 | 0,191929 | |
| 10386 | 16101 | (G3) | (10386 | 3 | 16101 | 1) | 0,564425 | -0,521337 | 0,271792 | |
| 10386 | 16101 | (G1) | (10386 | 3 | 16101 | 2) | 0,61736 | -0,837641 | 0,701642 | |
| 10386 | 16101 | (G2) | (10386 | 3 | 16101 | 2) | 0,662173 | -0,766209 | 0,587077 | |
| 10386 | 16101 | (G3) | (10386 | 3 | 16101 | 2) | 0,437354 | -1,5094 | 2,27829 | |
| 10386 | 16101 | (G1) | (10386 | 3 | 16101 | 3) | 0,701808 | -0,836068 | 0,699009 | |
| 10386 | 16101 | (G2) | (10386 | 3 | 16101 | 3) | 0,310578 | -2,21781 | 4,9187 | |
| 10386 | 16101 | (G3) | (10386 | 3 | 16101 | 3) | 0,596328 | -1,3288 | 1,7657 | |
| 10788 | 26873 | (G1) | (10788 | 1 | 26873 | 1) | 1 | 0 | 0 | |
| 10788 | 26873 | (G2) | (10788 | 1 | 26873 | 1) | 1 | 0 | 0 | |
| 10788 | 26873 | (G3) | (10788 | 1 | 26873 | 1) | 1 | 0 | 0 | |
| 10788 | 26873 | (G1) | (10788 | 1 | 26873 | 2) | 1 | 0 | 0 | |
| 10788 | 26873 | (G2) | (10788 | 1 | 26873 | 2) | 1 | 0 | 0 | |
| 10788 | 26873 | (G3) | (10788 | 1 | 26873 | 2) | 1 | 0 | 0 | |
| 10788 | 26873 | (G1) | (10788 | 1 | 26873 | 3) | 1 | 0 | 0 | |
| 10788 | 26873 | (G2) | (10788 | 1 | 26873 | 3) | 1 | 0 | 0 | |
| 10788 | 26873 | (G3) | (10788 | 1 | 26873 | 3) | 1 | 0 | 0 | |
| 10788 | 26873 | (G1) | (10788 | 2 | 26873 | 1) | 0,810743 | -0,646896 | 0,418474 | |
| 10788 | 26873 | (G2) | (10788 | 2 | 26873 | 1) | 0,643325 | -1,31979 | 1,74183 | |
| 10788 | 26873 | (G3) | (10788 | 2 | 26873 | 1) | 2,11389 | 2,3333 | 5,4443 | |
| 10788 | 26873 | (G1) | (10788 | 2 | 26873 | 2) | 1,1613 | 0,386298 | 0,149226 | |
| 10788 | 26873 | (G2) | (10788 | 2 | 26873 | 2) | 1,14668 | 0,369664 | 0,136652 | |
| 10788 | 26873 | (G3) | (10788 | 2 | 26873 | 2) | 1,24529 | 0,572372 | 0,32761 | |
| 10788 | 26873 | (G1) | (10788 | 2 | 26873 | 3) | 0,525922 | -0,777535 | 0,604561 | |
| 10788 | 26873 | (G2) | (10788 | 2 | 26873 | 3) | 0,238358 | -1,46172 | 2,13662 | |
| 10788 | 26873 | (G3) | (10788 | 2 | 26873 | 3) | 1,44555 | 0,424857 | 0,180503 | |
| 10788 | 26873 | (G1) | (10788 | 3 | 26873 | 1) | 2,58301 | 2,84456 | 8,09152 | |
| 10788 | 26873 | (G2) | (10788 | 3 | 26873 | 1) | 2,58301 | 2,84456 | 8,09152 | |
| 10788 | 26873 | (G3) | (10788 | 3 | 26873 | 1) | 0,355521 | -2,85016 | 8,12342 | |
| 10788 | 26873 | (G1) | (10788 | 3 | 26873 | 2) | 0,396857 | -2,16107 | 4,67022 | |
| 10788 | 26873 | (G2) | (10788 | 3 | 26873 | 2) | 0,563571 | -1,39394 | 1,94306 | |
| 10788 | 26873 | (G3) | (10788 | 3 | 26873 | 2) | 0,666067 | -0,999765 | 0,99953 | |
| 10788 | 26873 | (G1) | (10788 | 3 | 26873 | 3) | 0,0958079 | -1,28084 | 1,64054 | |
| 10788 | 26873 | (G2) | (10788 | 3 | 26873 | 3) | 0,564425 | -0,521337 | 0,271792 | |
| 10788 | 26873 | (G3) | (10788 | 3 | 26873 | 3) | 2,36798 | 1,07768 | 1,16139 | |
| 11993 | 22099 | (G1) | (11993 | 1 | 22099 | 1) | 1,77171 | 0,521337 | 0,271792 | |
| 11993 | 22099 | (G2) | (11993 | 1 | 22099 | 1) | 0,977358 | -0,0243966 | 0,0005952 | |
| 11993 | 22099 | (G3) | (11993 | 1 | 22099 | 1) | 0,250929 | -0,708813 | 0,502415 | |
| 11993 | 22099 | (G1) | (11993 | 1 | 22099 | 2) | 0,396857 | -2,16107 | 4,67022 | |
| 11993 | 22099 | (G2) | (11993 | 1 | 22099 | 2) | 0,970296 | -0,0885805 | 0,0078465 | |
| 11993 | 22099 | (G3) | (11993 | 1 | 22099 | 2) | 1,69242 | 1,47872 | 2,18661 | |
| 11993 | 22099 | (G1) | (11993 | 1 | 22099 | 3) | 2,64208 | 3,04325 | 9,26137 | |
| 11993 | 22099 | (G2) | (11993 | 1 | 22099 | 3) | 0,372626 | -2,94947 | 8,69939 | |
| 11993 | 22099 | (G3) | (11993 | 1 | 22099 | 3) | 0,311077 | -3,47004 | 12,0412 | |
| 11993 | 22099 | (G1) | (11993 | 2 | 22099 | 1) | 0,0728376 | -1,44553 | 2,08956 | |
| 11993 | 22099 | (G2) | (11993 | 2 | 22099 | 1) | 16,7194 | 1,56453 | 2,44775 | |
| 11993 | 22099 | (G3) | (11993 | 2 | 22099 | 1) | 7,38775 | 1,07169 | 1,14852 | |
| 11993 | 22099 | (G1) | (11993 | 2 | 22099 | 2) | 0,929619 | -0,190977 | 0,0364723 | |
| 11993 | 22099 | (G2) | (11993 | 2 | 22099 | 2) | 1,91262 | 1,33297 | 1,77681 | |
| 11993 | 22099 | (G3) | (11993 | 2 | 22099 | 2) | 2,94107 | 2,64854 | 7,01478 | |
| 11993 | 22099 | (G1) | (11993 | 2 | 22099 | 3) | 0,745546 | -0,854118 | 0,729518 | |
| 11993 | 22099 | (G2) | (11993 | 2 | 22099 | 3) | 1,63019 | 1,42569 | 2,03258 | |
| 11993 | 22099 | (G3) | (11993 | 2 | 22099 | 3) | 0,906235 | -0,275773 | 0,0760506 | |
| 11993 | 22099 | (G1) | (11993 | 3 | 22099 | 1) | 1 | 0 | 0 | |
| 11993 | 22099 | (G2) | (11993 | 3 | 22099 | 1) | 0,977859 | -0,00907804 | 8,24E-05 | |
| 11993 | 22099 | (G3) | (11993 | 3 | 22099 | 1) | 1,02264 | 0,00907804 | 8,24E-05 | |
| 11993 | 22099 | (G1) | (11993 | 3 | 22099 | 2) | 1 | 0 | 0 | |
| 11993 | 22099 | (G2) | (11993 | 3 | 22099 | 2) | 0,977859 | -0,00907804 | 8,24E-05 | |
| 11993 | 22099 | (G3) | (11993 | 3 | 22099 | 2) | 1,02264 | 0,00907804 | 8,24E-05 | |
| 11993 | 22099 | (G1) | (11993 | 3 | 22099 | 3) | 1 | 0 | 0 | |
| 11993 | 22099 | (G2) | (11993 | 3 | 22099 | 3) | 0,977859 | -0,00907804 | 8,24E-05 | |
| 11993 | 22099 | (G3) | (11993 | 3 | 22099 | 3) | 1,02264 | 0,00907804 | 8,24E-05 | |
| 13145 | 27852 | (G1) | (13145 | 1 | 27852 | 1) | 1,02264 | 0,00907804 | 8,24E-05 | |
| 13145 | 27852 | (G2) | (13145 | 1 | 27852 | 1) | 0,988806 | -0,00456438 | 2,08E-05 | |
| 13145 | 27852 | (G3) | (13145 | 1 | 27852 | 1) | 0,40408 | -0,873765 | 0,763465 | |
| 13145 | 27852 | (G1) | (13145 | 1 | 27852 | 2) | 10,6822 | 1,29344 | 1,67298 | |
| 13145 | 27852 | (G2) | (13145 | 1 | 27852 | 2) | 1,42918 | 0,411621 | 0,169432 | |
| 13145 | 27852 | (G3) | (13145 | 1 | 27852 | 2) | 0,152179 | -1,98902 | 3,95622 | |
| 13145 | 27852 | (G1) | (13145 | 1 | 27852 | 3) | 1,0249 | 0,0478004 | 0,00228488 | |
| 13145 | 27852 | (G2) | (13145 | 1 | 27852 | 3) | 1,09681 | 0,20135 | 0,0405418 | |
| 13145 | 27852 | (G3) | (13145 | 1 | 27852 | 3) | 0,60515 | -0,949493 | 0,901538 | |
| 13145 | 27852 | (G1) | (13145 | 2 | 27852 | 1) | 0,141631 | -1,04742 | 1,09708 | |
| 13145 | 27852 | (G2) | (13145 | 2 | 27852 | 1) | 2,53276 | 0,896217 | 0,803205 | |
| 13145 | 27852 | (G3) | (13145 | 2 | 27852 | 1) | 1,03475 | 0,0138499 | 0,00019182 | |
| 13145 | 27852 | (G1) | (13145 | 2 | 27852 | 2) | 1,30974 | 0,668312 | 0,44664 | |
| 13145 | 27852 | (G2) | (13145 | 2 | 27852 | 2) | 2,68245 | 1,97818 | 3,9132 | |
| 13145 | 27852 | (G3) | (13145 | 2 | 27852 | 2) | 0,60515 | -0,949493 | 0,901538 | |
| 13145 | 27852 | (G1) | (13145 | 2 | 27852 | 3) | 0,35959 | -2,8778 | 8,28174 | |
| 13145 | 27852 | (G2) | (13145 | 2 | 27852 | 3) | 0,367961 | -3,11401 | 9,69704 | |
| 13145 | 27852 | (G3) | (13145 | 2 | 27852 | 3) | 2,97661 | 2,99041 | 8,94256 | |
| 13145 | 27852 | (G1) | (13145 | 3 | 27852 | 1) | 0,399399 | -0,885165 | 0,783517 | |
| 13145 | 27852 | (G2) | (13145 | 3 | 27852 | 1) | 7,14146 | 1,0535 | 1,10986 | |
| 13145 | 27852 | (G3) | (13145 | 3 | 27852 | 1) | 1,03516 | 0,0279643 | 0,000782 | |
| 13145 | 27852 | (G1) | (13145 | 3 | 27852 | 2) | 1,40811 | 0,740389 | 0,548175 | |
| 13145 | 27852 | (G2) | (13145 | 3 | 27852 | 2) | 0,858576 | -0,287612 | 0,0827209 | |
| 13145 | 27852 | (G3) | (13145 | 3 | 27852 | 2) | 1,24255 | 0,515241 | 0,265474 | |
| 13145 | 27852 | (G1) | (13145 | 3 | 27852 | 3) | 1,84331 | 1,86529 | 3,47932 | |
| 13145 | 27852 | (G2) | (13145 | 3 | 27852 | 3) | 1,51078 | 1,17273 | 1,37529 | |
| 13145 | 27852 | (G3) | (13145 | 3 | 27852 | 3) | 0,812548 | -0,644867 | 0,415853 | |
| 13221 | 22109 | (G1) | (13221 | 1 | 22109 | 1) | 1,77171 | 0,521337 | 0,271792 | |
| 13221 | 22109 | (G2) | (13221 | 1 | 22109 | 1) | 0,238102 | -1,46241 | 2,13863 | |
| 13221 | 22109 | (G3) | (13221 | 1 | 22109 | 1) | 0,303642 | -1,18843 | 1,41236 | |
| 13221 | 22109 | (G1) | (13221 | 1 | 22109 | 2) | 0,24648 | -2,70668 | 7,32612 | |
| 13221 | 22109 | (G2) | (13221 | 1 | 22109 | 2) | 0,542928 | -1,53645 | 2,36068 | |
| 13221 | 22109 | (G3) | (13221 | 1 | 22109 | 2) | 0,815765 | -0,517924 | 0,268245 | |
| 13221 | 22109 | (G1) | (13221 | 1 | 22109 | 3) | 2,69552 | 3,15262 | 9,93904 | 8 |
| 13221 | 22109 | (G2) | (13221 | 1 | 22109 | 3) | 2,59388 | 2,93895 | 8,6374 | 8 |
| 13221 | 22109 | (G3) | (13221 | 1 | 22109 | 3) | 2,44322 | 2,84432 | 8,09014 | 8 |
| 13221 | 22109 | (G1) | (13221 | 2 | 22109 | 1) | 1 | 0 | 0 | |
| 13221 | 22109 | (G2) | (13221 | 2 | 22109 | 1) | 4,07645 | 0,720397 | 0,518972 | |
| 13221 | 22109 | (G3) | (13221 | 2 | 22109 | 1) | 0,173135 | -1,83659 | 3,37305 | |
| 13221 | 22109 | (G1) | (13221 | 2 | 22109 | 2) | 1,65472 | 1,21363 | 1,4729 | |
| 13221 | 22109 | (G2) | (13221 | 2 | 22109 | 2) | 0,451255 | -1,83246 | 3,35793 | |
| 13221 | 22109 | (G3) | (13221 | 2 | 22109 | 2) | 0,472003 | -1,6494 | 2,72051 | |
| 13221 | 22109 | (G1) | (13221 | 2 | 22109 | 3) | 0,444657 | -2,41486 | 5,83155 | |
| 13221 | 22109 | (G2) | (13221 | 2 | 22109 | 3) | 1,05907 | 0,169385 | 0,0286912 | |
| 13221 | 22109 | (G3) | (13221 | 2 | 22109 | 3) | 1,02927 | 0,0808805 | 0,00654165 | |
| 13221 | 22109 | (G1) | (13221 | 3 | 22109 | 1) | 1 | 0 | 0 | |
| 13221 | 22109 | (G2) | (13221 | 3 | 22109 | 1) | 1 | 0 | 0 | |
| 13221 | 22109 | (G3) | (13221 | 3 | 22109 | 1) | 1,02264 | 0,00907804 | 8,24E-05 | |
| 13221 | 22109 | (G1) | (13221 | 3 | 22109 | 2) | 1 | 0 | 0 | |
| 13221 | 22109 | (G2) | (13221 | 3 | 22109 | 2) | 1 | 0 | 0 | |
| 13221 | 22109 | (G3) | (13221 | 3 | 22109 | 2) | 1,02264 | 0,00907804 | 8,24E-05 | |
| 13221 | 22109 | (G1) | (13221 | 3 | 22109 | 3) | 1 | 0 | 0 | |
| 13221 | 22109 | (G2) | (13221 | 3 | 22109 | 3) | 1 | 0 | 0 | |
| 13221 | 22109 | (G3) | (13221 | 3 | 22109 | 3) | 1,02264 | -0,00907804 | 8,24E-05 | |
| 13221 | 22112 | (G1) | (13221 | 1 | 22112 | 1) | 2,69552 | 3,15262 | 9,93904 | 8 |
| 13221 | 22112 | (G2) | (13221 | 1 | 22112 | 1) | 2,54021 | 2,88047 | 8,29711 | 8 |
| 13221 | 22112 | (G3) | (13221 | 1 | 22112 | 1) | 2,44322 | 2,84432 | 8,09014 | 8 |
| 13221 | 22112 | (G1) | (13221 | 1 | 22112 | 2) | 0,24648 | -2,70668 | 7,32612 | |
| 13221 | 22112 | (G2) | (13221 | 1 | 22112 | 2) | 0,586961 | -1,36685 | 1,86829 | |
| 13221 | 22112 | (G3) | (13221 | 1 | 22112 | 2) | 0,815765 | -0,517924 | 0,268245 | |
| 13221 | 22112 | (G1) | (13221 | 1 | 22112 | 3) | 1,77171 | 0,521337 | 0,271792 | |
| 13221 | 22112 | (G2) | (13221 | 1 | 22112 | 3) | 0,235406 | -1,47411 | 2,17299 | |
| 13221 | 22112 | (G3) | (13221 | 1 | 22112 | 3) | 0,303642 | -1,18843 | 1,41236 | |
| 13221 | 22112 | (G1) | (13221 | 2 | 22112 | 1) | 0,444657 | -2,41486 | 5,83155 | |
| 13221 | 22112 | (G2) | (13221 | 2 | 22112 | 1) | 1,04286 | 0,12399 | 0,0153736 | |
| 13221 | 22112 | (G3) | (13221 | 2 | 22112 | 1) | 1,02927 | 0,0808805 | 0,00654165 | |
| 13221 | 22112 | (G1) | (13221 | 2 | 22112 | 2) | 1,65472 | 1,21363 | 1,4729 | |
| 13221 | 22112 | (G2) | (13221 | 2 | 22112 | 2) | 0,445638 | -1,86209 | 3,46737 | |
| 13221 | 22112 | (G3) | (13221 | 2 | 22112 | 2) | 0,472003 | -1,6494 | 2,72051 | |
| 13221 | 22112 | (G1) | (13221 | 2 | 22112 | 3) | 1 | 0 | 0 | |
| 13221 | 22112 | (G2) | (13221 | 2 | 22112 | 3) | 4,0303 | 0,714572 | 0,510613 | |
| 13221 | 22112 | (G3) | (13221 | 2 | 22112 | 3) | 0,173135 | -1,83659 | 3,37305 | |
| 13221 | 22112 | (G1) | (13221 | 3 | 22112 | 1) | 1 | 0 | 0 | |
| 13221 | 22112 | (G2) | (13221 | 3 | 22112 | 1) | 0,988806 | -0,00456438 | 2,08E-05 | |
| 13221 | 22112 | (G3) | (13221 | 3 | 22112 | 1) | 1,02264 | 0,00907804 | 8,24E-05 | |
| 13221 | 22112 | (G1) | (13221 | 3 | 22112 | 2) | 1 | 0 | 0 | |
| 13221 | 22112 | (G2) | (13221 | 3 | 22112 | 2) | 0,988806 | -0,00456438 | 2,08E-05 | |
| 13221 | 22112 | (G3) | (13221 | 3 | 22112 | 2) | 1,02264 | 0,00907804 | 8,24E-05 | |
| 13221 | 22112 | (G1) | (13221 | 3 | 22112 | 3) | 1 | 0 | 0 | |
| 13221 | 22112 | (G2) | (13221 | 3 | 22112 | 3) | 0,988806 | -0,00456438 | 2,08E-05 | |
| 13221 | 22112 | (G3) | (13221 | 3 | 22112 | 3) | 1,02264 | 0,00907804 | 8,24E-05 | |
| 13221 | 22113 | (G1) | (13221 | 1 | 22113 | 1) | 1,77171 | 0,521337 | 0,271792 | |
| 13221 | 22113 | (G2) | (13221 | 1 | 22113 | 1) | 0,235406 | -1,47411 | 2,17299 | |
| 13221 | 22113 | (G3) | (13221 | 1 | 22113 | 1) | 0,303642 | -1,18843 | 1,41236 | |
| 13221 | 22113 | (G1) | (13221 | 1 | 22113 | 2) | 0,24648 | -2,70668 | 7,32612 | |
| 13221 | 22113 | (G2) | (13221 | 1 | 22113 | 2) | 0,586961 | -1,36685 | 1,86829 | |
| 13221 | 22113 | (G3) | (13221 | 1 | 22113 | 2) | 0,815765 | 0,517924 | 0,268245 | |
| 13221 | 22113 | (G1) | (13221 | 1 | 22113 | 3) | 2,69552 | 3,15262 | 9,93904 | 8 |
| 13221 | 22113 | (G2) | (13221 | 1 | 22113 | 3) | 2,54021 | 2,88047 | 8,29711 | 8 |
| 13221 | 22113 | (G3) | (13221 | 1 | 22113 | 3) | 2,44322 | 2,84432 | 8,09014 | 8 |
| 13221 | 22113 | (G1) | (13221 | 2 | 22113 | 1) | 1 | 0 | 0 | |
| 13221 | 22113 | (G2) | (13221 | 2 | 22113 | 1) | 4,0303 | 0,714572 | 0,510613 | |
| 13221 | 22113 | (G3) | (13221 | 2 | 22113 | 1) | 0,173135 | -1,83659 | 3,37305 | |
| 13221 | 22113 | (G1) | (13221 | 2 | 22113 | 2) | 1,65472 | 1,21363 | 1,4729 | |
| 13221 | 22113 | (G2) | (13221 | 2 | 22113 | 2) | 0,445638 | -1,86209 | 3,46737 | |
| 13221 | 22113 | (G3) | (13221 | 2 | 22113 | 2) | 0,472003 | -1,6494 | 2,72051 | |
| 13221 | 22113 | (G1) | (13221 | 2 | 22113 | 3) | 0,444657 | -2,41486 | 5,83155 | |
| 13221 | 22113 | (G2) | (13221 | 2 | 22113 | 3) | 1,04286 | 0,12399 | 0,0153736 | |
| 13221 | 22113 | (G3) | (13221 | 2 | 22113 | 3) | 1,02927 | 0,0808805 | 0,00654165 | |
| 13221 | 22113 | (G1) | (13221 | 3 | 22113 | 1) | 1 | 0 | 0 | |
| 13221 | 22113 | (G2) | (13221 | 3 | 22113 | 1) | 0,988806 | -0,00456438 | 2,08E-05 | |
| 13221 | 22113 | (G3) | (13221 | 3 | 22113 | 1) | 1,02264 | 0,00907804 | 8,24E-05 | |
| 13221 | 22113 | (G1) | (13221 | 3 | 22113 | 2) | 1 | 0 | 0 | |
| 13221 | 22113 | (G2) | (13221 | 3 | 22113 | 2) | 0,988806 | -0,00456438 | 2,08E-05 | |
| 13221 | 22113 | (G3) | (13221 | 3 | 22113 | 2) | 1,02264 | 0,00907804 | 8,24E-05 | |
| 13221 | 22113 | (G1) | (13221 | 3 | 22113 | 3) | 1 | 0 | 0 | |
| 13221 | 22113 | (G2) | (13221 | 3 | 22113 | 3) | 0,988806 | -0,00456438 | 2,08E-05 | |
| 13221 | 22113 | (G3) | (13221 | 3 | 22113 | 3) | 1,02264 | 0,00907804 | 8,24E-05 | |
| 13221 | 22114 | (G1) | (13221 | 1 | 22114 | 1) | 2,69552 | 3,15262 | 9,93904 | 8 |
| 13221 | 22114 | (G2) | (13221 | 1 | 22114 | 1) | 2,54021 | 2,88047 | 8,29711 | 8 |
| 13221 | 22114 | (G3) | (13221 | 1 | 22114 | 1) | 2,44322 | 2,84432 | 8,09014 | 8 |
| 13221 | 22114 | (G1) | (13221 | 1 | 22114 | 2) | 0,24648 | -2,70668 | 7,32612 | |
| 13221 | 22114 | (G2) | (13221 | 1 | 22114 | 2) | 0,586961 | -1,36685 | 1,86829 | |
| 13221 | 22114 | (G3) | (13221 | 1 | 22114 | 2) | 0,815765 | -0,517924 | 0,268245 | |
| 13221 | 22114 | (G1) | (13221 | 1 | 22114 | 3) | 1,77171 | 0,521337 | 0,271792 | |
| 13221 | 22114 | (G2) | (13221 | 1 | 22114 | 3) | 0,235406 | -1,47411 | 2,17299 | |
| 13221 | 22114 | (G3) | (13221 | 1 | 22114 | 3) | 0,303642 | -1,18843 | 1,41236 | |
| 13221 | 22114 | (G1) | (13221 | 2 | 22114 | 1) | 0,466129 | -2,26867 | 5,14686 | |
| 13221 | 22114 | (G2) | (13221 | 2 | 22114 | 1) | 1,04286 | 0,12399 | 0,0153736 | |
| 13221 | 22114 | (G3) | (13221 | 2 | 22114 | 1) | 1,02927 | 0,0808805 | 0,00654165 | |
| 13221 | 22114 | (G1) | (13221 | 2 | 22114 | 2) | 1,50135 | 0,999765 | 0,99953 | |
| 13221 | 22114 | (G2) | (13221 | 2 | 22114 | 2) | 0,445638 | -1,86209 | 3,46737 | |
| 13221 | 22114 | (G3) | (13221 | 2 | 22114 | 2) | 0,472003 | -1,6494 | 2,72051 | |
| 13221 | 22114 | (G1) | (13221 | 2 | 22114 | 3) | 1 | 0 | 0 | |
| 13221 | 22114 | (G2) | (13221 | 2 | 22114 | 3) | 4,0303 | 0,714572 | 0,510613 | |
| 13221 | 22114 | (G3) | (13221 | 2 | 22114 | 3) | 0,173135 | -1,83659 | 3,37305 | |
| 13221 | 22114 | (G1) | (13221 | 3 | 22114 | 1) | 1 | 0 | 0 | |
| 13221 | 22114 | (G2) | (13221 | 3 | 22114 | 1) | 0,988806 | -0,00456438 | 2,0BE-05 | |
| 13221 | 22114 | (G3) | (13221 | 3 | 22114 | 1) | 1,02264 | 0,00907804 | 8,24E-05 | |
| 13221 | 22114 | (G1) | (13221 | 3 | 22114 | 2) | 1 | 0 | 0 | |
| 13221 | 22114 | (G2) | (13221 | 3 | 22114 | 2) | 0,988806 | -0,00456438 | 2,08E-05 | |
| 13221 | 22114 | (G3) | (13221 | 3 | 22114 | 2) | 1,02264 | 0,00907804 | 8,24E-05 | |
| 13221 | 22114 | (G1) | (13221 | 3 | 22114 | 3) | 1 | 0 | 0 | |
| 13221 | 22114 | (G2) | (13221 | 3 | 22114 | 3) | 0,988806 | -0,00456438 | 2,08E-05 | |
| 13221 | 22114 | (G3) | (13221 | 3 | 22114 | 3) | 1,02264 | 0,00907804 | 8,24E-05 | |
| 13861 | 22083 | (G1) | (13861 | 1 | 22083 | 1) | 1 | 0 | 0 | |
| 13861 | 22083 | (G2) | (13861 | 1 | 22083 | 1) | 1,01119 | 0,00451366 | 2,04E-05 | |
| 13861 | 22083 | (G3) | (13861 | 1 | 22083 | 1) | 1,05929 | 0,0233527 | 0,00054535 | |
| 13861 | 22083 | (G1) | (13861 | 1 | 22083 | 2) | 1 | 0 | 0 | |
| 13861 | 22083 | (G2) | (13861 | 1 | 22083 | 2) | 1,01119 | 0,00451366 | 2,04E-05 | |
| 13861 | 22083 | (G3) | (13861 | 1 | 22083 | 2) | 1,05929 | 0,0233527 | 0,00054535 | |
| 13861 | 22083 | (G1) | (13861 | 1 | 22083 | 3) | 1 | 0 | 0 | |
| 13861 | 22083 | (G2) | (13861 | 1 | 22083 | 3) | 1,01119 | 0,00451366 | 2,04E-05 | |
| 13861 | 22083 | (G3) | (13861 | 1 | 22083 | 3) | 1,05929 | 0,0233527 | 0,00054535 | |
| 13861 | 22083 | (G1) | (13861 | 2 | 22083 | 1) | 0,691688 | -0,424934 | 0,180569 | |
| 13861 | 22083 | (G2) | (13861 | 2 | 22083 | 1) | 1,65754 | 0,706762 | 0,499512 | |
| 13861 | 22083 | (G3) | (13861 | 2 | 22083 | 1) | 1,53462 | 0,493477 | 0,243519 | |
| 13861 | 22083 | (G1) | (13861 | 2 | 22083 | 2) | 0,654031 | -0,788996 | 0,622515 | |
| 13861 | 22083 | (G2) | (13861 | 2 | 22083 | 2) | 1,5759 | 0,956248 | 0,914411 | |
| 13861 | 22083 | (G3) | (13861 | 2 | 22083 | 2) | 0,976135 | -0,0564573 | 0,00318742 | |
| 13861 | 22083 | (G1) | (13861 | 2 | 22083 | 3) | 0,4369 | -1,3757 | 1,89256 | |
| 13861 | 22083 | (G2) | (13861 | 2 | 22083 | 3) | 1,3645 | 0,565365 | 0,319637 | |
| 13861 | 22083 | (G3) | (13861 | 2 | 22083 | 3) | 2,09504 | 1,06457 | 1,13332 | |
| 13861 | 22083 | (G1) | (13861 | 3 | 22083 | 1) | 1,07976 | 0,195825 | 0,0383473 | |
| 13861 | 22083 | (G2) | (13861 | 3 | 22083 | 1) | 1,88014 | 1,3608 | 1,85178 | |
| 13861 | 22083 | (G3) | (13861 | 3 | 22083 | 1) | 2,54751 | 2,16435 | 4,68442 | |
| 13861 | 22083 | (G1) | (13861 | 3 | 22083 | 2) | 0,622131 | -1,53206 | 2,34721 | |
| 13861 | 22083 | (G2) | (13861 | 3 | 22083 | 2) | 1,22527 | 0,668174 | 0,446457 | |
| 13861 | 22083 | (G3) | (13861 | 3 | 22083 | 2) | 1,14919 | 0,438646 | 0,19241 | |
| 13861 | 22083 | (G1) | (13861 | 3 | 22083 | 3) | 2,96446 | 2,99748 | 8,98488 | |
| 13861 | 22083 | (G2) | (13861 | 3 | 22083 | 3) | 0,301946 | -3,16886 | 10,0416 | |
| 13861 | 22083 | (G3) | (13861 | 3 | 22083 | 3) | 0,331386 | -2,97949 | 8,87737 | |
| 15993 | 30662 | (G1) | (15993 | 1 | 30662 | 1) | 0,751237 | -0,384056 | 0,147499 | |
| 15993 | 30662 | (G2) | (15993 | 1 | 30662 | 1) | 0,385986 | -0,918164 | 0,843024 | |
| 15993 | 30662 | (G3) | (15993 | 1 | 30662 | 1) | 1,75188 | 0,511048 | 0,26117 | |
| 15993 | 30662 | (G1) | (15993 | 1 | 30662 | 2) | 1,68862 | 1,2199 | 1,48816 | |
| 15993 | 30662 | (G2) | (15993 | 1 | 30662 | 2) | 1,22967 | 0,440287 | 0,193853 | |
| 15993 | 30662 | (G3) | (15993 | 1 | 30662 | 2) | 0,906158 | -0,238292 | 0,0567833 | |
| 15993 | 30662 | (G1) | (15993 | 1 | 30662 | 3) | 0,986899 | -0,0313978 | 0,00098582 | |
| 15993 | 30662 | (G2) | (15993 | 1 | 30662 | 3) | 0,692491 | -0,86719 | 0,752019 | |
| 15993 | 30662 | (G3) | (15993 | 1 | 30662 | 3) | 1,1653 | 0,374774 | 0,140455 | |
| 15993 | 30662 | (G1) | (15993 | 2 | 30662 | 1) | 4,15008 | 1,45036 | 2,10353 | |
| 15993 | 30662 | (G2) | (15993 | 2 | 30662 | 1) | 0,822286 | -0,321972 | 0,103666 | |
| 15993 | 30662 | (G3) | (15993 | 2 | 30662 | 1) | 2,62304 | 1,4471 | 2,09409 | |
| 15993 | 30662 | (G1) | (15993 | 2 | 30662 | 2) | 0,292593 | -3,15248 | 9,93814 | |
| 15993 | 30662 | (G2) | (15993 | 2 | 30662 | 2) | 2,99933 | 2,89015 | 8,35296 | |
| 15993 | 30662 | (G3) | (15993 | 2 | 30662 | 2) | 0,34077 | -2,82255 | 7,9668 | |
| 15993 | 30662 | (G1) | (15993 | 2 | 30662 | 3) | 2,30548 | 2,15232 | 4,63248 | |
| 15993 | 30662 | (G2) | (15993 | 2 | 30662 | 3) | 1,26071 | 0,658795 | 0,434012 | |
| 15993 | 30662 | (G3) | (15993 | 2 | 30662 | 3) | 1,83795 | 1,69858 | 2,88519 | |
| 15993 | 30662 | (G1) | (15993 | 3 | 30662 | 1) | 0,557891 | -0,531865 | 0,282881 | |
| 15993 | 30662 | (G2) | (15993 | 3 | 30662 | 1) | 0,988679 | -0,0121294 | 0,00014712 | |
| 15993 | 30662 | (G3) | (15993 | 3 | 30662 | 1) | 0,988806 | -0,00911231 | 8,30E-05 | |
| 15993 | 30662 | (G1) | (15993 | 3 | 30662 | 2) | 1,39487 | 0,688686 | 0,474288 | |
| 15993 | 30662 | (G2) | (15993 | 3 | 30662 | 2) | 0,202575 | -2,15308 | 4,63576 | |
| 15993 | 30662 | (G3) | (15993 | 3 | 30662 | 2) | 0,843094 | -0,302471 | 0,0914888 | |
| 15993 | 30662 | (G1) | (15993 | 3 | 30662 | 3) | 0,537404 | -1,09906 | 1,20793 | |
| 15993 | 30662 | (G2) | (15993 | 3 | 30662 | 3) | 0,42982 | -1,66253 | 2,76401 | |
| 15993 | 30662 | (G3) | (15993 | 3 | 30662 | 3) | 1,15798 | 0,259937 | 0,067567 | |
| 16005 | 30662 | (G1) | (16005 | 1 | 30662 | 1) | 0,769053 | -0,352671 | 0,124377 | |
| 16005 | 30662 | (G2) | (16005 | 1 | 30662 | 1) | 0,381515 | -0,929342 | 0,863677 | |
| 16005 | 30662 | (G3) | (16005 | 1 | 30662 | 1) | 1,75188 | 0,511048 | 0,26117 | |
| 16005 | 30662 | (G1) | (16005 | 1 | 30662 | 2) | 1,60382 | 1,08966 | 1,18735 | |
| 16005 | 30662 | (G2) | (16005 | 1 | 30662 | 2) | 1,21436 | 0,413463 | 0,170951 | |
| 16005 | 30662 | (G3) | (16005 | 1 | 30662 | 2) | 0,906158 | -0,238292 | 0,0567833 | |
| 16005 | 30662 | (G1) | (16005 | 1 | 30662 | 3) | 0,930216 | -0,17493 | 0,0306004 | |
| 16005 | 30662 | (G2) | (16005 | 1 | 30662 | 3) | 0,683175 | -0,898696 | 0,807655 | |
| 16005 | 30662 | (G3) | (16005 | 1 | 30662 | 3) | 1,27632 | 0,587365 | 0,344997 | |
| 16005 | 30662 | (G1) | (16005 | 2 | 30662 | 1) | 5,10209 | 1,6871 | 2,84631 | |
| 16005 | 30662 | (G2) | (16005 | 2 | 30662 | 1) | 0,812419 | -0,341771 | 0,116807 | |
| 16005 | 30662 | (G3) | (16005 | 2 | 30662 | 1) | 2,62304 | 1,4471 | 2,09409 | |
| 16005 | 30662 | (G1) | (16005 | 2 | 30662 | 2) | 0,302456 | -3,073 | 9,44332 | |
| 16005 | 30662 | (G2) | (16005 | 2 | 30662 | 2) | 2,96054 | 2,85423 | 8,14662 | |
| 16005 | 30662 | (G3) | (16005 | 2 | 30662 | 2) | 0,34077 | -2,82255 | 7,9668 | |
| 16005 | 30662 | (G1) | (16005 | 2 | 30662 | 3) | 2,36589 | 2,22143 | 4,93477 | |
| 16005 | 30662 | (G2) | (16005 | 2 | 30662 | 3) | 1,24278 | 0,617561 | 0,381381 | |
| 16005 | 30662 | (G3) | (16005 | 2 | 30662 | 3) | 1,83795 | 1,69858 | 2,88519 | |
| 16005 | 30662 | (G1) | (16005 | 3 | 30662 | 1) | 0,570815 | -0,511048 | 0,26117 | |
| 16005 | 30662 | (G2) | (16005 | 3 | 30662 | 1) | 0,977358 | -0,0243966 | 0,0005952 | |
| 16005 | 30662 | (G3) | (16005 | 3 | 30662 | 1) | 0,988806 | -0,00911231 | 8,30E-05 | |
| 16005 | 30662 | (G1) | (16005 | 3 | 30662 | 2) | 1,26087 | 0,493641 | 0,243681 | |
| 16005 | 30662 | (G2) | (16005 | 3 | 30662 | 2) | 0,200021 | -2,16987 | 4,70834 | |
| 16005 | 30662 | (G3) | (16005 | 3 | 30662 | 2) | 0,843094 | -0,302471 | 0,0914888 | |
| 16005 | 30662 | (G1) | (16005 | 3 | 30662 | 3) | 0,550953 | -1,05551 | 1,11409 | |
| 16005 | 30662 | (G2) | (16005 | 3 | 30662 | 3) | 0,46468 | -1,4915 | 2,22458 | |
| 16005 | 30662 | (G3) | (16005 | 3 | 30662 | 3) | 0,988 | -0,0221534 | 0,00049077 | |
| 17177 | 22099 | (G1) | (17177 | 1 | 22099 | 1) | 1 | 0 | 0 | |
| 17177 | 22099 | (G2) | (17177 | 1 | 22099 | 1) | 1 | 0 | 0 | |
| 17177 | 22099 | (G3) | (17177 | 1 | 22099 | 1) | 1 | 0 | 0 | |
| 17177 | 22099 | (G1) | (17177 | 1 | 22099 | 2) | 0,245373 | -0,720315 | 0,518854 | |
| 17177 | 22099 | (G2) | (17177 | 1 | 22099 | 2) | 1 | 0 | 0 | |
| 17177 | 22099 | (G3) | (17177 | 1 | 22099 | 2) | 1 | 0 | 0 | |
| 17177 | 22099 | (G1) | (17177 | 1 | 22099 | 3) | 0,564425 | -0,521337 | 0,271792 | |
| 17177 | 22099 | (G2) | (17177 | 1 | 22099 | 3) | 7,22047 | 1,05945 | 1,12244 | |
| 17177 | 22099 | (G3) | (17177 | 1 | 22099 | 3) | 1 | 0 | 0 | |
| 17177 | 22099 | (G1) | (17177 | 2 | 22099 | 1) | 0,390457 | -0,907111 | 0,822851 | |
| 17177 | 22099 | (G2) | (17177 | 2 | 22099 | 1) | 1,90142 | 0,777535 | 0,604561 | |
| 17177 | 22099 | (G3) | (17177 | 2 | 22099 | 1) | 1 | 0 | 0 | |
| 17177 | 22099 | (G1) | (17177 | 2 | 22099 | 2) | 0,6159 | -0,970715 | 0,942288 | |
| 17177 | 22099 | (G2) | (17177 | 2 | 22099 | 2) | 1,12096 | 0,23876 | 0,0570065 | |
| 17177 | 22099 | (G3) | (17177 | 2 | 22099 | 2) | 1,9027 | 1,23638 | 1,52862 | |
| 17177 | 22099 | (G1) | (17177 | 2 | 22099 | 3) | 0,760749 | -0,637918 | 0,406939 | |
| 17177 | 22099 | (G2) | (17177 | 2 | 22099 | 3) | 2,29647 | 1,63744 | 2,68121 | |
| 17177 | 22099 | (G3) | (17177 | 2 | 22099 | 3) | 0,558814 | -1,18223 | 1,39768 | |
| 17177 | 22099 | (G1) | (17177 | 3 | 22099 | 1) | 0,564425 | -0,521337 | 0,271792 | |
| 17177 | 22099 | (G2) | (17177 | 3 | 22099 | 1) | 10,4376 | 1,28084 | 1,64054 | |
| 17177 | 22099 | (G3) | (17177 | 3 | 22099 | 1) | 1,77171 | 0,521337 | 0,271792 | |
| 17177 | 22099 | (G1) | (17177 | 3 | 22099 | 2) | 0,653614 | -1,21167 | 1,46815 | |
| 17177 | 22099 | (G2) | (17177 | 3 | 22099 | 2) | 1,26024 | 0,678855 | 0,460844 | |
| 17177 | 22099 | (G3) | (17177 | 3 | 22099 | 2) | 2,49048 | 2,79982 | 7,83902 | |
| 17177 | 22099 | (G1) | (17177 | 3 | 22099 | 3) | 2,35469 | 2,80854 | 7,88789 | |
| 17177 | 22099 | (G2) | (17177 | 3 | 22099 | 3) | 0,424684 | -2,80854 | 7,88789 | |
| 17177 | 22099 | (G3) | (17177 | 3 | 22099 | 3) | 0,423143 | -2,81359 | 7,91628 | |
| 18014 | 30063 | (G1) | (18014 | 1 | 30063 | 1) | 0,7868 | -0,487981 | 0,238126 | |
| 18014 | 30063 | (G2) | (18014 | 1 | 30063 | 1) | 1,60082 | 0,816996 | 0,667483 | |
| 18014 | 30063 | (G3) | (18014 | 1 | 30063 | 1) | 0,708426 | -0,714034 | 0,509844 | |
| 18014 | 30063 | (G1) | (18014 | 1 | 30063 | 2) | 0,760247 | -0,368182 | 0,135558 | |
| 18014 | 30063 | (G2) | (18014 | 1 | 30063 | 2) | 0,988417 | -0,0167266 | 0,00027978 | |
| 18014 | 30063 | (G3) | (18014 | 1 | 30063 | 2) | 1,96852 | 0,975963 | 0,952505 | |
| 18014 | 30063 | (G1) | (18014 | 1 | 30063 | 3) | 4,07542 | 0,720315 | 0,518854 | |
| 18014 | 30063 | (G2) | (18014 | 1 | 30063 | 3) | 0,242627 | -0,726074 | 0,527184 | |
| 18014 | 30063 | (G3) | (18014 | 1 | 30063 | 3) | 1 | 0 | 0 | |
| 18014 | 30063 | (G1) | (18014 | 2 | 30063 | 1) | 3,334 | 3,47776 | 12,0948 | |
| 18014 | 30063 | (G2) | (18014 | 2 | 30063 | 1) | 0,371301 | -2,83131 | 8,01634 | |
| 18014 | 30063 | (G3) | (18014 | 2 | 30063 | 1) | 2,76212 | 3,01018 | 9,06119 | |
| 18014 | 30063 | (G1) | (18014 | 2 | 30063 | 2) | 0,38459 | -2,48901 | 6,19517 | |
| 18014 | 30063 | (G2) | (18014 | 2 | 30063 | 2) | 1,65524 | 1,29462 | 1,67603 | |
| 18014 | 30063 | (G3) | (18014 | 2 | 30063 | 2) | 0,474529 | -1,78189 | 3,17513 | |
| 18014 | 30063 | (G1) | (18014 | 2 | 30063 | 3) | 1,77171 | 0,521337 | 0,271792 | |
| 18014 | 30063 | (G2) | (18014 | 2 | 30063 | 3) | 0,683857 | -0,438097 | 0,191929 | |
| 18014 | 30063 | (G3) | (18014 | 2 | 30063 | 3) | 0,296844 | -1,21116 | 1,46691 | |
| 18014 | 30063 | (G1) | (18014 | 3 | 30063 | 1) | 0,574478 | -1,46938 | 2,15907 | |
| 18014 | 30063 | (G2) | (18014 | 3 | 30063 | 1) | 1,55001 | 1,29261 | 1,67085 | |
| 18014 | 30063 | (G3) | (18014 | 3 | 30063 | 1) | 1,07233 | 0,186817 | 0,0349005 | |
| 18014 | 30063 | (G1) | (18014 | 3 | 30063 | 2) | 1,37216 | 0,684972 | 0,469187 | |
| 18014 | 30063 | (G2) | (18014 | 3 | 30063 | 2) | 1,10718 | 0,212821 | 0,0452926 | |
| 18014 | 30063 | (G3) | (18014 | 3 | 30063 | 2) | 0,544134 | -1,43203 | 2,0507 | |
| 18014 | 30063 | (G1) | (18014 | 3 | 30063 | 3) | 0,138495 | -1,05945 | 1,12244 | |
| 18014 | 30063 | (G2) | (18014 | 3 | 30063 | 3) | 1,75188 | 0,511048 | 0,26117 | |
| 18014 | 30063 | (G3) | (18014 | 3 | 30063 | 3) | 0,245373 | -0,720315 | 0,518854 | |
| 18262 | 27805 | (G1) | (18262 | 1 | 27805 | 1) | 2,95232 | 2,99154 | 8,94933 | |
| 18262 | 27805 | (G2) | (18262 | 1 | 27805 | 1) | 4,23787 | 3,50085 | 12,2559 | |
| 18262 | 27805 | (G3) | (18262 | 1 | 27805 | 1) | 0,367164 | -2,81617 | 7,93083 | |
| 18262 | 27805 | (G1) | (18262 | 1 | 27805 | 2) | 0,484204 | -1,95464 | 3,82062 | |
| 18262 | 27805 | (G2) | (18262 | 1 | 27805 | 2) | 0,440807 | -2,22583 | 4,95431 | |
| 18262 | 27805 | (G3) | (18262 | 1 | 27805 | 2) | 1,83638 | 1,53259 | 2,34882 | |
| 18262 | 27805 | (G1) | (18262 | 1 | 27805 | 3) | 1 | 0 | 0 | |
| 18262 | 27805 | (G2) | (18262 | 1 | 27805 | 3) | 1,46229 | 0,438097 | 0,191929 | |
| 18262 | 27805 | (G3) | (18262 | 1 | 27805 | 3) | 3,36877 | 1,21116 | 1,46691 | |
| 18262 | 27805 | (G1) | (18262 | 2 | 27805 | 1) | 1 | 0 | 0 | |
| 18262 | 27805 | (G2) | (18262 | 2 | 27805 | 1) | 0,749306 | -0,825845 | 0,682019 | |
| 18262 | 27805 | (G3) | (18262 | 2 | 27805 | 1) | 0,858403 | -0,478239 | 0,228712 | |
| 18262 | 27805 | (G1) | (18262 | 2 | 27805 | 2) | 0,847048 | -0,406867 | 0,165541 | |
| 18262 | 27805 | (G2) | (18262 | 2 | 27805 | 2) | 1,27623 | 0,62071 | 0,38528 | |
| 18262 | 27805 | (G3) | (18262 | 2 | 27805 | 2) | 1,70371 | 1,13558 | 1,28953 | |
| 18262 | 27805 | (G1) | (18262 | 2 | 27805 | 3) | 0,525922 | -0,777535 | 0,604561 | |
| 18262 | 27805 | (G2) | (18262 | 2 | 27805 | 3) | 1,01172 | 0,0167266 | 0,00027978 | |
| 18262 | 27805 | (G3) | (18262 | 2 | 27805 | 3) | 2,5611 | 0,907111 | 0,822851 | |
| 18262 | 27805 | (G1) | (18262 | 3 | 27805 | 1) | 0,299878 | -1,56939 | 2,46298 | |
| 18262 | 27805 | (G2) | (18262 | 3 | 27805 | 1) | 0,427137 | -1,06334 | 1,13069 | |
| 18262 | 27805 | (G3) | (18262 | 3 | 27805 | 1) | 1 | 0 | 0 | |
| 18262 | 27805 | (G1) | (18262 | 3 | 27805 | 2) | 1,31536 | 0,368182 | 0,135558 | |
| 18262 | 27805 | (G2) | (18262 | 3 | 27805 | 2) | 0,0968804 | -1,27473 | 1,62495 | |
| 18262 | 27805 | (G3) | (18262 | 3 | 27805 | 2) | 2,5611 | 0,907111 | 0,822851 | |
| 18262 | 27805 | (G1) | (18262 | 3 | 27805 | 3) | 4,07542 | 0,720315 | 0,518854 | |
| 18262 | 27805 | (G2) | (18262 | 3 | 27805 | 3) | 0,570815 | -0,511048 | 0,26117 | |
| 18262 | 27805 | (G3) | (18262 | 3 | 27805 | 3) | 1 | 0 | 0 | |
| 18264 | 27805 | (G1) | (18264 | 1 | 27805 | 1) | 0,299878 | -1,56939 | 2,46298 | |
| 18264 | 27805 | (G2) | (18264 | 1 | 27805 | 1) | 0,422301 | -1,07768 | 1,16139 | |
| 18264 | 27805 | (G3) | (18264 | 1 | 27805 | 1) | 1 | 0 | 0 | |
| 18264 | 27805 | (G1) | (18264 | 1 | 27805 | 2) | 1,31536 | 0,368182 | 0,135558 | |
| 18264 | 27805 | (G2) | (18264 | 1 | 27805 | 2) | 0,0958079 | -1,28084 | 1,64054 | |
| 18264 | 27805 | (G3) | (18264 | 1 | 27805 | 2) | 2,5611 | 0,907111 | 0,822851 | |
| 18264 | 27805 | (G1) | (18264 | 1 | 27805 | 3) | 4,07542 | 0,720315 | 0,518854 | |
| 18264 | 27805 | (G2) | (18264 | 1 | 27805 | 3) | 0,564425 | -0,521337 | 0,271792 | |
| 18264 | 27805 | (G3) | (18264 | 1 | 27805 | 3) | 1 | 0 | 0 | |
| 18264 | 27805 | (G1) | (18264 | 2 | 27805 | 1) | 1 | 0 | 0 | |
| 18264 | 27805 | (G2) | (18264 | 2 | 27805 | 1) | 0,738801 | -0,866952 | 0,751606 | |
| 18264 | 27805 | (G3) | (18264 | 2 | 27805 | 1) | 0,858403 | -0,478239 | 0,228712 | |
| 18264 | 27805 | (G1) | (18264 | 2 | 27805 | 2) | 0,847048 | -0,406867 | 0,165541 | |
| 18264 | 27805 | (G2) | (18264 | 2 | 27805 | 2) | 1,34987 | 0,771377 | 0,595023 | |
| 18264 | 27805 | (G3) | (18264 | 2 | 27805 | 2) | 1,70371 | 1,13558 | 1,28953 | |
| 18264 | 27805 | (G1) | (18264 | 2 | 27805 | 3) | 0,525922 | -0,777535 | 0,604561 | |
| 18264 | 27805 | (G2) | (18264 | 2 | 27805 | 3) | 1 | 0 | 0 | |
| 18264 | 27805 | (G3) | (18264 | 2 | 27805 | 3) | 2,5611 | 0,907111 | 0,822851 | |
| 18264 | 27805 | (G1) | (18264 | 3 | 27805 | 1) | 2,95232 | 2,99154 | 8,94933 | |
| 18264 | 27805 | (G2) | (18264 | 3 | 27805 | 1) | 4,16877 | 3,46375 | 11,9975 | |
| 18264 | 27805 | (G3) | (18264 | 3 | 27805 | 1) | 0,367164 | -2,81617 | 7,93083 | |
| 18264 | 27805 | (G1) | (18264 | 3 | 27805 | 2) | 0,484204 | -1,95464 | 3,82062 | |
| 18264 | 27805 | (G2) | (18264 | 3 | 27805 | 2) | 0,435032 | -2,26311 | 5,12169 | |
| 18264 | 27805 | (G3) | (18264 | 3 | 27805 | 2) | 1,83638 | 1,53259 | 2,34882 | |
| 18264 | 27805 | (G1) | (18264 | 3 | 27805 | 3) | 1 | 0 | 0 | |
| 18264 | 27805 | (G2) | (18264 | 3 | 27805 | 3) | 1,44555 | 0,424857 | 0,180503 | |
| 18264 | 27805 | (G3) | (18264 | 3 | 27805 | 3) | 3,36877 | 1,21116 | 1,46691 | |
| 18266 | 27805 | (G1) | (18266 | 1 | 27805 | 1) | 0,299878 | -1,56939 | 2,46298 | |
| 18266 | 27805 | (G2) | (18266 | 1 | 27805 | 1) | 0,525922 | -0,777535 | 0,604561 | |
| 18266 | 27805 | (G3) | (18266 | 1 | 27805 | 1) | 1 | 0 | 0 | |
| 18266 | 27805 | (G1) | (18266 | 1 | 27805 | 2) | 1,31536 | 0,368182 | 0,135558 | |
| 18266 | 27805 | (G2) | (18266 | 1 | 27805 | 2) | 0,0958079 | -1,28084 | 1,64054 | |
| 18266 | 27805 | (G3) | (18266 | 1 | 27805 | 2) | 2,5611 | 0,907111 | 0,822851 | |
| 18266 | 27805 | (G1) | (18266 | 1 | 27805 | 3) | 4,07542 | 0,720315 | 0,518854 | |
| 18266 | 27805 | (G2) | (18266 | 1 | 27805 | 3) | 0,564425 | -0,521337 | 0,271792 | |
| 18266 | 27805 | (G3) | (18266 | 1 | 27805 | 3) | 1 | 0 | 0 | |
| 18266 | 27805 | (G1) | (18266 | 2 | 27805 | 1) | 1 | 0 | 0 | |
| 18266 | 27805 | (G2) | (18266 | 2 | 27805 | 1) | 0,698935 | -1,03171 | 1,06443 | |
| 18266 | 27805 | (G3) | (18266 | 2 | 27805 | 1) | 0,858403 | -0,478239 | 0,228712 | |
| 18266 | 27805 | (G1) | (18266 | 2 | 27805 | 2) | 0,847048 | -0,406867 | 0,165541 | |
| 18266 | 27805 | (G2) | (18266 | 2 | 27805 | 2) | 1,34987 | 0,771377 | 0,595023 | |
| 18266 | 27805 | (G3) | (18266 | 2 | 27805 | 2) | 1,70371 | 1,13558 | 1,28953 | |
| 18266 | 27805 | (G1) | (18266 | 2 | 27805 | 3) | 0,525922 | -0,777535 | 0,604561 | |
| 18266 | 27805 | (G2) | (18266 | 2 | 27805 | 3) | 1 | 0 | 0 | |
| 18266 | 27805 | (G3) | (18266 | 2 | 27805 | 3) | 2,5611 | 0,907111 | 0,822851 | |
| 18266 | 27805 | (G1) | (18266 | 3 | 27805 | 1) | 2,95232 | 2,99154 | 8,94933 | |
| 18266 | 27805 | (G2) | (18266 | 3 | 27805 | 1) | 4,16877 | 3,46375 | 11,9975 | |
| 18266 | 27805 | (G3) | (18266 | 3 | 27805 | 1) | 0,367164 | -2,81617 | 7,93083 | |
| 18266 | 27805 | (G1) | (18266 | 3 | 27805 | 2) | 0,484204 | -1,95464 | 3,82062 | |
| 18266 | 27805 | (G2) | (18266 | 3 | 27805 | 2) | 0,435032 | -2,26311 | 5,12169 | |
| 18266 | 27805 | (G3) | (18266 | 3 | 27805 | 2) | 1,83638 | 1,53259 | 2,34882 | |
| 18266 | 27805 | (G1) | (18266 | 3 | 27805 | 3) | 1 | 0 | 0 | |
| 18266 | 27805 | (G2) | (18266 | 3 | 27805 | 3) | 1,44555 | 0,424857 | 0,180503 | |
| 18266 | 27805 | (G3) | (18266 | 3 | 27805 | 3) | 3,36877 | 1,21116 | 1,46691 | |
| 18267 | 27805 | (G1) | (18267 | 1 | 27805 | 1) | 0,299878 | -1,56939 | 2,46298 | |
| 18267 | 27805 | (G2) | (18267 | 1 | 27805 | 1) | 0,525922 | -0,777535 | 0,604561 | |
| 18267 | 27805 | (G3) | (18267 | 1 | 27805 | 1) | 1 | 0 | 0 | |
| 18267 | 27805 | (G1) | (18267 | 1 | 27805 | 2) | 1,31536 | 0,368182 | 0,135558 | |
| 18267 | 27805 | (G2) | (18267 | 1 | 27805 | 2) | 0,0958079 | -1,28084 | 1,64054 | |
| 18267 | 27805 | (G3) | (18267 | 1 | 27805 | 2) | 2,5611 | 0,907111 | 0,822851 | |
| 18267 | 27805 | (G1) | (18267 | 1 | 27805 | 3) | 4,07542 | 0,720315 | 0,518854 | |
| 18267 | 27805 | (G2) | (18267 | 1 | 27805 | 3) | 0,564425 | -0,521337 | 0,271792 | |
| 18267 | 27805 | (G3) | (18267 | 1 | 27805 | 3) | 1 | 0 | 0 | |
| 18267 | 27805 | (G1) | (18267 | 2 | 27805 | 1) | 1 | 0 | 0 | |
| 18267 | 27805 | (G2) | (18267 | 2 | 27805 | 1) | 0,698935 | -1,03171 | 1,06443 | |
| 18267 | 27805 | (G3) | (18267 | 2 | 27805 | 1) | 0,858403 | -0,478239 | 0,228712 | |
| 18267 | 27805 | (G1) | (18267 | 2 | 27805 | 2) | 0,847048 | -0,406867 | 0,165541 | |
| 18267 | 27805 | (G2) | (18267 | 2 | 27805 | 2) | 1,34987 | 0,771377 | 0,595023 | |
| 18267 | 27805 | (G3) | (18267 | 2 | 27805 | 2) | 1,70371 | 1,13558 | 1,28953 | |
| 18267 | 27805 | (G1) | (18267 | 2 | 27805 | 3) | 0,525922 | -0,777535 | 0,604561 | |
| 18267 | 27805 | (G2) | (18267 | 2 | 27805 | 3) | 1 | 0 | 0 | |
| 18267 | 27805 | (G3) | (18267 | 2 | 27805 | 3) | 2,5611 | 0,907111 | 0,822851 | |
| 18267 | 27805 | (G1) | (18267 | 3 | 27805 | 1) | 2,95232 | 2,99154 | 8,94933 | |
| 18267 | 27805 | (G2) | (18267 | 3 | 27805 | 1) | 4,16877 | 3,46375 | 11,9975 | |
| 18267 | 27805 | (G3) | (18267 | 3 | 27805 | 1) | 0,367164 | -2,81617 | 7,93083 | |
| 18267 | 27805 | (G1) | (18267 | 3 | 27805 | 2) | 0,484204 | -1,95464 | 3,82062 | |
| 18267 | 27805 | (G2) | (18267 | 3 | 27805 | 2) | 0,435032 | -2,26311 | 5,12169 | |
| 18267 | 27805 | (G3) | (18267 | 3 | 27805 | 2) | 1,83638 | 1,53259 | 2,34882 | |
| 18267 | 27805 | (G1) | (18267 | 3 | 27805 | 3) | 1 | 0 | 0 | |
| 18267 | 27805 | (G2) | (18267 | 3 | 27805 | 3) | 1,44555 | 0,424857 | 0,180503 | |
| 18267 | 27805 | (G3) | (18267 | 3 | 27805 | 3) | 3,36877 | 1,21116 | 1,46691 | |
| 18413 | 21070 | (G1) | (18413 | 1 | 21070 | 1) | 0,416656 | -1,09367 | 1,19611 | |
| 18413 | 21070 | (G2) | (18413 | 1 | 21070 | 1) | 0,555699 | -0,938593 | 0,880956 | |
| 18413 | 21070 | (G3) | (18413 | 1 | 21070 | 1) | 2,01725 | 1,14635 | 1,31413 | |
| 18413 | 21070 | (G1) | (18413 | 1 | 21070 | 2) | 4,99348 | 1,66409 | 2,76921 | |
| 18413 | 21070 | (G2) | (18413 | 1 | 21070 | 2) | 0,135217 | -1,07217 | 1,14955 | |
| 18413 | 21070 | (G3) | (18413 | 1 | 21070 | 2) | 0,138495 | -1,05945 | 1,12244 | |
| 18413 | 21070 | (G1) | (18413 | 1 | 21070 | 3) | 0,988549 | -0,00466932 | 2,18E-05 | |
| 18413 | 21070 | (G2) | (18413 | 1 | 21070 | 3) | 0,977358 | -0,00928557 | 8,62E-05 | |
| 18413 | 21070 | (G3) | (18413 | 1 | 21070 | 3) | 1 | 0 | 0 | |
| 18413 | 21070 | (G1) | (18413 | 2 | 21070 | 1) | 0,875651 | -0,387819 | 0,150404 | |
| 18413 | 21070 | (G2) | (18413 | 2 | 21070 | 1) | 0,581103 | -1,69093 | 2,85924 | |
| 18413 | 21070 | (G3) | (18413 | 2 | 21070 | 1) | 2,24136 | 2,49527 | 6,22636 | |
| 18413 | 21070 | (G1) | (18413 | 2 | 21070 | 2) | 3,23405 | 2,22224 | 4,93837 | |
| 18413 | 21070 | (G2) | (18413 | 2 | 21070 | 2) | 1,37523 | 0,539627 | 0,291198 | |
| 18413 | 21070 | (G3) | (18413 | 2 | 21070 | 2) | 1,12096 | 0,23876 | 0,0570065 | |
| 18413 | 21070 | (G1) | (18413 | 2 | 21070 | 3) | 0,988549 | -0,00466932 | 2,18E-05 | |
| 18413 | 21070 | (G2) | (18413 | 2 | 21070 | 3) | 0,977358 | -0,00928557 | 8,62E-05 | |
| 18413 | 21070 | (G3) | (18413 | 2 | 21070 | 3) | 1 | 0 | 0 | |
| 18413 | 21070 | (G1) | (18413 | 3 | 21070 | 1) | 0,409948 | -2,81333 | 7,91481 | |
| 18413 | 21070 | (G2) | (18413 | 3 | 21070 | 1) | 2,51567 | 2,83823 | 8,05554 | |
| 18413 | 21070 | (G3) | (18413 | 3 | 21070 | 1) | 0,369974 | -2,98512 | 8,91091 | |
| 18413 | 21070 | (G1) | (18413 | 3 | 21070 | 2) | 2,47769 | 1,91585 | 3,67048 | |
| 18413 | 21070 | (G2) | (18413 | 3 | 21070 | 2) | 0,681729 | -0,901918 | 0,813455 | |
| 18413 | 21070 | (G3) | (18413 | 3 | 21070 | 2) | 1 | 0 | 0 | |
| 18413 | 21070 | (G1) | (18413 | 3 | 21070 | 3) | 0,988549 | -0,00466932 | 2,18E-05 | |
| 18413 | 21070 | (G2) | (18413 | 3 | 21070 | 3) | 0,977358 | -0,00928557 | 8,62E-05 | |
| 18413 | 21070 | (G3) | (18413 | 3 | 21070 | 3) | 1 | 0 | 0 | |
| 21014 | 24281 | (G1) | (21014 | 1 | 24281 | 1) | 1,08412 | 0,0327342 | 0,00107153 | |
| 21014 | 24281 | (G2) | (21014 | 1 | 24281 | 1) | 1,18368 | 0,0683391 | 0,00467023 | |
| 21014 | 24281 | (G3) | (21014 | 1 | 24281 | 1) | 1,46991 | 0,156078 | 0,0243604 | |
| 21014 | 24281 | (G1) | (21014 | 1 | 24281 | 2) | 1,08412 | 0,0327342 | 0,00107153 | |
| 21014 | 24281 | (G2) | (21014 | 1 | 24281 | 2) | 1,18368 | 0,0683391 | 0,00467023 | |
| 21014 | 24281 | (G3) | (21014 | 1 | 24281 | 2) | 1,46991 | 0,156078 | 0,0243604 | |
| 21014 | 24281 | (G1) | (21014 | 1 | 24281 | 3) | 1,08412 | 0,0327342 | 0,00107153 | |
| 21014 | 24281 | (G2) | (21014 | 1 | 24281 | 3) | 1,18368 | 0,0683391 | 0,00467023 | |
| 21014 | 24281 | (G3) | (21014 | 1 | 24281 | 3) | 1,46991 | 0,156078 | 0,0243604 | |
| 21014 | 24281 | (G1) | (21014 | 2 | 24281 | 1) | 1,08412 | 0,0327342 | 0,00107153 | |
| 21014 | 24281 | (G2) | (21014 | 2 | 24281 | 1) | 1,18368 | 0,0683391 | 0,00467023 | |
| 21014 | 24281 | (G3) | (21014 | 2 | 24281 | 1) | 1,46991 | 0,156078 | 0,0243604 | |
| 21014 | 24281 | (G1) | (21014 | 2 | 24281 | 2) | 3,51951 | 2,14998 | 4,62243 | |
| 21014 | 24281 | (G2) | (21014 | 2 | 24281 | 2) | 1,20931 | 0,364436 | 0,132814 | |
| 21014 | 24281 | (G3) | (21014 | 2 | 24281 | 2) | 0,357809 | -1,59551 | 2,54564 | |
| 21014 | 24281 | (G1) | (21014 | 2 | 24281 | 3) | 1,29086 | 0,657338 | 0,432093 | |
| 21014 | 24281 | (G2) | (21014 | 2 | 24281 | 3) | 0,946958 | -0,144108 | 0,0207672 | |
| 21014 | 24281 | (G3) | (21014 | 2 | 24281 | 3) | 1,78598 | 1,38772 | 1,92577 | |
| 21014 | 24281 | (G1) | (21014 | 3 | 24281 | 1) | 1,08412 | 0,0327342 | 0,00107153 | |
| 21014 | 24281 | (G2) | (21014 | 3 | 24281 | 1) | 1,18368 | 0,0683391 | 0,00467023 | |
| 21014 | 24281 | (G3) | (21014 | 3 | 24281 | 1) | 1,46991 | 0,156078 | 0,0243604 | |
| 21014 | 24281 | (G1) | (21014 | 3 | 24281 | 2) | 0,310181 | -2,84273 | 8,08109 | 9 |
| 21014 | 24281 | (G2) | (21014 | 3 | 24281 | 2) | 0,244981 | -3,06189 | 9,37518 | 9 |
| 221014 | 24281 | (G3) | (21014 | 3 | 24281 | 2) | 0,109235 | -3,10685 | 9,65251 | 9 |
| 21014 | 24281 | (G1) | (21014 | 3 | 24281 | 3) | 1,21226 | 0,57824 | 0,334361 | |
| 21014 | 24281 | (G2) | (21014 | 3 | 24281 | 3) | 2,55447 | 2,66556 | 7,10522 | 9 |
| 21014 | 24281 | (G3) | (21014 | 3 | 24281 | 3) | 2,65162 | 2,71831 | 7,38922 | 9 |
| 21689 | 23137 | (G1) | (21689 | 1 | 23137 | 1) | 1,0123 | 0,0237601 | 0,00056454 | |
| 21689 | 23137 | (G2) | (21689 | 1 | 23137 | 1) | 2,53982 | 2,08023 | 4,32736 | |
| 21689 | 23137 | (G3) | (21689 | 1 | 23137 | 1) | 1,10215 | 0,220415 | 0,0485829 | |
| 21689 | 23137 | (G1) | (21689 | 1 | 23137 | 2) | 1,71928 | 1,74323 | 3,03885 | |
| 21689 | 23137 | (G2) | (21689 | 1 | 23137 | 2) | 1,34727 | 0,974061 | 0,948795 | |
| 21689 | 23137 | (G3) | (21689 | 1 | 23137 | 2) | 1,25294 | 0,749971 | 0,562456 | |
| 21689 | 23137 | (G1) | (21689 | 1 | 23137 | 3) | 0,375235 | -2,94009 | 8,64412 | 10 |
| 21689 | 23137 | (G2) | (21689 | 1 | 23137 | 3) | 0,312136 | -3,22997 | 10,4327 | 10 |
| 21689 | 23137 | (G3) | (21689 | 1 | 23137 | 3) | 0,370113 | -2,89695 | 8,39234 | 10 |
| 21689 | 23137 | (G1) | (21689 | 2 | 23137 | 1) | 2,39606 | 1,0923 | 1,19311 | |
| 21689 | 23137 | (G2) | (21689 | 2 | 23137 | 1) | 1,65754 | 0,706762 | 0,499512 | |
| 21689 | 23137 | (G3) | (21689 | 2 | 23137 | 1) | 1 | 0 | 0 | |
| 21689 | 23137 | (G1) | (21689 | 2 | 23137 | 2) | 1,64437 | 0,995801 | 0,99162 | |
| 21689 | 23137 | (G2) | (21689 | 2 | 23137 | 2) | 0,892819 | -0,225922 | 0,0510408 | |
| 21689 | 23137 | (G3) | (21689 | 2 | 23137 | 2) | 3,33469 | 1,56939 | 2,46298 | |
| 21689 | 23137 | (G1) | (21689 | 2 | 23137 | 3) | 1,012 | 0,0204637 | 0,00041876 | |
| 21689 | 23137 | (G2) | (21689 | 2 | 23137 | 3) | 1,33078 | 0,383785 | 0,147291 | |
| 21689 | 23137 | (G3) | (21689 | 2 | 23137 | 3) | 3,37242 | 1,87352 | 3,51007 | |
| 21689 | 23137 | (G1) | (21689 | 3 | 23137 | 1) | 1,01119 | 0,00451366 | 2,04E-05 | |
| 21689 | 23137 | (G2) | (21689 | 3 | 23137 | 1) | 1,01119 | 0,00451366 | 2,04E-05 | |
| 21689 | 23137 | (G3) | (21689 | 3 | 23137 | 1) | 1 | 0 | 0 | |
| 21689 | 23137 | (G1) | (21689 | 3 | 23137 | 2) | 1,01132 | 0,00911231 | 8,30E-05 | |
| 21689 | 23137 | (G2) | (21689 | 3 | 23137 | 2) | 1,01132 | 0,00911231 | 8,30E-05 | |
| 21689 | 23137 | (G3) | (21689 | 3 | 23137 | 2) | 1 | 0 | 0 | |
| 21689 | 23137 | (G1) | (21689 | 3 | 23137 | 3) | 1,01119 | 0,00451366 | 2,04E-05 | |
| 21689 | 23137 | (G2) | (21689 | 3 | 23137 | 3) | 4,12156 | 0,726074 | 0,527184 | |
| 21689 | 23137 | (G3) | (21689 | 3 | 23137 | 3) | 1 | 0 | 0 | |
| 22109 | 24450 | (G1) | (22109 | 1 | 24450 | 1) | 4,07542 | 0,720315 | 0,518854 | |
| 22109 | 24450 | (G2) | (22109 | 1 | 24450 | 1) | 1,792 | 0,531686 | 0,28269 | |
| 22109 | 24450 | (G3) | (22109 | 1 | 24450 | 1) | 0,0728376 | -1,44553 | 2,08956 | |
| 22109 | 24450 | (G1) | (22109 | 1 | 24450 | 2) | 0,564425 | -0,521337 | 0,271792 | |
| 22109 | 24450 | (G2) | (22109 | 1 | 24450 | 2) | 0,0968804 | -1,27473 | 1,62495 | |
| 22109 | 24450 | (G3) | (22109 | 1 | 24450 | 2) | 0,35142 | -1,3386 | 1,79185 | |
| 22109 | 24450 | (G1) | (22109 | 1 | 24450 | 3) | 4,07542 | 0,720315 | 0,518854 | |
| 22109 | 24450 | (G2) | (22109 | 1 | 24450 | 3) | 0,24812 | -0,714596 | 0,510647 | |
| 22109 | 24450 | (G3) | (22109 | 1 | 24450 | 3) | 0,245373 | -0,720315 | 0,518854 | |
| 22109 | 24450 | (G1) | (22109 | 2 | 24450 | 1) | 1 | 0 | 0 | |
| 22109 | 24450 | (G2) | (22109 | 2 | 24450 | 1) | 0,576877 | -0,879675 | 0,773828 | |
| 22109 | 24450 | (G3) | (22109 | 2 | 24450 | 1) | 1 | 0 | 0 | |
| 22109 | 24450 | (G1) | (22109 | 2 | 24450 | 2) | 0,396817 | -1,70693 | 2,91363 | |
| 22109 | 24450 | (G2) | (22109 | 2 | 24450 | 2) | 0,513829 | -1,57917 | 2,49379 | |
| 22109 | 24450 | (G3) | (22109 | 2 | 24450 | 2) | 0,372208 | -2,10819 | 4,44445 | |
| 22109 | 24450 | (G1) | (22109 | 2 | 24450 | 3) | 1 | 0 | 0 | |
| 22109 | 24450 | (G2) | (22109 | 2 | 24450 | 3) | 0,474236 | -1,55072 | 2,40473 | |
| 22109 | 24450 | (G3) | (22109 | 2 | 24450 | 3) | 0,907314 | -0,220415 | 0,0485829 | |
| 22109 | 24450 | (G1) | (22109 | 3 | 24450 | 1) | 0,82371 | -0,538532 | 0,290017 | |
| 22109 | 24450 | (G2) | (22109 | 3 | 24450 | 1) | 2,53164 | 1 ,96566 | 3,86383 | |
| 22109 | 24450 | (G3) | (22109 | 3 | 24450 | 1) | 1,50263 | 0,89411 | 0,799433 | |
| 22109 | 24450 | (G1) | (22109 | 3 | 24450 | 2) | 3,09935 | 3,31361 | 10,98 | 11 |
| 22109 | 24450 | (G2) | (22109 | 3 | 24450 | 2) | 2,53996 | 2,85499 | 8,15099 | 11 |
| 22109 | 24450 | (G3) | (22109 | 3 | 24450 | 2) | 2,72323 | 3,08036 | 9,48862 | 11 |
| 22109 | 24450 | (G1) | (22109 | 3 | 24450 | 3) | 0,372873 | -2,3202 | 5,38334 | |
| 22109 | 24450 | (G2) | (22109 | 3 | 24450 | 3) | 0,570766 | -1,36245 | 1,85628 | |
| 22109 | 24450 | (G3) | (22109 | 3 | 24450 | 3) | 0,716495 | -0,812023 | 0,659381 | |
| 22109 | 29832 | (G1) | (22109 | 1 | 29832 | 1) | 1 | 0 | 0 | |
| 22109 | 29832 | (G2) | (22109 | 1 | 29832 | 1) | 1,01119 | 0,00451366 | 2,04E-05 | |
| 22109 | 29832 | (G3) | (22109 | 1 | 29832 | 1) | 0,138495 | -1,05945 | 1,12244 | |
| 22109 | 29832 | (G1) | (22109 | 1 | 29832 | 2) | 4,07542 | 0,720315 | 0,518854 | |
| 22109 | 29832 | (G2) | (22109 | 1 | 29832 | 2) | 1,01145 | 0,0121294 | 0,00014712 | |
| 22109 | 29832 | (G3) | (22109 | 1 | 29832 | 2) | 0,564425 | -0,521337 | 0,271792 | |
| 22109 | 29832 | (G1) | (22109 | 1 | 29832 | 3) | 1 | 0 | 0 | |
| 22109 | 29832 | (G2) | (22109 | 1 | 29832 | 3) | 0,0968804 | -1,27473 | 1,62495 | |
| 22109 | 29832 | (G3) | (22109 | 1 | 29832 | 3) | 0,169259 | -1,86056 | 3,46169 | |
| 22109 | 29832 | (G1) | (22109 | 2 | 29832 | 1) | 0,296844 | -1,21116 | 1,46691 | |
| 22109 | 29832 | (G2) | (22109 | 2 | 29832 | 1) | 0,0968804 | -1,27473 | 1,62495 | |
| 22109 | 29832 | (G3) | (22109 | 2 | 29832 | 1) | 4,07542 | 0,720315 | 0,518854 | |
| 22109 | 29832 | (G1) | (22109 | 2 | 29832 | 2) | 1,15023 | 0,264209 | 0,0698063 | |
| 22109 | 29832 | (G2) | (22109 | 2 | 29832 | 2) | 0,693605 | -0,8343 | 0,696056 | |
| 22109 | 29832 | (G3) | (22109 | 2 | 29832 | 2) | 0,497179 | -1,52238 | 2,31764 | |
| 22109 | 29832 | (G1) | (22109 | 2 | 29832 | 3) | 0,680202 | -0,974651 | 0,949945 | |
| 22109 | 29832 | (G2) | (22109 | 2 | 29832 | 3) | 0,442613 | -2,03482 | 4,14048 | |
| 22109 | 29832 | (G3) | (22109 | 2 | 29832 | 3) | 0,680202 | -0,974651 | 0,949945 | |
| 22109 | 29832 | (G1) | (22109 | 3 | 29832 | 1) | 0,0584866 | -1,57703 | 2,48701 | |
| 22109 | 29832 | (G2) | (22109 | 3 | 29832 | 1) | 1,01158 | 0,0145838 | 0,00021269 | |
| 22109 | 29832 | (G3) | (22109 | 3 | 29832 | 1) | 0,691778 | -0,424857 | 0,180503 | |
| 22109 | 29832 | (G1) | (22109 | 3 | 29832 | 2) | 0,598949 | -1,41691 | 2,00765 | |
| 22109 | 29832 | (G2) | (22109 | 3 | 29832 | 2) | 1,09857 | 0,233875 | 0,0546976 | |
| 22109 | 29832 | (G3) | (22109 | 3 | 29832 | 2) | 0,876692 | -0,362506 | 0,13141 | |
| 22109 | 29832 | (G1) | (22109 | 3 | 29832 | 3) | 2,37414 | 2,81993 | 7,95202 | 12 |
| 22109 | 29832 | (G2) | (22109 | 3 | 29832 | 3) | 2,44519 | 2,89655 | 8,39 | 12 |
| 22109 | 29832 | (G3) | (22109 | 3 | 29832 | 3) | 3,01619 | 3,45915 | 11,9657 | 12 |
| 22112 | 29832 | (G1) | (22112 | 1 | 29832 | 1) | 0,0584866 | -1,57703 | 2,48701 | |
| 22112 | 29832 | (G2) | (22112 | 1 | 29832 | 1) | 1 | 0 | 0 | |
| 22112 | 29832 | (G3) | (22112 | 1 | 29832 | 1) | 0,691778 | -0,424857 | 0,180503 | |
| 22112 | 29832 | (G1) | (22112 | 1 | 29832 | 2) | 0,598949 | -1,41691 | 2,00765 | |
| 22112 | 29832 | (G2) | (22112 | 1 | 29832 | 2) | 1,08398 | 0,200739 | 0,0402963 | |
| 22112 | 29832 | (G3) | (22112 | 1 | 29832 | 2) | 0,876692 | -0,362506 | 0,13141 | |
| 22112 | 29832 | (G1) | (22112 | 1 | 29832 | 3) | 2,37414 | 2,81993 | 7,95202 | 12 |
| 22112 | 29832 | (G2) | (22112 | 1 | 29832 | 3) | 2,38742 | 2,82759 | 7,99529 | 12 |
| 22112 | 29832 | (G3) | (22112 | 1 | 29832 | 3) | 3,01619 | 3,45915 | 11,9657 | 12 |
| 22112 | 29832 | (G1) | (22112 | 2 | 29832 | 1) | 0,296844 | -1,21116 | 1,46691 | |
| 22112 | 29832 | (G2) | (22112 | 2 | 29832 | 1) | 0,0958079 | -1,28084 | 1,64054 | |
| 22112 | 29832 | (G3) | (22112 | 2 | 29832 | 1) | 4,07542 | 0,720315 | 0,518854 | |
| 22112 | 29832 | (G1) | (22112 | 2 | 29832 | 2) | 1,15023 | 0,264209 | 0,0698063 | |
| 22112 | 29832 | (G2) | (22112 | 2 | 29832 | 2) | 0,68497 | -0,863219 | 0,745146 | |
| 22112 | 29832 | (G3) | (22112 | 2 | 29832 | 2) | 0,497179 | -1,52238 | 2,31764 | |
| 22112 | 29832 | (G1) | (22112 | 2 | 29832 | 3) | 0,680202 | -0,974651 | 0,949945 | |
| 22112 | 29832 | (G2) | (22112 | 2 | 29832 | 3) | 0,481679 | -1,86575 | 3,48103 | |
| 22112 | 29832 | (G3) | (22112 | 2 | 29832 | 3) | 0,680202 | -0,974651 | 0,949945 | |
| 22112 | 29832 | (G1) | (22112 | 3 | 29832 | 1) | 1 | 0 | 0 | |
| 22112 | 29832 | (G2) | (22112 | 3 | 29832 | 1) | 1 | 0 | 0 | |
| 22112 | 29832 | (G3) | (22112 | 3 | 29832 | 1) | 0,138495 | -1,05945 | 1,12244 | |
| 22112 | 29832 | (G1) | (22112 | 3 | 29832 | 2) | 4,07542 | 0,720315 | 0,518854 | |
| 22112 | 29832 | (G2) | (22112 | 3 | 29832 | 2) | 1 | 0 | 0 | |
| 22112 | 29832 | (G3) | (22112 | 3 | 29832 | 2) | 0,564425 | -0,521337 | 0,271792 | |
| 22112 | 29832 | (G1) | (22112 | 3 | 29832 | 3) | 1 | 0 | 0 | |
| 22112 | 29832 | (G2) | (22112 | 3 | 29832 | 3) | 0,0958079 | -1,28084 | 1,64054 | |
| 22112 | 29832 | (G3) | (22112 | 3 | 29832 | 3) | 0,169259 | -1,86056 | 3,46169 | |
| 22113 | 29832 | (G1) | (22113 | 1 | 29832 | 1) | 1 | 0 | 0 | |
| 22113 | 29832 | (G2) | (22113 | 1 | 29832 | 1) | 1 | 0 | 0 | |
| 22113 | 29832 | (G3) | (22113 | 1 | 29832 | 1) | 0,138495 | -1,05945 | 1,12244 | |
| 22113 | 29832 | (G1) | (22113 | 1 | 29832 | 2) | 4,07542 | 0,720315 | 0,518854 | |
| 22113 | 29832 | (G2) | (22113 | 1 | 29832 | 2) | 1 | 0 | 0 | |
| 22113 | 29832 | (G3) | (22113 | 1 | 29832 | 2) | 0,564425 | -0,521337 | 0,271792 | |
| 22113 | 29832 | (G1) | (22113 | 1 | 29832 | 3) | 1 | 0 | 0 | |
| 22113 | 29832 | (G2) | (22113 | 1 | 29832 | 3) | 0,0958079 | -1,28084 | 1,64054 | |
| 22113 | 29832 | (G3) | (22113 | 1 | 29832 | 3) | 0,169259 | -1,86056 | 3,46169 | |
| 22113 | 29832 | (G1) | (22113 | 2 | 29832 | 1) | 0,296844 | -1,21116 | 1,46691 | |
| 22113 | 29832 | (G2) | (22113 | 2 | 29832 | 1) | 0,0958079 | -1,28084 | 1,64054 | |
| 22113 | 29832 | (G3) | (22113 | 2 | 29832 | 1) | 4,07542 | 0,720315 | 0,518854 | |
| 22113 | 29832 | (G1) | (22113 | 2 | 29832 | 2) | 1,15023 | 0,264209 | 0,0698063 | |
| 22113 | 29832 | (G2) | (22113 | 2 | 29832 | 2) | 0,68497 | -0,863219 | 0,745146 | |
| 22113 | 29832 | (G3) | (22113 | 2 | 29832 | 2) | 0,497179 | -1,52238 | 2,31764 | |
| 22113 | 29832 | (G1) | (22113 | 2 | 29832 | 3) | 0,680202 | -0,974651 | 0,949945 | |
| 22113 | 29832 | (G2) | (22113 | 2 | 29832 | 3) | 0,481679 | -1,86575 | 3,48103 | |
| 22113 | 29832 | (G3) | (22113 | 2 | 29832 | 3) | 0,680202 | -0,974651 | 0,949945 | |
| 22113 | 29832 | (G1) | (22113 | 3 | 29832 | 1) | 0,0584866 | -1,57703 | 2,48701 | |
| 22113 | 29832 | (G2) | (22113 | 3 | 29832 | 1) | 1 | 0 | 0 | |
| 22113 | 29832 | (G3) | (22113 | 3 | 29832 | 1) | 0,691778 | -0,424857 | 0,180503 | |
| 22113 | 29832 | (G1) | (22113 | 3 | 29832 | 2) | 0,598949 | -1,41691 | 2,00765 | |
| 22113 | 29832 | (G2) | (22113 | 3 | 29832 | 2) | 1,08398 | 0,200739 | 0,0402963 | |
| 22113 | 29832 | (G3) | (22113 | 3 | 2929832 | 2) | 0,876692 | -0,362506 | 0,13141 | |
| 22113 | 29832 | (G1) | (22113 | 3 | 29832 | 3) | 2,37414 | 2,81993 | 7,95202 | 12 |
| 22113 | 29832 | (G2) | (22113 | 3 | 29832 | 3) | 2,38742 | 2,82759 | 7,99529 | 12 |
| 22113 | 29832 | (G3) | (22113 | 3 | 29832 | 3) | 3,01619 | 3,45915 | 11,9657 | 12 |
| 22114 | 29832 | (G1) | (22114 | 1 | 29832 | 1) | 0,0728376 | -1,44553 | 2,08956 | |
| 22114 | 29832 | (G2) | (22114 | 1 | 29832 | 1) | 1 | 0 | 0 | |
| 22114 | 29832 | (G3) | (22114 | 1 | 29832 | 1) | 0,691778 | -0,424857 | 0,180503 | |
| 22114 | 29832 | (G1) | (22114 | 1 | 29832 | 2) | 0,598949 | -1,41691 | 2,00765 | |
| 22114 | 29832 | (G2) | (22114 | 1 | 29832 | 2) | 1,08398 | 0,200739 | 0,0402963 | |
| 22114 | 29832 | (G3) | (22114 | 1 | 29832 | 2) | 0,876692 | -0,362506 | 0,13141 | |
| 22114 | 29832 | (G1) | (22114 | 1 | 29832 | 3) | 2,37414 | -2,81993 | 7,95202 | 12 |
| 22114 | 29832 | (G2) | (22114 | 1 | 29832 | 3) | 2,38742 | 2,82759 | 7,99529 | 12 |
| 22114 | 29832 | (G3) | (22114 | 1 | 29832 | 3) | 3,01619 | 3,45915 | 11,9657 | 12 |
| 22114 | 29832 | (G1) | (22114 | 2 | 29832 | 1) | 0,238358 | -1.46172 | 2,13662 | |
| 22114 | 29832 | (G2) | (22114 | 2 | 29832 | 1) | 0,0958079 | -1,28084 | 1,64054 | |
| 22114 | 29832 | (G3) | (22114 | 2 | 29832 | 1) | 4,07542 | 0,720315 | 0,518854 | |
| 22114 | 29832 | (G1) | (22114 | 2 | 29832 | 2) | 1,15023 | 0,264209 | 0,0698063 | |
| 22114 | 29832 | (G2) | (22114 | 2 | 29832 | 2) | 0,68497 | -0,863219 | 0,745146 | |
| 22114 | 29832 | (G3) | (22114 | 2 | 29832 | 2) | 0,497179 | -1,52238 | 2,31764 | |
| 22114 | 29832 | (G1) | (22114 | 2 | 29832 | 3) | 0,680202 | -0,974651 | 0,949945 | |
| 22114 | 29832 | (G2) | (22114 | 2 | 29832 | 3) | 0,481679 | -1,86575 | 3,48103 | |
| 22114 | 29832 | (G3) | (22114 | 2 | 29832 | 3) | 0,680202 | -0,974651 | 0,949945 | |
| 22114 | 29832 | (G1) | (22114 | 3 | 29832 | 1) | 1 | 0 | 0 | |
| 22114 | 29832 | (G2) | (22114 | 3 | 29832 | 1) | 1 | 0 | 0 | |
| 22114 | 29832 | (G3) | (22114 | 3 | 29832 | 1) | 0,138495 | -1,05945 | 1,12244 | |
| 22114 | 29832 | (G1) | (22114 | 3 | 29832 | 2) | 4,07542 | 0,720315 | 0,518854 | |
| 22114 | 29832 | (G2) | (22114 | 3 | 29832 | 2) | 1 | 0 | 0 | |
| 22114 | 29832 | (G3) | (22114 | 3 | 29832 | 2) | 0,564425 | -0,521337 | 0,271792 | |
| 22114 | 29832 | (G1) | (22114 | 3 | 29832 | 3) | 1 | 0 | 0 | |
| 22114 | 29832 | (G2) | (22114 | 3 | 29832 | 3) | 0,0958079 | -1,28084 | 1,64054 | |
| 22114 | 29832 | (G3) | (22114 | 3 | 29832 | 3) | 0,169259 | -1,86056 | 3,46169 | |
| 22216 | 28979 | (G1) | (22216 | 1 | 28979 | 1) | 0,205128 | -2,13649 | 4,56461 | |
| 22216 | 28979 | (G2) | (22216 | 1 | 28979 | 1) | 0,495723 | -1,14635 | 1,31413 | |
| 22216 | 28979 | (G3) | (22216 | 1 | 28979 | 1) | 1,01158 | 0,0145838 | 0,00021269 | |
| 22216 | 28979 | (G1) | (22216 | 1 | 28979 | 2) | 0,890933 | -0,339671 | 0,115376 | |
| 22216 | 28979 | (G2) | (22216 | 1 | 28979 | 2) | 1,78479 | 1,49947 | 2,24841 | |
| 22216 | 28979 | (G3) | (22216 | 1 | 28979 | 2) | 1,50959 | 1,12369 | 1,26267 | |
| 22216 | 28979 | (G1) | (22216 | 1 | 28979 | 3) | 3,03811 | 2,92563 | 8,55931 | |
| 22216 | 28979 | (G2) | (22216 | 1 | 28979 | 3) | 0,378982 | -2,86975 | 8,23549 | |
| 22216 | 28979 | (G3) | (22216 | 1 | 28979 | 3) | 2,58328 | 2,8749 | 8,26507 | |
| 22216 | 28979 | (G1) | (22216 | 2 | 28979 | 1) | 0,691778 | -0,424857 | 0,180503 | |
| 22216 | 28979 | (G2) | (22216 | 2 | 28979 | 1) | 1,96852 | 0,975963 | 0,952505 | |
| 22216 | 28979 | (G3) | (22216 | 2 | 28979 | 1) | 0,43851 | -1,21506 | 1,47637 | |
| 22216 | 28979 | (G1) | (22216 | 2 | 28979 | 2) | 1,40039 | 0,913146 | 0,833836 | |
| 22216 | 28979 | (G2) | (22216 | 2 | 28979 | 2) | 1,2335 | 0,559968 | 0,313564 | |
| 22216 | 28979 | (G3) | (22216 | 2 | 28979 | 2) | 0,570729 | -1,25996 | 1,58751 | |
| 22216 | 28979 | (G1) | (22216 | 2 | 28979 | 3) | 0,596328 | -1,3288 | 1,7657 | |
| 22216 | 28979 | (G2) | (22216 | 2 | 28979 | 3) | 1,50263 | 0,89411 | 0,799433 | |
| 22216 | 28979 | (G3) | (22216 | 2 | 28979 | 3) | 0,476105 | -1,95363 | 3,81668 | |
| 22216 | 28979 | (G1) | (22216 | 3 | 28979 | 1) | 1 | 0 | 0 | |
| 22216 | 28979 | (G2) | (22216 | 3 | 28979 | 1) | 1 | 0 | 0 | |
| 22216 | 28979 | (G3) | (22216 | 3 | 28979 | 1) | 1,01119 | 0,00451366 | 2,04E-05 | |
| 22216 | 28979 | (G1) | (22216 | 3 | 28979 | 2) | 0,296844 | -1,21116 | 1,46691 | |
| 22216 | 28979 | (G2) | (22216 | 3 | 28979 | 2) | 1,75379 | 0,898461 | 0,807233 | |
| 22216 | 28979 | (G3) | (22216 | 3 | 28979 | 2) | 0,241056 | -1,45015 | 2,10293 | |
| 22216 | 28979 | (G1) | (22216 | 3 | 28979 | 3) | 0,245373 | -0,720315 | 0,518854 | |
| 22216 | 28979 | (G2) | (22216 | 3 | 28979 | 3) | 1,77171 | 0,521337 | 0,271792 | |
| 22216 | 28979 | (G3) | (22216 | 3 | 28979 | 3) | 1,01119 | 0,00451366 | 2,04E-05 | |
| 22235 | 23137 | (G1) | (22235 | 1 | 23137 | 1) | 1,01119 | 0,00451366 | 2,04E-05 | |
| 22235 | 23137 | (G2) | (22235 | 1 | 23137 | 1) | 1,01119 | 0,00451366 | 2,04E-05 | |
| 22235 | 23137 | (G3) | (22235 | 1 | 23137 | 1) | 1 | 0 | 0 | |
| 22235 | 23137 | (G1) | (22235 | 1 | 23137 | 2) | 0,140045 | -1,05347 | 1,1098 | |
| 22235 | 23137 | (G2) | (22235 | 1 | 23137 | 2) | 1,01119 | 0,00451366 | 2,04E-05 | |
| 22235 | 23137 | (G3) | (22235 | 1 | 23137 | 2) | 0,138495 | -1,05945 | 1,12244 | |
| 22235 | 23137 | (G1) | (22235 | 1 | 23137 | 3) | 1,01119 | 0,00451366 | 2,04E-05 | |
| 22235 | 23137 | (G2) | (22235 | 1 | 23137 | 3) | 1,01119 | 0,00451366 | 2,04E-05 | |
| 22235 | 23137 | (G3) | (22235 | 1 | 23137 | 3) | 1 | 0 | 0 | |
| 22235 | 23137 | (G1) | (22235 | 2 | 23137 | 1) | 0,570815 | -0,511048 | 0,26117 | |
| 22235 | 23137 | (G2) | (22235 | 2 | 23137 | 1) | 1,01132 | 0,00911231 | 8,30E-05 | |
| 22235 | 23137 | (G3) | (22235 | 2 | 23137 | 1) | 1 | 0 | 0 | |
| 22235 | 23137 | (G1) | (22235 | 2 | 23137 | 2) | 1,01132 | 0,00911231 | 8,30E-05 | |
| 22235 | 23137 | (G2) | (22235 | 2 | 23137 | 2) | 1,42535 | 0,600868 | 0,361043 | |
| 22235 | 23137 | (G3) | (22235 | 2 | 23137 | 2) | 10,4376 | 1,28084 | 1,64054 | |
| 22235 | 23137 | (G1) | (22235 | 2 | 23137 | 3) | 1,01158 | 0,0145838 | 0,00021269 | |
| 22235 | 23137 | (G2) | (22235 | 2 | 23137 | 3) | 7,30316 | 1,06554 | 1,13537 | |
| 22235 | 23137 | (G3) | (22235 | 2 | 23137 | 3) | 4,34894 | 1,96431 | 3,85851 | |
| 22235 | 23137 | (G1) | (22235 | 3 | 23137 | 1) | 1,5759 | 0,956248 | 0,914411 | |
| 22235 | 23137 | (G2) | (22235 | 3 | 23137 | 1) | 2,57904 | 2,30537 | 5,31471 | |
| 22235 | 23137 | (G3) | (22235 | 3 | 23137 | 1) | 1,0891 | 0,206507 | 0,0426453 | |
| 22235 | 23137 | (G1) | (22235 | 3 | 23137 | 2) | 2,20115 | 2,59078 | 6,71215 | |
| 22235 | 23137 | (G2) | (22235 | 3 | 23137 | 2) | 1,16797 | 0,515815 | 0,266065 | |
| 22235 | 23137 | (G3) | (22235 | 3 | 23137 | 2) | 1,49546 | 1,34105 | 1,79842 | |
| 22235 | 23137 | (G1) | (22235 | 3 | 23137 | 3) | 0,395846 | -2.87226 | 8,24988 | 13 |
| 22235 | 23137 | (G2) | (22235 | 3 | 23137 | 3) | 0,34229 | -3,13339 | 9,81816 | 13 |
| 22235 | 23137 | (G3) | (22235 | 3 | 23137 | 3) | 0,378982 | -2,86975 | 8,23549 | 13 |
| 22293 | 23036 | (G1) | (22293 | 1 | 23036 | 1) | 1,24537 | 0,330111 | 0,108973 | |
| 22293 | 23036 | (G2) | (22293 | 1 | 23036 | 1) | 1 | 0 | 0 | |
| 22293 | 23036 | (G3) | (22293 | 1 | 23036 | 1) | 1 | 0 | 0 | |
| 22293 | 23036 | (G1) | (22293 | 1 | 23036 | 2) | 0,680202 | -0,974651 | 0,949945 | |
| 22293 | 23036 | (G2) | (22293 | 1 | 23036 | 2) | 0,666067 | -0,999765 | 0,99953 | |
| 22293 | 23036 | (G3) | (22293 | 1 | 23036 | 2) | 0,746003 | -0,659854 | 0,435407 | |
| 22293 | 23036 | (G1) | (22293 | 1 | 23036 | 3) | 0,510294 | -1,38377 | 1,91481 | |
| 22293 | 23036 | (G2) | (22293 | 1 | 23036 | 3) | 1,17187 | 0,28114 | 0,0790396 | |
| 22293 | 23036 | (G3) | (22293 | 1 | 23036 | 3) | 0,708426 | -0,714034 | 0,509844 | |
| 22293 | 23036 | (G1) | (22293 | 2 | 23036 | 1) | 1,37216 | 0,684972 | 0,489187 | |
| 22293 | 23036 | (G2) | (22293 | 2 | 23036 | 1) | 0,828534 | -0,432875 | 0,18738 | |
| 22293 | 23036 | (G3) | (22293 | 2 | 23036 | 1) | 1,41158 | 0,714034 | 0,509844 | |
| 22293 | 23036 | (G1) | (22293 | 2 | 23036 | 2) | 3,03811 | 2,92563 | 8,55931 | 14 |
| 22293 | 23036 | (G2) | (22293 | 2 | 23036 | 2) | 2,81278 | 2,85016 | 8,12342 | 14 |
| 22293 | 23036 | (G3) | (22293 | 2 | 23036 | 2) | 3,18284 | 2,86963 | 8,23475 | 14 |
| 22293 | 23036 | (G1) | (22293 | 2 | 23036 | 3) | 0,468545 | -1,57631 | 2,48475 | |
| 22293 | 23036 | (G2) | (22293 | 2 | 23036 | 3) | 0,816935 | -0,448633 | 0,201272 | |
| 22293 | 23036 | (G3) | (22293 | 2 | 23036 | 3) | 0,444862 | -1,94932 | 3,79984 | |
| 22293 | 23036 | (G1) | (22293 | 3 | 23036 | 1) | 1 | 0 | 0 | |
| 22293 | 23036 | (G2) | (22293 | 3 | 23036 | 1) | 0,238358 | -1,46172 | 2,13662 | |
| 22293 | 23036 | (G3) | (22293 | 3 | 23036 | 1) | 1,63812 | 0,690368 | 0,476608 | |
| 22293 | 23036 | (G1) | (22293 | 3 | 23036 | 2) | 0,390035 | -1,58742 | 2,51989 | |
| 22293 | 23036 | (G2) | (22293 | 3 | 23036 | 2) | 0,6159 | -0,970715 | 0,942288 | |
| 22293 | 23036 | (G3) | (22293 | 3 | 23036 | 2) | 0,61736 | -0,837641 | 0,701642 | |
| 22293 | 23036 | (G1) | (22293 | 3 | 23036 | 3) | 1,46191 | 0,748081 | 0,559625 | |
| 22293 | 23036 | (G2) | (22293 | 3 | 23036 | 3) | 0,832154 | -0,302401 | 0,0914462 | |
| 22293 | 23036 | (G3) | (22293 | 3 | 23036 | 3) | 0,741882 | -0,543268 | 0,29514 | |
| 22296 | 23036 | (G1) | (22296 | 1 | 23036 | 1) | 1 | 0 | 0 | |
| 22296 | 23036 | (G2) | (22296 | 1 | 23036 | 1) | 0,238358 | -1,46172 | 2,13662 | |
| 22296 | 23036 | (G3) | (22296 | 1 | 23036 | 1) | 1,65754 | 0,706762 | 0,499512 | |
| 22296 | 23036 | (G1) | (22296 | 1 | 23036 | 2) | 0,390035 | -1,58742 | 2,51989 | |
| 22296 | 23036 | (G2) | (22296 | 1 | 23036 | 2) | 0,6159 | -0,970715 | 0,942288 | |
| 22296 | 23036 | (G3) | (22296 | 1 | 23036 | 2) | 0,624681 | -0,816996 | 0,667483 | |
| 22296 | 23036 | (G1) | (22296 | 1 | 23036 | 3) | 1,46191 | 0,748081 | 0,559625 | |
| 22296 | 23036 | (G2) | (22296 | 1 | 23036 | 3) | 0,832154 | -0,302401 | 0,0914462 | |
| 22296 | 23036 | (G3) | (22296 | 1 | 23036 | 3) | 0,750785 | -0,521439 | 0,271899 | |
| 22296 | 23036 | (G1) | (22296 | 2 | 23036 | 1) | 1,37216 | 0,684972 | 0,469187 | |
| 22296 | 23036 | (G2) | (22296 | 2 | 23036 | 1) | 0,828534 | -0,432875 | 0,18738 | |
| 22296 | 23036 | (G3) | (22296 | 2 | 23036 | 1) | 1,4296 | 0,740056 | 0,547682 | |
| 22296 | 23036 | (G1) | (22296 | 2 | 23036 | 2) | 3,03811 | 2,92563 | 8,55931 | 14 |
| 22296 | 23036 | (G2) | (22296 | 2 | 23036 | 2) | 2,81278 | 2,85016 | 8,12342 | 14 |
| 22296 | 23036 | (G3) | (22296 | 2 | 23036 | 2) | 3,2331 | 2,90627 | 8,44639 | 14 |
| 22296 | 23036 | (G1) | (22296 | 2 | 23036 | 3) | 0,468545 | -1,57631 | 2,48475 | |
| 22296 | 23036 | (G2) | (22296 | 2 | 23036 | 3) | 0,816935 | -0,448633 | 0,201272 | |
| 22296 | 23036 | (G3) | (22296 | 2 | 23036 | 3) | 0,401797 | -2,13125 | 4,54221 | |
| 22296 | 23036 | (G1) | (22296 | 3 | 23036 | 1) | 1,24537 | 0,330111 | 0,108973 | |
| 22296 | 23036 | (G2) | (22296 | 3 | 23036 | 1) | 1 | 0 | 0 | |
| 22296 | 23036 | (G3) | (22296 | 3 | 23036 | 1) | 1,01245 | 0,025301 | 0,00064014 | |
| 22296 | 23036 | (G1) | (22296 | 3 | 23036 | 2) | 0,680202 | -0,974651 | 0,949945 | |
| 22296 | 23036 | (G2) | (22296 | 3 | 23036 | 2) | 0,666067 | -0,999765 | 0,99953 | |
| 22296 | 23036 | (G3) | (22296 | 3 | 23036 | 2) | 0,755407 | -0,631394 | 0,398659 | |
| 22296 | 23036 | (G1) | (22296 | 3 | 23036 | 3) | 0,510294 | -1,38377 | 1,91481 | |
| 22296 | 23036 | (G2) | (22296 | 3 | 23036 | 3) | 1,17187 | 0,28114 | 0,0790396 | |
| 22296 | 23036 | (G3) | (22296 | 3 | 23036 | 3) | 0,717136 | -0,688499 | 0,474031 | |
| 22300 | 23036 | (G1) | (22300 | 1 | 23036 | 1) | 1 | 0 | 0 | |
| 22300 | 23036 | (G2) | (22300 | 1 | 23036 | 1) | 0,238358 | -1,46172 | 2,13662 | |
| 22300 | 23036 | (G3) | (22300 | 1 | 23036 | 1) | 1,63812 | 0,690368 | 0,476608 | |
| 22300 | 23036 | (G1) | (22300 | 1 | 23036 | 2) | 0,390035 | -1,58742 | 2,51989 | |
| 22300 | 23036 | (G2) | (22300 | 1 | 23036 | 2) | 0,6159 | -0,970715 | 0,942288 | |
| 22300 | 23036 | (G3) | (22300 | 1 | 23036 | 2) | 0,61736 | -0,837641 | 0,701642 | |
| 22300 | 23036 | (G1) | (22300 | 1 | 23036 | 3) | 1,27097 | 0,487981 | 0,238126 | |
| 22300 | 23036 | (G2) | (22300 | 1 | 23036 | 3) | 0,832154 | -0,302401 | 0,0914462 | |
| 22300 | 23036 | (G3) | (22300 | 1 | 23036 | 3) | 0,741882 | -0,543268 | 0,29514 | |
| 22300 | 23036 | (G1) | (22300 | 2 | 23036 | 1) | 1,37216 | 0,684972 | 0,469187 | |
| 22300 | 23036 | (G2) | (22300 | 2 | 23036 | 1) | 0,828534 | -0,432875 | 0,18738 | |
| 22300 | 23036 | (G3) | (22300 | 2 | 23036 | 1) | 1,41158 | 0,714034 | 0,509844 | |
| 22300 | 23036 | (G1) | (22300 | 2 | 23036 | 2) | 3,03811 | 2,92563 | 8,55931 | 14 |
| 22300 | 23036 | (G2) | (22300 | 2 | 23036 | 2) | 3,0634 | 3,0277 | 9,16695 | 14 |
| 22300 | 23036 | (G3) | (22300 | 2 | 23036 | 2) | 3,18284 | 2,86963 | 8,23475 | 14 |
| 22300 | 23036 | (G1) | (22300 | 2 | 23036 | 3) | 0,510294 | -1,38377 | 1,91481 | |
| 22300 | 23036 | (G2) | (22300 | 2 | 23036 | 3) | 0,816935 | -0,448633 | 0,201272 | |
| 22300 | 23036 | (G3) | (22300 | 2 | 23036 | 3) | 0,444862 | -1,94932 | 3,79984 | |
| 22300 | 23036 | (G1) | (22300 | 3 | 23036 | 1) | 1,24537 | 0,330111 | 0,108973 | |
| 22300 | 23036 | (G2) | (22300 | 3 | 23036 | 1) | 1 | 0 | 0 | |
| 22300 | 23036 | (G3) | (22300 | 3 | 23036 | 1) | 1 | 0 | 0 | |
| 22300 | 23036 | (G1) | (22300 | 3 | 23036 | 2) | 0,680202 | -0,974651 | 0,949945 | |
| 22300 | 23036 | (G2) | (22300 | 3 | 23036 | 2) | 0,621142 | -1,1822 | 1,39761 | |
| 22300 | 23036 | (G3) | (22300 | 3 | 23036 | 2) | 0,746003 | -0,659854 | 0,435407 | |
| 22300 | 23036 | (G1) | (22300 | 3 | 23036 | 3) | 0,510294 | -1,38377 | 1,91481 | |
| 22300 | 23036 | (G2) | (22300 | 3 | 23036 | 3) | 1,17187 | 0,28114 | 0,0790396 | |
| 22300 | 23036 | (G3) | (22300 | 3 | 23036 | 3) | 0,708426 | -0,714034 | 0,509844 | |
| 22879 | 24281 | (G1) | (22879 | 1 | 24281 | 1) | 1,08412 | 0,0327342 | 0,00107153 | |
| 22879 | 24281 | (G2) | (22879 | 1 | 24281 | 1) | 1,18368 | 0,0683391 | 0,00467023 | |
| 22879 | 24281 | (G3) | (22879 | 1 | 24281 | 1) | 1,46991 | 0,156078 | 0,0243604 | |
| 22879 | 24281 | (G1) | (22879 | 1 | 24281 | 2) | 1,09046 | 0,136219 | 0,0185556 | |
| 22879 | 24281 | (G2) | (22879 | 1 | 24281 | 2) | 0,995548 | -0,00727406 | 5,29E-05 | |
| 22879 | 24281 | (G3) | (22879 | 1 | 24281 | 2) | 0,215501 | -1,61425 | 2,60582 | |
| 22879 | 24281 | (G1) | (22879 | 1 | 24281 | 3) | 1,72968 | 1,13654 | 1,29171 | |
| 22879 | 24281 | (G2) | (22879 | 1 | 24281 | 3) | 0,783208 | -0,569337 | 0,324144 | |
| 22879 | 24281 | (G3) | (22879 | 1 | 24281 | 3) | 1,13842 | 0,304945 | 0,0929912 | |
| 22879 | 24281 | (G1) | (22879 | 2 | 24281 | 1) | 1,08412 | 0,0327342 | 0,00107153 | |
| 22879 | 24281 | (G2) | (22879 | 2 | 24281 | 1) | 1,18368 | 0,0683391 | 0,00467023 | |
| 22879 | 24281 | (G3) | (22879 | 2 | 24281 | 1) | 1,46991 | 0,156078 | 0,0243604 | |
| 22879 | 24281 | (G1) | (22879 | 2 | 24281 | 2) | 0,586357 | -1,23318 | 1,52074 | |
| 22879 | 24281 | (G2) | (22879 | 2 | 24281 | 2) | 0,400697 | -1,82047 | 3,31412 | |
| 22879 | 24281 | (G3) | (22879 | 2 | 24281 | 2) | 0,162181 | -2,51969 | 6,34885 | |
| 22879 | 24281 | (G1) | (22879 | 2 | 24281 | 3) | 0,424685 | -2,31307 | 5,3503 | |
| 22879 | 24281 | (G2) | (22879 | 2 | 24281 | 3) | 1,37089 | 0,886712 | 0,786258 | |
| 22879 | 24281 | (G3) | (22879 | 2 | 24281 | 3) | 1,5289 | 1,13819 | 1,29549 | |
| 22879 | 24281 | (G1) | (22879 | 3 | 24281 | 1) | 1,08412 | 0,0327342 | 0,00107153 | |
| 22879 | 24281 | (G2) | (22879 | 3 | 24281 | 1) | 1,18368 | 0,0683391 | 0,00467023 | |
| 22879 | 24281 | (G3) | (22879 | 3 | 24281 | 1) | 1,46991 | 0,156078 | 0,0243604 | |
| 22879 | 24281 | (G1) | (22879 | 3 | 24281 | 2) | 0,834488 | -0,345797 | 0,119575 | |
| 22879 | 24281 | (G2) | (22879 | 3 | 24281 | 2) | 0,374087 | -1,67731 | 2,81338 | |
| 22879 | 24281 | (G3) | (22879 | 3 | 24281 | 2) | 0,30168 | -1,60316 | 2,57012 | |
| 22879 | 24281 | (G1) | (22879 | 3 | 24281 | 3) | 3,7675 | 2,96947 | 8,81774 | 15 |
| 22879 | 24281 | (G2) | (22879 | 3 | 24281 | 3) | 5,15833 | 2,85731 | 8,1642 | 15 |
| 22879 | 24281 | (G3) | (22879 | 3 | 24281 | 3) | 4,32256 | 3,15681 | 9,96543 | 15 |
| 24281 | 29792 | (G1) | (24281 | 1 | 29792 | 1) | 1,08412 | 0,0327342 | 0,00107153 | |
| 24281 | 29792 | (G2) | (24281 | 1 | 29792 | 1) | 1,18368 | 0,0683391 | 0,00467023 | |
| 24281 | 29792 | (G3) | (24281 | 1 | 29792 | 1) | 1,46991 | 0,156078 | 0,0243604 | |
| 24281 | 29792 | (G1) | (24281 | 1 | 29792 | 2) | 1,08412 | 0,0327342 | 0,00107153 | |
| 24281 | 29792 | (G2) | (24281 | 1 | 29792 | 2) | 1,18368 | 0,0683391 | 0,00467023 | |
| 24281 | 29792 | (G3) | (24281 | 1 | 29792 | 2) | 1,46991 | 0,156078 | 0,0243604 | |
| 24281 | 29792 | (G1) | (24281 | 1 | 29792 | 3) | 1,08412 | 0,0327342 | 0,00107153 | |
| 24281 | 29792 | (G2) | (24281 | 1 | 29792 | 3) | 1,18368 | 0,0683391 | 0,00467023 | |
| 24281 | 29792 | (G3) | (24281 | 1 | 29792 | 3) | 1,46991 | 0,156078 | 0,0243604 | |
| 24281 | 29792 | (G1) | (24281 | 2 | 29792 | 1) | 0,456064 | -0,977552 | 0,955608 | |
| 24281 | 29792 | (G2) | (24281 | 2 | 29792 | 1) | 0,112751 | -1,1908 | 1,41801 | |
| 24281 | 29792 | (G3) | (24281 | 2 | 29792 | 1) | 1,13646 | 0,170437 | 0,0290486 | |
| 24281 | 29792 | (G1) | (24281 | 2 | 29792 | 2) | 1,09945 | 0,207804 | 0,0431824 | |
| 24281 | 29792 | (G2) | (24281 | 2 | 29792 | 2) | 0,665827 | -0,813484 | 0,661755 | |
| 24281 | 29792 | (G3) | (24281 | 2 | 29792 | 2) | 0,14088 | -2,72909 | 7,44794 | |
| 24281 | 29792 | (G1) | (24281 | 2 | 29792 | 3) | 0,608611 | -1,09809 | 1,20581 | |
| 24281 | 29792 | (G2) | (24281 | 2 | 29792 | 3) | 0,438453 | -1,81498 | 3,29414 | |
| 24281 | 29792 | (G3) | (24281 | 2 | 29792 | 3) | 0,033536 | -1,9148 | 3,66644 | |
| 24281 | 29792 | (G1) | (24281 | 3 | 29792 | 1) | 0,661339 | -0,575413 | 0,331101 | |
| 24281 | 29792 | (G2) | (24281 | 3 | 29792 | 1) | 0,602027 | -0,726164 | 0,527314 | |
| 24281 | 29792 | (G3) | (24281 | 3 | 29792 | 1) | 0,350574 | -1,06372 | 1,1315 | |
| 24281 | 29792 | (G1) | (24281 | 3 | 29792 | 2) | 2,91755 | 2,92226 | 8,53963 | 16 |
| 24281 | 29792 | (G2) | (24281 | 3 | 29792 | 2) | 3,35391 | 3,14919 | 9,91742 | 16 |
| 24281 | 29792 | (G3) | (24281 | 3 | 29792 | 2) | 3,96581 | 3,73004 | 13,9132 | 16 |
| 24281 | 29792 | (G1) | (24281 | 3 | 29792 | 3) | 0,562235 | -1,53041 | 2,34217 | |
| 24281 | 29792 | (G2) | (24281 | 3 | 29792 | 3) | 0,971616 | -0,0784901 | 0,0061607 | |
| 24281 | 29792 | (G3) | (24281 | 3 | 29792 | 3) | 1,33884 | 0,742519 | 0,551334 | |

| | | | | | | | | | | |
|---|---|---|---|---|---|---|---|---|---|---|
| *epist: a sequence number is assigned each time that a combination with homogeneous direction of effect appears in the same stratum in all the groups in which there appears a marker not previously detected in the Table. | | | | | | | | | | |

This simple study allowed it to be determined that only 25 of the 55 remaining combinations (45%) had a homogenous direction of effect. In other words, for the same stratum, in the comparison groups G1, G2 and G3 (each one of which includes the comparison of Parkinson's disease with healthy controls), the OR is in all of these greater than 1 or in all of these less than 1, furthermore, fulfilling the requirement that Z²>7.879. These 25 combinations (indicated in the above Table by spotted lines) included the combined effect of 31 different markers. Therefore, only the combination of 31 markers of the 31,532 studied (0.096%) can be selected, under the initial HFCC criteria for Parkinson's disease (homogeneity of effect between the three groups, HARD). This permits their direct prioritisation for future studies with the validation engine.
These markers and their correspondence with the molecular markers of Coriell's database, along with their position in the genome, are summarised in Table 19.

**Table 19: variables selected by the HFCC software following the evaluation of the post-hoc analysis of the raw data of chromosome 1 for Parkinson's disease. The markers prioritised by Fung et al., (2006) for chromosome 1 are underlined.**

| **Marker** | **Identification Code** | **Position on Chromosome 1** |
|---|---|---|
| 271 | rs2493278 | 3330903 |
| 1221 | rs2071414 | 8854978 |
| 1223 | rs2274971 | 8865415 |
| 2311 | rs3817269 | 15801670 |
| 3597 | rs1555024 | 23531509 |
| 4283 | rs602345 | 28895908 |
| 5375 | rs4653210 | 36743444 |
| 9353 | rs12089806 | 64866616 |
| 10386 | rs988421 | 72261857 |
| 13221 | rs10493872 | 94602439 |
| 15323 | rs7543509 | 110764041 |
| 16101 | rs10494171 | 115413951 |
| 21014 | rs3795320 | 173322948 |
| 21689 | rs4652572 | 177859863 |
| 22109 | rs2986574 | 180638271 |
| 22112 | rs3010040 | 180639879 |
| 22113 | rs2296713 | 180641374 |
| 22114 | rs1887279 | 180641817 |
| 22235 | rs10489697 | 181399388 |
| 22293 | rs6700484 | 181821558 |
| 22296 | rs1322646 | 181842524 |
| 22300 | rs2146329 | 181864499 |
| 22879 | rs703934 | 187194947 |
| 23036 | rs10921046 | 188625465 |
| 23188 | rs7554157 | 190220826 |
| 23137 | rs842796 | 189634072 |
| 24281 | rs2820304 | 198650184 |
| 24450 | rs1342387 | 199646013 |
| 27090 | rs4339871 | 215821425 |
| 29792 | rs1876084 | 222186730 |
| 29832 | rs622625 | 234141587 |

### 2.6. Evaluation of the positives obtained.

From the study based on this first approach to the use of HFCC with high-volume genomic data, it is concluded that the system allows the selection of only 25 combinations of markers potentially involved in Parkinson's disease on chromosome 1 of the nearly 500 million possible by taking the 31,532 elements two by two. As a result, it can be proved that the procedure drastically reduces the complexity in the interpretation of multilocus studies in the human genome. In addition, it is worth pointing out that ALL the markers selected by the diverse conventional statistical analysis techniques employed by the investigators who generated this raw data (Fung et al., 2006) appear in the prioritisation table (underlined), without exception. Therefore, the HFCC system did not omit any of the markers statistically observable using conventional methods in this series. However, HFCC identified a group of new, potentially significant markers, which had been completely omitted by the methods employed in the conventional study. This demonstrates that the HFCC system can lead to localisations and genetic models that are completely new and inaccessible through the classical analysis techniques usually employed during the process of whole genome mapping and can be a useful tool for the multilocus dissection of complex pathologies.

### 2.7. Performance of the study without noise filter

In order to evaluate the potential of the system without a noise filter especially for situations in which, as in this case, the number of controls available is limited and can not be applied in optimal conditions, the study described in sections 2.1 to 2.6 was repeated with the same individuals and using the same matrixes F1, F2, F3, Cc1, Cc2, Cc3. Comparison between the control group matrixes was not carried out. This meant that the study had to be performed with two differences in the parameters of the calculation module:
○ Input of the maximum number of controls in Cf: (not applicable)
○ Input of the noise filter application=No
The results obtained were identical to the previous ones, which is not surprising as the parameters employed in the study are very restrictive and this results in an extensive noise scan. This demonstrates the applicability of the HFCC system without using a noise filter.

### - Example 3: Employing the HFCC technology for the prioritisation of genetic markers in genetic association studies for Parkinson's disease on a massive genomic scale using the noise filter and "fuzzy logic" analysis

### 3.1. Aim of the study:

In order to verify the capacity of the noise filter developed and the application of the fuzzy logic system of HFCC technology, it was decided to repeat the experiment described in Example 2, modifying the parameters of the study. The objective is to prove the absence of redundancy between the two systems of analysis implemented and the potential of the noise filter designed. In order to evaluate the capacity of the filter of the system, the fuzzy logic analysis system was used which, due to its *modus operandi*, generates a much greater number of positive results than the *hard* mode. Furthermore, the threshold of significance was reduced to Z²>6.65 (equivalent to p<0.01). The idea of this experiment is to calculate the rate of variables that get past the noise filter under these conditions.

### 3.2. Description of the study phenotype

Identical to Example 2.

### 3.3. Obtaining genotypes in the patients and controls in the study

Identical to Example 2.

### 3.4. Calculation module parameters applied to the study

Employing the same input matrixes used in the initial test described in Example 2, the HFCC software was applied in accordance with the input values introduced in the various parameters of the calculation module, i.e.:
○ Input of the maximum number of cases in F=90
○ Input of the maximum number of controls in Cc=90.
○ Input of the maximum number of controls in Cf=90
○ Input of number of comparison groups=3
○ Input of the number of genetic markers of the study=31,532
○ Input of the statistical threshold for the selection: Z²>6.65
○ Input of the correction factor for null values=0.33
○ Input of the location path for F, Cc, Cf. and output files
   ■ /home/aruiz/hfcc/pruebas chromosome 1-3 groups/
○ Input of the application of the noise filter=Yes
○ Input of multilocus model selected=Digenic
○ Input of printing of intermediate data (y/n)=No
○ Input of the type of analysis=Fuzzy logic
○ Input of the statistical model applied=Exhaustive

### 3.5. Obtaining specific results

Applying the parameters displayed above and using exactly the same matrixes as those employed in example 2, the system would produce (as expected) 11,202 positive variables in total in the results file if the noise filter is not applied. However, our system automatically eliminated 11,117 variables in which positive strata were detected in some of the control against control comparison groups (Ccl versus Cfl, etc). Therefore, by employing the noise filter, 99.24% of the associations were eliminated due to the detection of the existence of differences between the control groups by the noise filter. The remaining 0.76% (85 variables) were analysed using post-hoc analysis, employing an identical strategy to that employed in the previous example. The positive results of this study are shown in Table 20.

**Table 20: Strata of variables of chromosome 1 positive for Parkinson disease with Fuzzy Logic type analysis**

| | | | | |
|---|---|---|---|---|
| 126 1 2395 1 | 126 1 2395 2 | 126 1 2395 3 | 126 2 2395 1 | 126 2 2395 2 |
| 126 2 2395 3 | 126 3 2395 1 | 126 3 2395 2 | 126 3 2395 3 | 126 1 2397 1 |
| 126 1 2397 2 | 126 1 2397 3 | 126 2 2397 1 | 126 2 2397 2 | 126 2 2397 3 |
| 126 3 2397 1 | 126 3 2397 2 | 126 3 2397 3 | 126 1 2399 1 | 126 1 2399 2 |
| 126 1 2399 3 | 126 2 2399 1 | 126 2 2399 2 | 126 2 2399 3 | 126 3 2399 1 |
| 126 3 2399 2 | 126 3 2399 3 | 126 1 2401 1 | 126 1 2401 2 | 126 1 2401 3 |
| 126 2 2401 1 | 126 2 2401 2 | 126 2 2401 3 | 126 3 2401 1 | 126 3 2401 2 |
| 126 3 2401 3 | 126 1 2404 1 | 126 1 2404 2 | 126 1 2404 3 | 126 2 2404 1 |
| 126 2 2404 2 | 126 2 2404 3 | 126 3 2404 1 | 126 3 2404 2 | 126 3 2404 3 |
| 134 1 27743 1 | 134 1 27743 2 | 134 1 27743 3 | 134 2 27743 1 | 134 2 27743 2 |
| 134 2 27743 3 | 134 3 27743 1 | 134 3 27743 2 | 134 3 27743 3 | 287 1 22118 1 |
| 2871 22118 2 | 287 1 22118 3 | 287 2 22118 1 | 287 2 22118 2 | 287 2 22118 3 |
| 287 3 22118 1 | 287 3 22118 2 | 287 3 22118 3 | 548 1 29316 1 | 548 1 29316 2 |
| 548 1 29316 3 | 548 2 29316 1 | 548 2 29316 2 | 548 2 29316 3 | 548 3 29316 1 |
| 548 3 29316 2 | 548 3 29316 3 | 769 1 13501 1 | 769 1 13501 2 | 769 1 13501 3 |
| 769 2 13501 1 | 769 2 13501 2 | 769 2 13501 3 | 769313501 1 | 769 3 13501 2 |
| 769 3 13501 3 | 1321 1 14740 1 | 1321 1 14740 2 | 1321 1 14740 3 | 1321 2 14740 1 |
| 1321 2 47440 2 | 1321 2 14740 3 | 1321 3 14740 1 | 1321 3 14740 2 | 1321 3 14740 3 |
| 1322 1 12814 1 | 13221 12814 2 | 1322 1 12814 3 | 1322 2 12814 1 | 13322 2 142814 2 |
| 1322 2 12814 3 | 1322 3 12814 1 | 1322 3 12814 2 | 1322 3 12814 3 | 1322 1 19677 1 |
| 1322 1 19677 2 | 1322 1 19677 3 | 1322 2 19677 1 | 1322 2 19677 2 | 1322 2 19677 3 |
| 1322 3 19677 1 | 1322 3 19677 2 | 1322 3 19677 3 | 1501 1 11993 1 | 1501 1 11993 2 |
| 1501 1 11993 3 | 1501 2 11993 1 | 1501 211993 2 | 1501 2 11993 3 | 1501 3 11993 1 |
| 1501 3 11993 2 | 1501 3 11993 3 | 1501 1 12740 1 | 1501 1 12740 2 | 1501 1 12740 3 |
| 1501 2 12740 1 | 1501 2 12740 2 | 1501 2 12740 3 | 1501 3 12740 1 | 1501 3 12740 2 |
| 1501 3 12740 3 | 1946 1 22115 1 | 1946 1 22115 2 | 1946 1 22115 3 | 1946 2 22115 1 |
| 1946 2 22115 2 | 1946 2 22115 3 | 1946 3 22115 1 | 1946 3 22115 2 | 1946 3 22115 3 |
| 1956 1 26076 1 | 1956 1 26076 2 | 1956 1 26076 3 | 1956 2 26076 1 | 1956 2 26076 2 |
| 1956 2 26076 3 | 1956 3 26076 1 | 1956 3 26076 2 | 1956 3 26076 3 | 2019 1 8223 1 |
| 2019 1 8223 2 | 2019 1 8223 3 | 2019 2 8223 1 | 2019 2 8223 2 | 2019 2 8223 3 |
| 2019 3 8223 1 | 2019 3 8223 2 | 2019 3 8223 3 | 2242 1 10919 1 | 2242 1 10919 2 |
| 2242 1 10919 3 | 2242 2 10919 1 | 2242 2 10919 2 | 2242 2 10919 3 | 2242 3 10919 1 |
| 2242 3 10919 2 | 2242 3 10919 3 | 2666 1 7818 1 | 2666 1 7818 2 | 2666 7 7818 3 |
| 2666 2 7818 1 | 2666 2 7818 2 | 2666 2 7818 3 | 2666 3 7818 1 | 2666 3 7818 2 |
| 2666 3 7818 3 | 2684 1 18178 1 | 2684 1 18178 2 | 2684 1 18178 3 | 2684 2 18178 1 |
| 2684 2 18178 2 | 2684 2 18178 3 | 2684 3 18178 1 | 2684 3 18178 2 | 2684 3 18178 3 |
| 3168 1 4595 1 | 3168 1 4595 2 | 3168 1 4595 3 | 3168 2 4595 1 | 3168 2 4595 2 |
| 3168 2 4595 3 | 3168 3 4595 1 | 3168 3 4595 2 | 3168 3 4595 3 | 3168 1 4597 1 |
| 3168 1 4597 2 | 3168 1 4597 3 | 3168 2 4597 1 | 3168 2 4597 2 | 3168 2 4597 3 |
| 3168 3 4597 1 | 3168 3 4597 2 | 3168 3 4597 3 | 3168 1 11942 1 | 3168 1 11942 2 |
| 3168 1 11942 3 | 3168 2 11942 1 | 3168 2 11942 2 | 3168 2 11942 3 | 3168 3 11942 1 |
| 3168 3 11942 2 | 3168 3 11942 3 | 3799 1 13145 1 | 3799 1 13145 2 | 3799 1 13145 3 |
| 3799 2 13145 1 | 3799 2 13145 2 | 3799 2 13145 3 | 3799 3 13145 1 | 3799 3 13145 2 |
| 3799 3 13145 3 | 3819 1 7445 1 | 3819 1 7445 2 | 3819 1 7445 3 | 3819 2 7445 1 |
| 3819 2 7445 2 | 3819 2 7445 3 | 3819 3 7445 1 | 3819 3 7445 2 | 3819 3 7445 3 |
| 4051 1 19015 1 | 4051 1 19015 2 | 4051 1 19015 3 | 4051 2 19015 1 | 4051 2 19015 2 |
| 4051 2 19015 3 | 4051 3 19015 1 | 4051 3 19015 2 | 4051 3 19015 3 | 4427 1 26313 1 |
| 4427 1 26313 2 | 4427 1 26313 3 | 4427 2 26313 1 | 4427 2 26313 2 | 4427 2 26313 3 |
| 4427 3 26313 1 | 4427 3 26313 2 | 4427 3 26313 3 | 4585 1 10683 1 | 4585 1 10683 2 |
| 4585 1 10683 3 | 4585 2 10683 1 | 4585 2 10683 2 | 4585 2 10683 3 | 4585 3 10683 1 |
| 4585 3 10683 2 | 4585 3 10683 3 | 5196 1 27805 1 | 5196 1 27805 2 | 51961 27805 3 |
| 5196 2 27805 1 | 5196 2 27805 2 | 5196 2 27805 3 | 5196 3 27805 1 | 5196 3 27805 2 |
| 5196 3 27805 3 | 5763 1 22000 1 | 5763 1 22000 2 | 5763 1 22000 3 | 5763 2 22000 1 |
| 5763 2 22000 2 | 5763 2 22000 3 | 5763 3 22000 1 | 5763 3 22000 2 | 5763 3 22000 3 |
| 6004 1 21706 1 | 6004 1 21706 2 | 6004 1 21706 3 | 6004 2 21706 1 | 6004 2 21706 2 |
| 6004 2 21706 3 | 6004 3 21706 1 | 6004 3 21706 2 | 6004 3 21706 3 | 6321 1 8945 1 |
| 6321 1 8945 2 | 6321 1 8945 3 | 6321 2 8945 1 | 6321 2 8945 2 | 6321 2 8945 3 |
| 6321 3 8945 1 | 6321 3 8945 2 | 6321 3 8945 3 | 6562 1 9836 1 | 6562 1 9836 2 |
| 65621 9836 3 | 6562 2 9836 1 | 6562 2 9836 2 | 6562 2 9836 3 | 6562 3 98361 1 |
| 6562 3 9836 2 | 6562 3 9836 3 | 6563 1 9836 1 | 65631 9836 2 | 65631 9836 3 |
| 6563 2 9836 1 | 6563 2 9836 2 | 6563 2 9836 3 | 6563 3 9836 1 | 6563 3 9836 2 |
| 6563 3 9836 3 | 6564 1 9836 1 | 6564 1 9836 2 | 6564 1 9836 3 | 6564 2 9836 1 |
| 6564 2 9836 2 | 6564 2 9836 3 | 6564 3 98361 1 | 6564 3 9836 2 | 6564 3 9836 3 |
| 6565 1 9836 1 | 6565 1 9836 2 | 65651 9836 3 | 6565 2 9836 1 | 6565 2 9836 2 |
| 6565 2 9836 3 | 6565 3 9836 1 | 6565 3 9836 2 | 6565 3 9836 3 | 7269 1 24356 1 |
| 7269 1 24356 2 | 7269 1 24356 3 | 7269 2 24356 1 | 7269 2 24356 2 | 7269 2 24356 3 |
| 7269 3 24356 1 | 7269 3 24356 2 | 7269 3 24356 3 | 8087 1 25865 1 | 8087 1 25865 2 |
| 8087 1 25865 3 | 8087 2 25865 1 | 8087 2 25865 2 | 8087 2 25865 3 | 8087 3 25865 1 |
| 8087 3 25865 2 | 8087 3 25865 3 | 8235 1 22083 1 | 8235 1 22083 2 | 8235 1 22083 3 |
| 8235 2 22083 1 | 8235 2 22083 2 | 8235 2 22083 3 | 8235 3 22083 1 | 8235 3 22083 2 |
| 8235 3 22083 3 | 8330 1 11942 1 | 8330 1 11942 2 | 8330 1 11942 3 | 8330 2 11942 1 |
| 8330 2 11942 2 | 8330 2 11942 3 | 8330 3 11942 1 | 8330 3 11942 2 | 8330 3 11942 3 |
| 8853 1 21706 1 | 8853 1 21706 2 | 8853 1 21706 3 | 8853 2 21706 1 | 8853 2 21706 2 |
| 8853 2 21706 3 | 8853 3 21706 1 | 8853 3 21706 2 | 8853 3 21706 3 | 9437 1 18953 1 |
| 9437 1 18953 2 | 9437 1 18953 3 | 9437 2 18953 1 | 9437 2 18953 2 | 9437 2 18953 3 |
| 9437 3 18953 1 | 9437 3 18953 2 | 9437 3 18953 3 | 10150 1 15976 1 | 10150 1 15976 2 |
| 10150 1 15976 3 | 10150 2 15976 1 | 10150 2 15976 2 | 10150 2 15976 3 | 10150 3 15976 1 |
| 10150 3 15976 2 | 10150 3 15976 3 | 10152 1 15976 1 | 10152 1 15976 2 | 10152 1 15976 3 |
| 10152 2 15976 1 | 10152 2 15976 2 | 10152 2 15976 3 | 10152 3 15976 1 | 10152 3 15976 2 |
| 10152 3 15976 3 | 106651 30701 1 | 10665 1 30701 2 | 10665 1 30701 3 | 10665 2 30701 1 |
| 10665 2 30701 2 | 10665 2 30701 3 | 10665 3 30701 1 | 10665 3 30701 2 | 10665 3 30701 3 |
| 10672 1 30701 1 | 10672 1 30701 2 | 10672 1 30701 3 | 10672 2 30701 1 | 10672 2 30701 2 |
| 10672 2 30701 3 | 10672 3 30701 1 | 10672 3 30701 2 | 10672 3 30701 3 | 10788 1 22000 1 |
| 10788 1 22000 2 | 10788 1 22000 3 | 10788 2 22000 1 | 10788 2 22000 2 | 10788 2 22000 3 |
| 10788 3 22000 1 | 10788 3 22000 2 | 10788 3 22000 3 | 10871 1 27334 1 | 10871 1 27334 2 |
| 10871 1 27334 3 | 10871 2 27334 1 | 10871 2 27334 2 | 10871 2 27334 3 | 10871 3 27334 1 |
| 10871 3 27334 2 | 10871 3 27334 3 | 11561 1 16492 1 | 11561 1 16492 2 | 11561 1 16492 3 |
| 11561 2 16492 1 | 11561 2 16492 2 | 11561 216492 3 | 11561 3 16492 1 | 11561 3 16492 2 |
| 11561 3 16492 3 | 11778 1 27944 1 | 11778 1 27944 2 | 11778 1 27944 3 | 11778 2 27944 1 |
| 11778 2 27944 2 | 11778 2 27944 3 | 11778 3 27944 1 | 11778 3 27944 2 | 11778 3 27944 3 |
| 11787 1 27401 1 | 11787 1 27401 2 | 11187 1 27401 3 | 11787 2 27401 1 | 1178 2 27401 2 |
| 11787 2 27401 3 | 11787 3 27401 1 | 11787 3 27401 2 | 11787 3 27401 3 | 11993 1 27805 1 |
| 119931 27805 2 | 11993 1 27805 3 | 11993 2 27805 1 | 11993 2 27805 2 | 11993 2 27805 3 |
| 11993 3 27805 1 | 11993 3 27805 2 | 11993 3 27805 3 | 12873 1 27805 1 | 12873 1 27805 2 |
| 12873 1 27805 3 | 12873 2 27805 1 | 12873 2 27805 2 | 12873 2 27805 3 | 12873 3 27805 1 |
| 12873 3 27805 2 | 12873 3 27805 3 | 13143 1 25917 1 | 131431 25917 2 | 131431 25917 3 |
| 13143 2 25917 1 | 13143 2 25917 2 | 13143 2 25917 3 | 13143 3 25917 1 | 13143 3 25917 2 |
| 13143 3 25917 3 | 13145 1 13851 1 | 13145 1 13851 2 | 13145 1 13851 3 | 13145 213851 1 |
| 13145 213851 2 | 13145 2 13851 3 | 13145 3 13851 1 | 13145 3 13851 2 | 13145 3 13851 3 |
| 13145 1 25917 1 | 13145 1 25917 2 | 13145 1 25917 3 | 13145 2 25917 1 | 13145 2 25917 2 |
| 13145 2 25917 3 | 13145 3 25917 1 | 13145 3 25917 2 | 13145 3 25917 3 | 13320 1 22115 1 |
| 13320 1 22115 2 | 13320 1 22115 3 | 13320 2 22115 1 | 13320 2 22115 2 | 13320 2 22115 3 |
| 13320 3 22115 1 | 13320 3 22115 2 | 13320 3 22115 3 | 13852 1 25294 1 | 13852 1 25294 2 |
| 13852 1 25294 3 | 13852 2 25294 1 | 13852 2 25294 2 | 13852 2 25294 3 | 13852 3 25294 1 |
| 13852 3 25294 2 | 13852 3 25294 3 | 14081 1 21613 1 | 14081 1 21613 2 | 14081 1 21613 3 |
| 14081 2 21613 1 | 140812 21613 2 | 14081 2 21613 3 | 14081 3 21613 1 | 14081 3 21613 2 |
| 14081 3 21613 3 | 14433 1 22115 1 | 14433 1 22115 2 | 14433 1 22115 3 | 14433 2 22115 1 |
| 14433 2 22115 2 | 14433 2 22115 3 | 14433 3 22115 1 | 14433 3 22115 2 | 14433 3 22115 3 |
| 15215 1 21439 1 | 15215 1 21439 2 | 15215 1 21439 3 | 15215 2 21439 1 | 15215 2 21439 2 |
| 15215 2 21439 3 | 15215 3 21439 1 | 15215 3 21439 2 | 15215 3 21439 3 | 16256 1 31360 1 |
| 162561 1 31360 2 | 16256 1 31360 3 | 16256 2 31360 1 | 16256 2 31360 2 | 16256 2 31360 3 |
| 16256 3 31360 1 | 16256 3 31360 2 | 16256 3 31360 3 | 16653 1 27186 1 | 16653 1 27186 2 |
| 166531 27186 3 | 16653 2 27186 1 | 16653 2 27186 2 | 16653 2 27186 3 | 16653 3 27186 1 |
| 16653 3 27186 2 | 16653 3 27186 3 | 16654 1 27186 1 | 16654 1 27186 2 | 16654 1 27186 3 |
| 16654 2 27186 1 | 16654 2 27186 2 | 16654 2 27186 3 | 16654 3 27186 1 | 16654 3 27186 2 |
| 16654 3 27186 3 | 17171 1 26834 1 | 17171 1 26834 2 | 17171 1 26834 3 | 17171 2 26834 1 |
| 17171 2 26834 2 | 17171 2 26834 3 | 17171 3 26834 1 | 17171 3 26834 2 | 17171 3 26834 3 |
| 18953 1 28612 1 | 18953 1 28612 2 | 18953 1 28612 3 | 18953 2 28612 1 | 18953 2 28612 2 |
| 18953 2 28612 3 | 18953 3 28612 1 | 18953 3 28612 2 | 18953 3 28612 3 | 19089 1 24490 1 |
| 19089 1 24490 2 | 19089 1 24490 3 | 19089 2 24490 1 | 19089 2 24490 2 | 19089 2 24490 3 |
| 19089 3 24490 1 | 19089 3 24490 2 | 19089 3 24490 3 | 20207 1 22000 1 | 202071 22000 2 |
| 20207 1 22000 3 | 20207 2 22000 1 | 20207 2 22000 2 | 20207 2 22000 3 | 20207 3 22000 1 |
| 20207 3 22000 2 | 20207 3 22000 3 | 20223 1 22000 1 | 202231 22000 2 | 20223 1 22000 3 |
| 20223 2 22000 1 | 20223 2 22000 2 | 20223 2 22000 3 | 20223 3 22000 1 | 20223 3 22000 2 |
| 20223 3 22000 3 | 20285 1 21036 1 | 20285 1 21036 2 | 20285 1 21036 3 | 20285 2 21036 1 |
| 20285 2 21036 2 | 20285 2 21036 3 | 20285 3 21036 1 | 20285 3 21036 2 | 20285 3 21036 3 |
| 20285 1 21037 1 | 20285 1 21037 2 | 20285 1 21037 3 | 20285 2 21037 1 | 20285 2 21037 2 |
| 20285 2 21037 3 | 20285 3 21037 1 | 20285 3 21037 2 | 20285 3 21037 3 | 20285 1 21038 1 |
| 20285 1 21038 2 | 202851 21038 3 | 20285 2 21038 1 | 20285 2 21038 2 | 20285 2 21038 3 |
| 20285 3 21038 1 | 20285 3 21038 2 | 20285 3 21038 3 | 21017 1 22115 1 | 21017 1 22115 2 |
| 210171 22115 3 | 21017 2 22115 1 | 21017 2 22115 2 | 21017 2 22115 3 | 21017 3 22115 1 |
| 21017 3 22115 2 | 21017 3 22115 3 | 21613 1 28781 1 | 21613 1 28781 2 | 21613 1 28781 3 |
| 21613 2 28781 1 | 21613 2 28781 2 | 21613 2 28781 3 | 21613 3 28781 1 | 21613 3 28781 2 |
| 21613 3 28781 3 | 21638 1 29192 1 | 21638 1 29192 2 | 21638 1 29192 3 | 21638 2 29192 1 |
| 21638 2 29192 2 | 21638 2 29192 3 | 21638 3 29192 1 | 21638 3 29192 2 | 21638 3 29192 3 |
| 21831 1 23912 1 | 21831 1 23912 2 | 21831 1 23912 3 | 21831 2 23912 1 | 21831 2 23912 2 |
| 21831 2 23912 3 | 21831 3 23912 1 | 21831 3 23912 2 | 21831 3 23912 3 | 22000 1 23060 1 |
| 22000 1 23060 2 | 22000 1 23060 3 | 22000 2 23060 1 | 22000 2 23060 2 | 22000 2 23060 3 |
| 22000 3 23060 1 | 22000 3 23060 2 | 22000 3 23060 3 | 22000 1 27338 1 | 22000 1 27338 2 |
| 22000 1 27338 3 | 22000 2 27338 1 | 22000 2 27338 2 | 22000 2 27338 3 | 22000 3 27338 1 |
| 22000 3 27338 2 | 22000 3 27338 3 | 22000 1 27805 1 | 22000 1 27805 2 | 22000 1 27805 3 |
| 22000 2 27805 1 | 22000 2 27805 2 | 22000 2 27805 3 | 22000 3 27805 1 | 22000 3 27805 2 |
| 22000 3 27805 3 | 22118 1 28707 1 | 22118 1 28707 2 | 22118 1 28707 3 | 22118 2 28707 1 |
| 22118 2 28707 2 | 22118 2 28707 3 | 22118 3 28707 1 | 22118 3 28707 2 | 22118 3 28707 3 |
| 22118 1 31207 1 | 22118 1 31207 2 | 22118 1 31207 3 | 22118 2 31207 1 | 22118 2 31207 2 |
| 22118 2 31207 3 | 22118 3 31207 1 | 22118 3 31207 2 | 22118 3 31207 3 | 24048 1 30692 1 |
| 24048 1 30692 2 | 24048 1 30692 3 | 24048 2 30692 1 | 24048 2 30692 2 | 24048 2 30692 3 |
| 24048 3 30692 1 | 24048 3 30692 2 | 24048 3 30692 3 | 24297 1 26436 1 | 24297 1 26436 2 |
| 24297 1 26436 3 | 24297 2 26436 1 | 24297 2 26436 2 | 24297 2 26436 3 | 24297 3 26436 1 |
| 24297 3 26436 2 | 24297 3 26436 3 | 267381 27681 1 | 26738 1 27681 2 | 26738 1 27681 3 |
| 26738 2 27681 1 | 26738 2 27681 2 | 26738 2 27681 3 | 26738 3 27681 1 | 26738 3 27681 2 |
| 26738 3 27681 3 | 26739 1 27681 1 | 26739 1 27681 2 | 267391 27681 3 | 26739 2 27681 1 |
| 26739 2 27681 2 | 26739 2 27681 3 | 26739 3 27681 1 | 26739 3 27681 2 | 26739 3 27681 3 |

Notably, marker 6321 appears in ONE combination of the 85 positives in this study. The 85 variables were subjected to a post-hoc analysis in order to evaluate the direction and genetic model of the potential interactions detected. As previously explained, the post-hoc analysis of HFCC allows the systematic evaluation of all the strata and positive groups of simultaneous studies. In order to do this, a printout similar to that shown in Table 18 of Example 2 was obtained, consisting of 2,296 lines (85 variables x 9 strata x 3 groups = 2,295 lines of data, to which the header must be added).
The study of the data contained in the aforementioned 2,295 lines allowed it to be determined that only 13 of the 85 remaining combinations (15.29%) had a direction of effect compatible with a genetic model (dominant, recessive, etc), eliminating all those variables that have in the same stratum of distinct groups OR in opposed directions (variables that would be marked as "R", for *ruido* (noise), in the column R/CS/E). In this case (*fuzzy logic*), the positive strata do not necessarily have to be consecutive, as long as they are different in the different groups. The data referring to these combinations and the corresponding strata selected are shown below in Table 21.

**Table 21.- Data referring to the combinations selected following the post-hoc analysis**

| **Variable** | | **Group** | **Stratum** | **OR** | **Z** | **Z²** | **Epist.^{a}** | **R/CS/E^{b}** | **HWD^{c}** |
|---|---|---|---|---|---|---|---|---|---|
| 134 | 27743 | (G2) | (134 2 27743 3) | 4,38022 | 2,62258 | 6,87792 | 1 | E | |
| 134 | 27743 | (G3) | (134 3 27743 2) | 3,153 | 3,03059 | 9,1845 | 1 | E | |
| 134 | 27743 | (G1) | (134 3 27743 3) | 3,71892 | 3,28714 | 10,8053 | 1 | E | |
| 1501 | 12740 | (G3) | (1501 1 12740 1) | 0,401581 | -2,8531 | 8,14024 | 2 | E | Yes |
| 1501 | 12740 | (G2) | (1501 1 12740 2) | 0,216087 | -2,9889 | 8,93362 | 2 | E | Yes |
| 1501 | 12740 | (G1) | (1501 2 12740 1) | 0,432419 | -2,6201 | 6,8651 | 2 | E | Yesl |
| 1956 | 26076 | (G3) | (1956 2 26076 2) | 5,16536 | 3,4645 | 12,0028 | 3 | E | |
| 1956 | 26076 | (G1) | (1956 2 26076 3) | 3,18284 | 2,86963 | 8,23475 | 3 | E | |
| 1956 | 26076 | (G2) | (1956 3 26076 2) | 4,71184 | 2,7668 | 7,6552 | 3 | E | |
| 2019 | 8223 | (G1) | (2019 1 8223 2) | 6,79858 | 2,63535 | 6,94508 | 4 | E | |
| 2019 | 8223 | (G3) | (2019 1 8223 3) | 7,1171 | 2,71267 | 7,35858 | 4 | E | |
| 2019 | 8223 | (G2) | (2019 3 8223 3) | 0,260703 | -2,5831 | 6,67248 | 4 | E | |
| 2666 | 7818 | (G1) | (2666 2 7818 2) | 4,35367 | 3,07228 | 9,43888 | 5 | E | |
| 2666 | 7818 | (G3) | (2666 3 7818 1) | 2,5604 | 2,63342 | 6,93488 | 5 | E | |
| 2666 | 7818 | (G2) | (2666 3 7818 2) | 0,345297 | -2,6126 | 6,8254 | 5 | E | |
| 2684 | 18178 | (G1) | (2684 1 18178 2) | 0,222542 | -2,6797 | 7,18091 | 6 | E | |
| 2684 | 18178 | (G3) | (2684 1 18178 2) | 0,196367 | -2,6038 | 6,7799 | 6 | E | |
| 2684 | 18178 | (G2) | (2684 2 18178 2) | 0,228187 | -3,594 | 12,917 | 6 | E | |
| 2684 | 18178 | (G1) | (2684 3 18178 1) | 6,89129 | 2,65351 | 7,04114 | 6 | E | |
| 5196 | 27805 | (G3) | (5196 1 27805 1) | 0,282926 | -3,1506 | 9,92603 | 7 | E | |
| 5196 | 27805 | (G2) | (5196 1 27805 2) | 0,304303 | -2,585 | 6,68197 | 7 | E | |
| 5196 | 27805 | (G1) | (5196 2 27805 1) | 2,72358 | 2,81617 | 7,93083 | 7 | E | |
| 5196 | 27805 | (G1) | (5196 2 27805 2) | 0,425535 | -2,3797 | 5,66313 | 7 | E | |
| 5196 | 27805 | (G3) | (5196 2 27805 2) | 2,54129 | 2,27095 | 5,15723 | 7 | E | |
| 6321 | 8945 | (G1) | (6321 1 8945 3) | 4,9054 | 2,45472 | 6,02564 | 8 | C1 | Yes |
| 6321 | 8945 | (G2) | (6321 1 8945 3) | 0,339712 | -1,8674 | 3,48714 | 8 | C1 | Yes |
| 6321 | 8945 | (G1) | (6321 2 8945 3) | 0,386055 | -2,1329 | 4,54921 | 8 | C1 | Yes |
| 6321 | 8945 | (G2) | (6321 2 8945 3) | 0,375514 | -2,2757 | 5,1786 | 8 | C1 | Yes |
| 6321 | 8945 | (G3) | (6321 2 8945 3) | 0,385594 | -2,2188 | 4,92317 | 8 | C1 | Yes |
| 6321 | 8945 | (G2) | (6321 3 8945 2) | 3,4854 | 2,43298 | 5,91938 | 8 | C1 | Yes |
| 6321 | 8945 | (G3) | (6321 3 8945 2) | 7,94752 | 3,24766 | 10,5473 | 8 | C1 | Yes |
| 6321 | 8945 | (G1) | (6321 3 8945 3) | 2,57725 | 2,70413 | 7,31231 | 8 | C1 | Yes |
| 6321 | 8945 | (G2) | (6321 3 8945 3) | 3,07958 | 2,9627 | 8,77759 | 8 | C1 | Yes |
| 6562 | 9836 | (G2) | (6562 1 9836 3) | 0,386789 | -3,0986 | 9,60143 | 9 | E | |
| 6562 | 9836 | (G3) | (6562 2 9836 2) | 0,197917 | -2,9052 | 8,44005 | 9 | E | |
| 6562 | 9836 | (G1) | (6562 2 9836 3) | 0,387145 | -2,8446 | 8,09152 | 9 | E | |
| 6563 | 9836 | (G2) | (6563 1 9836 3) | 0,423143 | -2,8136 | 7,91628 | 9 | E | |
| 6563 | 9836 | (G3) | (6563 2 9836 2) | 0,197917 | -2,9052 | 8,44005 | 9 | E | |
| 6563 | 9836 | (G1) | (6563 2 9836 3) | 0,412962 | -2,6794 | 7,17933 | 9 | E | |
| 6564 | 9836 | (G2) | (6564 1 9836 3) | 0,423143 | -2,8136 | 7,91628 | 9 | E | |
| 6564 | 9836 | (G3) | (6564 2 9836 2) | 0,197917 | -2,9052 | 8,44005 | 9 | E | |
| 6564 | 9836 | (G1) | (6564 2 9836 3) | 0,412962 | -2,6794 | 7,17933 | 9 | E | |
| 6565 | 9836 | (G3) | (6565 2 9836 2) | 0,197917 | -2,9052 | 8,44005 | 9 | E | |
| 6565 | 9836 | (G1) | (6565 2 9836 3) | 0,412962 | -2,6794 | 7,17933 | 9 | E | |
| 6565 | 9836 | (G2) | (6565 3 9836 3) | 0,423143 | -2,8136 | 7,91628 | 9 | E | |
| 11787 | 27401 | (G1) | (11787 1 27401 2) | 0,309606 | -2,6785 | 7,17413 | 10 | S | |
| 11787 | 27401 | (G2) | (11787 1 27401 3) | 0,171045 | -3,1914 | 10,1853 | 10 | S | |
| 11787 | 27401 | (G3) | (1187 2 27401 2) | 2,59485 | 2,67272 | 7,14344 | 10 | S | |
| 13145 | 13851 | (G3) | (13145 1 13851 3) | 0,145243 | -2,665 | 7,1024 | 11 | E | |
| 13145 | 13851 | (G2) | (13145 2 13851 3) | 0,406797 | -2,8356 | 8,04048 | 11 | E | |
| 13145 | 13851 | (G1) | (13145 3 13851 3) | 2,96006 | 3,05018 | 9,30362 | 11 | E | |
| 22000 | 23060 | (G3) | (22000 1 23060 2) | 0,415824 | -2,5846 | 6,68034 | 12 | E | |
| 22000 | 23060 | (G1) | (22000 1 23060 3) | 0,295875 | -2,9173 | 8,51061 | 12 | E | |
| 22000 | 23060 | (G2) | (22000 2 23060 2) | 0,377893 | -2,7846 | 7,75371 | 12 | E | |
| 22000 | 23060 | (G3) | (22000 2 23060 2) | 2,01013 | 1,82937 | 3,34658 | 12 | E | |
| 22000 | 23060 | (G1) | (22000 2 23060 3) | 5,96743 | 2,44363 | 5,97132 | 12 | E | |
| 22000 | 27338 | (G1) | (22000 1 27338 2) | 0,396882 | -2,7279 | 7,44163 | 13 | E | |
| 22000 | 27338 | (G2) | (22000 1 27338 3) | 2,81278 | 2,85016 | 8,12342 | 13 | E | |
| 22000 | 27338 | (G1) | (22000 2 27338 2) | 2,33022 | 2,18392 | 4,76953 | 13 | E | |
| 22000 | 27338 | (G3) | (22000 2 27338 2) | 3,2862 | 2,58495 | 6,68197 | 13 | E | |
| 22000 | 27338 | (G1) | (22000 2 27338 3) | 2,12789 | 1,98907 | 3,95638 | 13 | E | |
| 22118 | 31207 | (G2) | (22118 1 31207 1) | 0,403322 | -2,6234 | 6,88236 | 13 | E | |
| 22118 | 31207 | (G1) | (22118 2 31207 1) | 0,326589 | -2,761 | 7,62296 | 13 | E | |
| 22118 | 31207 | (G2) | (22118 2 31207 1) | 2,21604 | 1,83246 | 3,35793 | 13 | E | |
| 22118 | 31207 | (G3) | (22118 2 31207 2) | 3,61817 | 2,64752 | 7,00934 | 13 | E | |

| | | | | | | | | | |
|---|---|---|---|---|---|---|---|---|---|
| ^{a}: Number assigned to each one of the variables selected ^{b}: C: conditional, one of the markers of the pair (1 or 2) has a marginal or individual effect S: simultaneous, both markers have a marginal individual effect E: epistatic, none of the markers has a marginal individual effect ^{c}: HWD="Yes": there is Hardy-Weinberg disequilibrium in one of the markers of the pair | | | | | | | | | |

These 13 combinations include the combined effect of 29 different markers. Therefore, the combination of only 29 markers of the 31,532 studied (0.091%) can be selected under the initial HFCC criteria for Parkinson's disease (Fuzzy logic: some positive stratum in cases versus controls and no positive stratum in controls versus controls). This permits their direct prioritisation for future studies with the validation engine. These markers and their correlation with the molecular markers of Coriell's database, along with their position on the genome, are summarised in Table 22.

**Table 22: Markers selected by the HFCC software following the evaluation of the post-hoc analysis of the raw data from chromosome 1 for Parkinson's disease obtained in Fuzzy Logic mode with noise filter.**

| **Marker** | **Identification Code** | **Position on Chromosome** 1 |
|---|---|---|
| 134 | rs6659405 | 2405251 |
| 1501 | rs7514751 | 10696698 |
| 1956* | rss2038095* | 13970305* |
| 2019 | rs517269 | 14256655 |
| 2666 | rs4920425 | 18153445 |
| 2684 | rs2027530 | 18220829 |
| 5196 | rs2025005 | 34986565 |
| 6321 | rs12069733 | 43610601 |
| 6562 | rs11211059 | 45099311 |
| 6563 | rs263989 | 45111009 |
| 6564 | rs264025 | 45114089 |
| 6565 | rs264022 | 45115552 |
| 11787 | rs12563303 | 83806432 |
| 13145 | ras10874819 | 94131820 |
| 22000 | rs684527 | 179887139 |
| 22118 | rs2986550 | 180654092 |
| 27743 | rs11578981 | 221862247 |
| 12740 | rs12407088 | 90488881 |
| 26076 | rs2644548 | 209410757 |
| 8223 | rs2691461 | 57749052 |
| 7618 | rs6697414 | 54467112 |
| 18128 | rs1217060 | 154771742 |
| 27805 | rs2615069 | 222244320 |
| 8945 | rs2457829 | 62384567 |
| 9836 | rs675327 | 67869617 |
| 27401 | rs10495169 | 218120909 |
| 13851 | rs302788 | 99340459 |
| 23060 | rs1175111 | 188937689 |
| 27338 | rs7542375 | 217500175 |
| 31207 | rs1197627 | 242793946 |

| | | |
|---|---|---|
| * marker coinciding with a new locus for Parkinson's disease detected in an independent study performed by scientists of the Mayo Clinic of Rochester, Minnesota (Maraganore et al., 2005) | | |

### 3.6. Evaluation of the positives obtained.

From the study based on the second approach to the use of HFCC with high-volume genomic data, it is concluded that, changing the system of analysis, the system selects new combinations of markers potentially involved in Parkinson's disease on chromosome 1 of which nearly 500 million are possible taking the 31,532 elements two by two. This second analysis draws the conclusion that the two systems of analysis are not overlapping but complementary, providing different results. It is worth pointing out that the original work of Fung et al., 2006, on this data indicates the absence of replication of the results previously published by Maraganore et al., in 2005. However, our system, functioning in *fuzzy logic* mode, detects a positive marker (rs2038095) right in the vicinity of the gene PRDM2 (at 30Kb from the candidate gene identified) which is completely compatible with the results presented by Maraganore et al. for this chromosome. These results are the first independent replication of this locus and, paradoxically, this replication is obtained using a prior study with a panel of markers different to that employed by Maraganore et al. (2005) and a group of cases and controls that have been published without showing any link to this vicinity (Fung et al., 2006). This result, once again, confirms that the HFCC method has the capacity to identify loci that are completely invisible or undetectable when employing conventional statistical techniques.

### BIBLIOGRAPHIC REFERENCES:

Altshuler D, et al., Guilt by association, Nat Genet. 2000 Oct;26(2):135-7
Becker, KG, The common variants/multiple disease hypothesis of common complex genetic disorders. Med Hypotheses. 2004; V. 62 (2): pp. 309-17
Cargill et al. Characterization of single-nucleotide polymorphisms in coding regions of human genes. Nature Genetics. 1999 Jul; V. 22(3): pp. 231-8. Erratum in: Nat Genet 1999 Nov;23(3):373
Craig D.W. et al. Applications of whole-genome high-density SNP genotyping. Expert Rev Mol Diagn. 2005 Mar;5(2):159-70. Review
De Castro F, Moron FJ, Montoro L, Real LM, Ruiz A. Pharmacogenetics of controlled ovarian hyperstimulation. Pharmacogenomics. 2005a Sep;6(6):629-37. Review
De Castro F, Moron FJ, Montoro L, Galan JJ, Real LM, Ruiz A. Re: Polymorphisms associated with circulating sex hormone levels in postmenopausal women. J Natl Cancer Inst. 2005b Jan 19;97(2):152-3
De Castro F, Moron FJ, Montoro L, Galan JJ, Hernandez DP, Padilla ES, Ramirez-Lorca R, Real LM, Ruiz A. Human controlled ovarian hyperstimulation outcome is a polygenic trait. Pharmacogenetics. 2004 May;14(5):285-93
De Castro F, Ruiz R, Montoro L, Perez-Hernandez D, Sanchez-Casas Padilla E, Real LM, Ruiz A. Role of follicle-stimulating hormone receptor Ser680Asn polymorphism in the efficacy of follicle-stimulating hormone. Fertil Steril. 2003 Sep;80(3):571-6
Di Pasquale E, Rossetti R, Marozzi A, Bodega B, Borgato S, Cavallo L, Einaudi S, Radetti G, Russo G, Sacco M, Wasniewska M, Cole T, Beck-Peccoz P, Nelson LM, Persani L. Identification of new variants of human BMP15 gene in a large cohort of women with premature ovarian failure. J Clin Endocrinol Metab. 2006 May;91(5):1976-9
Dixit H, Rao LK, Padmalatha VV, Kanakavalli M, Deenadayal M, Gupta N, Chakrabarty B, Singh L. Missense mutations in the BMP15 gene are associated with ovarian failure. Hum Genet. 2006 May;119(4):408-15. Epub 2006 Mar 1
Dryja TP, et al. A point mutation of the rhodopsin gene in one form of retinitis pigmentosa. Nature. 1990 Jan 25;343(6256):364-6
Farrer MJ. Genetics of Parkinson disease: paradigm shifts and future prospects. Nat Rev Genet. 2006 Apr;7(4):306-18. Review
Fung HC, Scholz S, Matarin M, Simon-Sanchez J, Hernandez D, Britton A, Gibbs JR, Langefeld C, Stiegert ML, Schymick J, Okun MS, Mandel RJ, Fernandez HH, Foote KD, Rodriguez RL, Peckham E, De Vrieze FW, Gwinn-Hardy K, Hardy JA, Singleton A. Genome-wide genotyping in Parkinson's disease and neurologically normal controls: first stage analysis and public release of data. Lancet Neurol. 2006 Nov;5(11):911-6
Hardy GH (1908). "Mendelian proportions in a mixed population". Science 28:49-50.
Hirschhorn JN, et al. Once and again-issues surrounding replication in genetic association studies. J Clin Endocrinol Metab. 2002 Oct;87(10):4438-41
Hirschhorn JN, et al., 2002 A comprehensive review of genetic association studies. Genet Med 4:45-61
Hoh J y Ott J. Mathematical Multi-Locus Approaches to Localizing Complex Human Trait Genes, Nature Reviews Genetics 2003, V. 4, p. 701-709
Horikawa Y, et al. Genetic variation in the gene encoding calpain-10 is associated with type 2 diabetes mellitus. Nat Genet. 2000 Oct;26(2):163-75
Laissue P, Christin-Maitre S, Touraine P, Kuttenn F, Ritvos O, Aittomaki K, Bourcigaux N, Jacquesson L, Bouchard P, Frydman R, Dewailly D, Reyss AC, Jeffery L, Bachelot A, Massin N, Fellous M, Veitia RA. Mutations and sequence variants in GDF9 and BMP15 in patients with premature ovarian failure. Eur J Endocrinol. 2006 May;154(5):739-44
Maraganore DM, de Andrade M, Lesnick TG, Strain KJ, Farrer MJ, Rocca WA, Pant PV, Frazer KA, Cox DR, Ballinger DG. High-resolution whole-genome association study of Parkinson disease. Am J Hum Genet. 2005 Nov;77(5):685-93
Marchini et al, Genome-Wide Strategies For Detecting Multiple Loci That Influence Complex Diseases, Nature Genetics Abril 2005, V. 37 No. 4, p. 413-417
Moron FJ, de Castro F, Royo JL, Montoro L, Mira E, Saez ME, Real LM, Gonzalez A, Manes S, Ruiz A. Bone morphogenetic protein 15 (BMP15) alleles predict over-response to recombinant follicle stimulation hormone and iatrogenic ovarian hyperstimulation syndrome (OHSS). Pharmacogenet Genomics. 2006 Jul;16(7):485-95
Neurology, 2001; 57: 30-1354
Ott J, Hoh J. Set association analysis of SNP case-control and microarray data. J Comput Biol. 2003;10(3-4):569-74
Pericak-Vance MA, et al. Linkage studies in familial Alzheimer disease: evidence for chromosome 19 linkage. Am J Hum Genet. 1991 Jun;48(6):1034-50
Rothman KJ y Boice Jr JD : Epidemiologic Analysis with a Programmable Calculator. U.S. Department of Health, Education and Welfare, Public Health Service, National Institutes of Health, NIH Publication No. 79-1649, 1979.
Rothman KJ. Epidemiology: an introduction. Nueva York. Oxford University Press, 2002.
Syvanen A.C., Toward genome-wide SNP genotyping, Nat Genet. 2005 Jun; 37 Suppl:S5-10
Weinberg W. (1908). "Über den Nachweis der Verebung beim Menschen". Jahresh. Verein f. Vaterl. Naturk in Wüttemberg 64:368-82.
Weir, B.S., A.H.D. Brown y D.R. Marshall. 1976. Testing for selective neutrality of electrophoretically detectable protein polymorphisms. Genetics 84:639-659.
Weiss KM, et al., How many diseases does it take to map a gene with SNPs? Nat Genet. 2000 Oct;26(2):151-7
Zee RY, et al. Association of a polymorphism of the angiotensin I-converting enzyme gene with essential hypertension. Biochem Biophys Res Commun. 1992 Apr 15;184(1):9-15
Zondervan KT, et al., The complex interplay among factors that influence allelic association. Nat Rev Genet. 2004 Feb;5(2):89-100

## Claims

1. A method to determine the association between one or more *loci* in the genome of a species and a phenotype exhibited by a subgroup of individuals of that species, with the method comprising the following stages:
a) obtaining, from the genomes of multiple individuals of the species, which form a control group that does not show the phenotype, data indicating the presence of a multiplicity of predetermined genetic markers situated in separate *loci* in these genomes;
b) correlating the presence of markers at different *loci* of different members of a first subgroup of the previously mentioned control group with the presence of markers at these *loci* in different members of a second subgroup of the same control group, in order to use this to generate a noise filter;
c) obtaining, from the genomes of multiple individuals of the species that form a study group that shows the phenotype (F), data indicating the presence of this multiplicity of predetermined genetic markers situated at separate *loci.*
d) formulating various hypothetical correlations between this phenotype and the *loci* of the genomes of the individuals of each study group; and
e) filtering these hypothetical correlations with the noise filter in order to reject spurious correlations.

2. The method of claim 1, in which the study group comprises various study subgroups composed of human beings that show different biological states but that have a phenotype or risk factor in common, and the stage c) comprises determining an association between this phenotype or risk factor and one or more *loci* inside and common to the genomes of the members of each one of these study subgroups.

3. The method of claim 2, in which the subgroups of human beings present different diagnosed illnesses but have a common clinical phenotype.

4. The method of claim 1, in which the study group is composed of human beings that exhibit the same illness or biological state and stage c) implies the determination of an association between this illness or biological state and one or more *loci* inside and common to the genomes of the members of each one of the study subgroups.

5. The method of claim 4, in which the distribution of individuals of the study group in study subgroups is performed at random.

6. The method of claim 4, in which the distribution of the individuals of the study group in study subgroups is performed in such a way that each study subgroup is **characterised by** a distinctive phenotypic trait such as a particular evolution of the illness or biological state or a particular response to a drug.

7. The method of any one of claims 1 to 6, in which the study group is composed of at least three study subgroups.

8. The method of any one of claims 1 to 6, which includes (i) computing combinations of two *loci* in the first and second control subgroups and in the study subgroups; (ii) specifying combinations of two *loci* between these first and second control subgroups **characterised by** a level of confidence below a threshold level for determining a noise set (R₀); (iii) comparing each study subgroup with the results of (ii) in order to produce sets of diverse and potentially valid digenic associations (R₁, R₂, ... Rₙ); and (iv) selecting shared positive associations of R₁ to Rₙ not present in R₀, in order to thus determine an association between a pair of *loci* in the genome of the study group and the phenotype.

9. The method of any of the claims 1 to 6, which includes (i) computing combinations of three *loci* in the first and second control subgroups and in the study subgroups; (ii) specifying combinations of three *loci* between the first and second control subgroups **characterised by** a level of confidence below a threshold level for determining a noise set (R₀); (iii) comparing each study subgroup with the results of (ii) in order to produce diverse and potentially valid trigenic associations (R₁, R₂, ... Rₙ); and (iv) selecting positive shared associations of R₁ to Rₙ not present in R₀, in order to thus determine an association between a group of three *loci* inside the genome of this study group and this phenotype.

10. The method of any one of the claims 1 to 9, in which the hypothetical correlations between the phenotype of the study group and the *loci* of the genomes of the individuals of the study group are formulated, taking into account each one of the possible strata of a combination of markers and comparing it with all the other possible strata pertaining to any combination of the predetermined genetic markers situated at the *loci.*

11. The method of claim 10, in which it is formulated the existence of a correlation between a stratum of a combination of markers and the phenotype of the study group when the stratum gives rise to a positive association in each and every one of the comparisons of a study subgroup with a subgroup from the control group.

12. The method of claim 10, in which it is formulated the existence of a correlation between a combination of markers and the phenotype of the study group when the combination of markers presents at least a stratum of the same that gives rise to a positive association in each and every one of the comparisons of a study subgroup with a subgroup from the control group.

13. The method of any one of the claims 1 to 9, in which the hypothetical correlations between the phenotype of the study group and the *loci* of the genomes of the individuals of the study group are formulated taking into account all the strata of a combination of markers that present at least one copy of each one of the markers that form part of the combination, and comparing this against the rest of the combinations of strata.

14. The method of any one of the claims 1 to 13, that includes the additional stages of comparing the *loci* of the markers that comprise these hypothetical correlations filtered with a map of the genome of the species in order to identify genes close to these markers and to consult the related bibliography in order to limit the hypotheses.

15. The method of any one of the claims 1 to 13, that includes the additional stages of comparing the *loci* of markers that comprise these hypothetical correlations filtered with a map of the genome of the species, in order to determine additional markers that flank these markers and to reanalyse the correlations in order to limit the hypotheses.

16. The method of any one of the claims 1 to 13, which includes the additional stage of retesting a hypothetical correlation in a larger group of individuals.

17. The method of any one of the claims 1 to 13, in which the subgroups comprise less than 1000 members.

18. The method of any one of the claims 1 to 13, in which the subgroups comprise less than 100 members.

19. The method of any one of the claims 1 to 13, in which the markers are polymorphisms of one single nucleotide.

20. The method of any one of claims 1 to 13, in which the stage of obtaining data indicating the presence of predetermined multiple genetic markers includes the application of a sample derived from genomic DNA of the individuals to an array of oligonucleotides that includes these predetermined genetic markers situated at separate *loci* in these genomes.

21. The method of claim 20, in which the array comprises 3,500, 10,000, 50,000 or more separate oligonucleotides.

22. A noise filter to limit hypothetical associations between *loci* of the genome of a species and phenotypes shown by a subgroup of individuals of that species. The filter should comprise:
a database in which random noise associations are specified, a genotyping error or other spurious associations which comprise multi-*locus* combinations of genetic markers common to the control subgroups of individuals of the species below the threshold level of confidence, and
procedures to eliminate from a set of these hypothetical associations combinations that correspond to these noise associations.

23. A method to determine an association between one or more *loci* in the genome of a species and a phenotype exhibited by a subgroup of individuals of the species, comprising the following stages:
a) obtaining, from the genomes of multiple individuals of the species, which form a control group not showing the phenotype, data indicative of the presence of a multiplicity of predetermined genetic markers situated in these genomes at separate *loci;*
b) correlating the presence of markers at diverse *loci* of various members of a first subgroup of this control group with the presence of markers at these *loci* in various members of a second subgroup of this control group, in order to thus generate a noise filter;
c) obtaining, from the genomes of multiple individuals of the species that form the diverse study subgroups showing different biological states but having this phenotype in common, data indicative of the presence of this multiplicity of predetermined genetic markers common to the genomes of the members of each one of these study subgroups;
d) formulating diverse hypothetical correlations between *loci* of the genomes of the individuals of these study subgroups and this phenotype; and
e) filtering these hypothetical correlations with the noise filter in order to reject correlations due to noise.

24. A tool to determine hypothetical associations between one or more *loci* in the genome of a species and a phenotype shown by a subgroup of individuals of the species. The tool should include a programmed computer that comprises:
procedures to receive data indicating the presence of a multiplicity of predetermined genetic markers situated at separate *loci* along the length of the genomes of a multiplicity of study individuals of this species that show this phenotype;
procedures to record associations due to noise that comprise multilocus combinations of genetic markers common to two groups of control individuals of the species below a threshold level of confidence;
procedures to calculate, based on data indicating the presence of a multiplicity of predetermined genetic markers for these test individuals, the hypothetical associations between *loci* that carry the genetic markers and the phenotype;
procedures to eliminate from a set of these calculated hypothetical associations, the calculated hypothetical combinations that correspond to noise associations.

25. The tool of claim 24, designed so that it is optional the use of the different procedures in order to eliminate from the set of hypothetical calculated associations those combinations that correspond to noise.

26. Use of the tool of claim 24 or 25, in order to generate hypotheses for associations between one or more loci of the genome of a species and a phenotype exhibited by a subgroup of individuals of this species.

27. The use of claim 26, in which the hypotheses of association are generated following elimination of the noise associations common to two groups of control individuals of the species from the calculated hypothetical associations between *loci* of the genome of the species and the subgroup of individuals that show this phenotype.

28. The use of claim 26, in which the association hypotheses are generated without eliminating noise associations common to the two groups of control individuals of the species from the hypothetical associations calculated between *loci* of the genomes of the species and the subgroup of individuals that show this phenotype.

29. A computer program that comprises a computer readable medium and a computer readable program code, recorded on this computer readable medium, appropriate for giving instructions to a computer or computer system included in the tool of claim 24 or 25 in order to perform the following stages:
a) receiving data indicating the presence of a multiplicity of predetermined genetic markers situated at separate *loci* along the length of the genomes of a multiplicity of study individuals that show a particular phenotype;
b) receiving the data indicating the presence of the same multiplicity of genetic markers in two control groups that do not show the phenotype of the study individuals;
c) calculating hypothetical associations between the presence of genetic markers in each of the two control groups and the phenotype of the study individuals, considering one of the control groups as a group of individuals that show this phenotype;
d) calculating hypothetical associations between the presence of genetic markers in the genomes in the study individuals that show the phenotype and this phenotype;
e) eliminating from the hypothetical associations calculated in stage d) the associations calculated in stage c).
